# EUROPEAN PATENT SPECIFICATION

(11) **EP 4 501 934 B1**
(45) Date of publication and mention of the grant of the patent: **24.12.2025**
(21) Application number: 24191379.7
(22) Date of filing: 29.07.2024
(51) Int. Cl.: C07F 5/02, C09K 11/06, H10K 50/10

(54) **CONDENSED CYCLIC COMPOUND, LIGHT-EMITTING DEVICE INCLUDING THE SAME, AND ELECTRONIC APPARATUS INCLUDING THE LIGHT-EMITTING DEVICE**
KONDENSIERTE CYCLISCHE VERBINDUNG, LICHTEMITTIERENDE VORRICHTUNG DAMIT UND ELEKTRONISCHE VORRICHTUNG MIT DER LICHTEMITTIERENDEN VORRICHTUNG
COMPOSÉ CYCLIQUE CONDENSÉ, DISPOSITIF ÉLECTROLUMINESCENT LE COMPRENANT ET APPAREIL ÉLECTRONIQUE COMPRENANT LE DISPOSITIF ÉLECTROLUMINESCENT

(30) Priority: 31.07.2023 KR 20230099834
(43) Date of publication of application: 05.02.2025
(73) Proprietor: Samsung Display Co., Ltd., Yongin-si, Gyeonggi-do 17113 (KR)
(72) Inventor: LEE, Eunkyung, 16678 Suwon-si (KR); MARUYAMA, Yusuke, 16678 Suwon-si (KR); JUNG, Yongsik, 16678 Suwon-si (KR); LEE, Halim, 16678 Suwon-si (KR); NAM, Sungho, 16678 Suwon-si (KR); KIM, Joonghyuk, 16678 Suwon-si (KR); KIM, Jiwhan, 16678 Suwon-si (KR); JU, Kyeongsik, 16678 Suwon-si (KR); SON, Youngmok, 16678 Suwon-si (KR); CHO, Hwayoung, 16678 Suwon-si (KR)
(74) Representative: Elkington and Fife LLP

(56) References cited:
- US-A1- 2023 097 942

## Description

### FIELD OF THE INVENTION

The disclosure relates to a condensed cyclic compound, a light-emitting device including the same, and an electronic apparatus including the light-emitting device.

### BACKGROUND OF THE INVENTION

Organic light-emitting devices among light-emitting devices are self-emissive devices that have wide viewing angles, high contrast ratios, short response times, and excellent characteristics in terms of luminance, driving voltage, and response speed, compared to devices in the art, and can produce full-color images.

In an example, an organic light-emitting device includes an anode, a cathode, and an interlayer that is arranged between the anode and the cathode and includes an emission layer. A hole transport region may be provided between the anode and the emission layer, and an electron transport region may be provided between the emission layer and the cathode. Holes provided from the first electrode move toward the emission layer through the hole transport region, and electrons provided from the second electrode move toward the emission layer through the electron transport region. The holes and the electrons recombine in the emission layer to produce excitons. The excitons may transition from an excited state to a ground state, thereby generating light.

US2023097942 discloses polycyclic compounds and an organic light-emitting device including the same.

### SUMMARY OF THE INVENTION

Provided are a novel condensed cyclic compound, a light-emitting device including the same, and an electronic apparatus including the light-emitting device.

Additional aspects will be set forth in part in the description which follows and, in part, will be apparent from the description, or may be learned by practice of the presented embodiments of the disclosure.

According to an aspect of the disclosure, provided is a condensed cyclic compound represented by Formula 1: 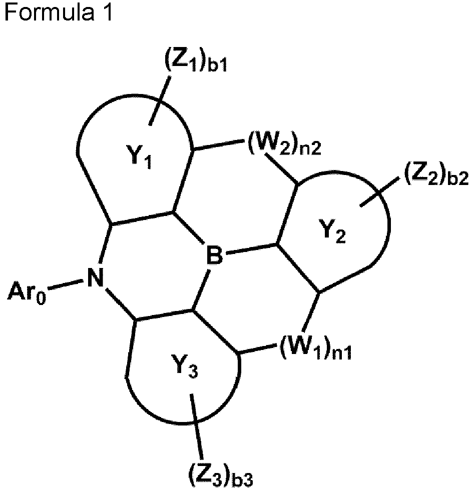 wherein, in Formula 1, Ar₀ is a group represented by Formula 2, 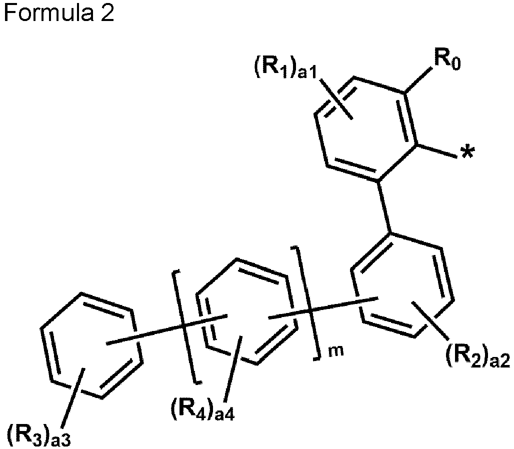 wherein, in Formulae 1 and 2,
ring Y₁ to ring Y₃ are each independently a C₅-C₆₀ carbocyclic group or a C₃-C₆₀ heterocyclic group,
W₁ is a single bond, O, S, N(Ar₁), N(T₁₁), C(T₁₂)(T₁₃), or Si(T₁₂)(T₁₃),
W₂ is a single bond, O, S, N(Ar₂), N(T₂₁), C(T₂₂)(T₂₃), or Si(T₂₂)(T₂₃),
n1 and n2 are each independently 0 or 1,
when n1 is 0, *-(W₁)ₙ₁-*' is absent,
when n2 is 0, *-(W₂)ₙ₂-*' is absent,
the sum of n1 and n2 is 1 or greater,
each of Ar₁ and Ar₂ is a group represented by Formula 2,
R₀ is
hydrogen, deuterium, -F, or a cyano group, or
a C₁-C₆₀ alkyl group unsubstituted or substituted with deuterium, -F, a cyano group, or any combination thereof,
R₁ to R₄ are each independently
hydrogen, deuterium, -F, or a cyano group,
a C₁-C₆₀ alkyl group unsubstituted or substituted with deuterium, -F, a cyano group, or any combination thereof, or
a C₆-C₆₀ aryl group unsubstituted or substituted with deuterium, -F, a cyano group, a C₁-C₆₀ alkyl group, a C₁-C₆₀ aryl group, or any combination thereof,
a1 is an integer from 0 to 3,
a2 and a4 are each independently an integer from 0 to 4,
a3 and m are each independently an integer from 0 to 5,
* in Formula 2 indicates a binding site to Formula 1,
Z₁ to Z₃, T₁₁ to T₁₃, and T₂₁ to T₂₃ are each independently hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), - Ge(Q₃)(Q₄)(Q₅), -B(Q₆)(Q₇), -P(=O)(Q₈)(Q₉), -P(Q₈)(Q₉), or a group represented by Formula 2,
b1 and b2 are each independently an integer from 0 to 10,
b3 is an integer from 1 to 5,
Formula 1 satisfies at least one of Conditions 1 and 2:
   Condition 1
      b1 is 1 or greater, and at least one of Z₁ in the number of b1 includes a carbazole group; and
   Condition 2
      b2 is 1 or greater, and at least one of Z₂ in the number of b2 includes a carbazole group,
at least one of Z₃ in the number of b3 is a C₁-C₆₀ alkyl group unsubstituted or substituted with deuterium, -F, a cyano group, or any combination thereof,
two or more of Z₁, Z₂, Z₃, T₁₁, T₁₂, T₁₃, T₂₁, T₂₂ and T₂₃ are optionally linked together to form a substituted or unsubstituted C₅-C₃₀ carbocyclic group or a substituted or unsubstituted C₁-C₃₀ heterocyclic group,
at least one substituent of the substituted C₅-C₃₀ carbocyclic group, the substituted C₁-C₃₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₁-C₆₀ alkylthio group, the substituted C₃-C₁₀ cycloalkyl group, the substituted C₁-C₁₀ heterocycloalkyl group, the substituted C₃-C₁₀ cycloalkenyl group, the substituted C₂-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group is:
   deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, or a C₁-C₆₀ alkylthio group,
   a C₁-C₆₀ alkyl group, a C₂-C₆₀ alkenyl group, a C₂-C₆₀ alkynyl group, a C₁-C₆₀ alkoxy group, or a C₁-C₆₀ alkylthio group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), - Ge(Q₁₃)(Q₁₄)(Q₁₅), -B(Q₁₆)(Q₁₇), -P(=O)(Q₁₈)(Q₁₉), -P(Q₁₈)(Q₁₉), or any combination thereof,
   a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₁-C₆₀ alkylthio group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), -Ge(Q₂₃)(Q₂₄)(Q₂₅), - B(Q₂₆)(Q₂₇), -P(=O)(Q₂₈)(Q₂₉), -P(Q₂₈)(Q₂₉), or any combination thereof,
   -N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), - P(=O)(Q₃₈)(Q₃₉), or -P(Q₃₈)(Q₃₉), or
   any combination thereof, and
   Q₁ to Q₉, Q₁₁ to Q₁₉, Q₂₁ to Q₂₉, and Q₃₁ to Q₃₉ are each independently hydrogen, deuterium, -F, or a C₁-C₆₀ alkyl group, a C₂-C₆₀ alkenyl group, a C₂-C₆₀ alkynyl group, a C₁-C₆₀ alkoxy group, a C₁-C₆₀ alkylthio group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with deuterium, -F, a cyano group, a C₁-C₆₀ alkyl group, a C₆-C₆₀ aryl group, or any combination thereof.

According to another aspect of the disclosure, a light-emitting device includes a first electrode, a second electrode, and an interlayer that is arranged between the first electrode and the second electrode and includes an emission layer, wherein the interlayer includes at least one of the condensed cyclic compound.

According to another aspect of the disclosure, an electronic apparatus includes the light-emitting device.

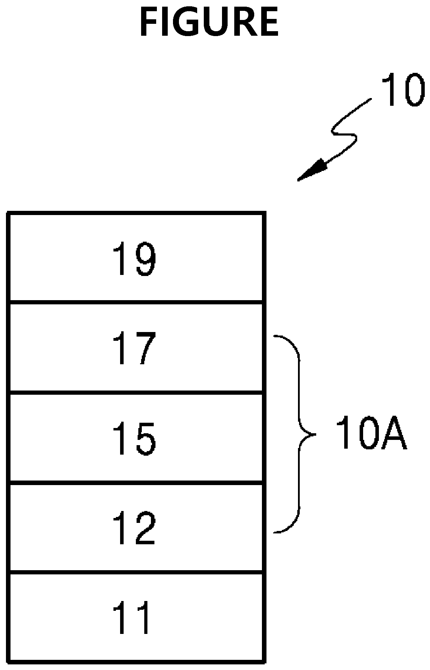

### BRIEF DESCRIPTION OF THE DRAWING

The above and other aspects, features, and advantages of certain embodiments of the disclosure will be more apparent from the following description taken in conjunction with the FIGURE which is a schematic cross-sectional view of a light-emitting device according to an embodiment.

### DETAILED DESCRIPTION OF THE EMBODIMENTS

Reference will now be made in detail to embodiments, examples of which are illustrated in the accompanying drawing, wherein like reference numerals refer to like elements throughout. In this regard, the present embodiments may have different forms and should not be construed as being limited to the descriptions set forth herein. Accordingly, the embodiments are merely described below, by referring to the figures, to explain aspects.

It will be understood that when an element is referred to as being "on" another element, it can be directly on the other element or intervening elements may be present therebetween In contrast, when an element is referred to as being "directly on" another element, there are no intervening elements present.

It will be understood that, although the terms "first," "second," "third" etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, "a first element," "component," "region," "layer" or "section" discussed below could be termed a second element, component, region, layer or section without departing from the teachings herein.

The terminology used herein is for the purpose of describing particular embodiments only and is not intended to be limiting. As used herein, "a," "an," "the," and "at least one" do not denote a limitation of quantity, and are intended to cover both the singular and plural, unless the context clearly indicates otherwise. For example, "an element" has the same meaning as "at least one element," unless the context clearly indicates otherwise.

Expressions such as "at least one of," when preceding a list of elements, modify the entire list of elements and do not modify the individual elements of the list.

"Or" means "and/or." As used herein, the term "and/or" includes any and all combinations of one or more of the associated listed items. It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Furthermore, relative terms, such as "lower" or "bottom" and "upper" or "top," may be used herein to describe one element's relationship to another element as illustrated in the Figures. It will be understood that relative terms are intended to encompass different orientations of the device in addition to the orientation depicted in the Figures. For example, if the device in one of the figures is turned over, elements described as being on the "lower" side of other elements would then be oriented on "upper" sides of the other elements. The exemplary term "lower," can therefore, encompasses both an orientation of "lower" and "upper," depending on the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ± 30%, 20%, 10% or 5% of the stated value.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded Thus, the regions illustrated in the figures are schematic in nature and their shapes are not intended to illustrate the precise shape of a region and are not intended to limit the scope of the present claims.

### Description of condensed cyclic compound

A condensed cyclic compound provided herein may be represented by Formula 1: 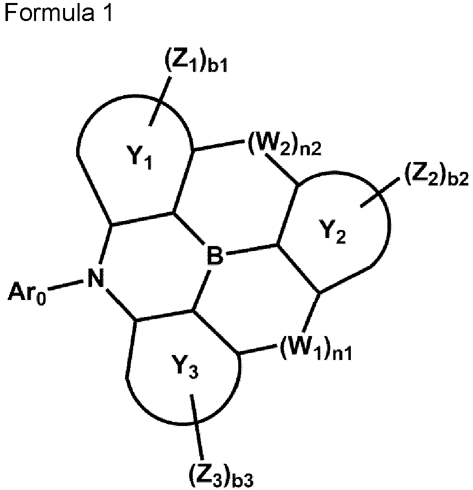 wherein, in Formula 1, Ar₀ may be a group represented by Formula 2, 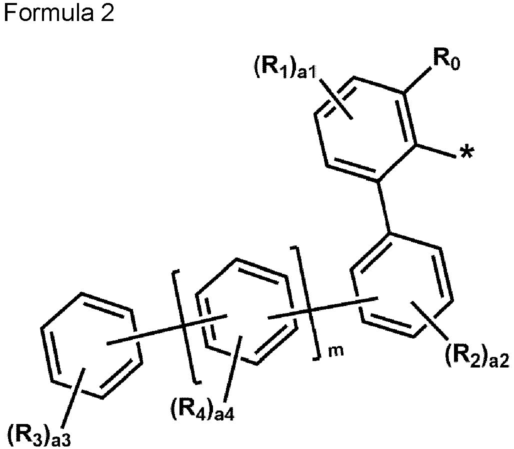 wherein a detailed description of Formula 2 may be the same as described herein.

In Formula 1, ring Y₁ to ring Y₃ may each independently be a C₅-C₆₀ carbocyclic group or a C₃-C₆₀ heterocyclic group.

In an embodiment, ring Y₁ to ring Y₃ may each independently be a benzene group, a naphthalene group, a phenanthrene group, a pyridine group, a pyrimidine group, a pyridazine group, a pyrazine group, a quinoline group, an isoquinoline group, a dibenzofuran group, a dibenzothiophene group, a carbazole group, a fluorene group, a dibenzosilole group, an azadibenzofuran group, an azadibenzothiophene group, an azacarbazole group, an azafluorene group, or an azadibenzosilole group.

In one or more embodiments, ring Y₁ to ring Y₃ may each independently be a benzene group, a naphthalene group, a dibenzofuran group, a dibenzothiophene group, a carbazole group, a fluorene group, or a dibenzosilole group.

In Formula 1, W₁ may be a single bond, O, S, N(Ar₁), N(T₁₁), C(T₁₂)(T₁₃), or Si(T₁₂)(T₁₃), and W₂ may be a single bond, O, S, N(Ar₂), N(T₂₁), C(T₂₂)(T₂₃), or Si(T₂₂)(T₂₃). Here, each of Ar₁ and Ar₂ may be a group represented by Formula 2, and T₁₁ to T₁₃ and T₂₁ to T₂₃ may each be the same as described herein.

In Formula 1, n1 and n2 may each independently be 0 or 1, wherein, when n1 is 0, *-(W₁)ₙ₁-*' may be absent, and when n2 is 0, *-(W₂)ₙ₂-*' may be absent, wherein the sum of n1 and n2 may be 1 or greater.

For example, in Formula 1,
i) n1 may be 1, and n2 may be 0;
ii) n1 may be 0, and n2 may be 1; or
iii) each of n1 and n2 may be 1.

In an embodiment, in Formula 1, n1 may be 1, and W₁ may be N(Ar₁) or N(T₁₁).

In one or more embodiments, in Formula 1, n2 may be 1, and W₂ may be N(Ar₂) or N(T₂₁).

In one or more embodiments, in Formula 1, n1 may be 1, and W₁ may be N(Ar₁), wherein Ar₀ and Ar₁ may be identical to each other.

In one or more embodiments, in Formula 1, n1 may be 1, and W₁ may be N(Ar₁), wherein Ar₀ and Ar₁ may be different from each other.

In one or more embodiments, in Formula 1, n2 may be 1, and W₂ may be N(Ar₂), wherein Ar₀ and Ar₂ may be identical to each other.

In one or more embodiments, in Formula 1, n2 may be 1, and W₂ may be N(Ar₂), wherein Ar₀ and Ar₂ may be different from each other.

In Formula 2,
R₀ may be:
hydrogen, deuterium, -F, or a cyano group; or
a C₁-C₆₀ alkyl group unsubstituted or substituted with deuterium, -F, a cyano group, or any combination thereof, and
R₁ to R₄ may each independently be:
   hydrogen, deuterium, -F, or a cyano group;
   a C₁-C₆₀ alkyl group unsubstituted or substituted with deuterium, -F, a cyano group, or any combination thereof; or
   a C₆-C₆₀ aryl group unsubstituted or substituted with deuterium, -F, a cyano group, a C₁-C₆₀ alkyl group, a C₁-C₆₀ aryl group, or any combination thereof.

For example, in Formula 2,
R₀ may be:
hydrogen, deuterium, -F, or a cyano group; or
a C₁-C₂₀ alkyl group unsubstituted or substituted with deuterium, -F, a cyano group, or any combination thereof, and
R₁ to R₄ may each independently be:
   hydrogen, deuterium, -F, or a cyano group;
   a C₁-C₂₀ alkyl group unsubstituted or substituted with deuterium, -F, a cyano group, or any combination thereof; or
   a phenyl group unsubstituted or substituted with deuterium, -F, a cyano group, a C₁-C₂₀ alkyl group, a phenyl group, or any combination thereof.

In Formula 2, a1 to a4 and m indicate the numbers of R₁ to R₄ and a phenylene group, respectively, wherein a1 may be an integer from 0 to 3, a2 and a4 may each independently be an integer from 0 to 4, and a3 and m may each independently be an integer from 0 to 5. When a1 is 2 or greater, two or more of R₁ may be identical to or different from each other, when a2 is 2 or greater, two or more of R₂ may be identical to or different from each other, when a3 is 2 or greater, two or more of R₃ may be identical to or different from each other, and when a4 is 2 or greater, two or more of R₄ may be identical to or different from each other. For example, m may be 0 or 1.

In Formula 2, * indicates a binding site to Formula 1.

In an embodiment, in Formula 2, a2 may be 1, R₂ may be a phenyl group unsubstituted or substituted with deuterium, -F, a cyano group, a C₁-C₂₀ alkyl group, a phenyl group, or any combination thereof.

In the present specification, examples of the C₁-C₆₀ alkyl group or the C₁-C₂₀ alkyl group may include a methyl group, an ethyl group, an n-propyl group, an isopropyl group, an n-butyl group, a sec-butyl group, an iso-butyl group, a tert-butyl group, an n-pentyl group, a tert-pentyl group, a neo-pentyl group, an iso-pentyl group, a sec-pentyl group, a 3-pentyl group, a sec-iso-pentyl group, and the like.

In Formula 1, Z₁ to Z₃, T₁₁ to T₁₃, and T₂₁ to T₂₃ may each independently be hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), - P(=O)(Q₈)(Q₉), -P(Q₈)(Q₉), or a group represented by Formula 2.

For example, Z₁ to Z₃, T₁₁ to T₁₃, and T₂₁ to T₂₃ may each independently be:
hydrogen, deuterium, -F, or a cyano group;
a C₁-C₂₀ alkyl group, a phenyl group, a biphenyl group, a dibenzofuranyl group, a dibenzothiophenyl group, a carbazolyl group, a fluorenyl group, or a dibenzosilolyl group, each unsubstituted or substituted with deuterium, -F, a cyano group, a C₁-C₂₀ alkyl group, a phenyl group, a dibenzofuranyl group, a dibenzothiophenyl group, a carbazolyl group, a fluorenyl group, a dibenzosilolyl group, or any combination thereof; or
-N(Q₁)(Q₂), and
Q₁ and Q₂ may each independently be a C₁-C₂₀ alkyl group, a phenyl group, a biphenyl group, a dibenzofuranyl group, a dibenzothiophenyl group, a carbazolyl group, a fluorenyl group, or a dibenzosilolyl group, each unsubstituted or substituted with deuterium, -F, a cyano group, a C₁-C₂₀ alkyl group, a phenyl group, a dibenzofuranyl group, a dibenzothiophenyl group, a carbazolyl group, a fluorenyl group, a dibenzosilolyl group, or any combination thereof.

In Formula 1, b1 to b3 indicate the numbers of Z₁ to Z₃, respectively, wherein b1 and b2 may each independently be an integer from 0 to 10, and b3 may be an integer from 1 to 5. When b1 is 2 or more, two or more of Z₁ may be identical to or different from each other, when b2 is 2 or more, two or more of Z₂ may be identical to or different from each other, and when b3 is 2 or more, two or more of Z₃ may be identical to or different from each other. For example, b1 to b3 may each independently be 0, 1, or 2.

Formula 1 may satisfy at least one of Conditions 1 and 2:
Condition 1
   b1 is 1 or greater, and at least one of b1 Z₁(s) comprises a carbazole group; and
Condition 2
   b2 is 1 or greater, and at least one of b2 Z₂(s) comprises a carbazole group.

In an embodiment, Formula 1 may satisfy both Conditions 1 and 2.

In one or more embodiments, in Formula 1, each of b1 and b2 may be 1 or more, and at least one of Z₁ and Z₂ may be a group represented by Formula 3, for example, Z₁ and Z₂ may each independently be a group represented by Formula 3: 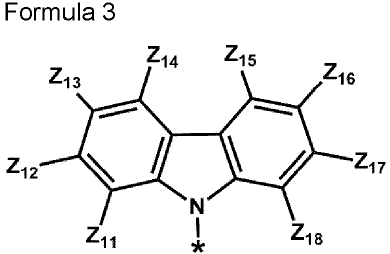 wherein, in Formula 3,
Z₁₁ to Z₁₈ may each independently be:
hydrogen, deuterium, -F, or a cyano group;
a C₁-C₂₀ alkyl group unsubstituted or substituted with deuterium, -F, a cyano group, or any combination thereof; or
a phenyl group unsubstituted or substituted with deuterium, -F, a cyano group, a C₁-C₂₀ alkyl group, a phenyl group, or any combination thereof, and
* indicates a binding site to Formula 1.

In one or more embodiments, each of Z₁₁ to Z₁₈ in Formula 3 may be hydrogen or deuterium.

In one or more embodiments, at least one of Z₁₃ and Z₁₆ (e.g., both Z₁₃ and Z₁₆) in Formula 3 may be:
a C₁-C₂₀ alkyl group unsubstituted or substituted with deuterium, -F, a cyano group, or any combination thereof (e.g., a tert-butyl group unsubstituted or substituted with deuterium); or
a phenyl group unsubstituted or substituted with deuterium, -F, a cyano group, a C₁-C₂₀ alkyl group, a phenyl group, or any combination thereof.

In one or more embodiments, at least one of Z₁₁ to Z₁₈ in Formula 3 may each independently be: deuterium; or Group 4 substituted with at least one deuterium, wherein Group 4 may be:
a C₁-C₂₀ alkyl group unsubstituted or substituted with -F, a cyano group, or any combination thereof; or
a phenyl group unsubstituted or substituted with -F, a cyano group, a C₁-C₂₀ alkyl group, a phenyl group, or any combination thereof.

The term "Group 4 substituted with at least one deuterium" as used herein refers to a substituent in which at least one hydrogen in Group 4 is substituted with deuterium. For example, "Group 4 substituted with at least one deuterium" may be - CD₃, -CD₂H, -CDH₂, -CD₂F, -C(CD₃)₃, -C₆D₅, -C₆D₂H₃, -C₆D₄(CH₃), -C₆H₄(CD₃), - C₆D₄(CD₃), -C₆F₄(CD₃), -C₆D₄(C₆H₅), or the like. The terms "Group 1 substituted with at least one deuterium", "Group 2 substituted with at least one deuterium", and "Group 3 substituted with at least one deuterium" may be also understood in the same way.

In one or more embodiments, in Formula 1, each of b1 and b2 may be 1, and each of Z₁ and Z₂ may be an N-carbazolyl group unsubstituted or substituted with deuterium, -F, a cyano group, a C₁-C₂₀ alkyl group, a phenyl group, or any combination thereof.

The term "N-carbazolyl group" as used herein refers to a monovalent group in which hydrogen is separated from N of a carbazole group so that the N is capable of being bonded to another group, and may be represented by 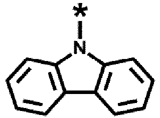 (wherein * indicates a binding site to a neighboring atom).

In Formula 1, at least one of Z₃ in the number of b3 may be a C₁-C₆₀ alkyl group (e.g., a C₁-C₂₀ alkyl group) unsubstituted or substituted with deuterium, -F, a cyano group, or any combination thereof.

In an embodiment, at least one of Z₃ in the number of b3 in Formula 1 may be a tert-butyl group unsubstituted or substituted with at least one deuterium.

In one or more embodiments, in Formula 1, b3 may be 1, and Z₃ may be a C₁-C₂₀ alkyl group unsubstituted or substituted with deuterium, -F, a cyano group, or any combination thereof (e.g., a tert-butyl group unsubstituted or substituted with at least one deuterium).

In one or more embodiments, the group represented by Formula 2 may be a group represented by one of Formulae 2-1 to 2-3: 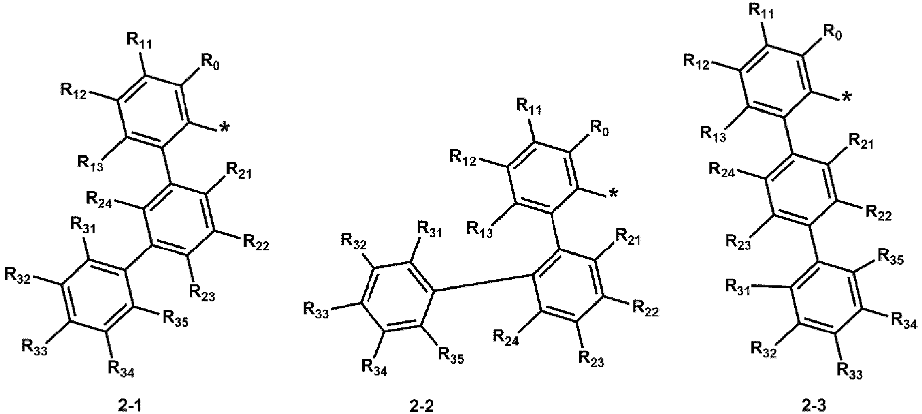 wherein, in Formulae 2-1 to 2-3,
R₀ may be the same as described herein,
R₁₁ to R₁₃ may each be the same as described in connection with R₁,
R₂₁ to R₂₄ may each be the same as described in connection with R₂,
R₃₁ to R₃₅ may each be the same as described in connection with R₃, and
* indicates a binding site to Formula 1 (e.g., nitrogen in Formula 1).

For example, each of Formulae 2-1 to 2-3 may satisfy Conditions 3 to 5:
Condition 3
   3a) each of R₁₁ to R₁₃ is hydrogen or deuterium,
   3b) R₁₂ is a C₁-C₂₀ alkyl group unsubstituted or substituted with deuterium, -F, a cyano group, or any combination thereof (e.g., a tert-butyl group unsubstituted or substituted with deuterium), or
   3c) at least one of R₁₁ to R₁₃ is each independently: deuterium; or Group 1 substituted with at least one deuterium, wherein Group 1 includes:
      a C₁-C₂₀ alkyl group unsubstituted or substituted with -F, a cyano group, or any combination thereof; or
      a phenyl group unsubstituted or substituted with -F, a cyano group, a C₁-C₂₀ alkyl group, a phenyl group, or any combination thereof;
Condition 4
   4a) each of R₂₁ to R₂₄ is hydrogen or deuterium,
   4b) R₂₂ is a C₁-C₂₀ alkyl group unsubstituted or substituted with deuterium, -F, a cyano group, or any combination thereof (e.g., a tert-butyl group unsubstituted or substituted with deuterium), or
   4c) R₂₂ is a phenyl group unsubstituted or substituted with deuterium, -F, a cyano group, a C₁-C₂₀ alkyl group, a phenyl group, or any combination thereof,
   4d) R₂₄ is a C₁-C₂₀ alkyl group unsubstituted or substituted with deuterium, -F, a cyano group, or any combination thereof (e.g., a tert-butyl group unsubstituted or substituted with deuterium),
   4e) R₂₄ is a phenyl group unsubstituted or substituted with deuterium, -F, a cyano group, a C₁-C₂₀ alkyl group, a phenyl group, or any combination thereof, or
   4f) at least one of R₂₁ to R₂₄ is each independently: deuterium; or Group 2 substituted with at least one deuterium, wherein Group 2 includes:
      a C₁-C₂₀ alkyl group unsubstituted or substituted with -F, a cyano group, or any combination thereof; or
      a phenyl group unsubstituted or substituted with -F, a cyano group, a C₁-C₂₀ alkyl group, a phenyl group, or any combination thereof; and
Condition 5
   5a) each of R₃₁ to R₃₅ is hydrogen or deuterium,
   5b) R₃₁ is a C₁-C₂₀ alkyl group unsubstituted or substituted with deuterium, -F, a cyano group, or any combination thereof (e.g., a tert-butyl group unsubstituted or substituted with deuterium),
   5c) R₃₁ is a phenyl group unsubstituted or substituted with deuterium, -F, a cyano group, a C₁-C₂₀ alkyl group, a phenyl group, or any combination thereof,
   5d) R₃₂ is a C₁-C₂₀ alkyl group unsubstituted or substituted with deuterium, -F, a cyano group, or any combination thereof (e.g., a tert-butyl group unsubstituted or substituted with deuterium),
   5e) R₃₂ is a phenyl group unsubstituted or substituted with deuterium, -F, a cyano group, a C₁-C₂₀ alkyl group, a phenyl group, or any combination thereof,
   5f) R₃₃ is a C₁-C₂₀ alkyl group unsubstituted or substituted with deuterium, -F, a cyano group, or any combination thereof,
   5g) R₃₃ is a phenyl group unsubstituted or substituted with deuterium, -F, a cyano group, a C₁-C₂₀ alkyl group, a phenyl group, or any combination thereof,
   5h) R₃₂ and R₃₄ are each independently:
      a C₁-C₂₀ alkyl group unsubstituted or substituted with deuterium, -F, a cyano group, or any combination thereof (e.g., a tert-butyl group unsubstituted or substituted with deuterium); or
      a phenyl group unsubstituted or substituted with deuterium, -F, a cyano group, a C₁-C₂₀ alkyl group, a phenyl group, or any combination thereof, or
   5i) at least one of R₃₁ to R₃₅ is each independently: deuterium; or Group 3 substituted with at least one deuterium, wherein Group 3 includes:
      a C₁-C₂₀ alkyl group unsubstituted or substituted with -F, a cyano group, or any combination thereof; or
      a phenyl group unsubstituted or substituted with -F, a cyano group, a C₁-C₂₀ alkyl group, a phenyl group, or any combination thereof.

In one or more embodiments, the number of benzene groups included in the group represented by Formula 2 (e.g., benzene groups that are included in the group represented by Formula 2, are not condensed with each other, and are linked to each other through single bonds) may be 4 or more, for example, 4, 5, or 6.

In one or more embodiments, the group represented by Formula 2 may be a group represented by Formula 2-1(1): 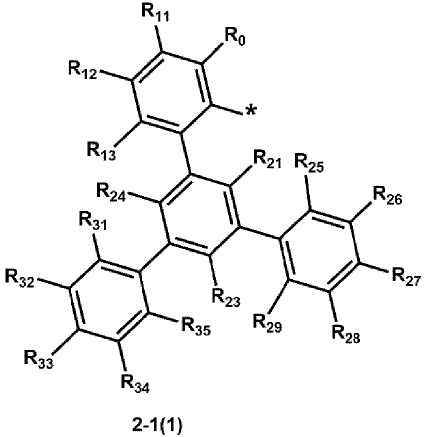 wherein, in Formula 2-1(1),
R₀ may be the same as described herein,
R₁₁ to R₁₃ may each be the same as described in connection with R₁,
R₂₁, R₂₃, and R₂₄ may each be the same as described in connection with R₂,
R₃₁ to R₃₅ may each be the same as described in connection with R₃, and
R₂₅ to R₂₉ may each independently be:
   hydrogen, deuterium, -F, or a cyano group; or
   a C₁-C₂₀ alkyl group unsubstituted or substituted with deuterium, -F, a cyano group, or any combination thereof, and
   * indicates a binding site to Formula 1.

For example, Formula 2-1(1) may satisfy Conditions 3 and 5 as described above with respect to Formula 2-1 as well as Conditions 6 and 7 below:
Condition 6
   6a) each of R₂₁, R₂₃, and R₂₄ is hydrogen or deuterium,
   6b) R₂₃ is a C₁-C₂₀ alkyl group unsubstituted or substituted with deuterium, -F, a cyano group, or any combination thereof (e.g., a tert-butyl group unsubstituted or substituted with deuterium),
   6c) at least one of R₂₁, R₂₃, and R₂₄ is each independently: deuterium; or Group 5 substituted with at least one deuterium, wherein Group 5 includes:
   a C₁-C₂₀ alkyl group unsubstituted or substituted with -F, a cyano group, or any combination thereof; or
   a phenyl group unsubstituted or substituted with -F, a cyano group, a C₁-C₂₀ alkyl group, a phenyl group, or any combination thereof; and
Condition 7
   7a) each of R₂₅ to R₂₉ is hydrogen or deuterium,
   7b) R₂₅ is a C₁-C₂₀ alkyl group unsubstituted or substituted with deuterium, -F, a cyano group, or any combination thereof (e.g., a tert-butyl group unsubstituted or substituted with deuterium),
   7c) R₂₅ is a phenyl group unsubstituted or substituted with deuterium, -F, a cyano group, a C₁-C₂₀ alkyl group, a phenyl group, or any combination thereof,
   7d) R₂₆ is a C₁-C₂₀ alkyl group unsubstituted or substituted with deuterium, -F, a cyano group, or any combination thereof (e.g., a tert-butyl group unsubstituted or substituted with deuterium),
   7e) R₂₆ is a phenyl group unsubstituted or substituted with deuterium, -F, a cyano group, a C₁-C₂₀ alkyl group, a phenyl group, or any combination thereof,
   7f) R₂₇ is a C₁-C₂₀ alkyl group unsubstituted or substituted with deuterium, -F, a cyano group, or any combination thereof,
   7g) R₂₇ is a phenyl group unsubstituted or substituted with deuterium, -F, a cyano group, a C₁-C₂₀ alkyl group, a phenyl group, or any combination thereof, or
   7h) R₂₆ and R₂₈ are each independently:
      a C₁-C₂₀ alkyl group unsubstituted or substituted with deuterium, -F, a cyano group, or any combination thereof (e.g., a tert-butyl group unsubstituted or substituted with deuterium); or
      a phenyl group unsubstituted or substituted with deuterium, -F, a cyano group, a C₁-C₂₀ alkyl group, a phenyl group, or any combination thereof,
   7i) at least one of R₂₅ to R₂₉ is each independently: deuterium; or Group 6 substituted with at least one deuterium, wherein Group 6 includes:
      a C₁-C₂₀ alkyl group unsubstituted or substituted with -F, a cyano group, or any combination thereof; or
      a phenyl group unsubstituted or substituted with -F, a cyano group, a C₁-C₂₀ alkyl group, a phenyl group, or any combination thereof.

In one or more embodiments, R₃₁ and R₂₅ in Formula 2-1(1)may each independently be:
a C₁-C₂₀ alkyl group unsubstituted or substituted with deuterium, -F, a cyano group, or any combination thereof (e.g., a tert-butyl group unsubstituted or substituted with deuterium); or
a phenyl group unsubstituted or substituted with deuterium, -F, a cyano group, a C₁-C₂₀ alkyl group, a phenyl group, or any combination thereof.

In one or more embodiments, R₃₂ and R₂₆ in Formula 2-1(1) may each independently be:
a C₁-C₂₀ alkyl group unsubstituted or substituted with deuterium, -F, a cyano group, or any combination thereof (e.g., a tert-butyl group unsubstituted or substituted with deuterium); or
a phenyl group unsubstituted or substituted with deuterium, -F, a cyano group, a C₁-C₂₀ alkyl group, a phenyl group, or any combination thereof.

In one or more embodiments, R₃₃ and R₂₇ in Formula 2-1(1) may each independently be:
a C₁-C₂₀ alkyl group unsubstituted or substituted with deuterium, -F, a cyano group, or any combination thereof (e.g., a tert-butyl group unsubstituted or substituted with deuterium); or
a phenyl group unsubstituted or substituted with deuterium, -F, a cyano group, a C₁-C₂₀ alkyl group, a phenyl group, or any combination thereof.

In Formula 1, two or more of Z₁, Z₂, Z₃, T₁₁, T₁₂, T₁₃, T₂₁, T₂₂ and T₂₃ may optionally be linked together to form a substituted or unsubstituted C₅-C₃₀ carbocyclic group or a substituted or unsubstituted C₁-C₃₀ heterocyclic group.

In an embodiment, in Formula 1, two or more of Z₁, Z₂, Z₃, T₁₁, T₁₂, T₁₃, T₂₁, T₂₂ and T₂₃ may be linked together to form a substituted or unsubstituted C₅-C₃₀ carbocyclic group or a substituted or unsubstituted C₁-C₃₀ heterocyclic group. As a result, the condensed cyclic compound may be represented by, for example, Formula 1-1, 1-2, or 1-3: 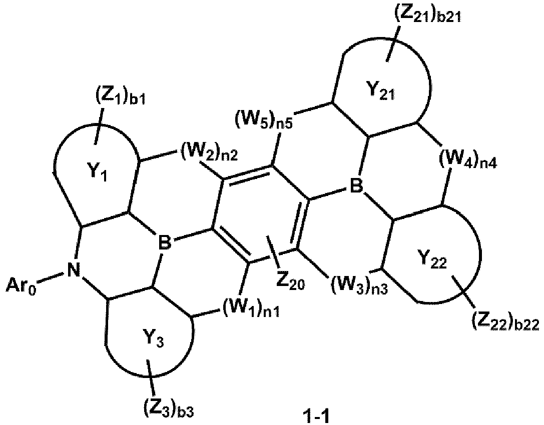 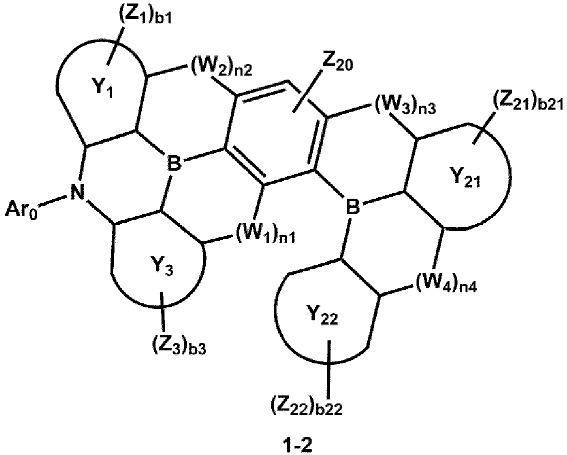 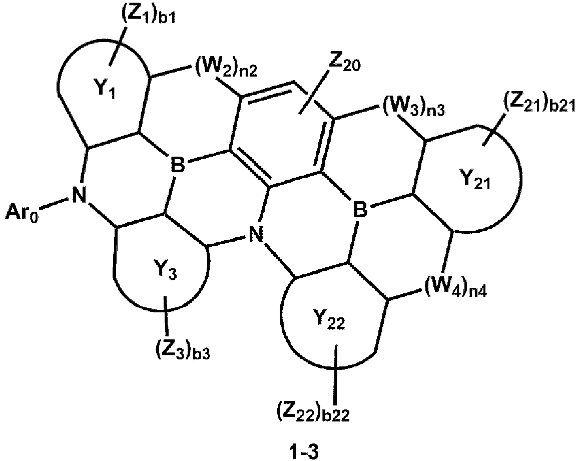 wherein, in Formulae 1-1, 1-2, and 1-3,
Ar₀, ring Y₁, ring Y₃, W₁, W₂, n1, n2, Z₁, Z₃, b1, and b3 may each be the same as described herein,
ring Y₂₁ and ring Y₂₂ may each independently be a C₅-C₆₀ carbocyclic group or a C₃-C₆₀ heterocyclic group,
W₃ may be a single bond, O, S, N(Ar₃), N(T₃₁), C(T₃₂)(T₃₃), or Si(T₃₂)(T₃₃),
W₄ may be a single bond, O, S, N(Ar₄), N(T₄₁), C(T₄₂)(T₄₃), or Si(T₄₂)(T₄₃),
W₅ may be a single bond, O, S, N(Ar₅), N(T₅₁), C(T₅₂)(T₅₃), or Si(T₅₂)(T₅₃),
n3 to n5 may each independently be 0 or 1,
when n3 is 0, *-(W₃)ₙ₃-*' may be absent,
when n4 is 0, *-(W₄)ₙ₄-*' may be absent,
when n5 is 0, *-(W₅)ₙ₅-*' may be absent,
the sum of n1 and n2 in Formulae 1-1 and 1-2 may be 1 or more,
the sum of n3, n4, and n5 in Formula 1-1 may be 1 or more,
the sum of n3 and n4 in Formulae 1-2 and 1-3 may be 1 or more,
each of Ar₃ to Ar₅ may be the group represented by Formula 2,
Z₂₀ to Z₂₂ may each be the same as described in connection with Z₂,
b21 and b22 may each independently be an integer from 0 to 5, and
each of Formulae 1-1, 1-2, and 1-3 may satisfy at least one of Conditions 1A and 2A:
   Condition 1A
      b1 is 1 or greater, and at least one of b1 Z₁(s) includes a carbazole group; and
   Condition 2A
      at least one of a), b), and c) is satisfied:
      a) b21 is 1 or more, and at least one of b21 Z₂₁(s) includes a carbazole group,
      b) Z₂₀ includes a carbazole group, and
      c) b22 is 1 or more, and at least one of b22 Z₂₂(s) includes a carbazole group.

Ring Y₂₁ and ring Y₂₂ may each be the same as described in connection with ring Y₁.

In an embodiment, the condensed cyclic compound represented by Formula 1 may include at least one deuterium, at least one tert-butyl group, or any combination thereof. For example, the condensed cyclic compound represented by Formula 1 may include at least one tert-butyl group and optionally at least one deuterium.

In one or more embodiments, the condensed cyclic compound represented by Formula 1 may have a symmetric structure.

In one or more embodiments, the condensed cyclic compound represented by Formula 1 may have an asymmetric structure.

In one or more embodiments, the condensed cyclic compound may be represented by Formula 1A: 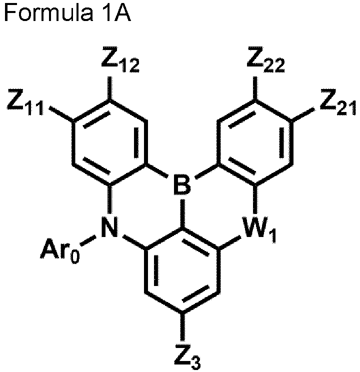 wherein, in Formula 1A,
W₁ may be O, S, N(Ar₁), N(T₁₁), C(T₁₂)(T₁₃), or Si(T₁₂)(T₁₃),
Ar₀, Ar₁, and T₁₁ to T₁₃ may each be the same as described herein,
Z₁₁ and Z₁₂ may each be the same as described herein in connection with Z₁,
Z₂₁ and Z₂₂ may each be the same as described in connection with Z₂,
at least one of Z₁₁, Z₁₂, Z₂₁, and Z₂₂ may be an N-carbazolyl group unsubstituted or substituted with deuterium, -F, a cyano group, a C₁-C₂₀ alkyl group, a phenyl group, or any combination thereof, and
Z₃ may be a C₁-C₂₀ alkyl group unsubstituted or substituted with deuterium, -F, a cyano group, or any combination thereof (e.g., a tert-butyl group unsubstituted or substituted with deuterium).

In an embodiment, in Formula 1A, each of Z₁₁ and Z₂₁ may be an N-carbazolyl group unsubstituted or substituted with deuterium, -F, a cyano group, a C₁-C₂₀ alkyl group, a phenyl group, or any combination thereof, and each of Z₁₂ and Z₂₂ may be hydrogen or deuterium.

In one or more embodiments, in Formula 1A, each of Z₁₂ and Z₂₂ may be an N-carbazolyl group unsubstituted or substituted with deuterium, -F, a cyano group, a C₁-C₂₀ alkyl group, a phenyl group, or any combination thereof, and each of Z₁₁ and Z₂₁ may be hydrogen or deuterium.

In one or more embodiments, the condensed cyclic compound may be a multiple resonance thermally activated delayed fluorescence material.

In one or more embodiments, the condensed cyclic compound may be one of Compounds 1 to 782:

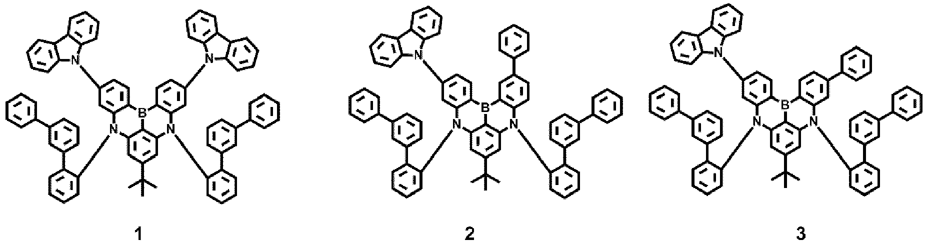

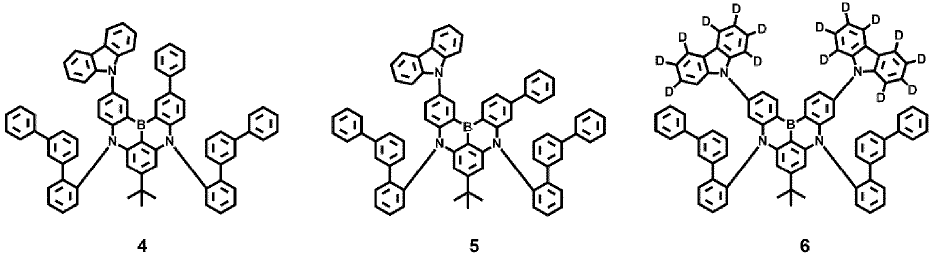

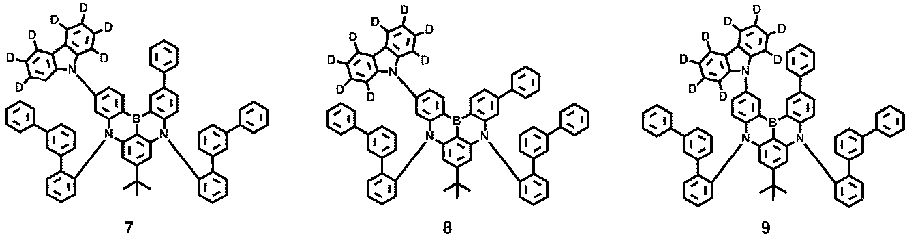

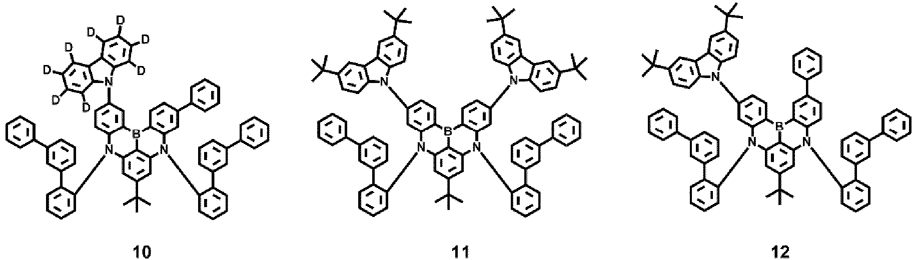

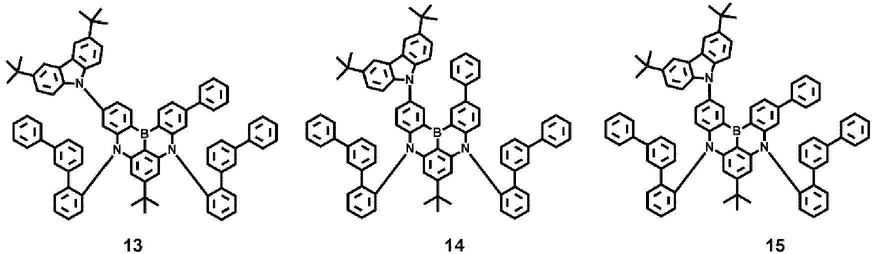

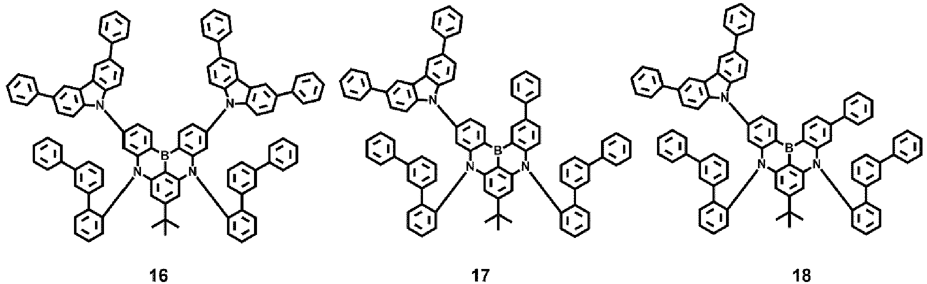

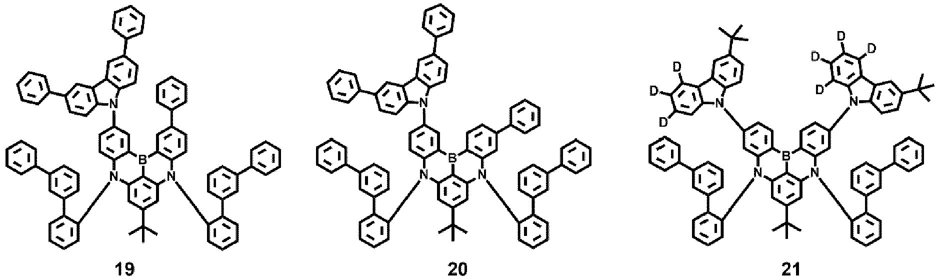

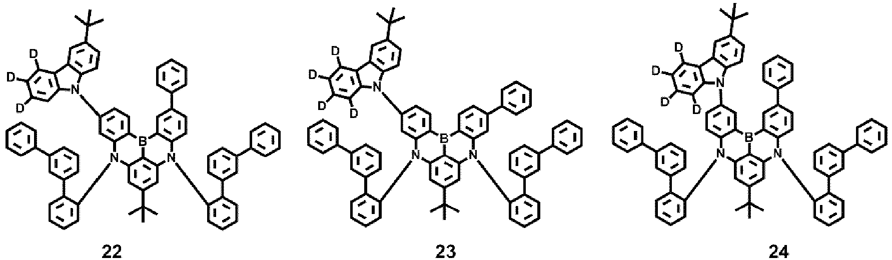

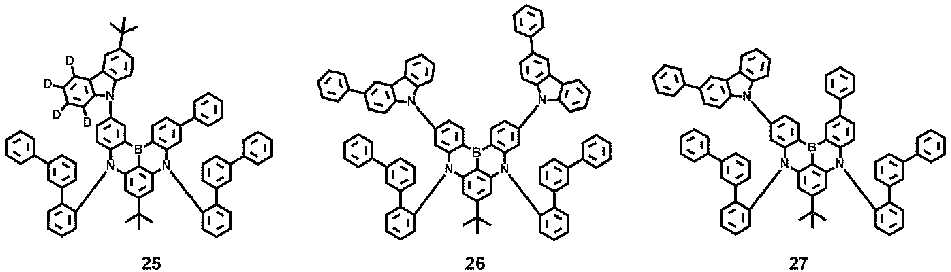

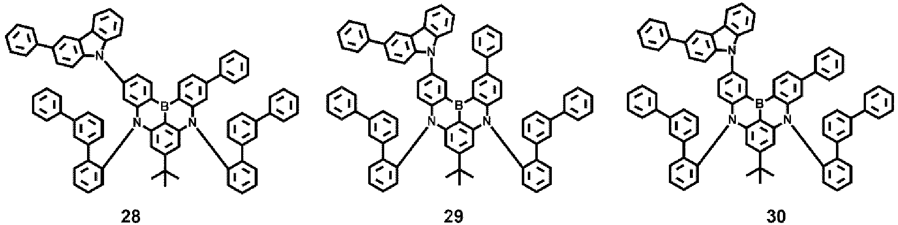

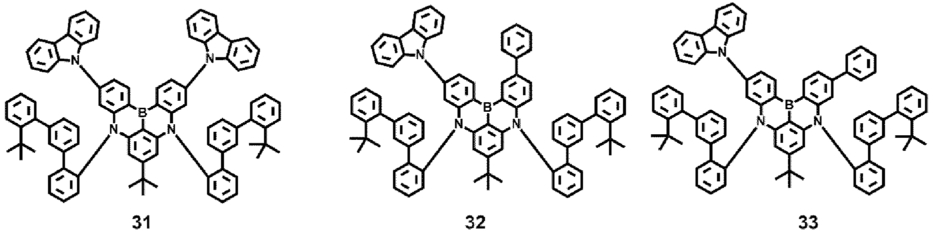

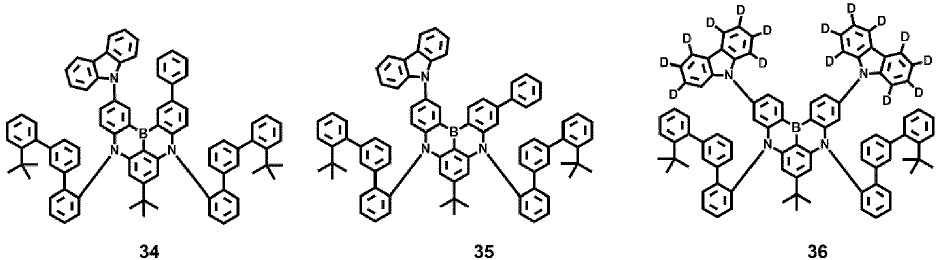

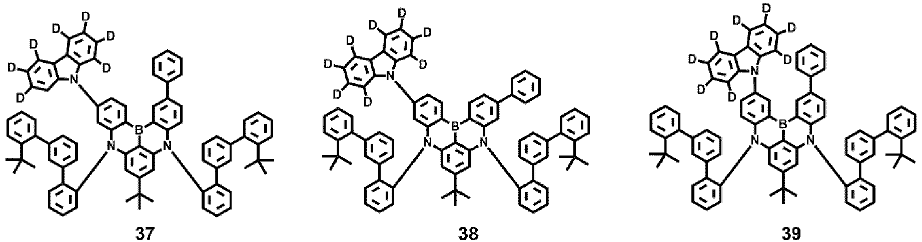

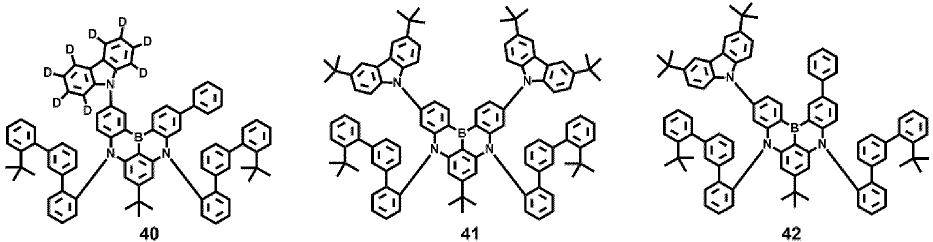

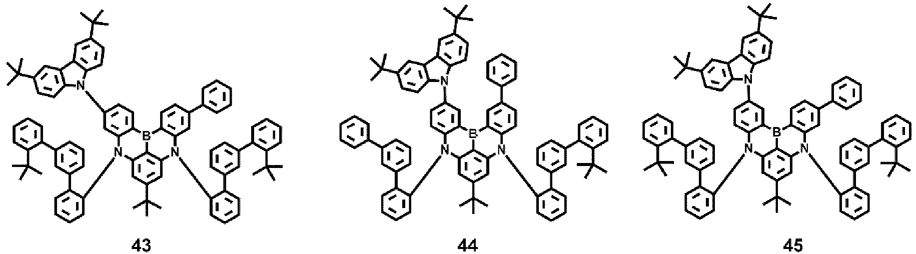

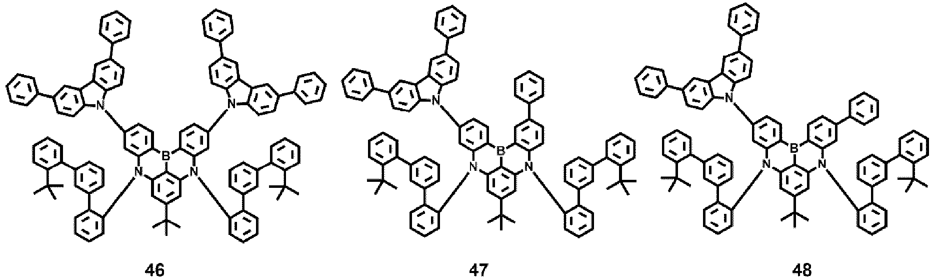

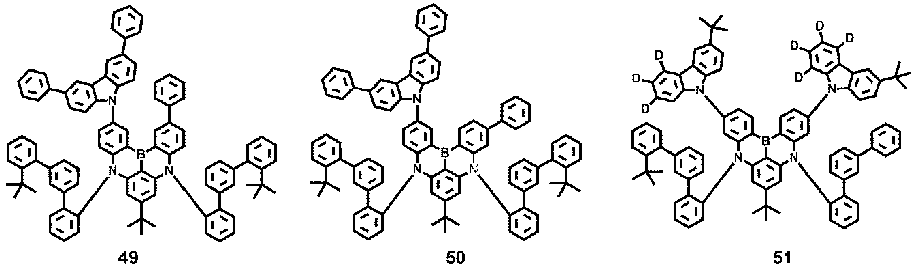

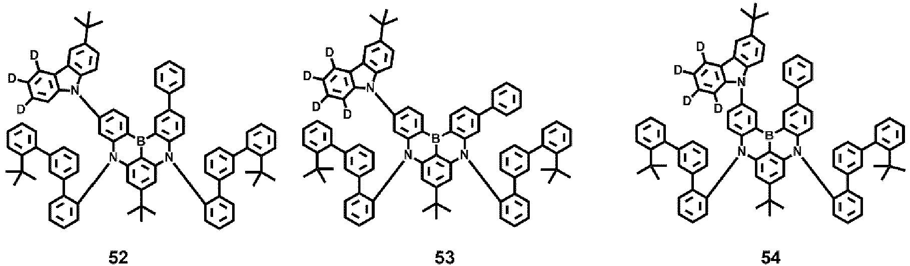

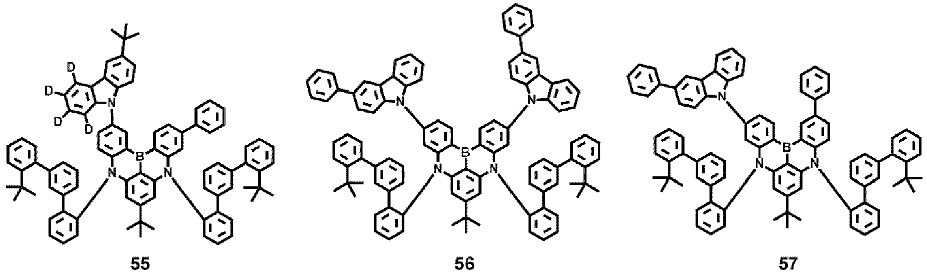

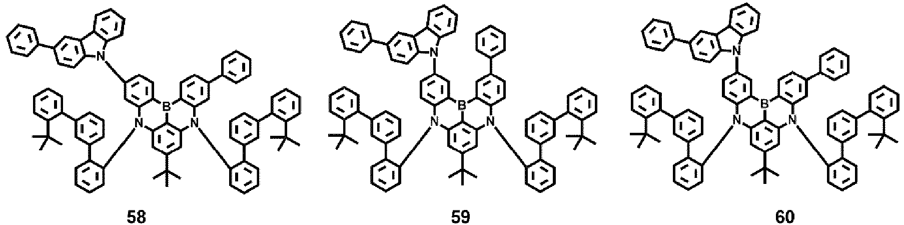

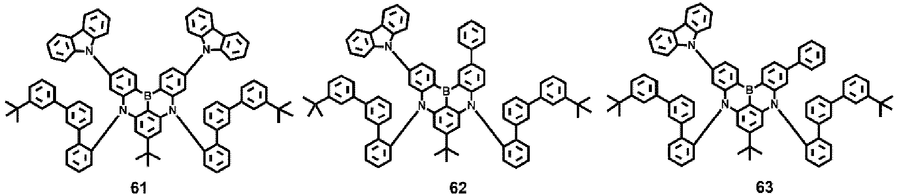

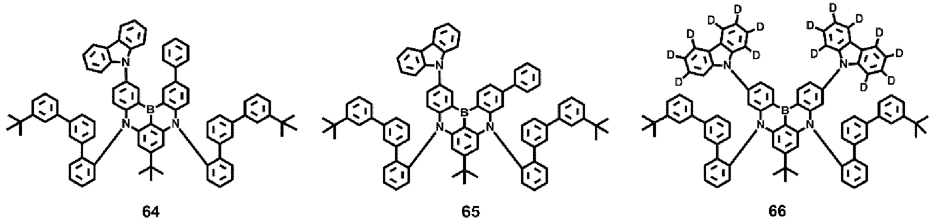

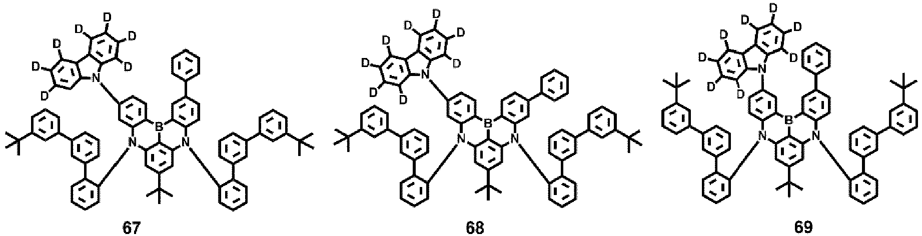

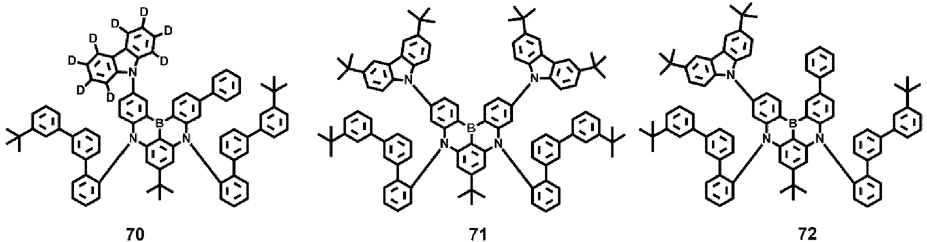

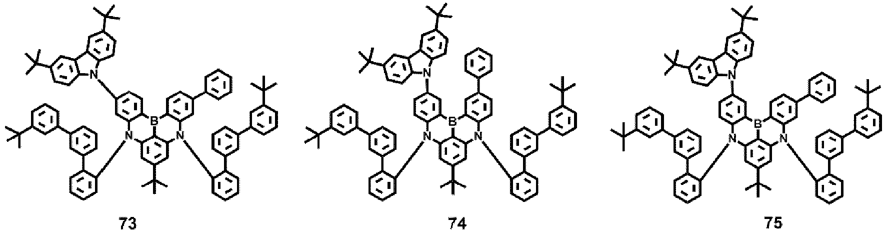

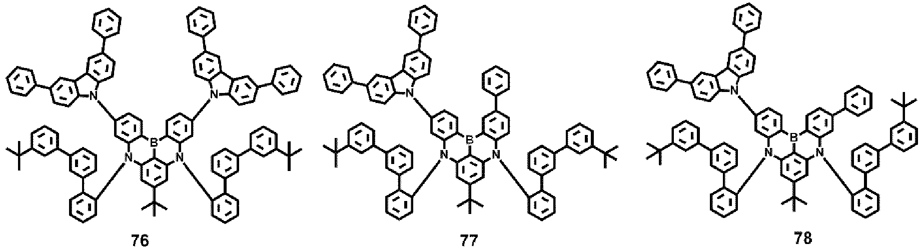

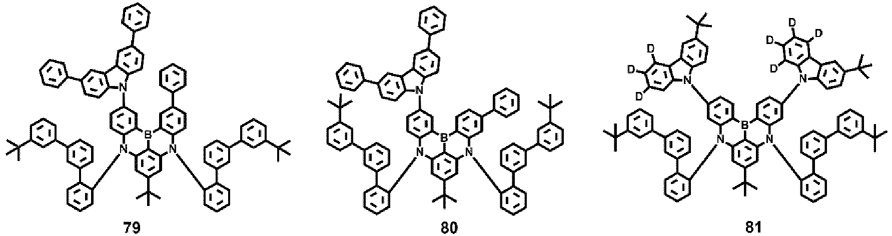

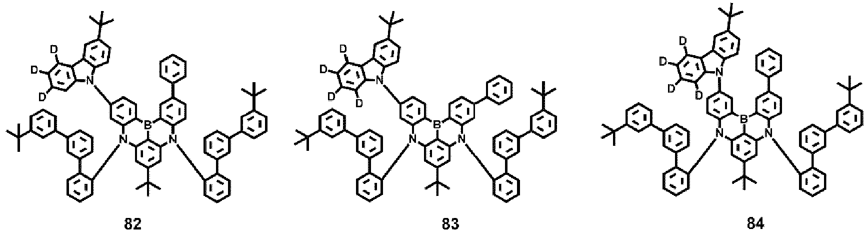

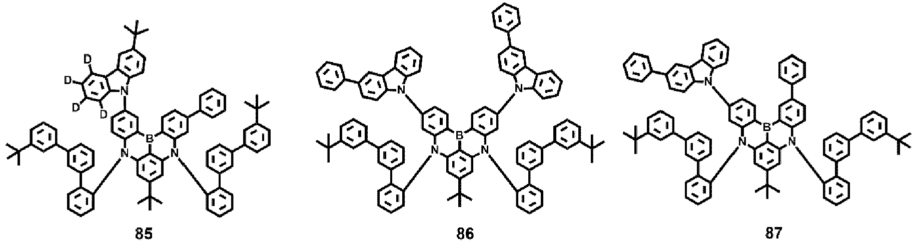

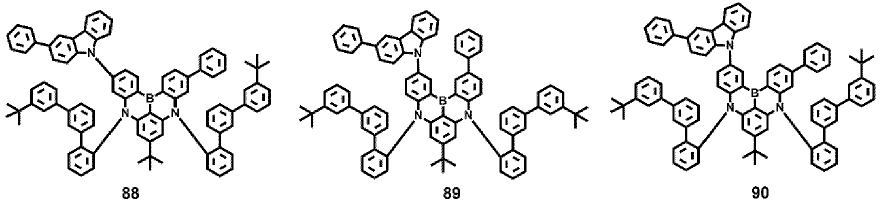

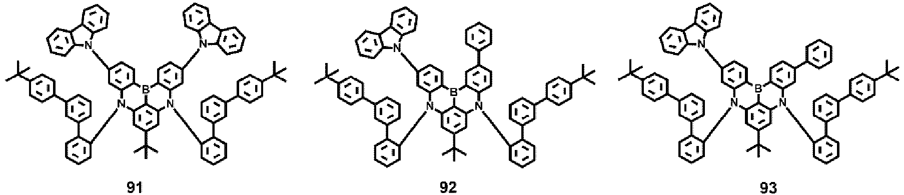

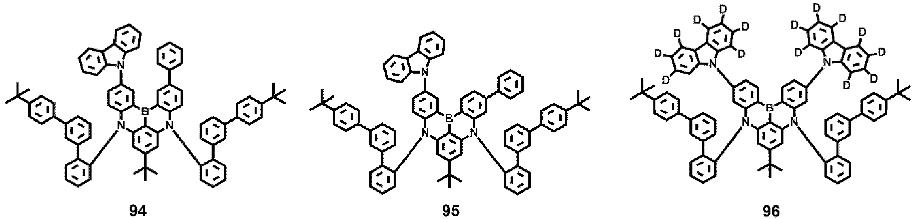

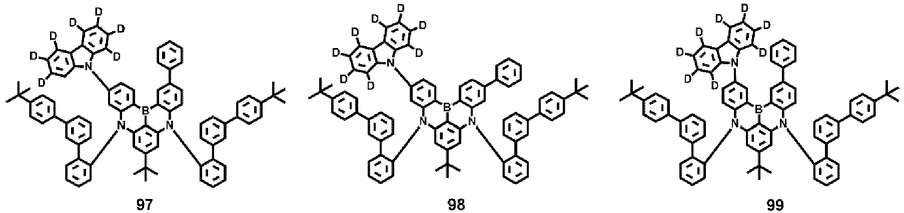

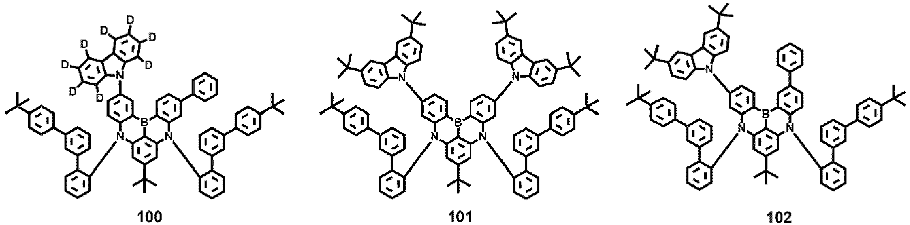

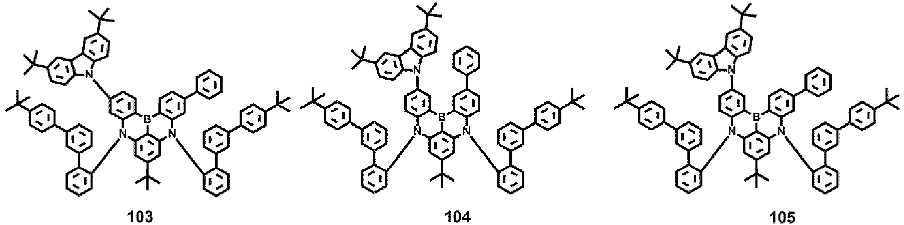

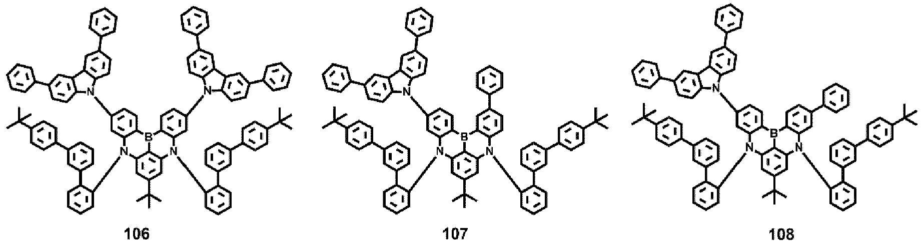

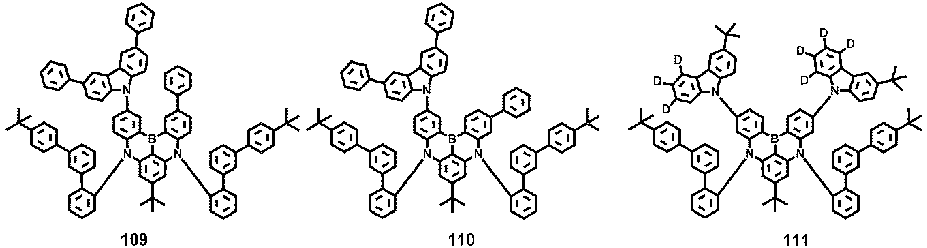

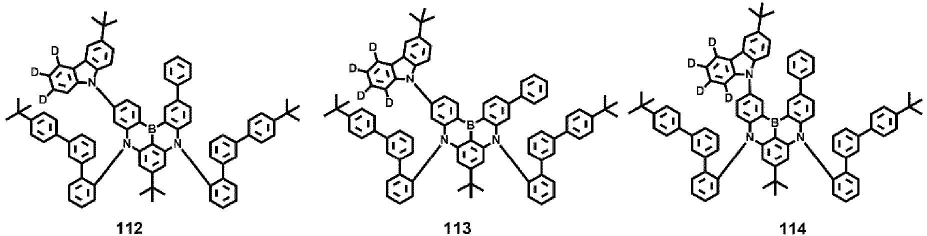

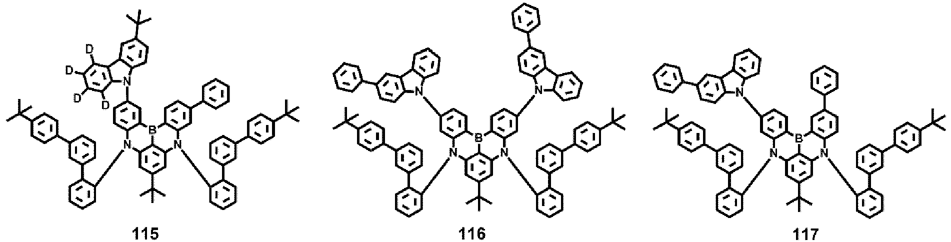

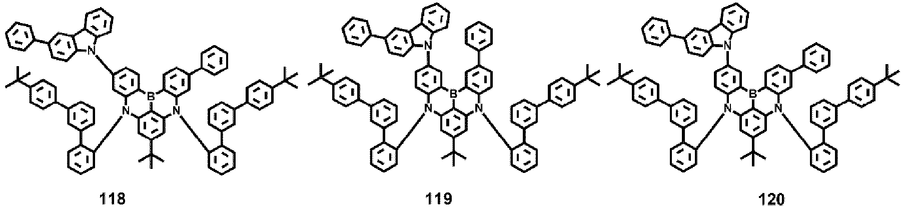

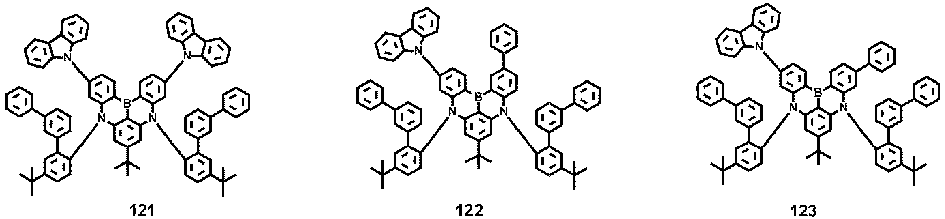

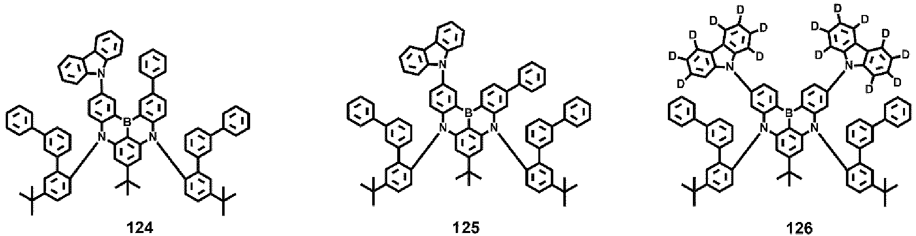

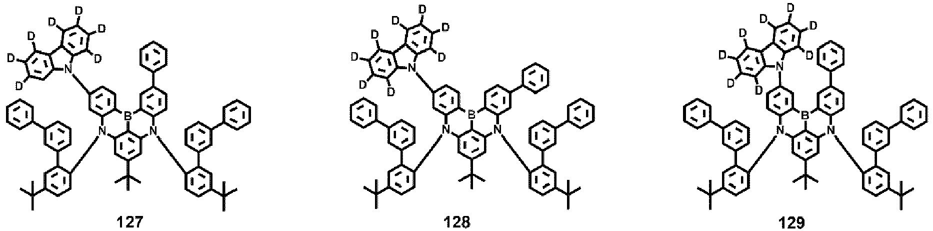

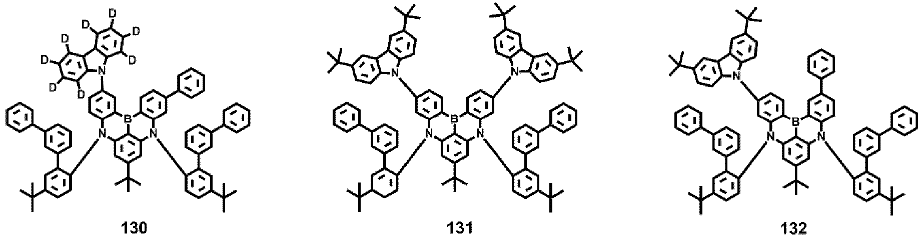

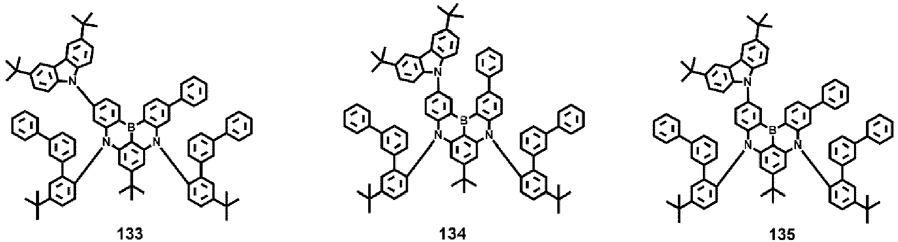

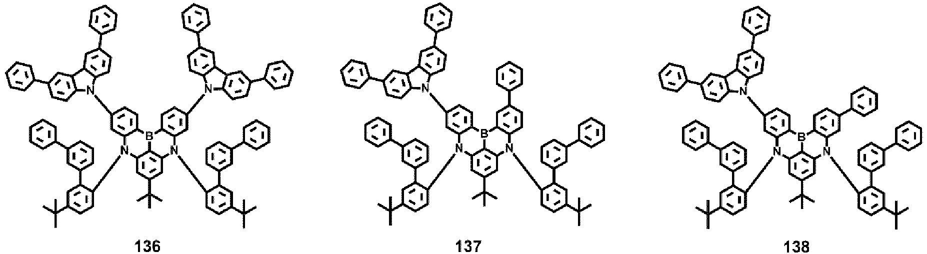

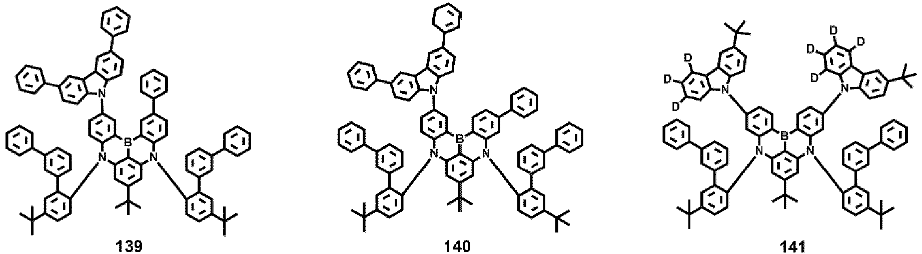

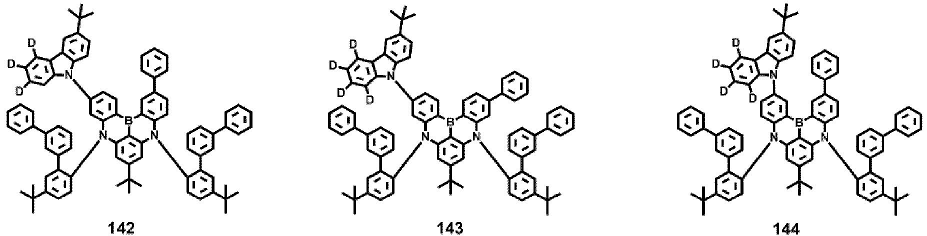

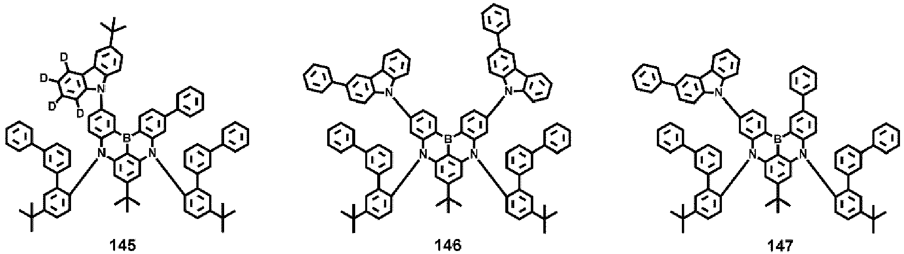

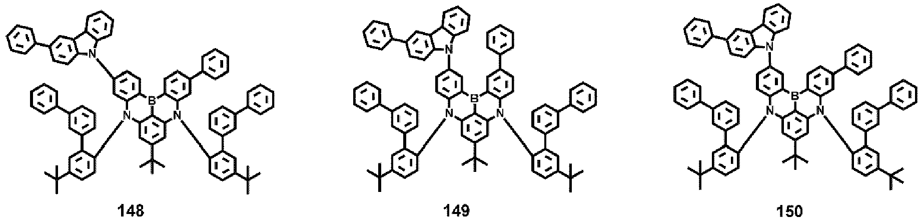

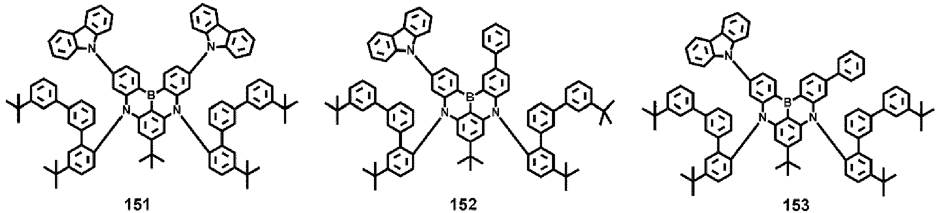

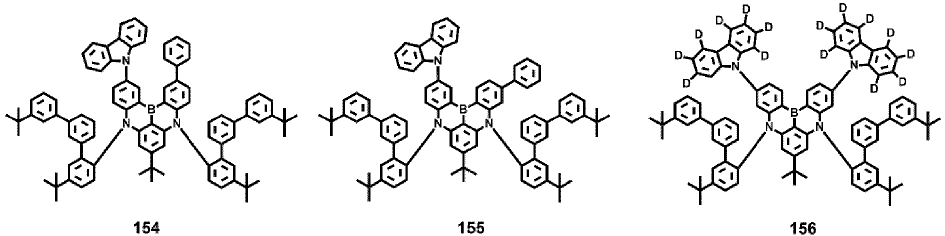

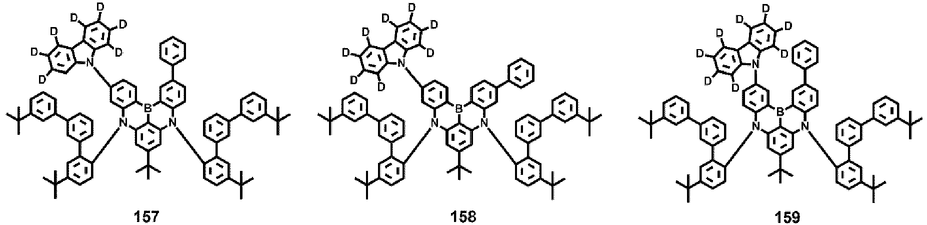

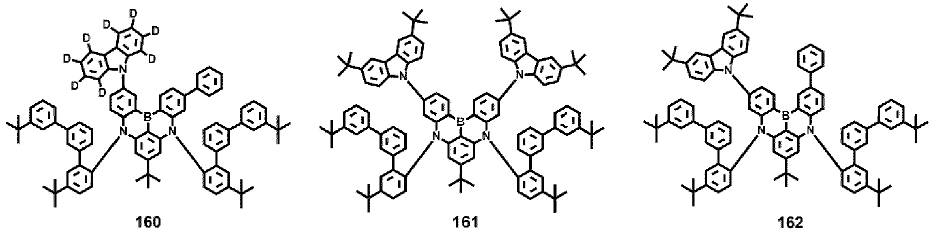

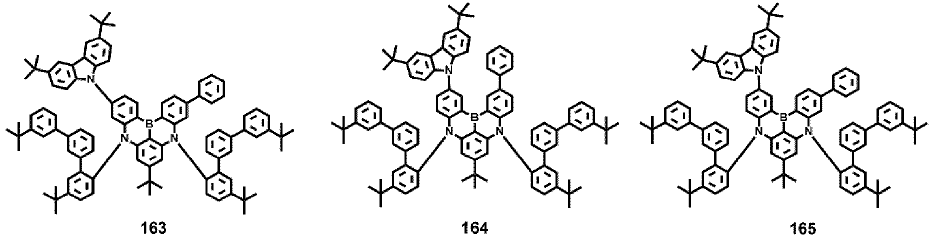

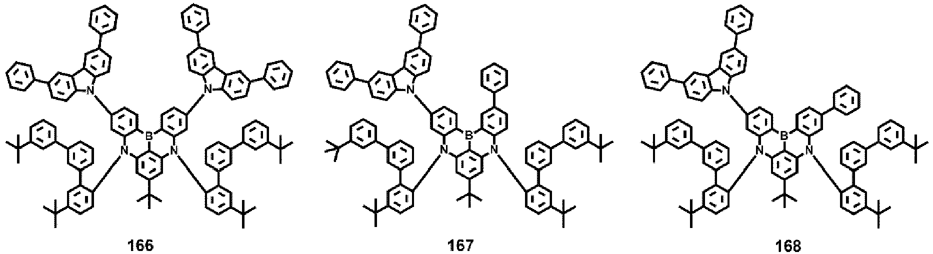

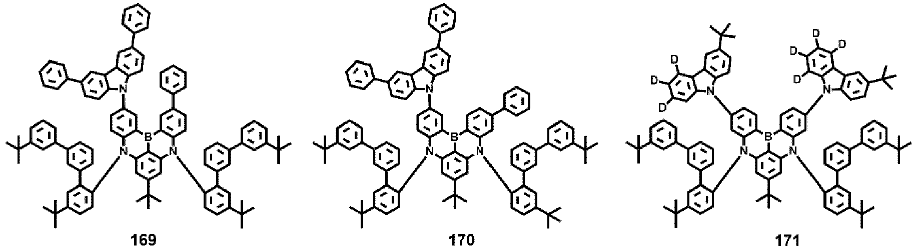

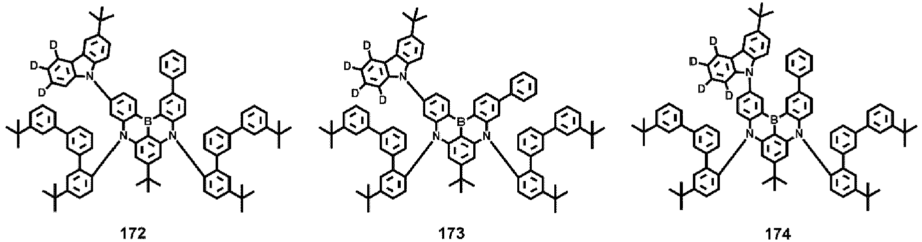

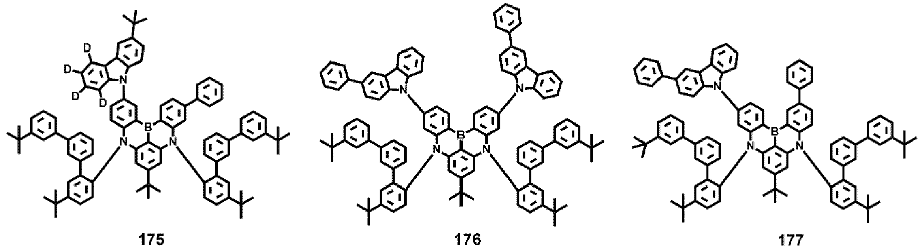

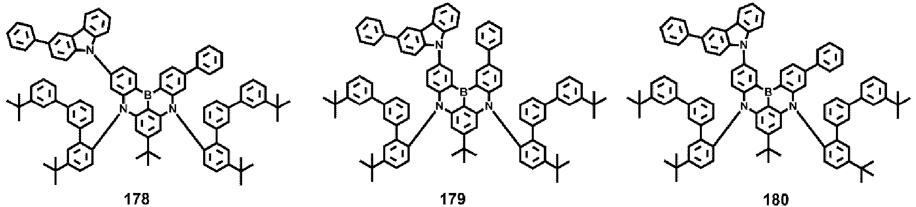

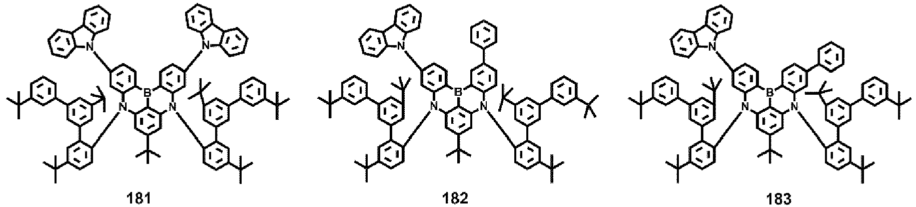

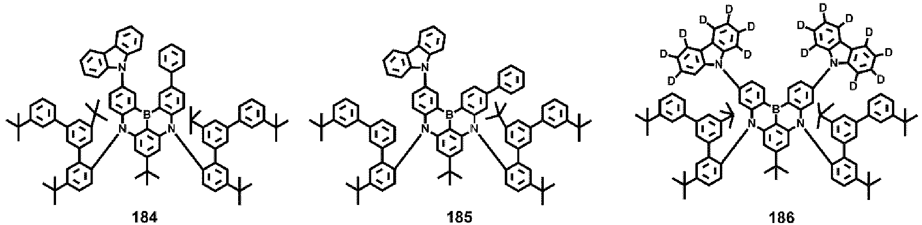

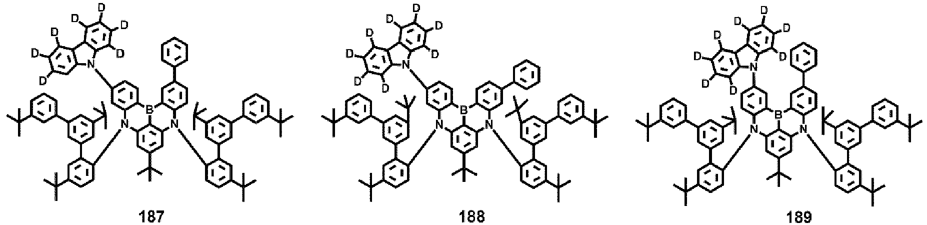

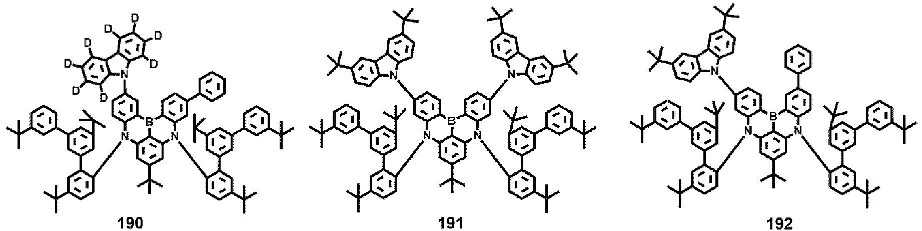

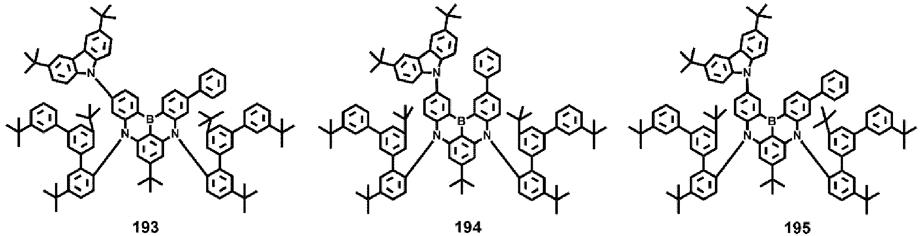

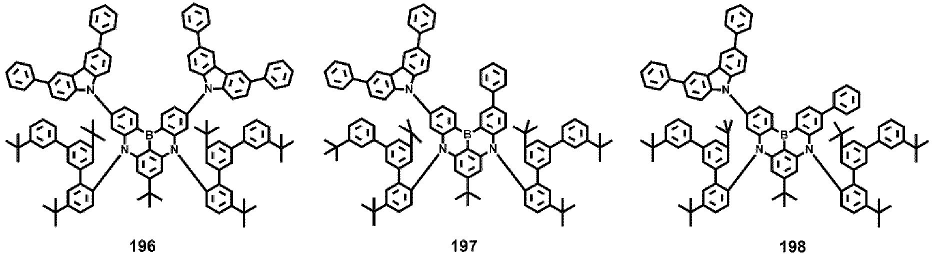

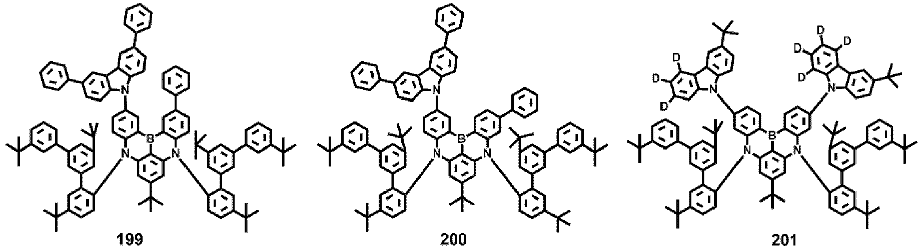

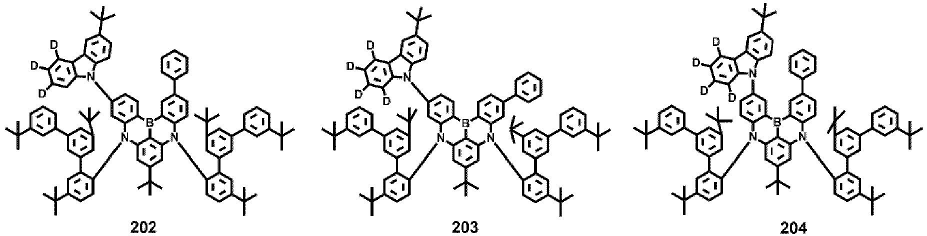

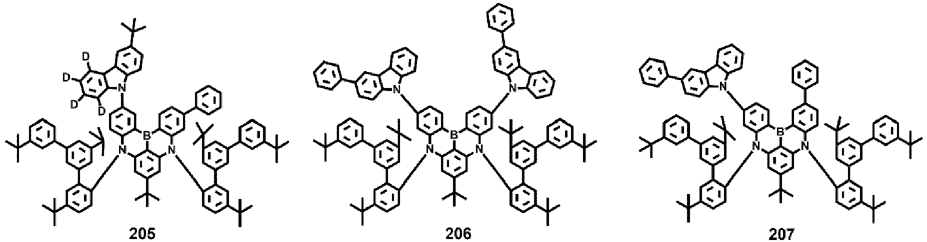

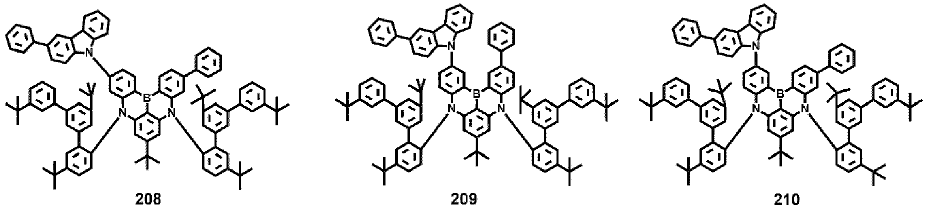

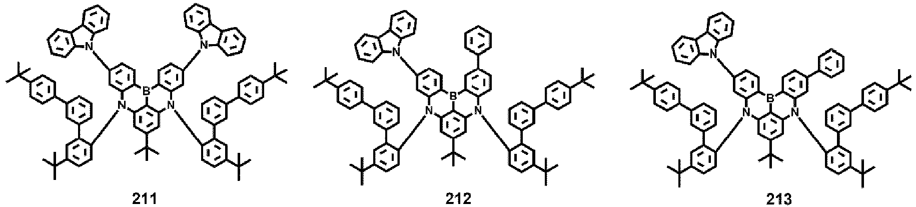

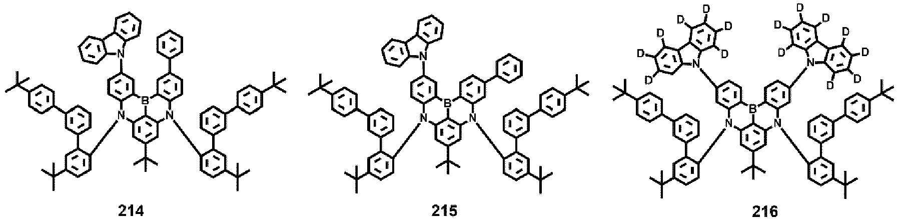

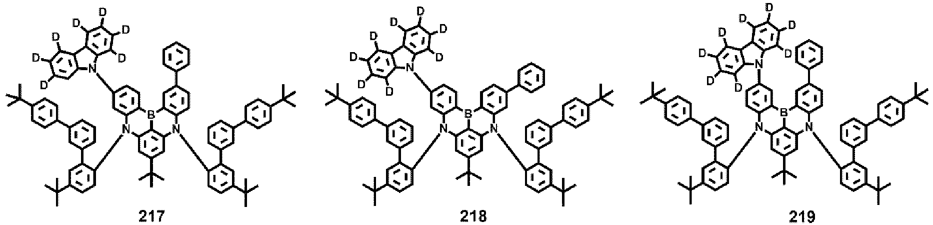

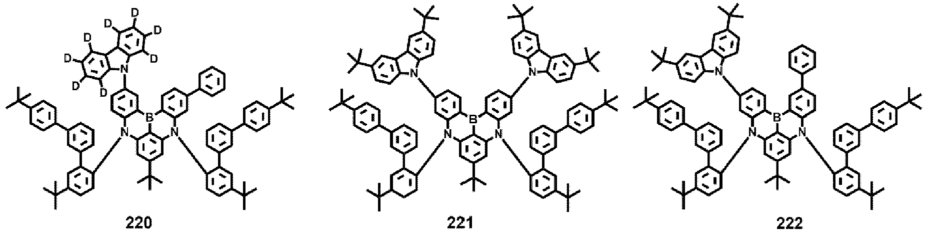

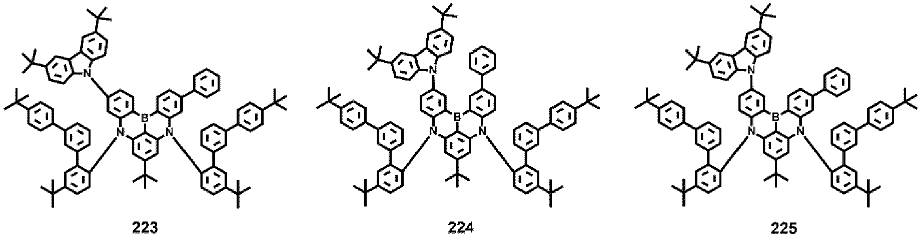

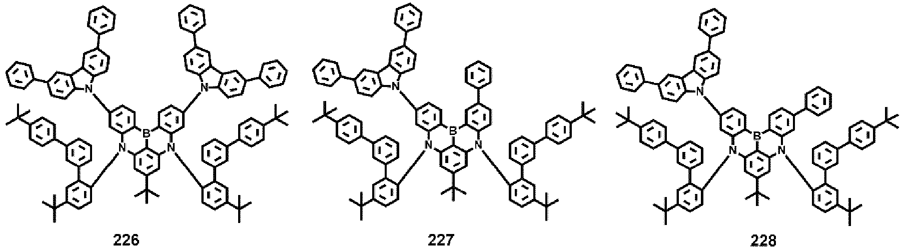

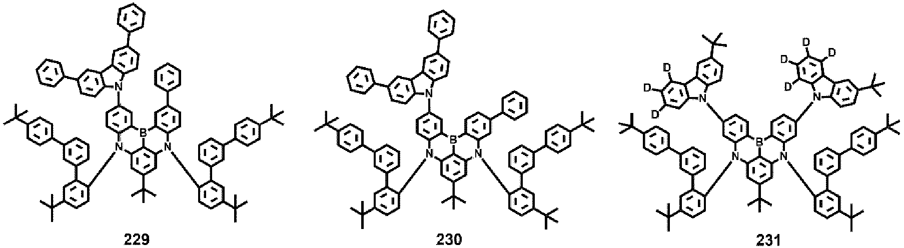

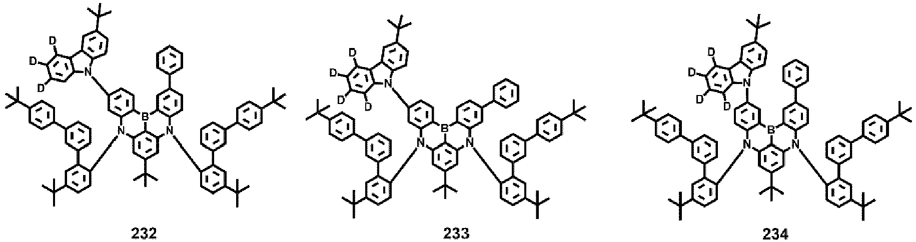

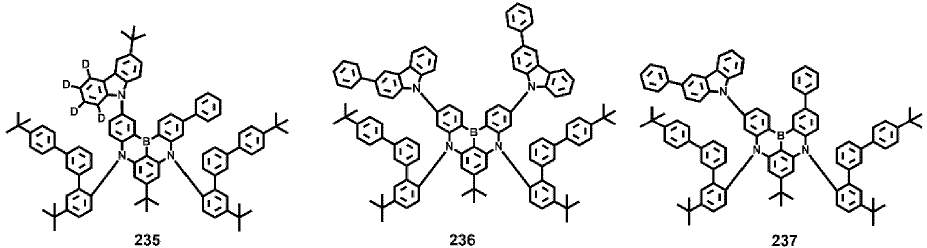

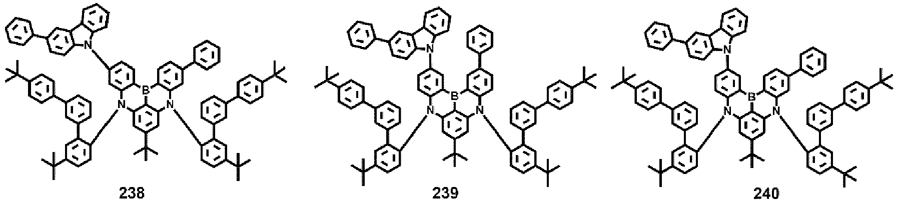

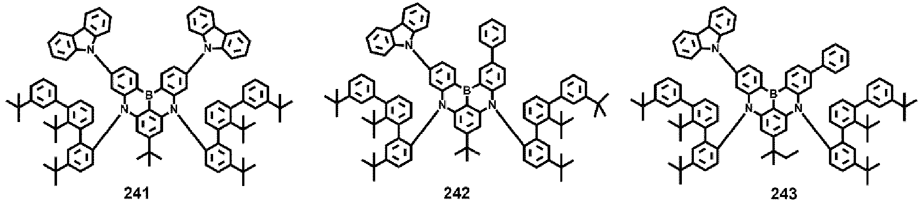

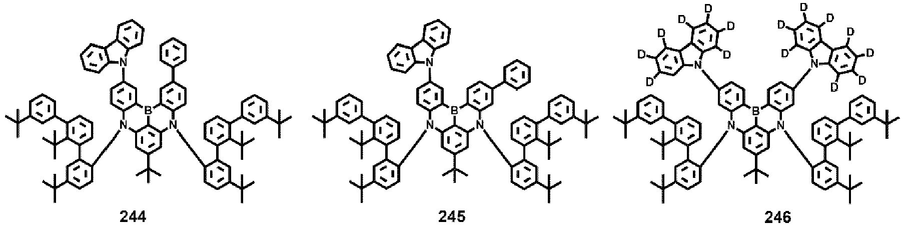

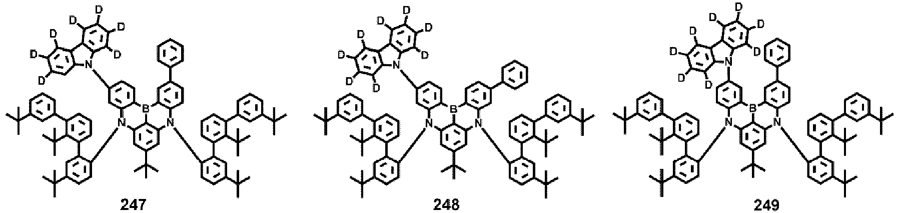

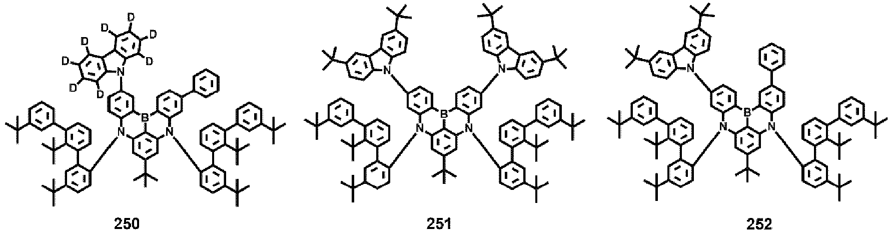

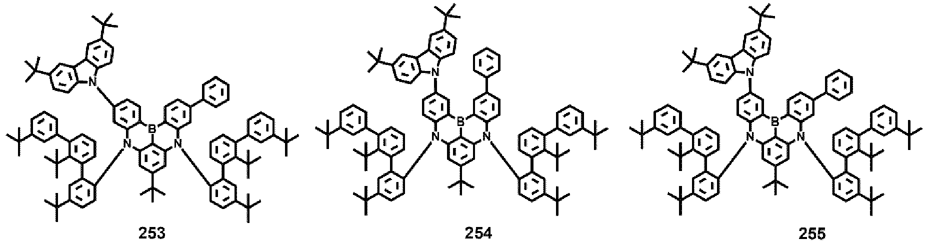

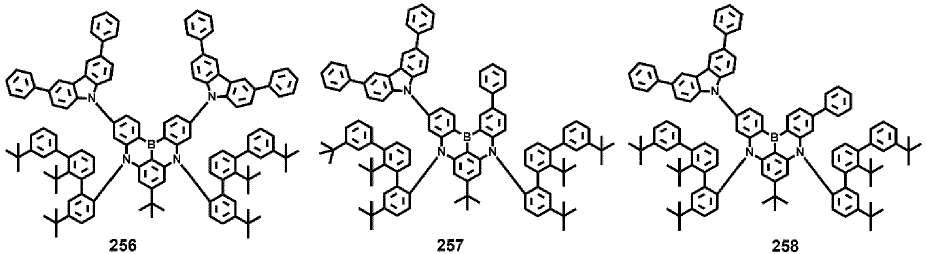

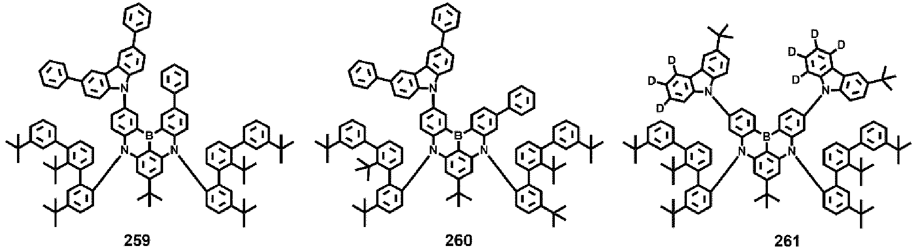

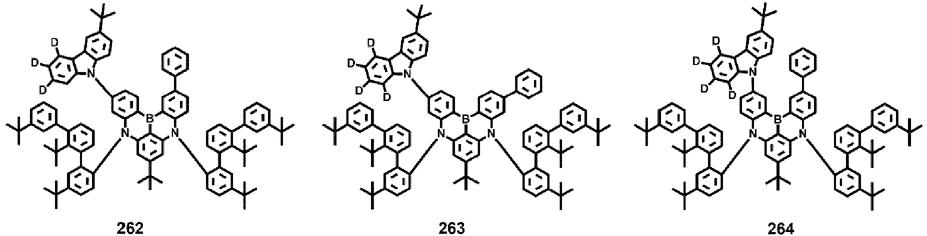

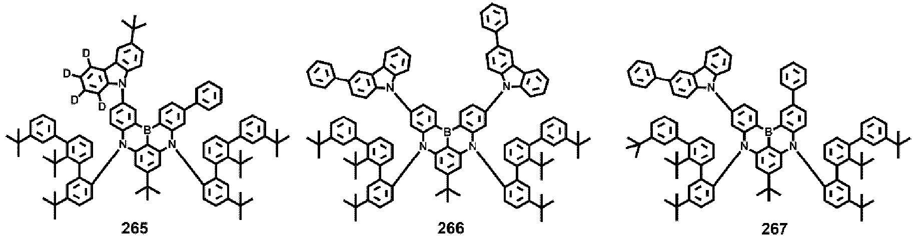

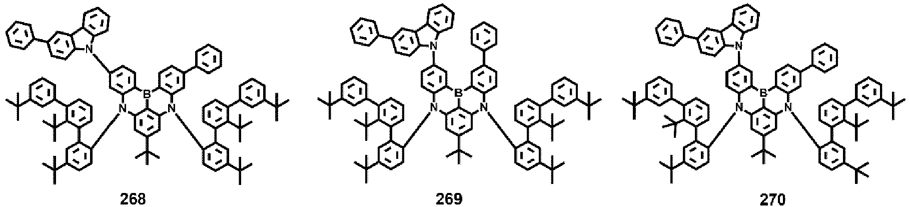

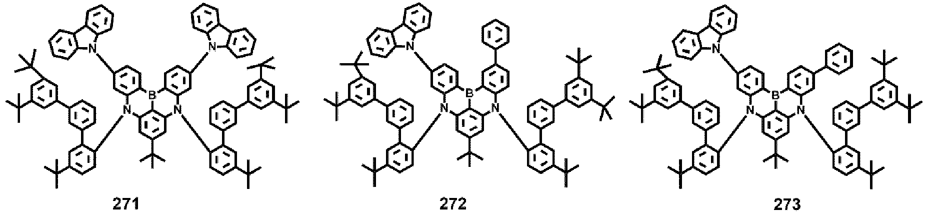

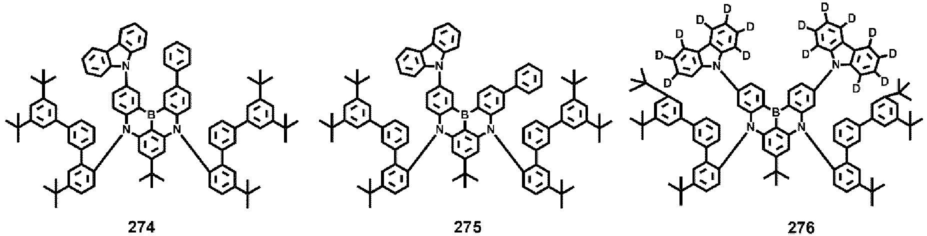

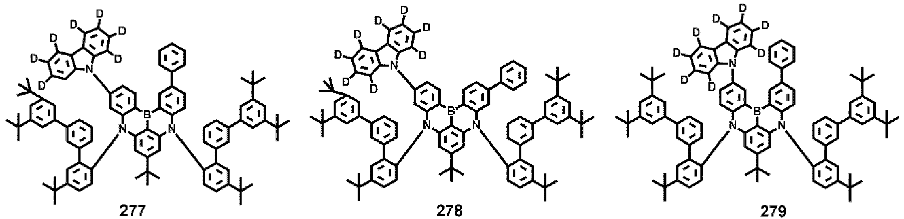

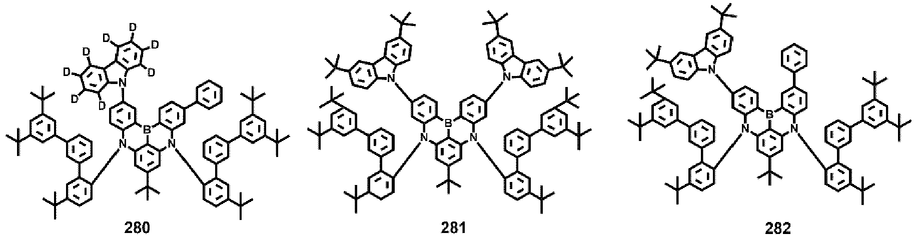

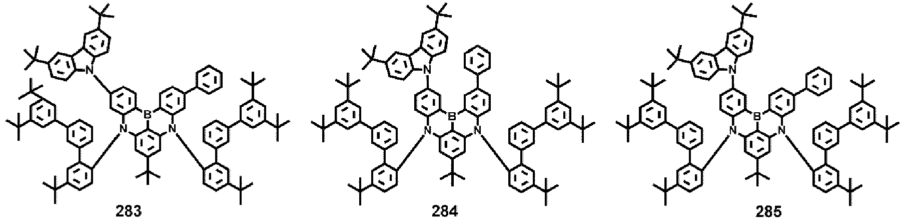

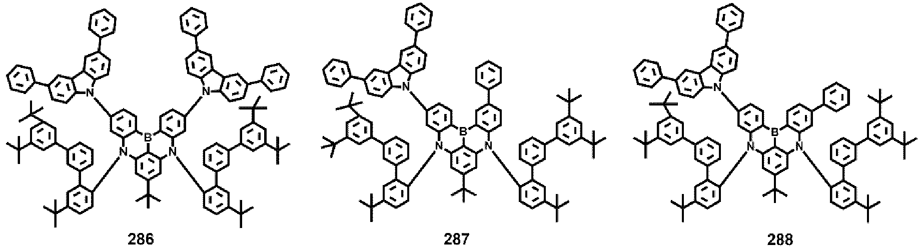

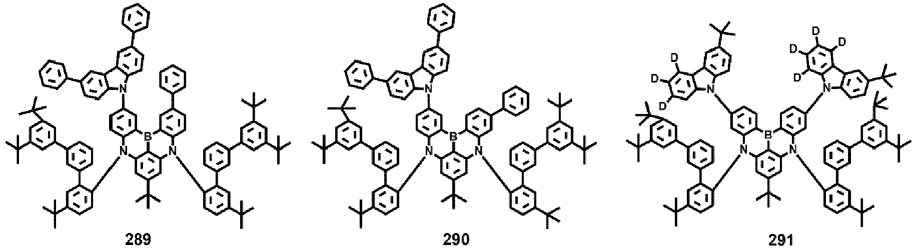

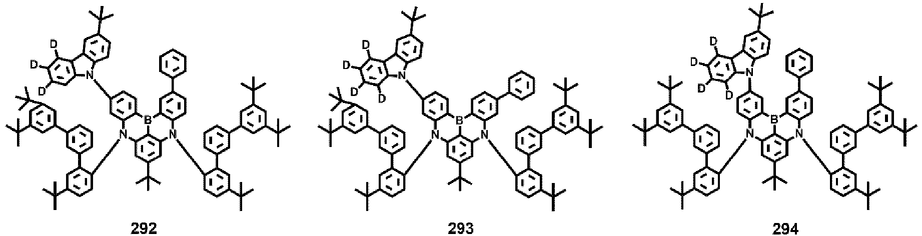

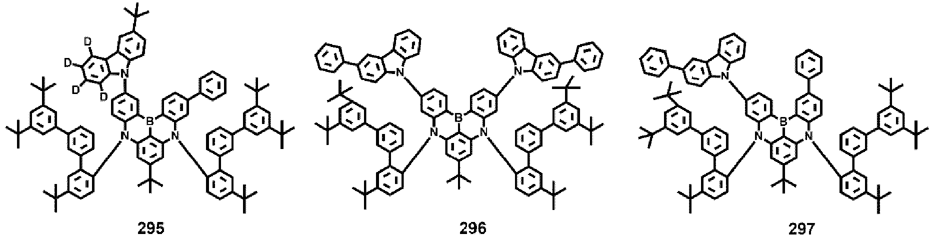

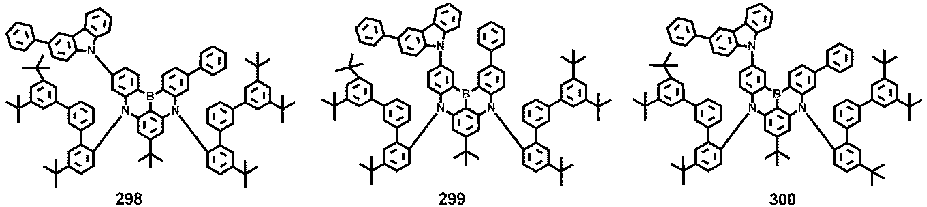

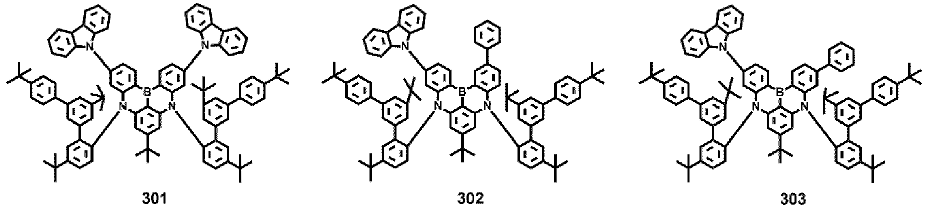

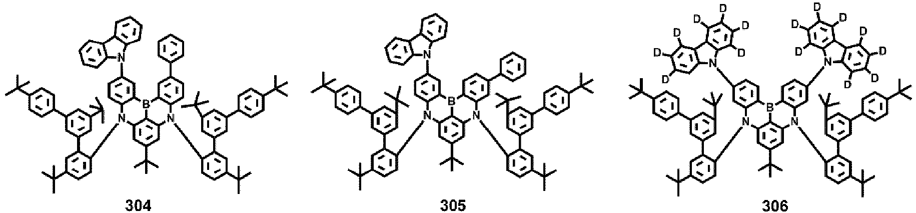

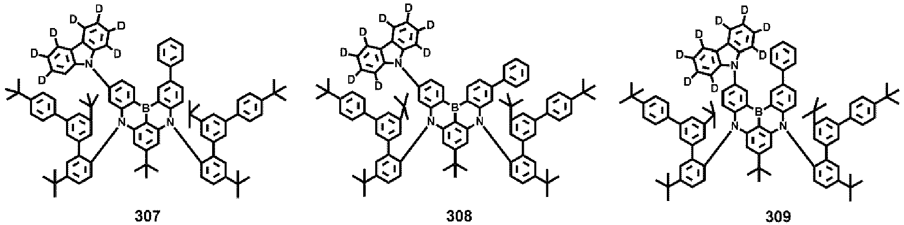

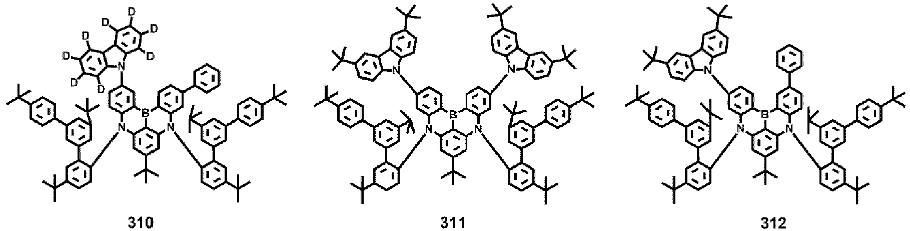

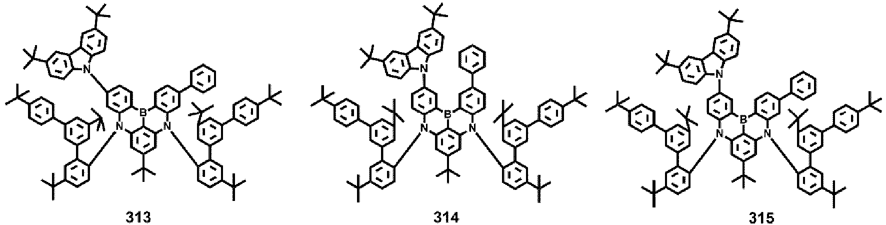

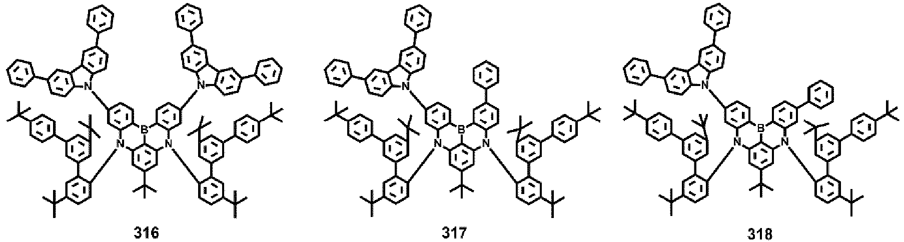

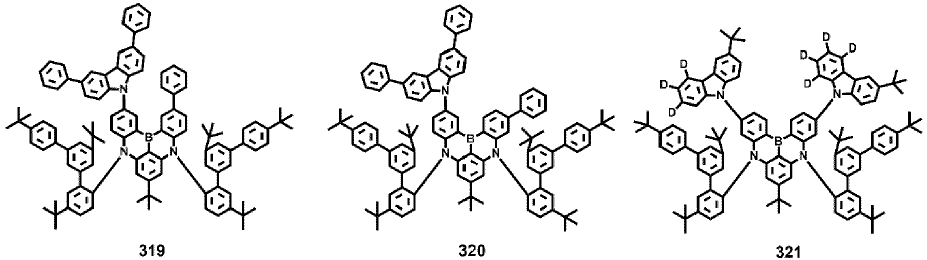

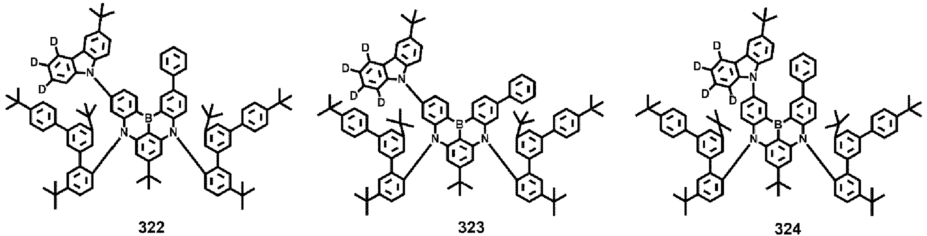

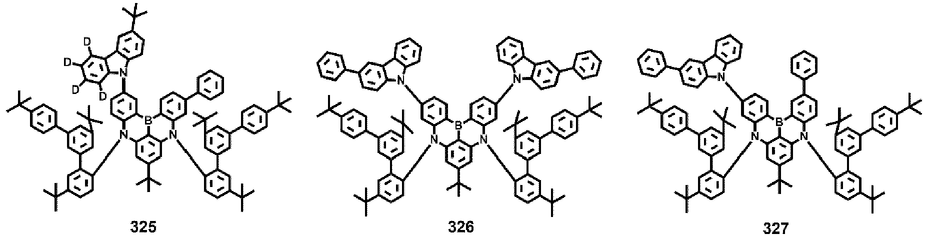

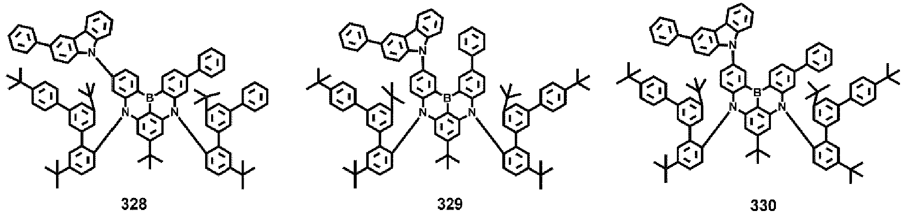

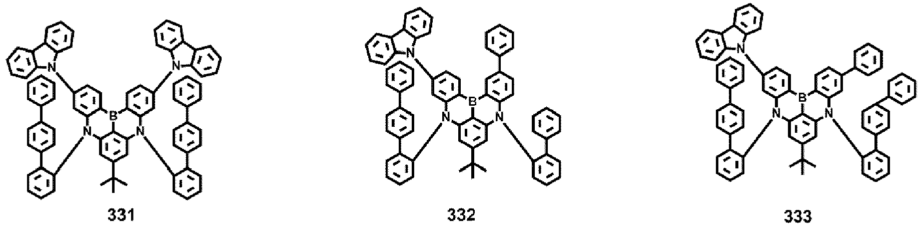

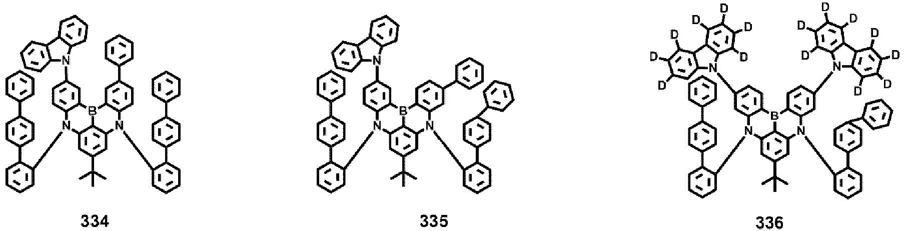

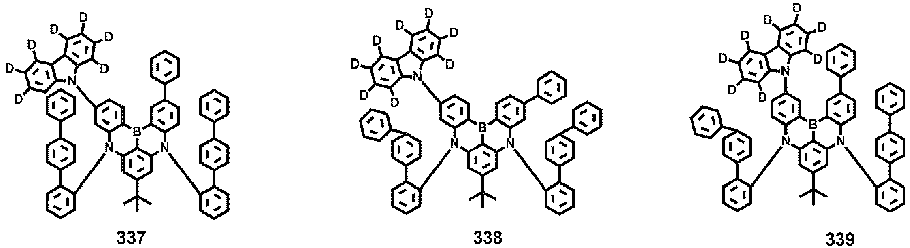

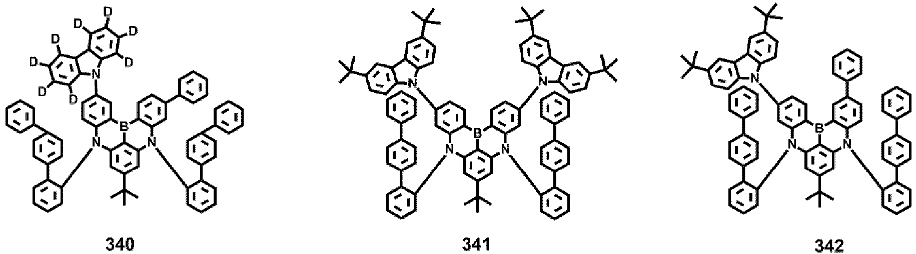

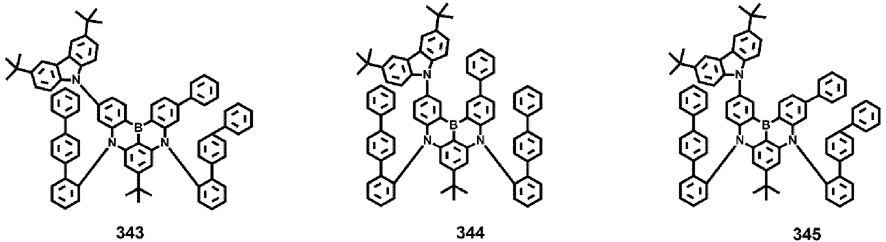

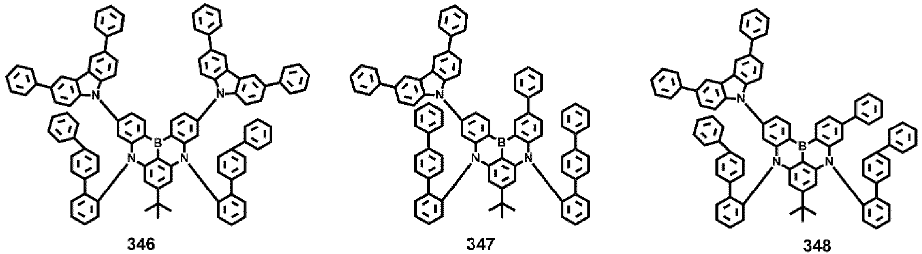

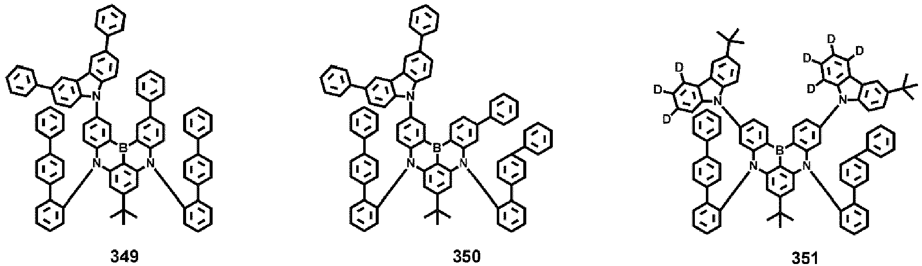

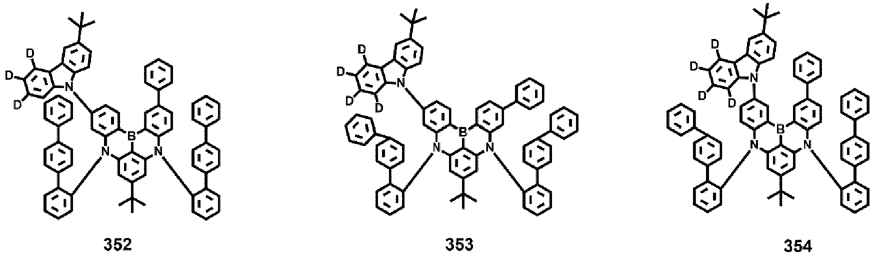

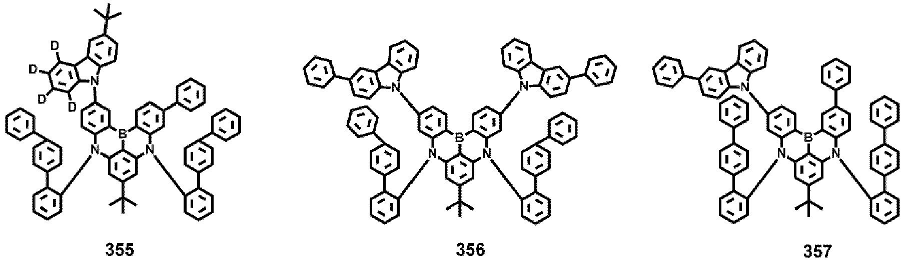

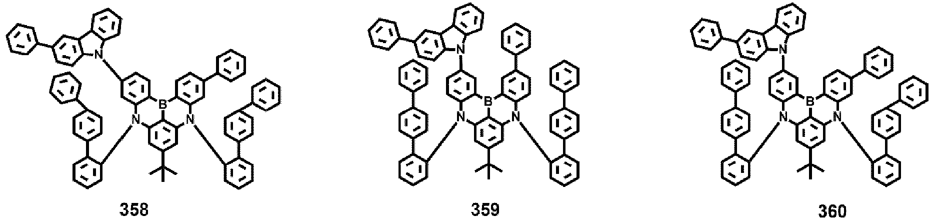

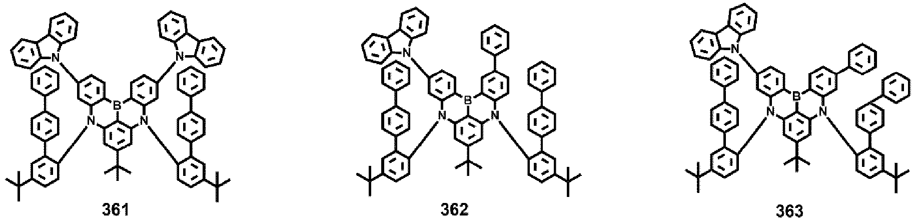

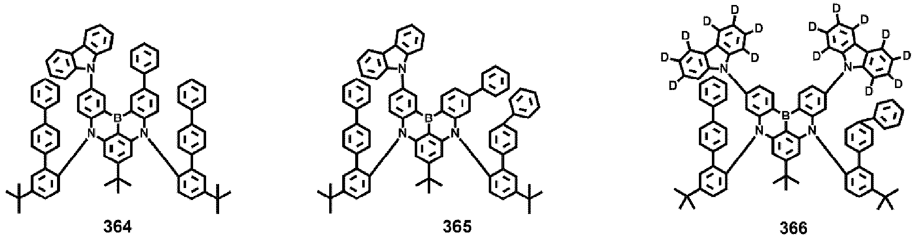

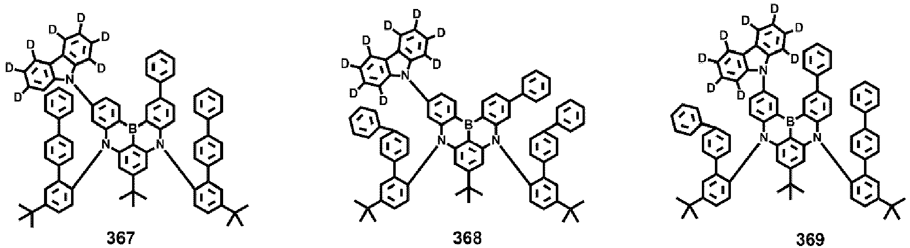

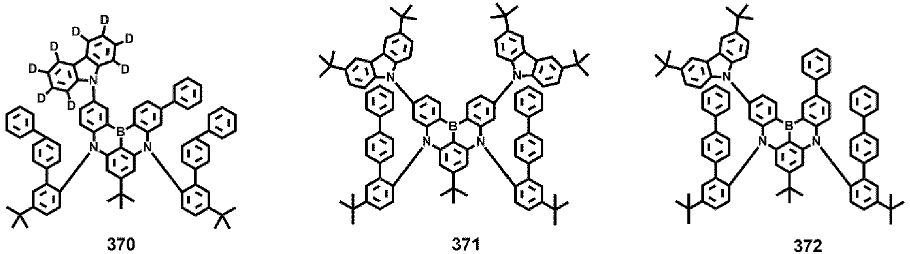

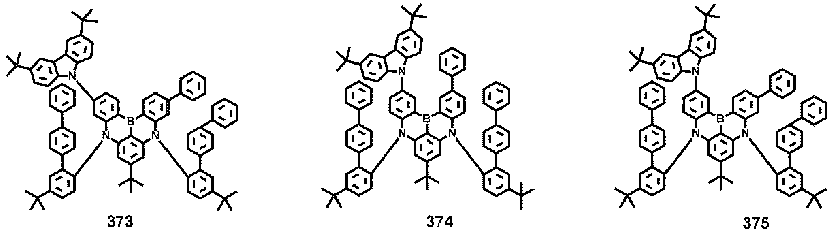

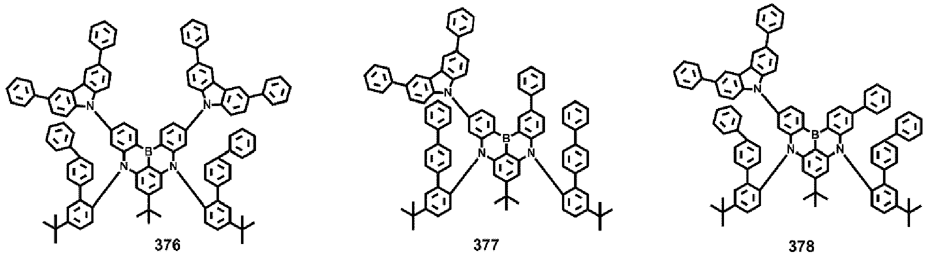

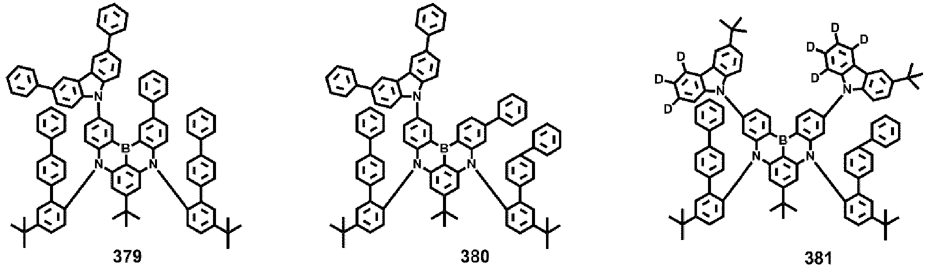

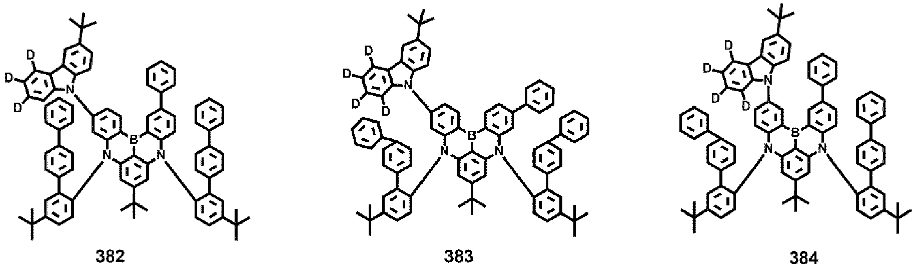

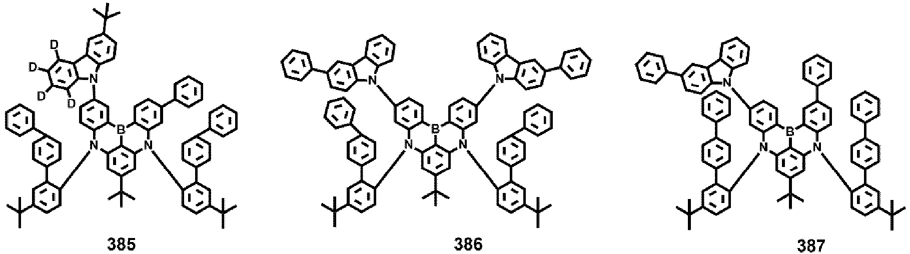

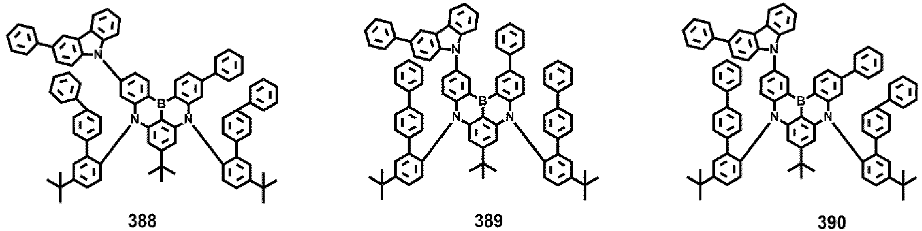

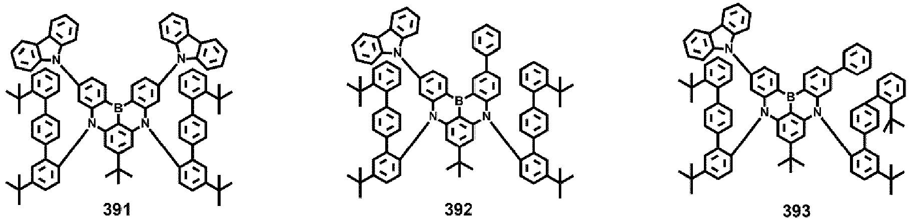

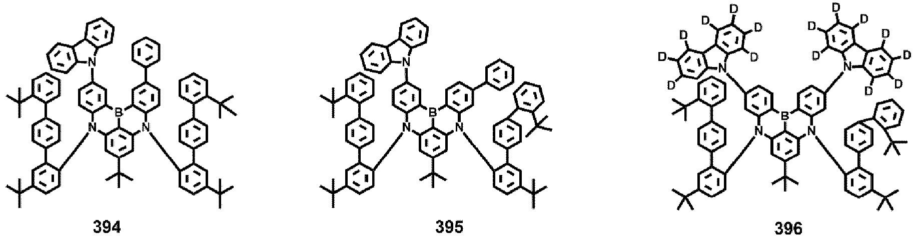

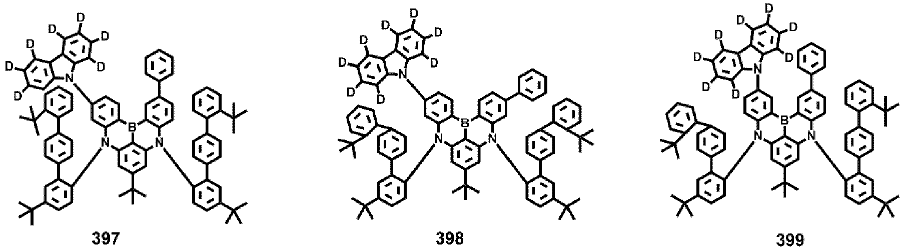

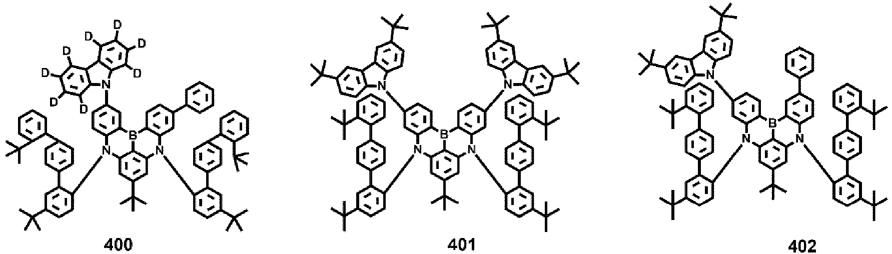

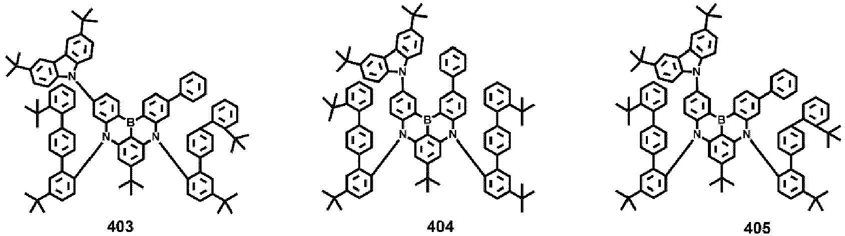

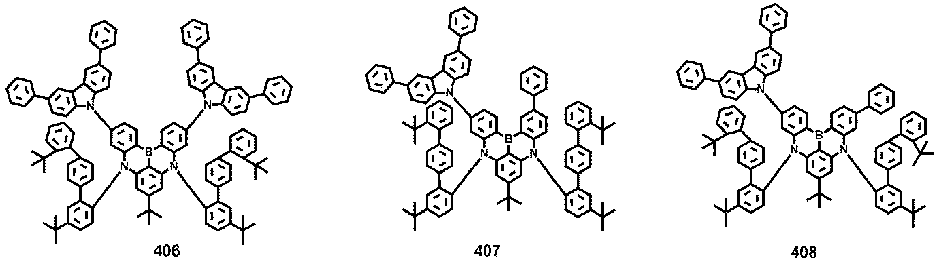

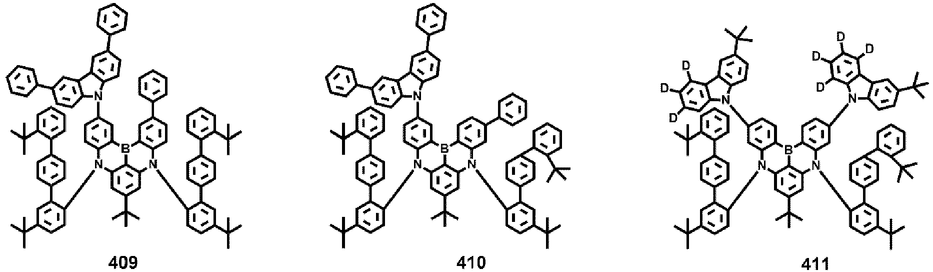

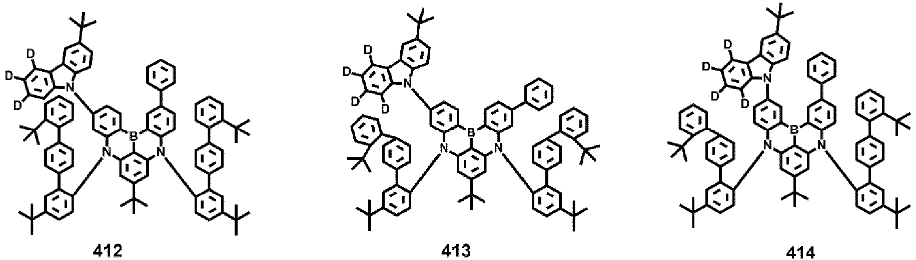

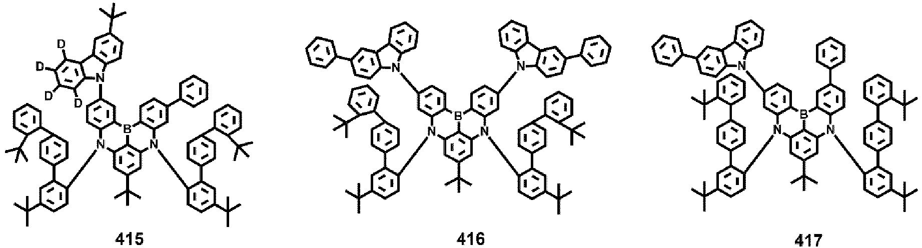

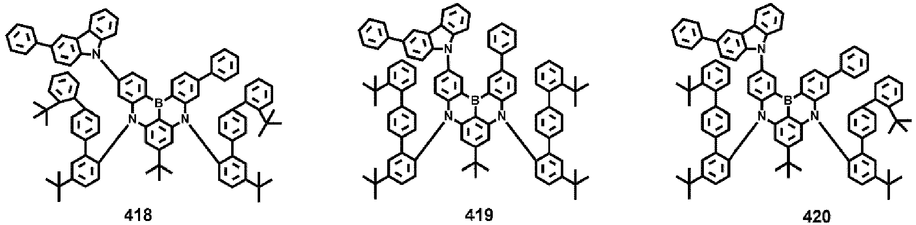

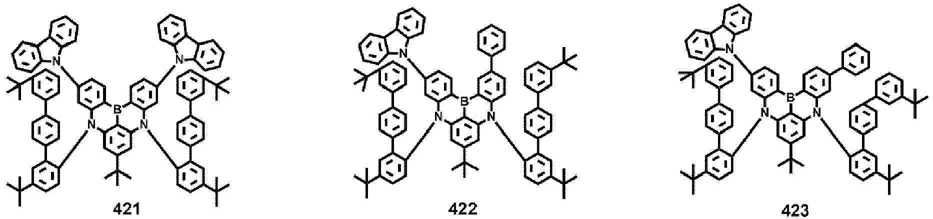

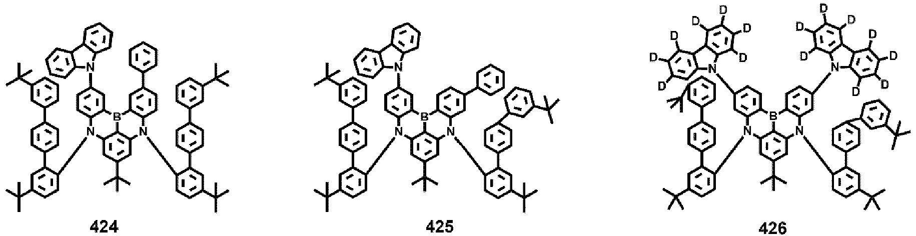

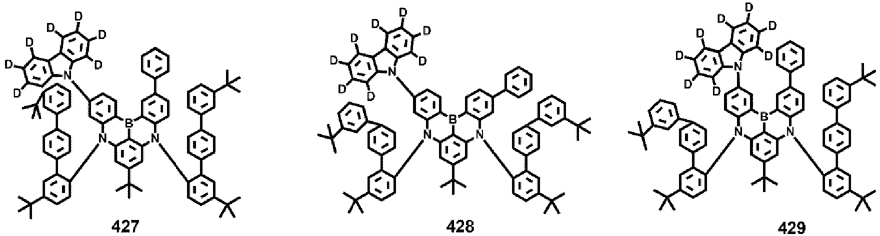

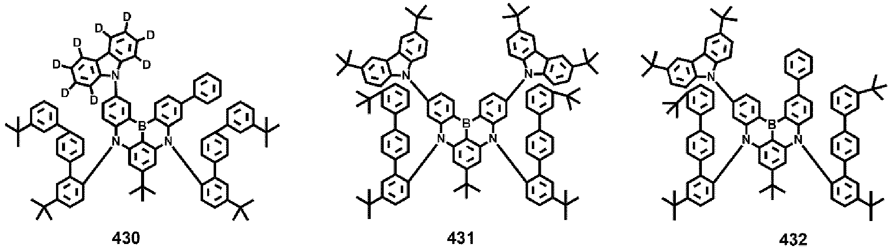

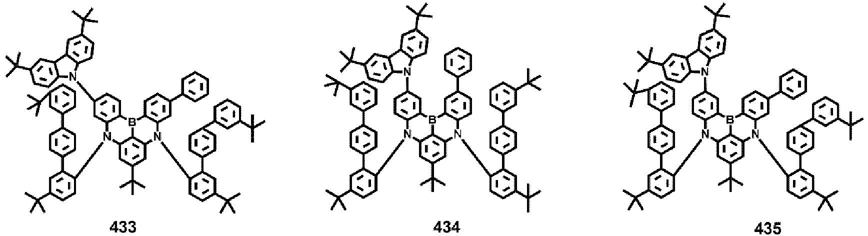

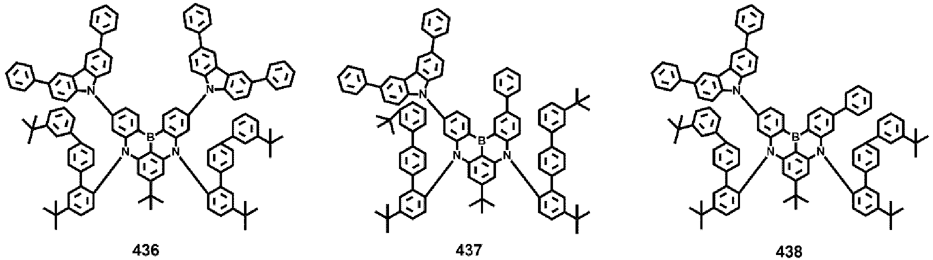

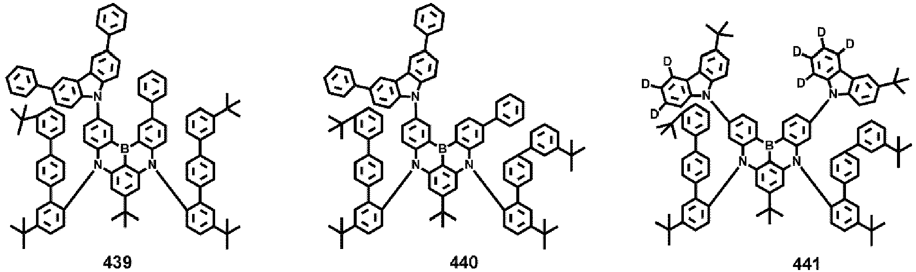

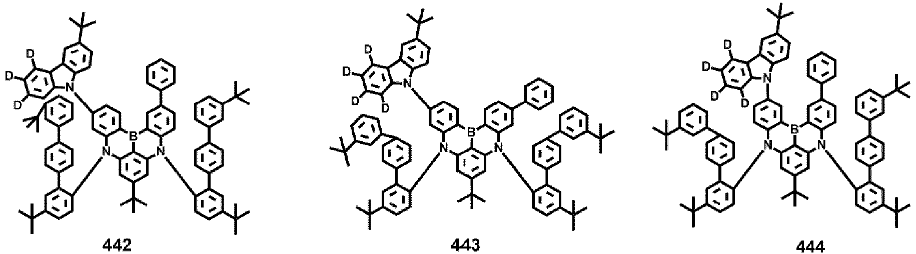

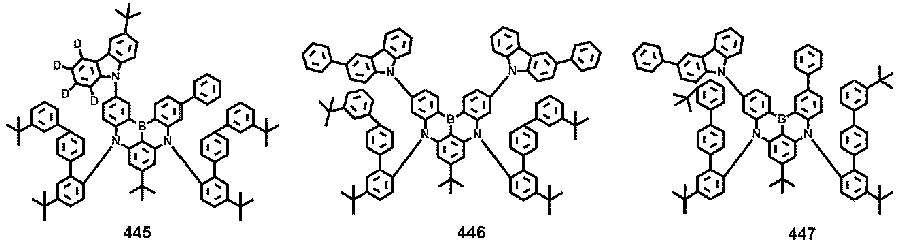

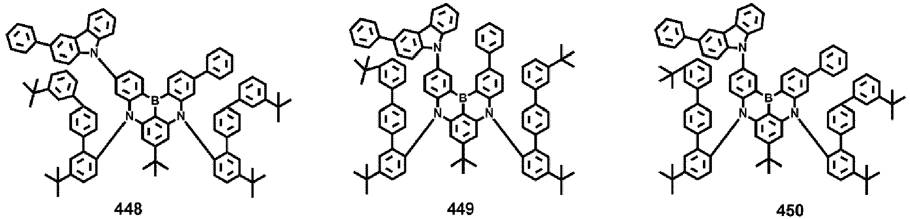

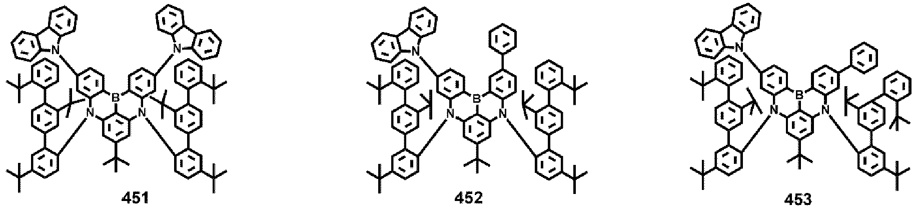

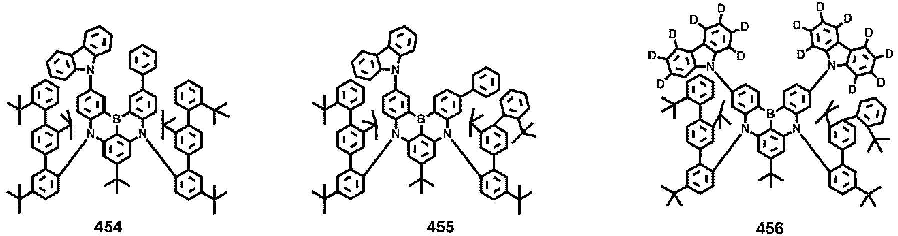

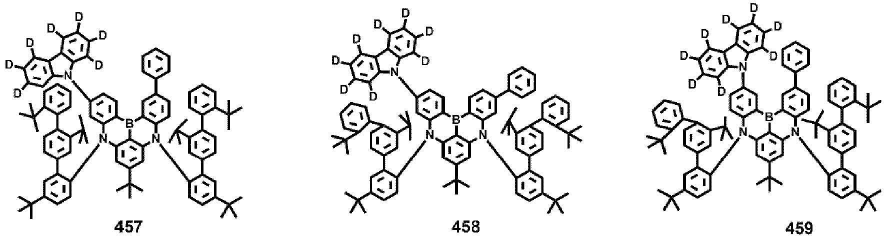

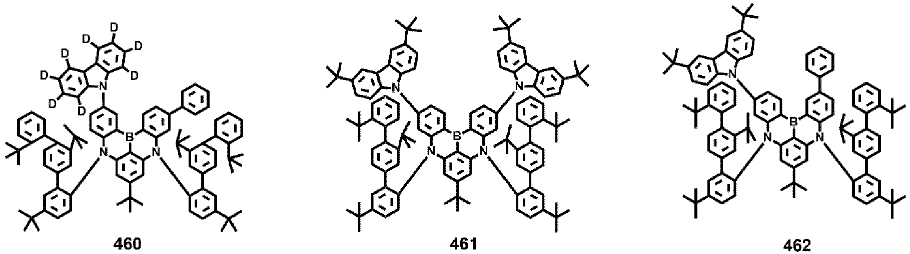

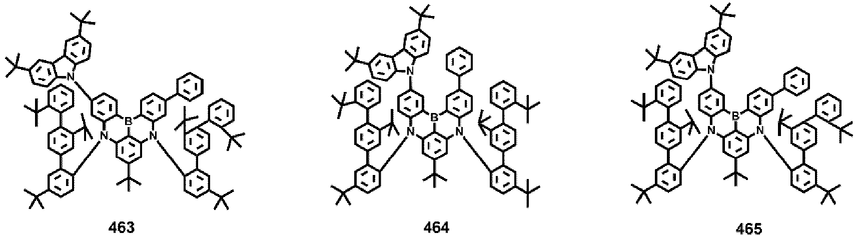

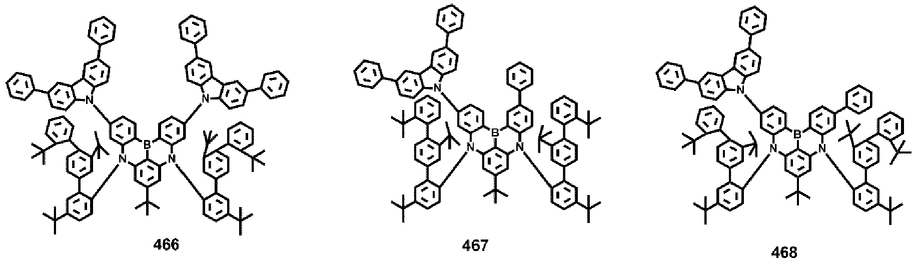

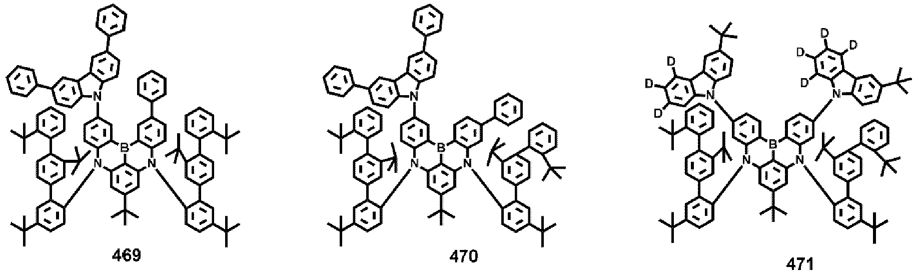

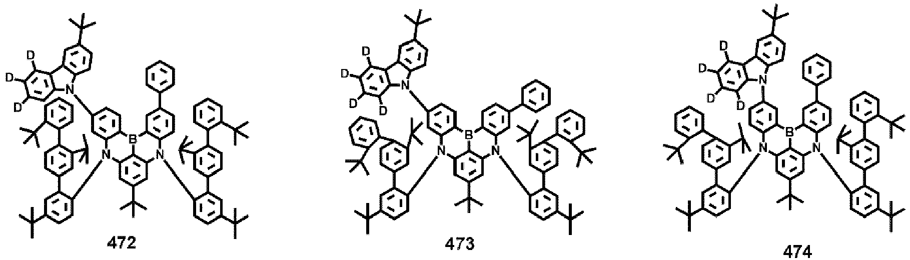

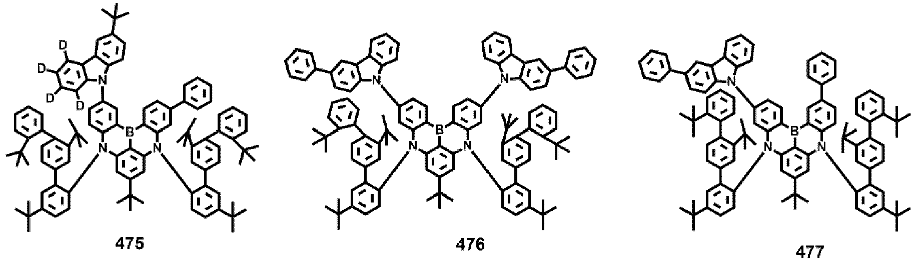

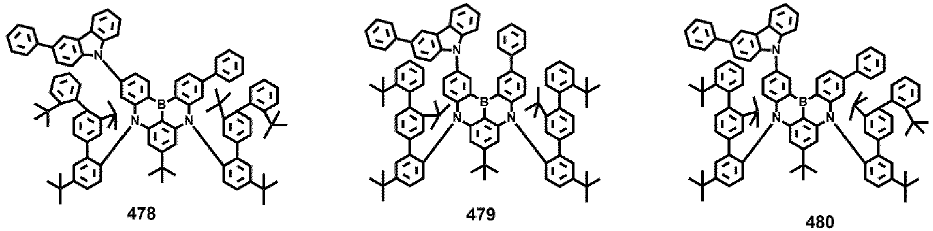

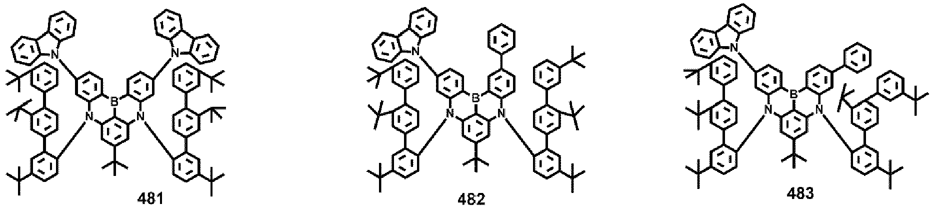

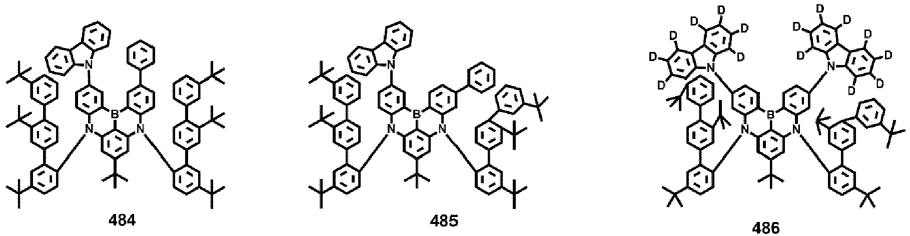

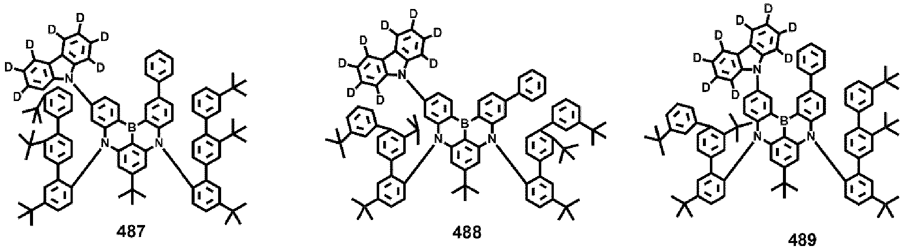

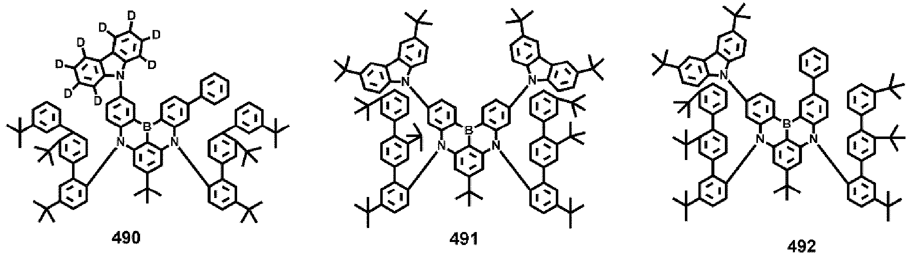

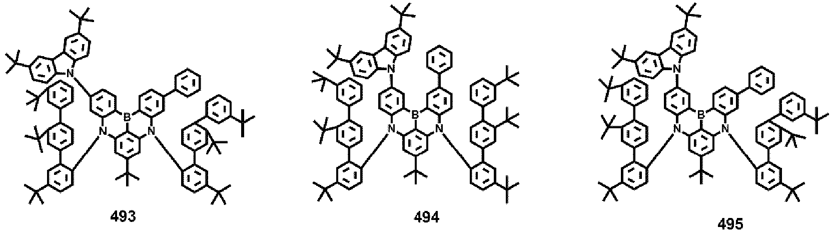

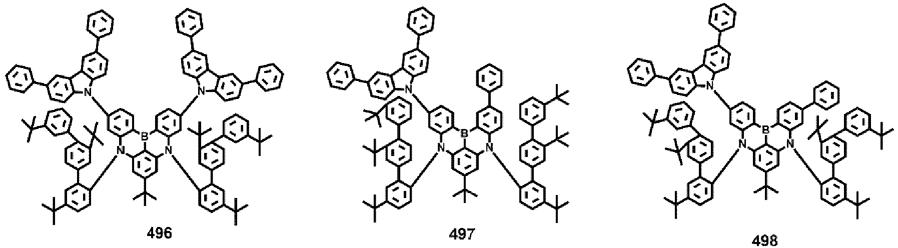

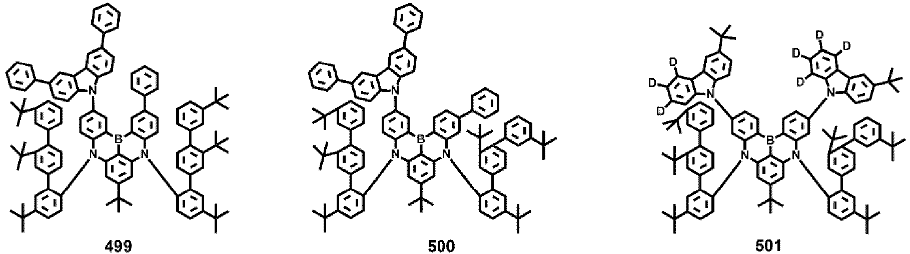

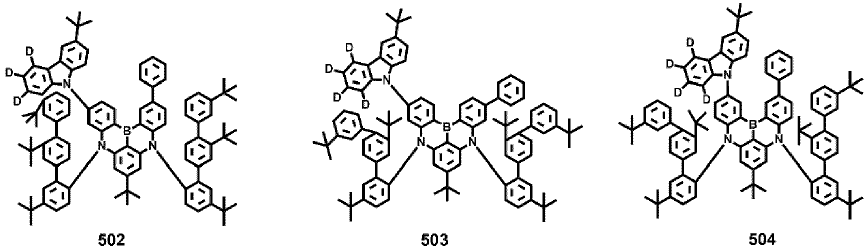

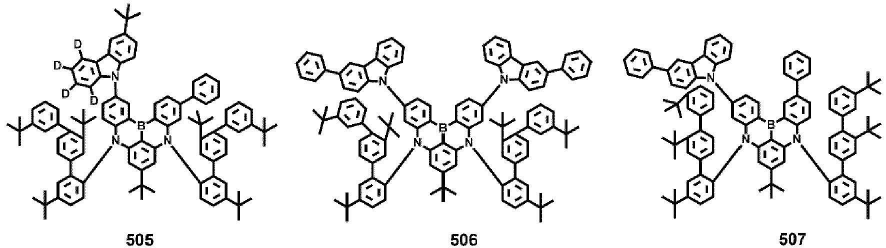

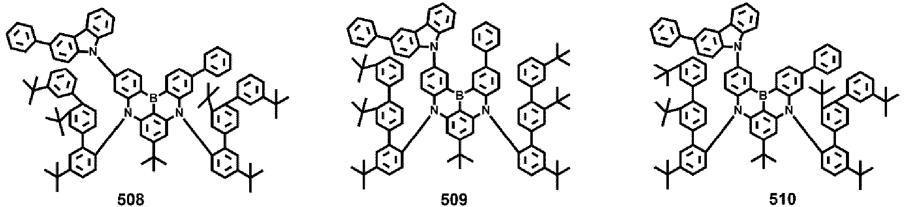

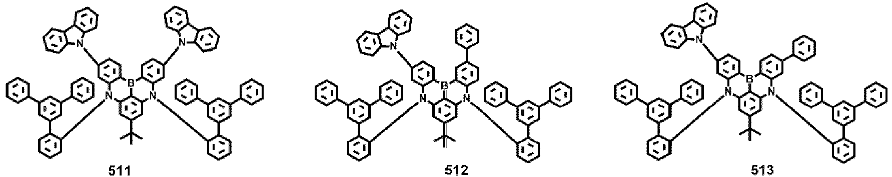

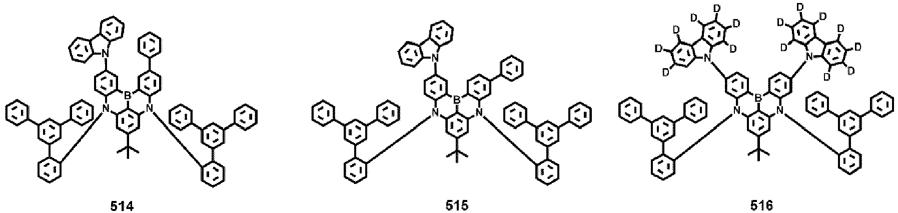

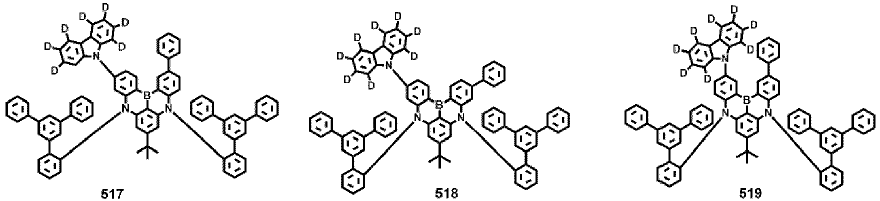

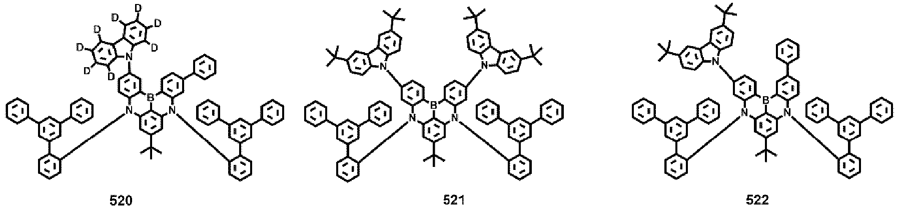

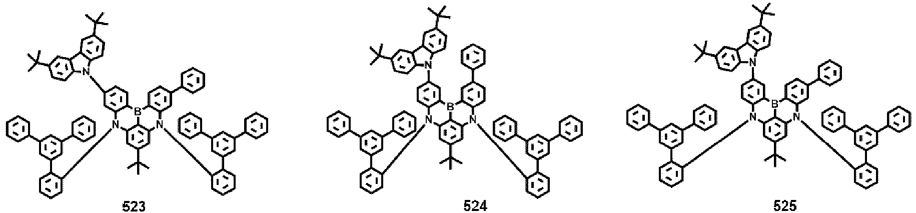

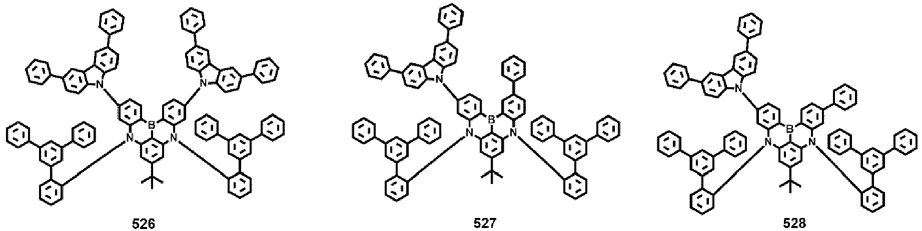

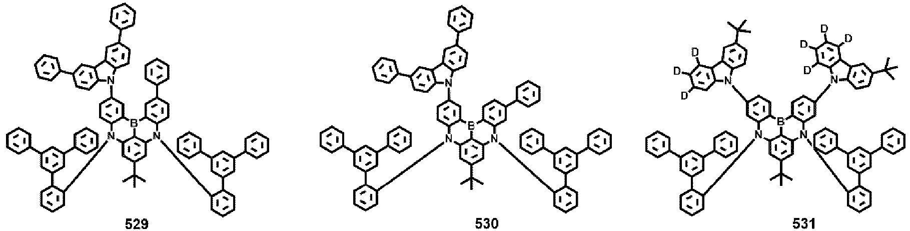

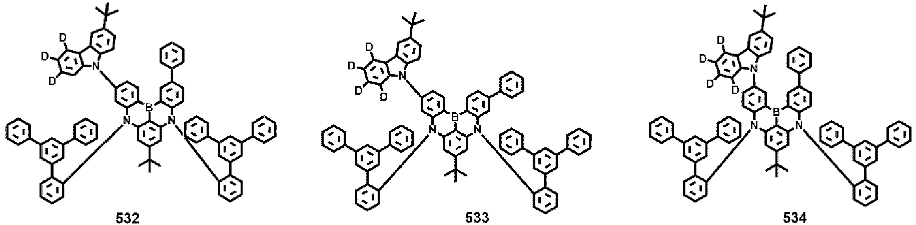

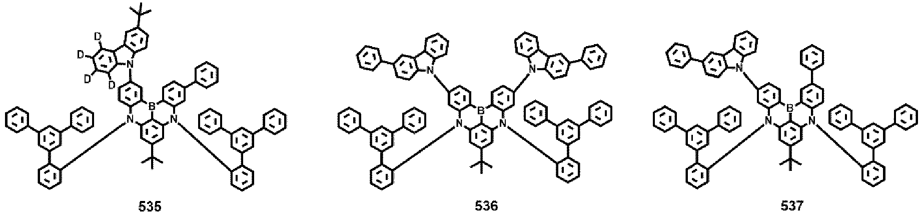

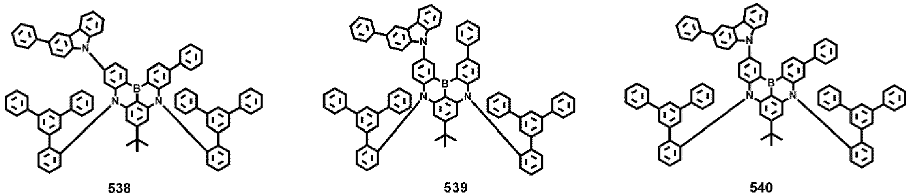

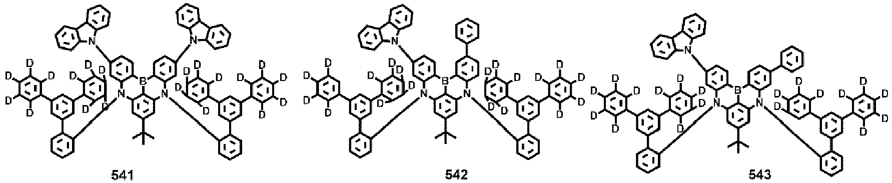

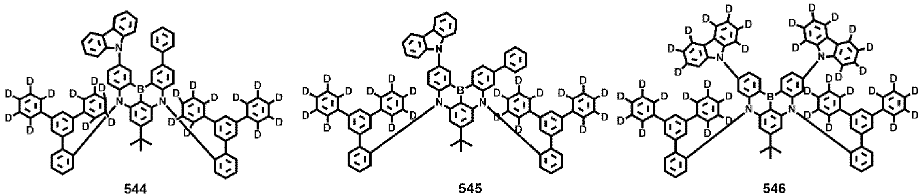

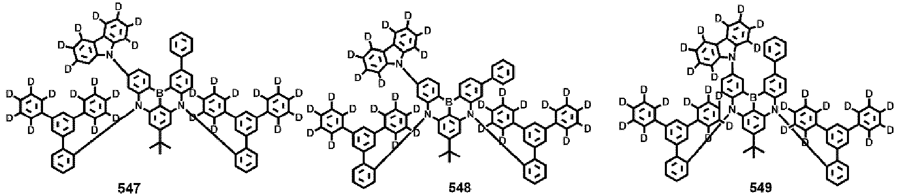

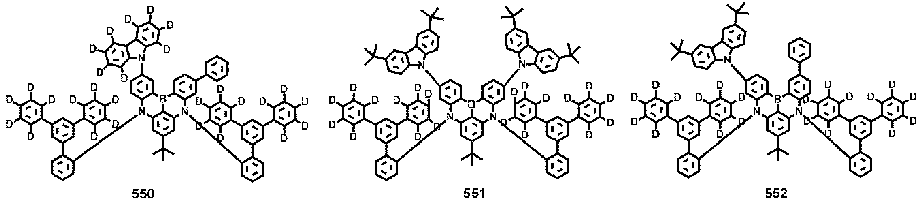

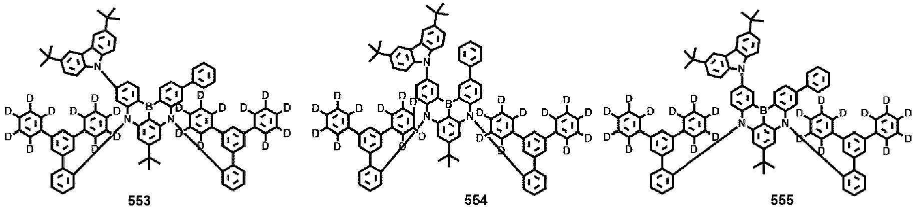

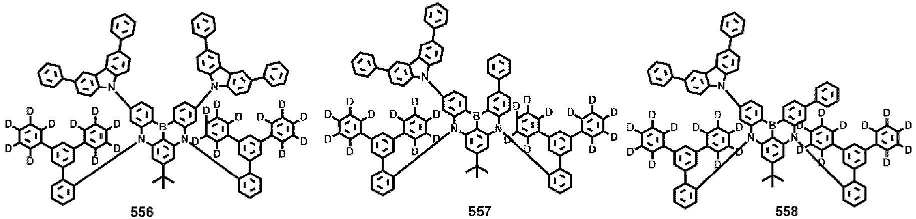

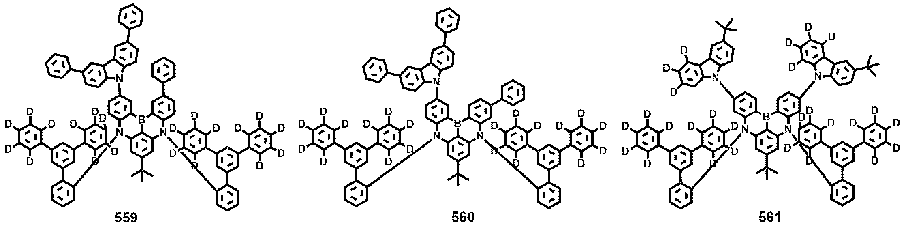

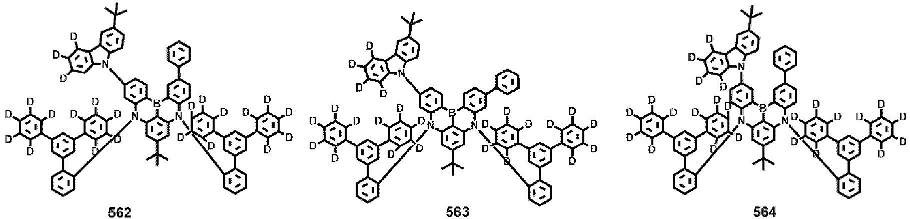

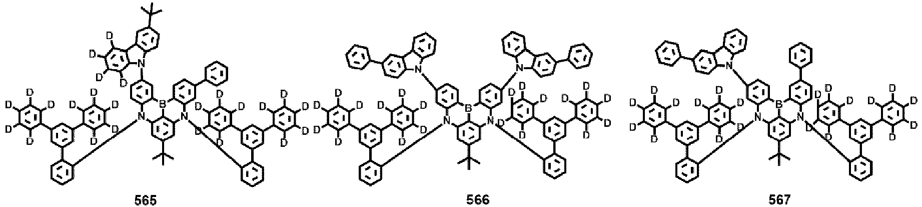

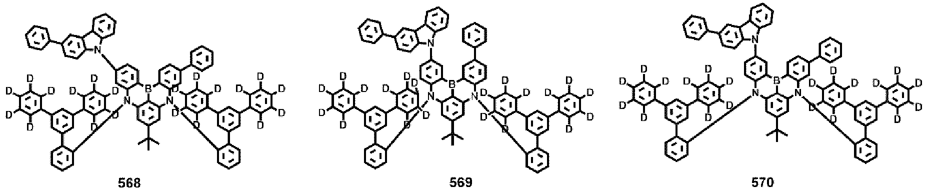

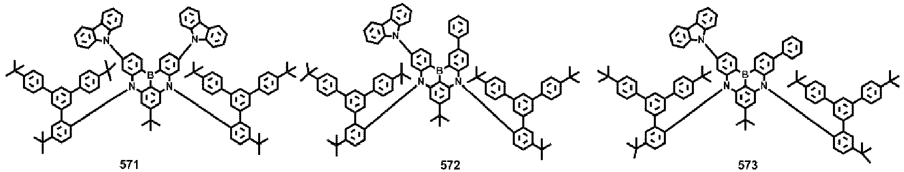

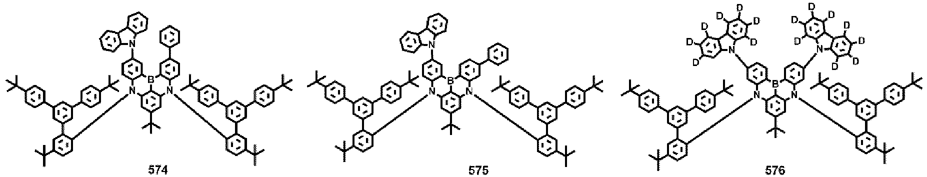

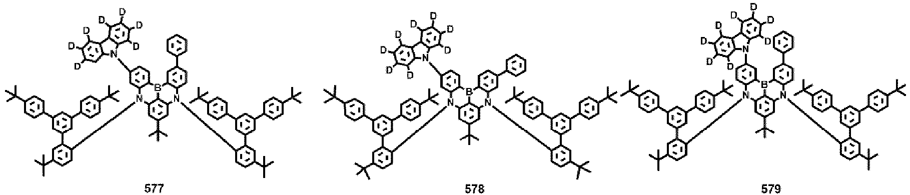

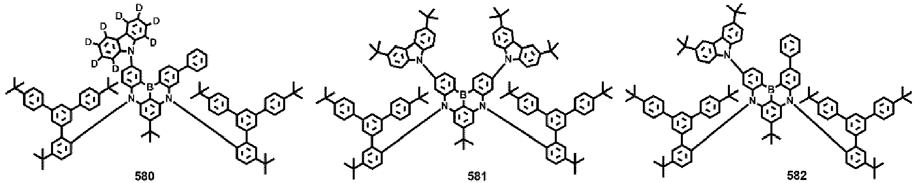

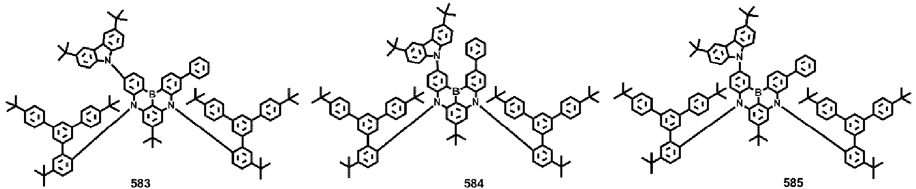

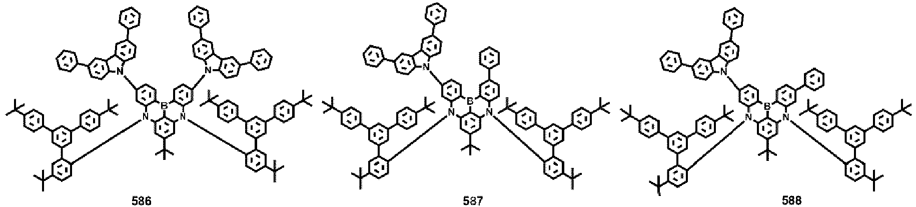

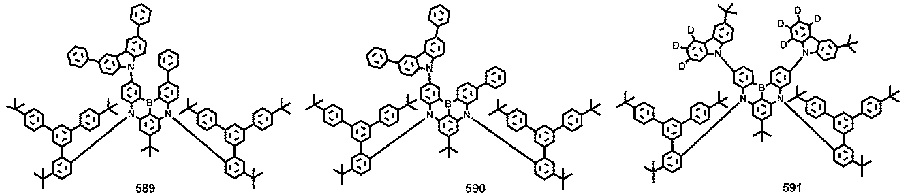

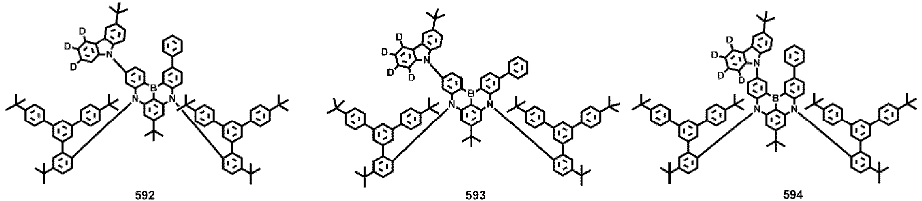

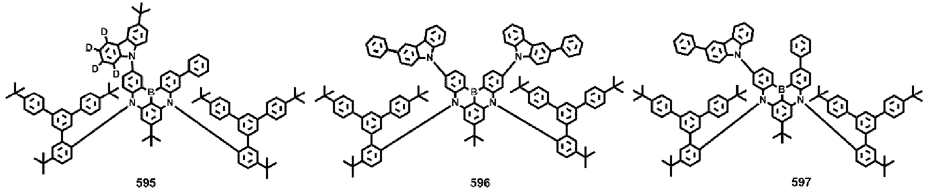

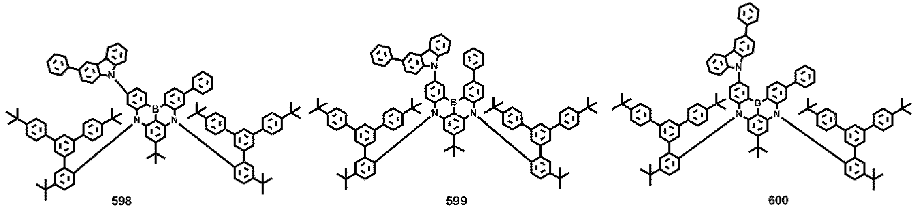

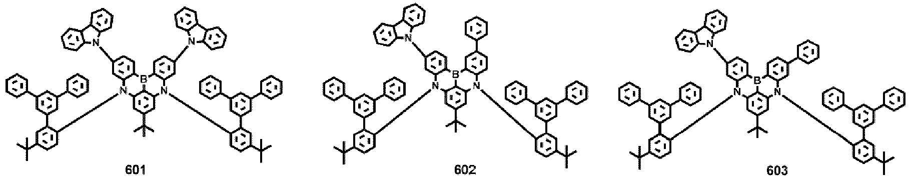

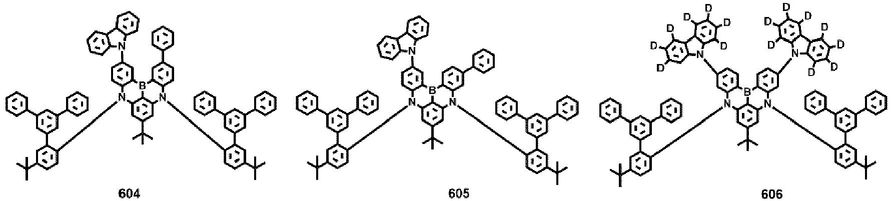

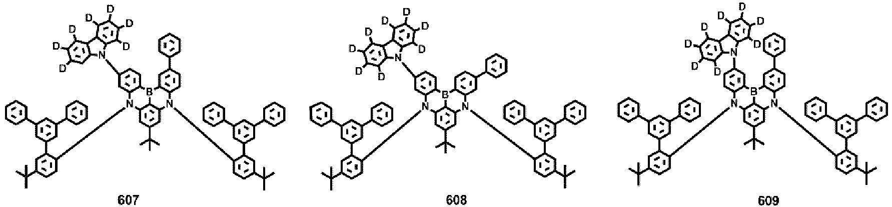

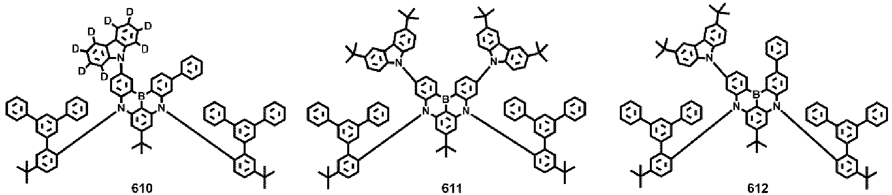

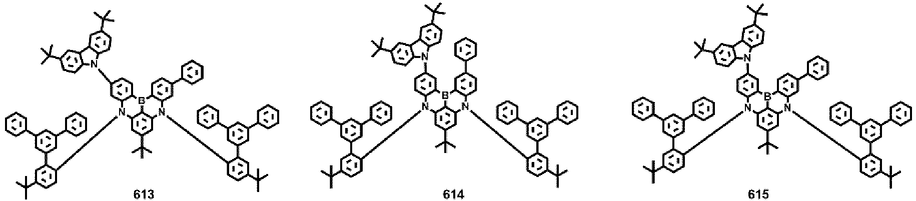

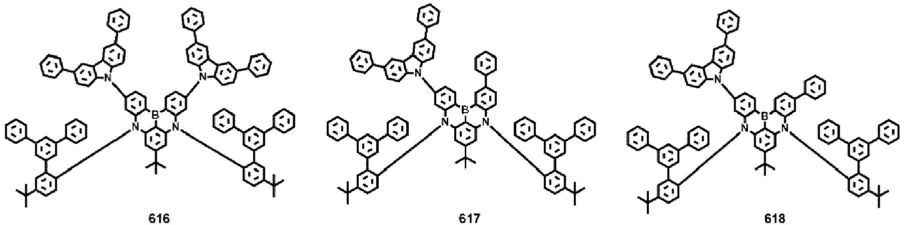

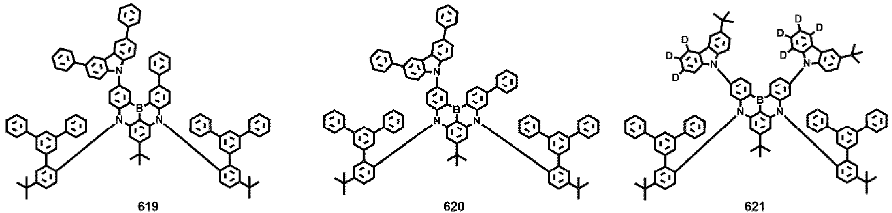

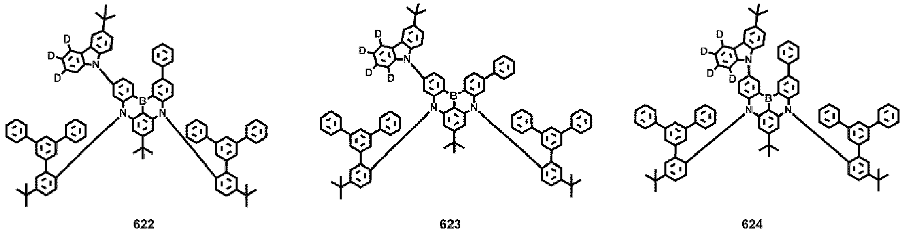

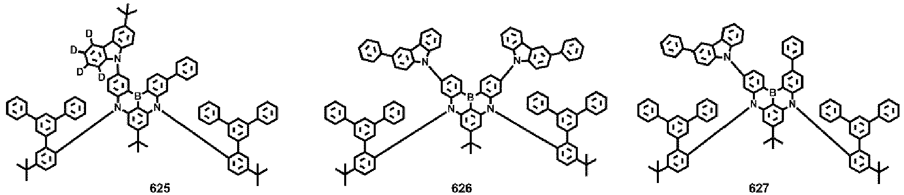

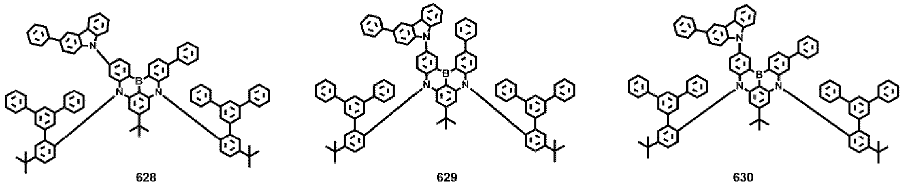

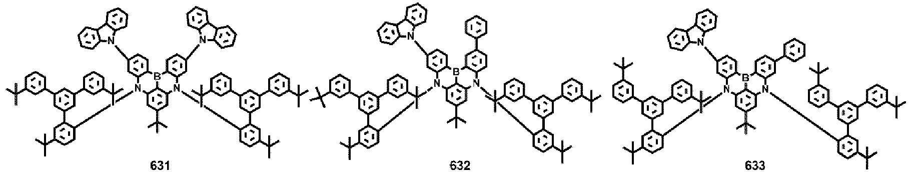

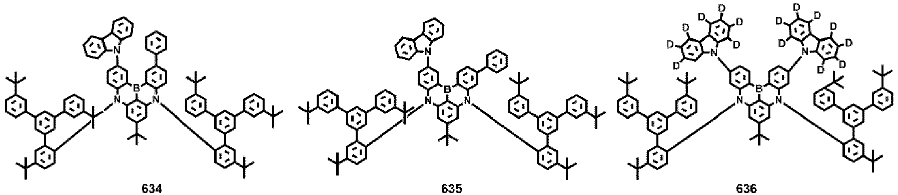

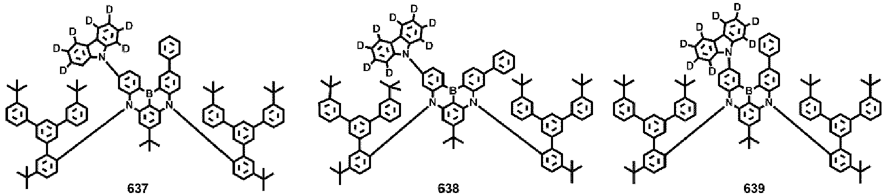

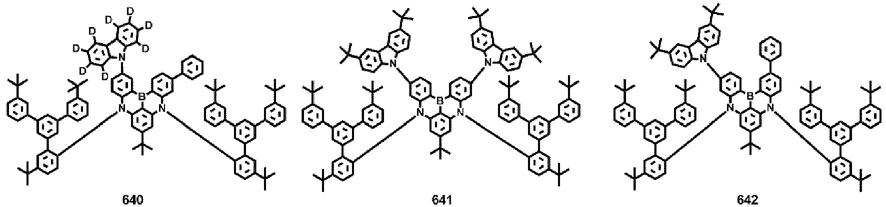

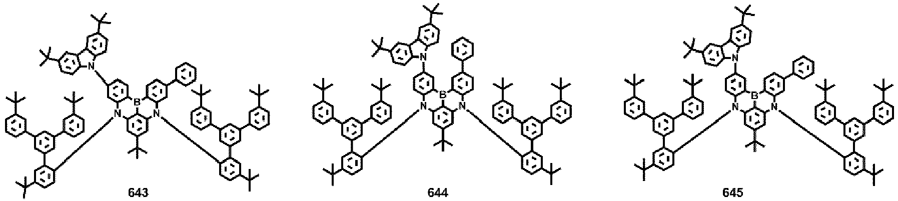

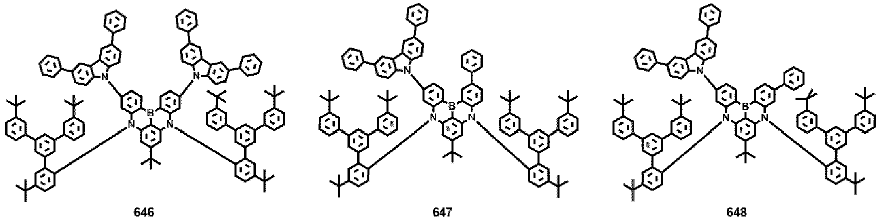

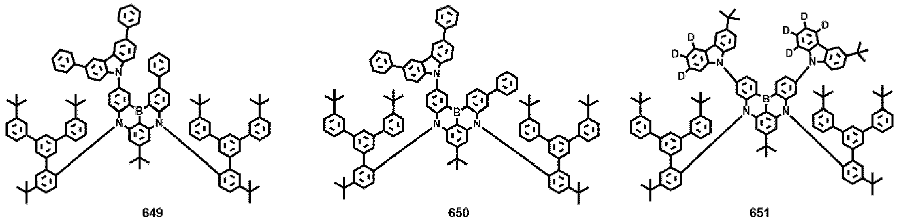

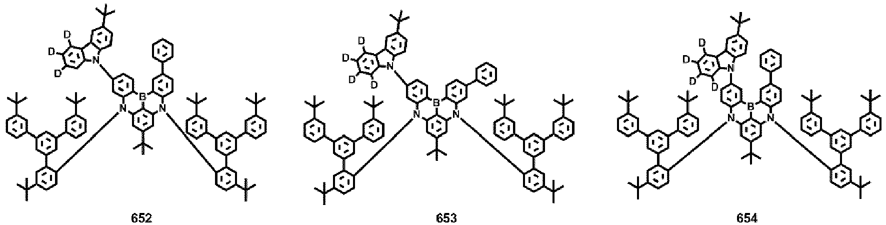

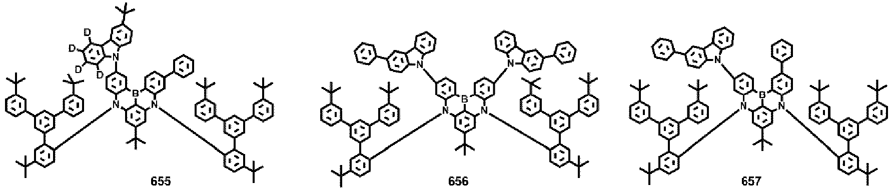

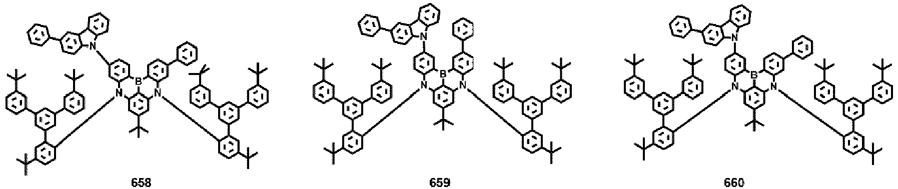

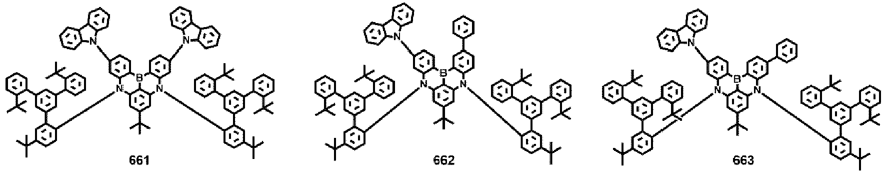

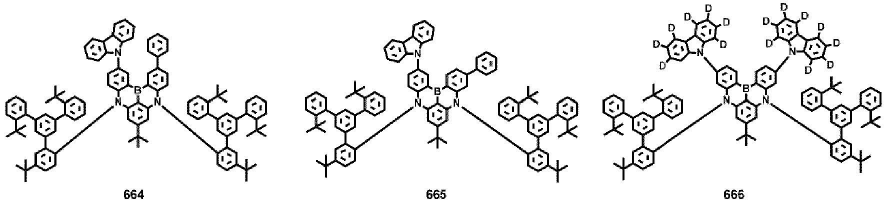

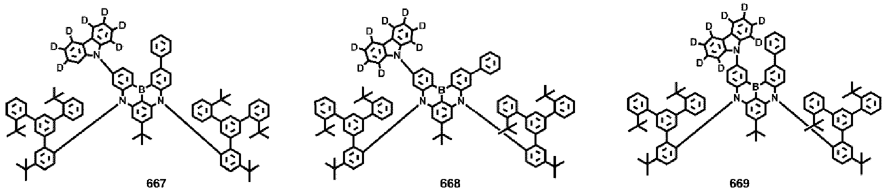

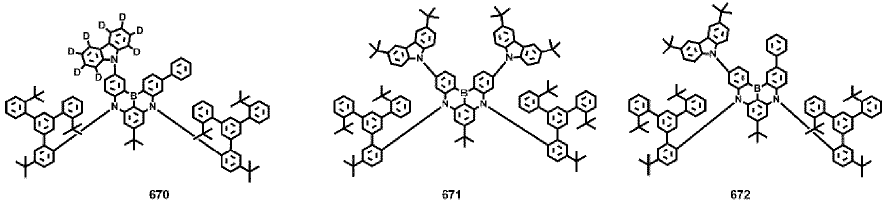

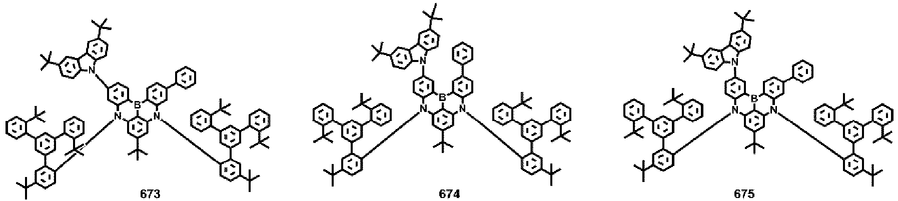

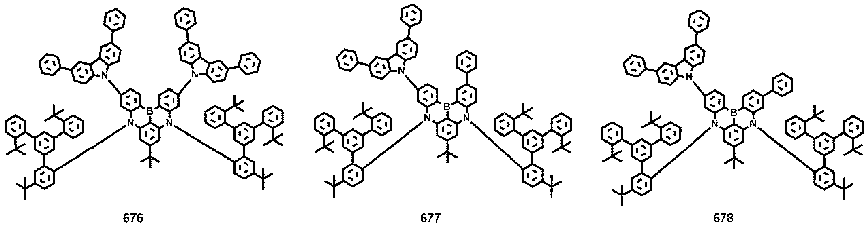

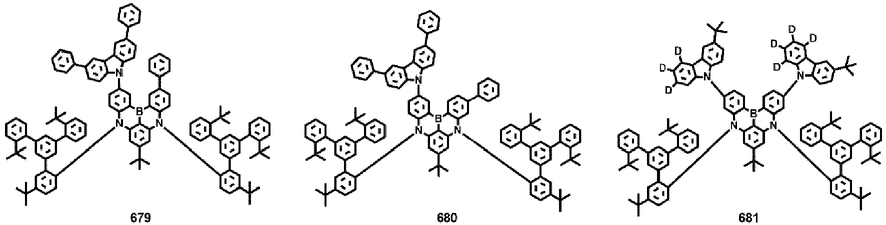

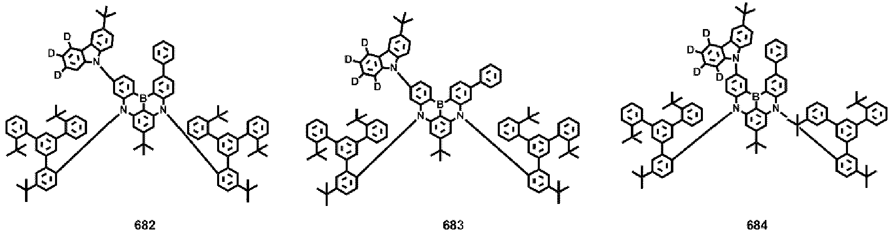

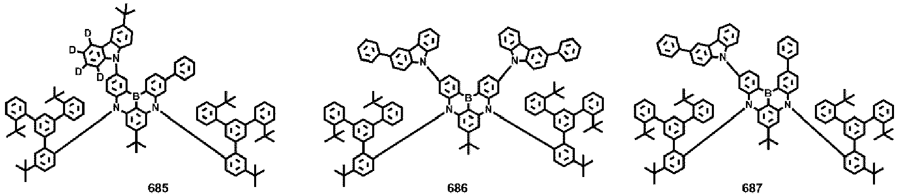

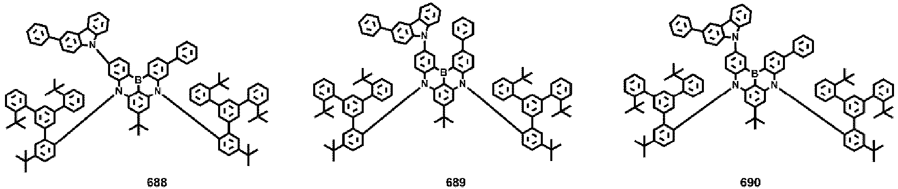

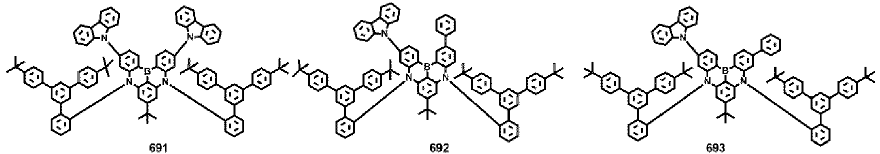

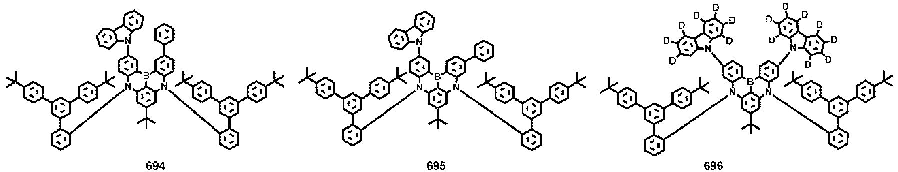

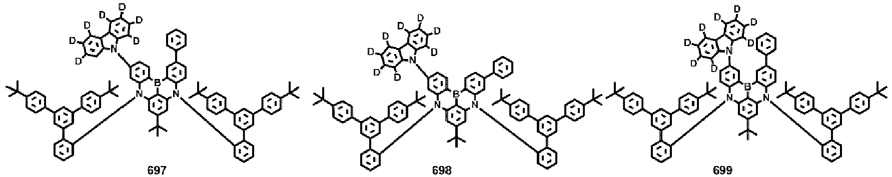

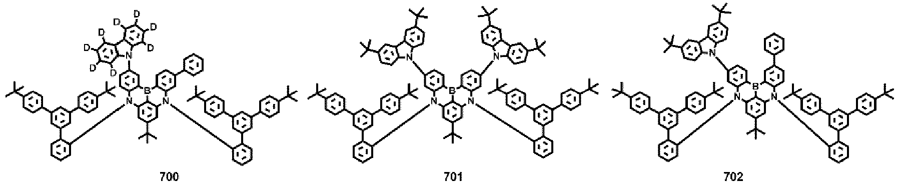

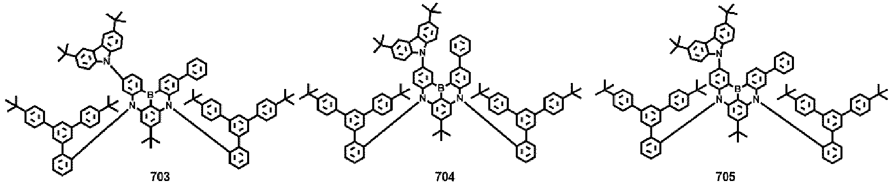

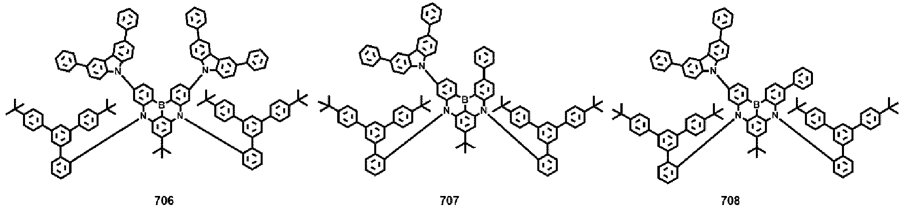

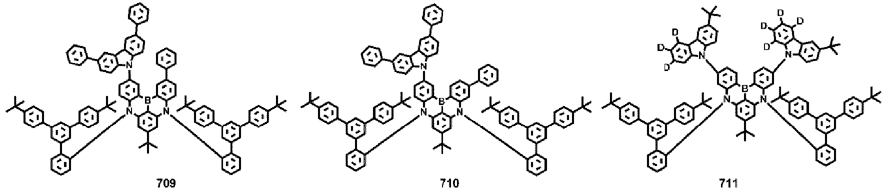

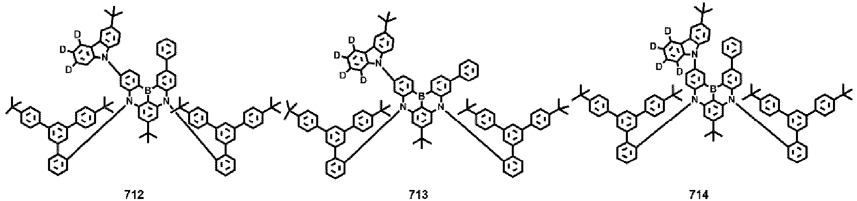

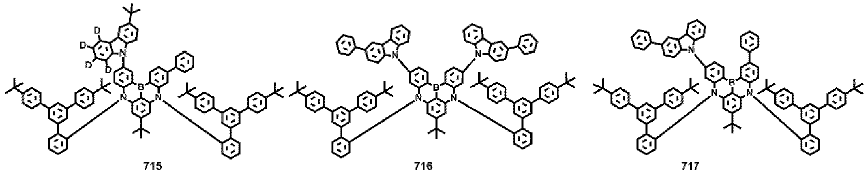

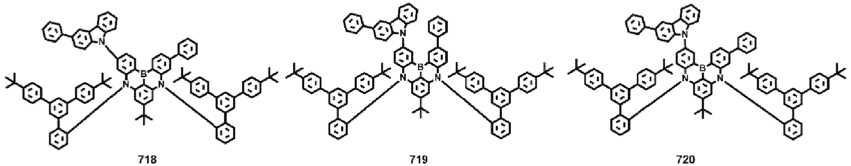

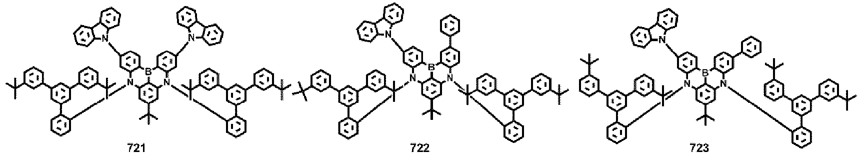

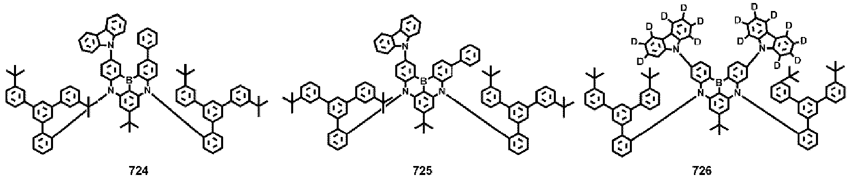

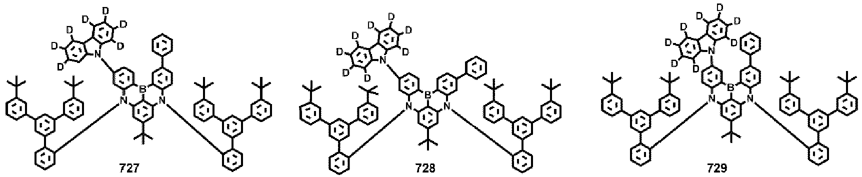

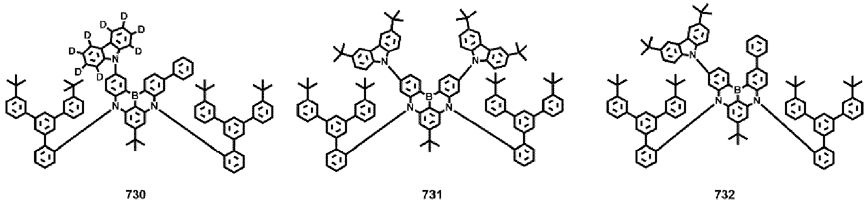

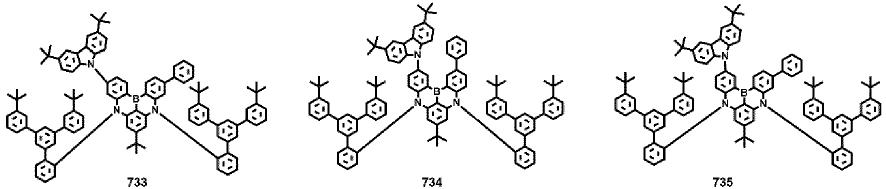

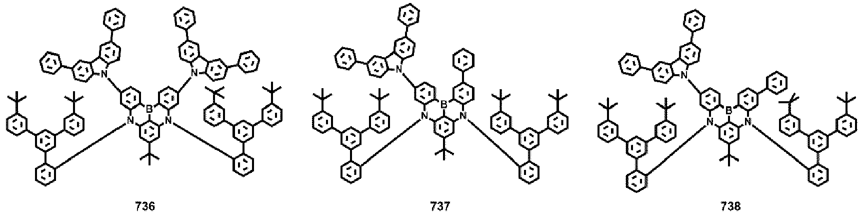

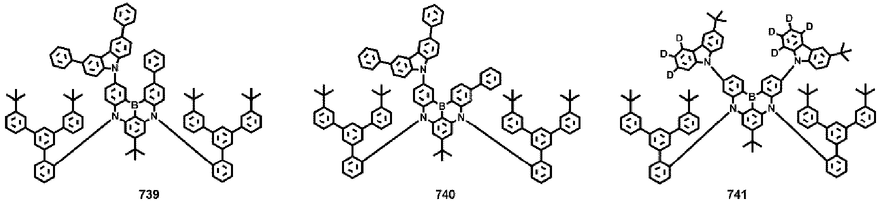

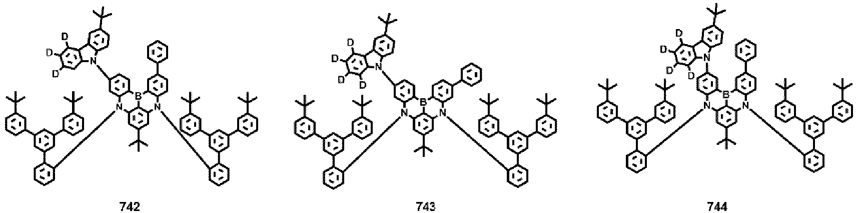

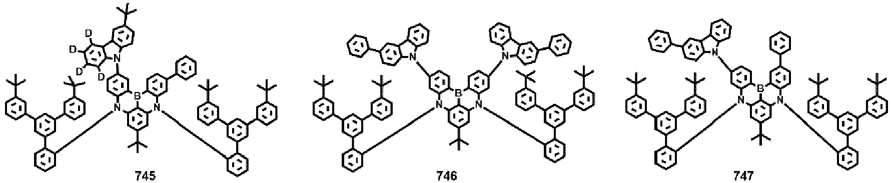

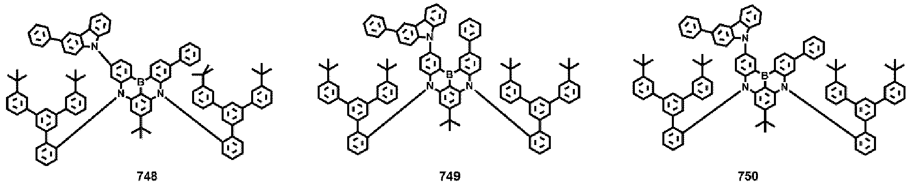

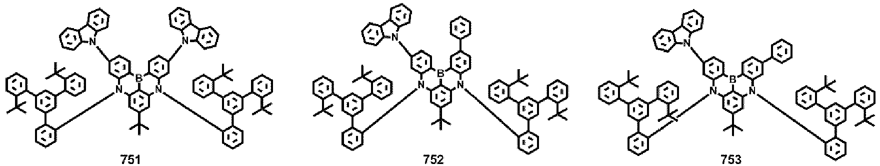

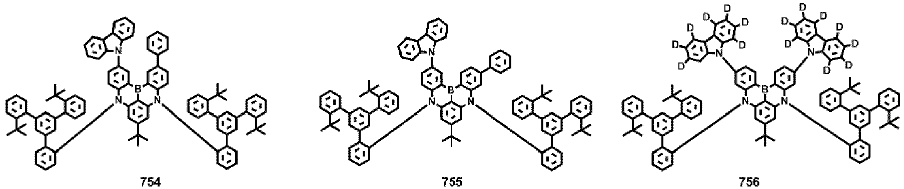

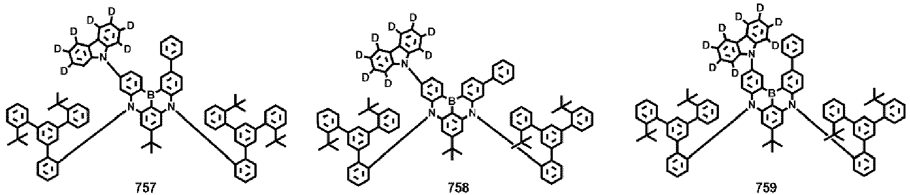

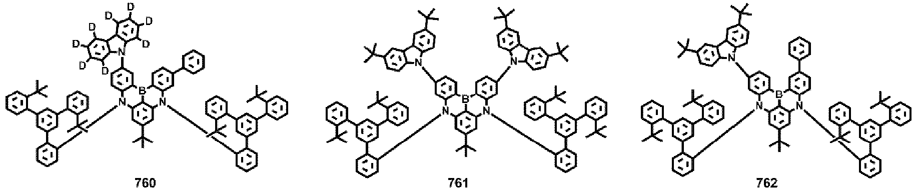

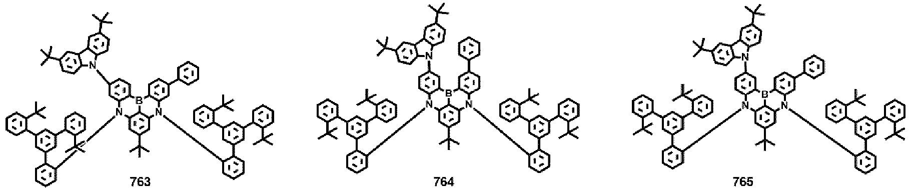

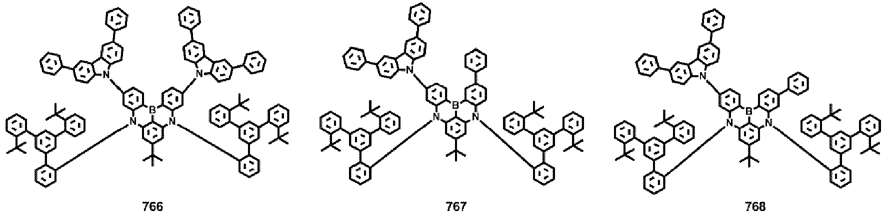

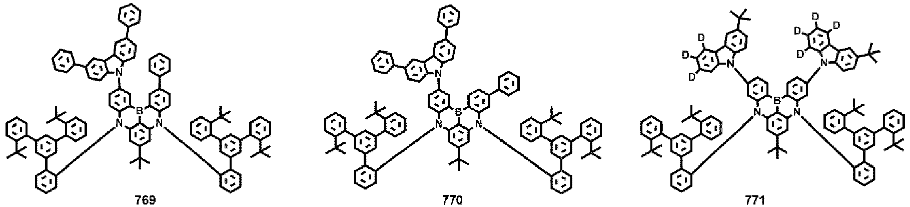

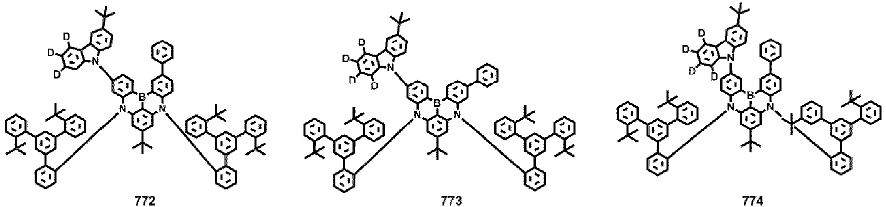

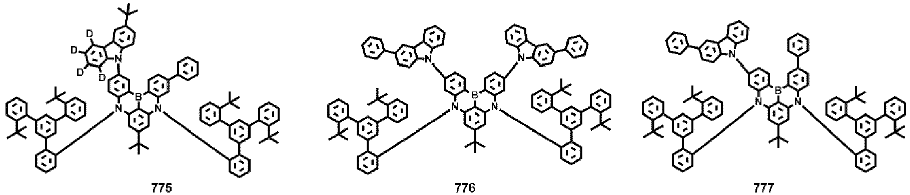

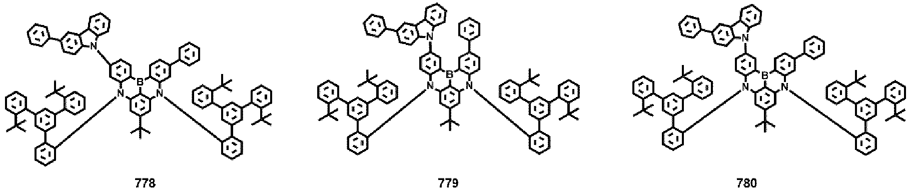

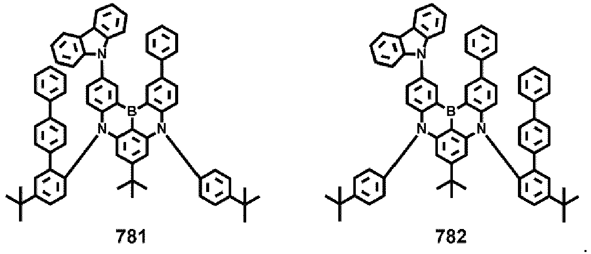

In Formula 1, Ar₀ may be the group represented by Formula 2, and at least one of Z₃ in the number of b3 may be a C₁-C₆₀ alkyl group unsubstituted or substituted with deuterium, -F, a cyano group, or any combination thereof. Also, Formula 1 may satisfy at least one of Conditions 1 and 2. Accordingly, the condensed cyclic compound represented by Formula 1 may have a relatively large absolute value of a highest occupied molecular orbital (HOMO) energy level and a relatively large absolute value of a lowest unoccupied molecular orbital (LUMO) energy level, as shown in Table 1. The HOMO and LUMO energy levels shown in Table 1 are evaluated according to Evaluation Example 1. Also, the condensed cyclic compound may emit blue light having a relatively large oscillator strength and a relatively narrow full width at half maximum (FWHM), and thus an electronic device, e.g., a light-emitting device, including the condensed cyclic compound may have improved driving voltage, improved external quantum efficiency, and improved lifespan characteristics.

**Table 1**

| Compound No. | HOMO energy level (electron volts, eV) | LUMO energy level (eV) |
|---|---|---|
| R4 | -4.576 | -1.013 |
| 361 | -4.906 | -1.379 |

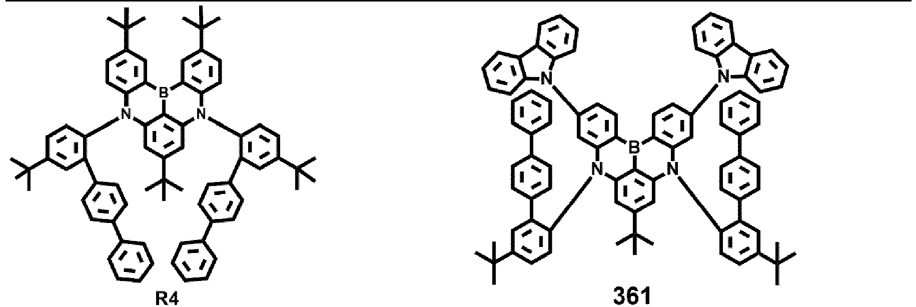

In an embodiment, the FWHM of the emission spectrum of the condensed cyclic compound may be in a range of about 5 nanometers (nm) to about 30 nm, for example, about 10 nm to about 25 nm.

In one or more embodiments, the emission peak wavelength (i.e., maximum emission wavelength or peak emission wavelength) of the emission spectrum of the condensed cyclic compound may be in a range of about 400 nm to about 500 nm, for example, about 440 nm to about 470 nm.

In one or more embodiments, the singlet (S₁) energy level of the condensed cyclic compound may be in a range of about 2.4 eV to about 3.1 eV.

In one or more embodiments, the absolute value of the difference between the singlet (S₁) energy level and the triplet (T₁) energy level of the condensed cyclic compound may be in a range of about 0 eV to about 1 eV.

In one or more embodiments, the absolute value of the **HOMO** energy level of the condensed cyclic compound may be in a range of about 4.0 eV to about 6.5 eV.

Synthesis methods for the condensed cyclic compound may be recognized by those skilled in the art with reference to Synthesis Examples to be described later.

### Light-emitting device

The condensed cyclic compound may be suitable for use as an interlayer of a light-emitting device, for example, as a material for an emission layer among interlayers. In this regard, another aspect of the disclosure provides a light-emitting device including: a first electrode; a second electrode; and an interlayer that is arranged between the first electrode and the second electrode and includes an emission layer, wherein the interlayer includes at least one condensed cyclic compound represented by Formula 1.

Such a light-emitting device is provided with an interlayer including at least one type of the aforementioned condensed cyclic compound. In this regard, the light-emitting device may be able to emit blue light having a relatively narrow FWHM and a short-wavelength shift, and may have improved driving voltage, improved external quantum efficiency, improved luminescence efficiency, and/or improved lifespan characteristics.

The condensed cyclic compound may be used between a pair of electrodes of the light-emitting device. For example, the condensed cyclic compound may be included in the emission layer. Here, the emission layer may further include a host. The amount (weight) of the host may be greater than that of the condensed cyclic compound. The emission layer may emit red light, green light, or blue light. For example, the emission layer may emit blue light.

In an embodiment, the CIEy value of light emitted from the emission layer may be in a range of about 0.040 to about 0.170, about 0.050 to about 0.170, about 0.060 to about 0.170, about 0.040 to about 0.165, about 0.050 to about 0.165, or about 0.060 to about 0.165.

In one or more embodiments, the emission peak wavelength of light emitted from the emission layer may be in a range of about 440 nm to about 470 nm, about 445 nm to about 470 nm, about 450 nm to about 470 nm, about 455 nm to about 470 nm, about 460 nm to about 470 nm, about 440 nm to about 465 nm, about 445 nm to about 465 nm, about 450 nm to about 465 nm, about 455 nm to about 465 nm, or about 460 nm to about 465 nm.

The emission layer may further include a host. A detailed description of the host may be the same as described herein.

The first electrode may be an anode, which is a hole injection electrode, and the second electrode may be a cathode, which is an electron injection electrode; or the first electrode may be a cathode, which is an electron injection electrode, and the second electrode may be an anode, which is a hole injection electrode.

For example, in the light-emitting device, the first electrode may be an anode, the second electrode may be a cathode, and the interlayer may further include a hole transport region arranged between the first electrode and the emission layer and an electron transport region arranged between the emission layer and the second electrode, wherein the hole transport region may include a hole injection layer, a hole transport layer, an electron blocking layer, an auxiliary layer, or any combination thereof, and the electron transport region may include a buffer layer, a hole blocking layer, an electron transport layer, an electron injection layer, or any combination thereof.

The term "interlayer" as used herein refers to a single layer and/or multiple layers arranged between the first electrode and the second electrode of the light-emitting device. The "interlayer" may include, in addition to an organic compound, an organometallic complex including metal or the like.

For example, the emission layer may include a first embodiment or a second embodiment:

### First embodiment

The emission layer may include at least one condensed cyclic compound represented by Formula 1, and the condensed cyclic compound may serve as an emitter, e.g., a delayed fluorescence emitter. That is, the condensed cyclic compound may be an emitter. For example, among the total luminescent components in the emission layer, luminescent components derived from the condensed cyclic compound may account for 80 % or more, 85 % or more, 90 % or more, or 95 % or more. Light emitted from the condensed cyclic compound may be blue light. The emission layer may further include a sensitizer, and the sensitizer and the condensed cyclic compound may be different from each other. The sensitizer may be an organometallic compound, a delayed fluorescence material, a prompt fluorescence material, or any combination thereof. The amount (weight) of the sensitizer may be about 0.01 parts by weight to about 10 parts by weight, per 100 parts by weight of the emission layer.

### Second embodiment

The emission layer may include at least one condensed cyclic compound, and the condensed cyclic compound may serve as a sensitizer or an auxiliary dopant. That is, the condensed cyclic compound may be a sensitizer or an auxiliary dopant. The emission layer may further include an emitter. The sensitizer or an auxiliary dopant may effectively transfer excitons from the host to the emitter. The emitter may be different from the condensed cyclic compound. The emitter may be an organometallic compound, a prompt fluorescence material, a delayed fluorescence material, or any combination thereof.

In the present specification, the emitter may be a material that receives excitons from a host, a sensitizer, and/or an auxiliary dopant and emits light through transition of the excitons to the ground state.

In the first embodiment and the second embodiment, the amount of the condensed cyclic compound may be about 0.01 parts by weight to about 40 parts by weight, about 0.1 parts by weight to about 20 parts by weight, or about 1 part by weight to about 20 parts by weight, per 100 parts by weight of the emission layer.

In the first embodiment and the second embodiment, the organometallic compound may include a transition metal and n ligand(s) bonded to the transition metal, wherein n may be an integer from 1 to 4.

In an embodiment, the transition metal in the organometallic compound may be platinum (Pt) or palladium (Pd), n may be 1, and the ligand may be a tetradentate ligand. The tetradentate ligand may include, for example, a carbene moiety bonded to the transition metal.

In one or more embodiments, the organometallic compound may include a transition metal and a tetradentate ligand bonded to the transition metal, wherein the transition metal may be Pt or Pd, and the tetradentate ligand may include a carbene moiety bonded to the transition metal.

In one or more embodiments, in the organometallic compound, the transition metal may be iridium (Ir) or osmium (Os), and n may be 3, wherein at least one ligand among the n ligand(s) may be: a bidentate ligand including -F, a cyano group, or a combination thereof; or a bidentate ligand including a carbene moiety bonded to the transition metal. For example, the bidentate ligand may further include an imidazole group or a triazole group.

In one or more embodiments, the organometallic compound may be an organometallic compound represented by Formula 30 and/or an organometallic compound represented by Formula 5. A detailed description of Formulae 30 and 5 will be provided below.

In the first embodiment and the second embodiment, the delayed fluorescence material may be, for example, a thermally activated delayed fluorescence material. In one or more embodiments, the delayed fluorescence material may be a multiple resonance thermally activated delayed fluorescence material.

The multiple resonance thermally activated delayed fluorescence material may be a polycyclic compound that i) does not include a transition metal, and ii) includes a core in which two or more C₃-C₆₀ cyclic groups are condensed with each other. Here, two C₃-C₆₀ cyclic groups of the core may be condensed with each other while sharing boron (B) or nitrogen (N).

In an embodiment, the delayed fluorescence material may be a polycyclic compound represented by Formula 4. A detailed description of Formula 4 will be provided below.

In the first embodiment and the second embodiment, the prompt fluorescence material may be an amino group-containing compound, a styryl group-containing compound, or the like. For example, the prompt fluorescence material may include a naphthalene group, a fluorene group, a spirobifluorene group, a benzofluorene group, a dibenzofluorene group, a phenanthrene group, an anthracene group, a fluoranthene group, a triphenylene group, a pyrene group, a chrysene group, a naphthacene group (i.e., a tetracene group), a picene group, a perylene group, a pentaphene group, an indenoanthracene group, a group represented by one of Formulae 501-1 to 501-21, or any combination thereof:

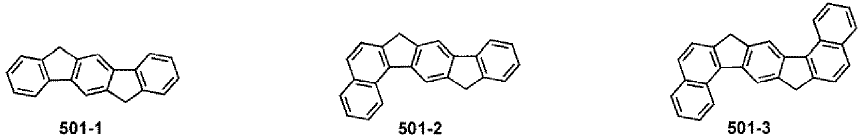

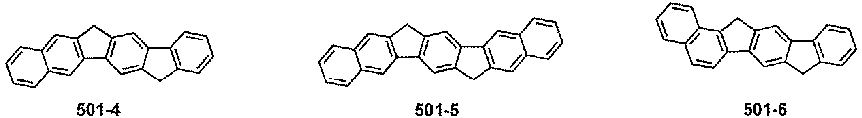

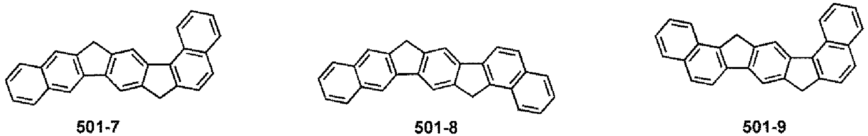

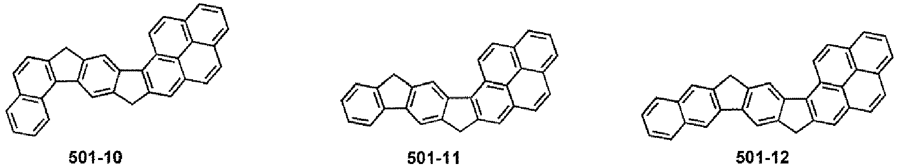

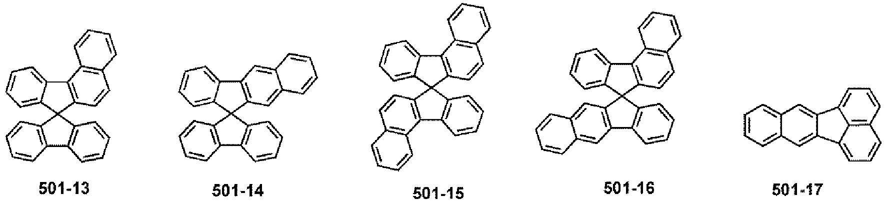

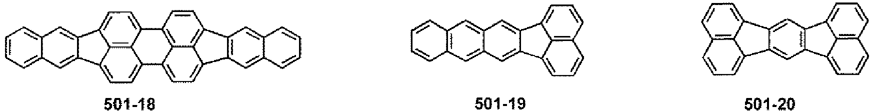

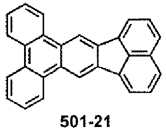

In one or more embodiments, the prompt fluorescence material may include a compound represented by Formula 501A or 501B: 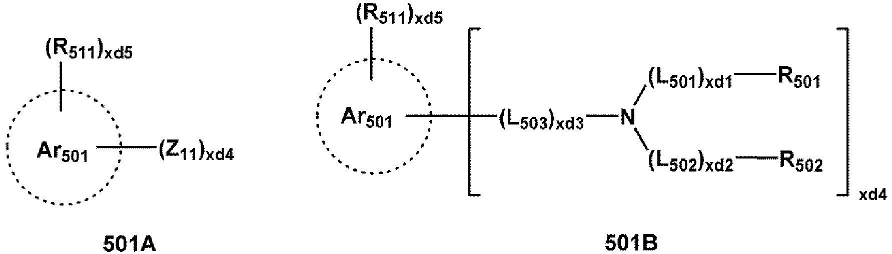 wherein, in Formulae 501A and 501B,
Ar₅₀₁ may be a naphthalene group, a fluorene group, a spirobifluorene group, a benzofluorene group, a dibenzofluorene group, a phenanthrene group, an anthracene group, a fluoranthene group, a triphenylene group, a pyrene group, a chrysene group, a naphthacene group, a picene group, a perylene group, a pentaphene group, an indenoanthracene group, a bisanthracene group, or a group represented by one of Formulae 501-1 to 501-21,
R₅₁₁ may be hydrogen, deuterium, -F, -Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, or -Si(Q₅₀₁)(Q₅₀₂)(Q₅₀₃),
xd5 may be an integer from 0 to 10,
L₅₀₁ to L₅₀₃ may each independently be:
   a single bond; and
   a C₃-C₁₀ cycloalkylene group, a C₁-C₁₀ heterocycloalkylene group, a C₃-C₁₀ cycloalkenylene group, a C₂-C₁₀ heterocycloalkenylene group, a C₆-C₆₀ arylene group, a C₁-C₆₀ heteroarylene group, a divalent non-aromatic condensed polycyclic group, or a divalent non-aromatic condensed heteropolycyclic group, each unsubstituted or substituted with deuterium, -F, - Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₅₀₁)(Q₅₀₂)(Q₅₀₃), or any combination thereof,
   xd1 to xd3 may each independently be 1, 2, or 3,
   R₅₀₁ and R₅₀₂ may each independently be a phenyl group, a naphthyl group, a fluorenyl group, a spiro-bifluorenyl group, a benzofluorenyl group, a dibenzofluorenyl group, a phenanthrenyl group, an anthracenyl group, a pyrenyl group, a chrysenyl group, a pyridinyl group, a pyrazinyl group, a pyrimidinyl group, a pyridazinyl group, a quinolinyl group, an isoquinolinyl group, a quinoxalinyl group, a quinazolinyl group, a carbazole group, a triazinyl group, a dibenzofuranyl group, a dibenzothiophenyl group, or a dibenzosilolyl group, each unsubstituted or substituted with deuterium, -F, -Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₅₀₁)(Q₅₀₂)(Q₅₀₃), or any combination thereof,
   Z₁₁ may be a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with deuterium, -F, - Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₅₀₁)(Q₅₀₂)(Q₅₀₃), or any combination thereof,
   xd4 may be 1, 2, 3, 4, 5, or 6, and
   Q₅₀₁ to Q₅₀₃ may each independently be hydrogen, a C₁-C₆₀ alkyl group, a C₁-C₆₀ alkoxy group, a C₆-C₆₀ aryl group, a C₁-C₆₀ heteroaryl group, a monovalent non-aromatic condensed polycyclic group, or a monovalent non-aromatic condensed heteropolycyclic group.

In one or more embodiments, the prompt fluorescence material may include a compound represented by Formula 501A or 501B, wherein xd4 in Formula 501A may be 1, 2, 3, 4, 5, or 6, and xd4 in Formula 501B may be 2, 3, or 4.

### Description of host

The emission layer may include m1 host(s) which may include a hole transport compound, an electron transport compound, a bipolar compound, or any combination thereof. m1 may be an integer from 1 to 3. The host may not include a transition metal.

For example, in the emission layer, m1 may be 2, and two hosts in the emission layer may each include a hole transport compound and an electron transport compound, wherein the hole transport compound and the electron transport compound may be different from each other.

In an embodiment, the hole transport compound may include at least one π electron-rich C₃-C₆₀ cyclic group and may not include an electron transport group. Examples of the electron transport group may include a cyano group, a fluoro group (-F), a π-electron deficient nitrogen-containing cyclic group, a phosphine oxide group, a sulfoxide group, and the like.

The term "π-electron deficient nitrogen-containing cyclic group" as used herein refers to a C₁-C₆₀ heterocyclic group including at least one *-N=*' moiety as a ring-forming moiety. Examples of the π-electron deficient nitrogen-containing cyclic group may include a triazine group, an imidazole group, and the like.

The term "π electron-rich C₃-C₆₀ cyclic group" as used herein refers to a C₃-C₆₀ cyclic group not including *-N=*' moiety as a ring-forming moiety. Examples of the π electron-rich C₃-C₆₀ cyclic group may include a benzene group, a naphthalene group, a triphenylene group, a dibenzofuran group, a dibenzothiophene group, a carbazole group, an indolodibenzofuran group, an indolodibenzothiophene group, an indolocarbazole group, a naphthobenzofuran group, a naphthobenzothiophene group, a benzocarbazole group, a phenanthrenobenzofuran group, a phenanthrenobenzothiophene group, a naphthocarbazole group, a dinaphthofuran group, a dinaphthothiophene group, a dibenzocarbazole group, and the like.

For example, the hole transport compound may include two or more carbazole groups.

In one or more embodiments, the electron transport compound may be a compound including at least one electron transport group. Examples of the electron transport group may include a cyano group, a fluoro group, a π electron deficient nitrogen-containing C₁-C₆₀ cyclic group, a phosphine oxide group, a sulfoxide group, or any combination thereof. In an embodiment, the electron transport compound may include a triazine group.

For example, the electron transport compound may include at least one electron transport group (e.g., a triazine group) and at least one π electron-rich C₃-C₆₀ cyclic group (e.g., a benzene group, a naphthalene group, a triphenylene group, a dibenzofuran group, a dibenzothiophene group, a carbazole group, an indolodibenzofuran group, an indolodibenzothiophene group, an indolocarbazole group, a naphthobenzofuran group, a naphthobenzothiophene group, a benzocarbazole group, a phenanthrenobenzofuran group, a phenanthrenobenzothiophene group, a naphthocarbazole group, a dinaphthofuran group, a dinaphthothiophene group, a dibenzocarbazole group, or any combination thereof).

In an embodiment, the hole transport compound may be a compound represented by Formula 6: 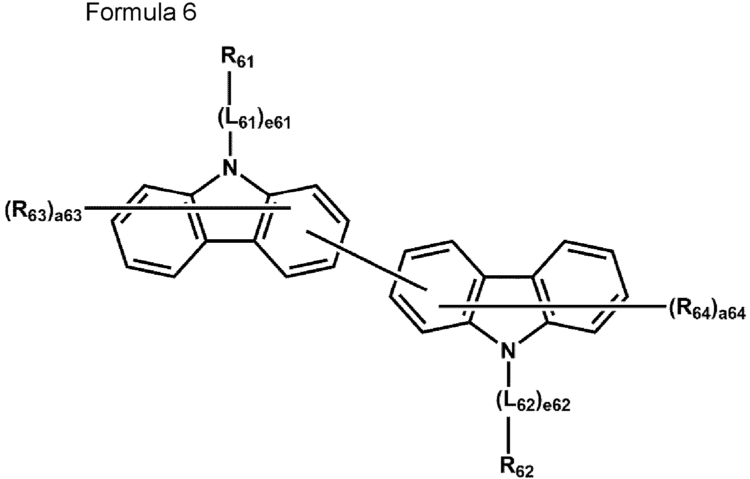 wherein, in Formula 6,
L₆₁ and L₆₂ may each independently be a π electron-rich C₃-C₆₀ cyclic group unsubstituted or substituted with deuterium, a C₁-C₂₀ alkyl group, a deuterated C₁-C₂₀ alkyl group, a phenyl group, a deuterated phenyl group, a (C₁-C₂₀ alkyl)phenyl group, a biphenyl group, a deuterated biphenyl group, a (C₁-C₂₀ alkyl)biphenyl group, -Si(Q₃₃)(Q₃₄)(Q₃₅), or any combination thereof,
e61 and e62 may each independently be an integer from 1 to 6,
R₆₁ to R₆₄ may each independently be:
   hydrogen, deuterium, a C₁-C₂₀ alkyl group, or a deuterated C₁-C₂₀ alkyl group;
   a π electron-rich C₃-C₆₀ cyclic group unsubstituted or substituted with deuterium, a C₁-C₂₀ alkyl group, a deuterated C₁-C₂₀ alkyl group, a phenyl group, a deuterated phenyl group, a (C₁-C₂₀ alkyl)phenyl group, a biphenyl group, a deuterated biphenyl group, a (C₁-C₂₀ alkyl)biphenyl group, - Si(Q₃₃)(Q₃₄)(Q₃₅), or any combination thereof; or
   -Si(Q₃)(Q₄)(Q₅), and
   a63 and a64 may each independently be an integer from 0 to 7.

Q₃ to Q₅ and Q₃₃ to Q₃₅ may each be the same as described herein.

**In** one or more embodiments, the hole transport compound may be a compound represented by Formula 6-1, 6-2, or 6-3: 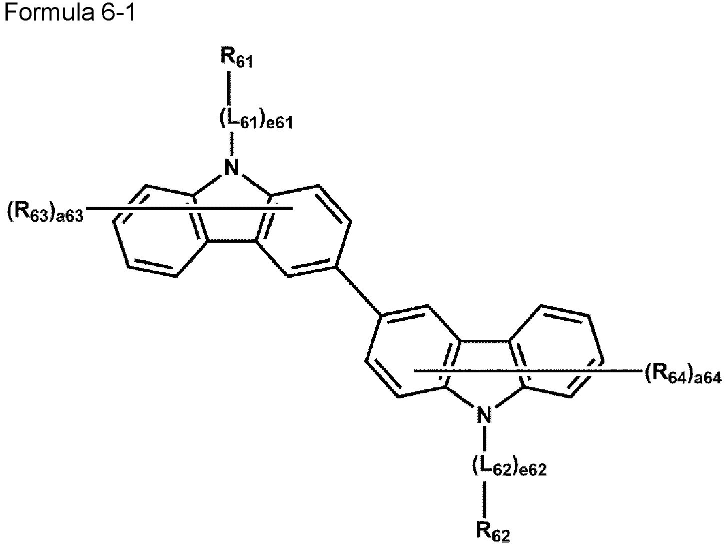 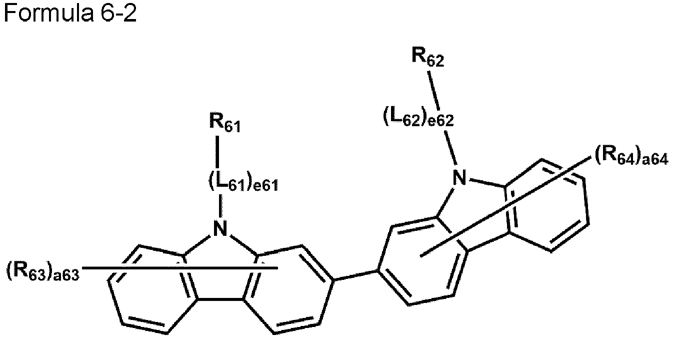 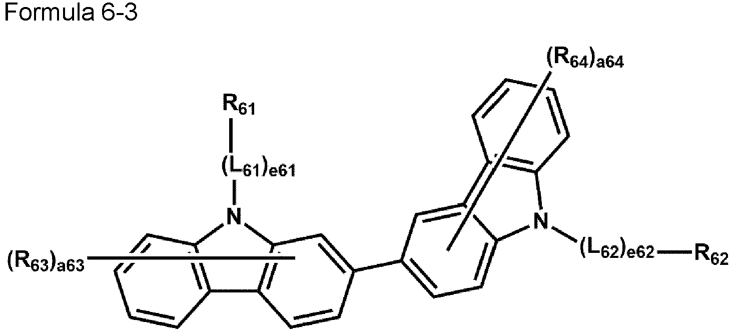 wherein, in Formulae 6-1 to 6-3, L₆₁, L₆₂, R₆₁ to R₆₄, e61, e62, a63, a64 may each be the same as described herein.

In one or more embodiments, the hole transport compound may be one of Compounds HTH1 to HTH6:

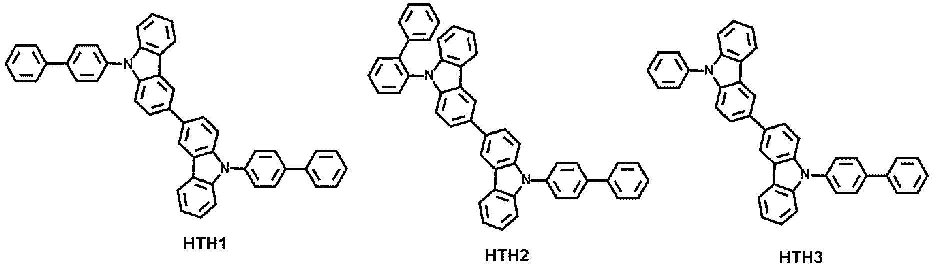

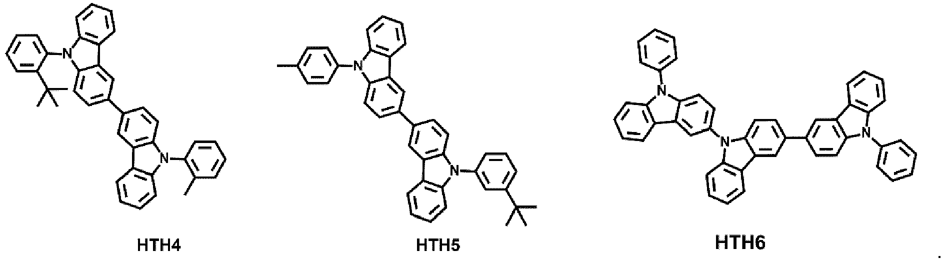

In one or more embodiments, the electron transport compound may be a compound represented by Formula 7: 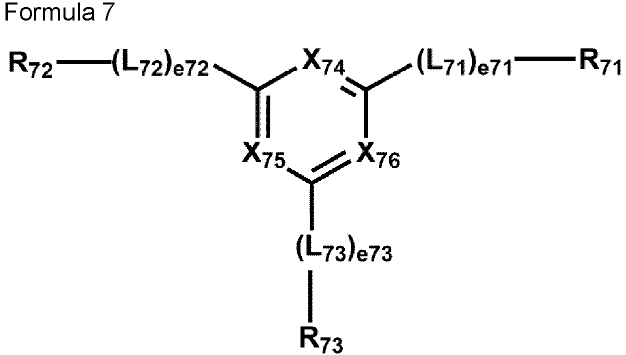 wherein, in Formula 7,
X₇₄ may be C(R₇₄) or N, X₇₅ may be C(R₇₅) or N, X₇₆ may be C(R₇₆) or N, and at least one of X₇₄ to X₇₆ may be N,
L₇₁ to L₇₃ may each independently be a C₅-C₃₀ carbocyclic group or a C₁-C₃₀ heterocyclic group, each unsubstituted or substituted with deuterium, - F, a cyano group, a C₁-C₂₀ alkyl group, a deuterated C₁-C₂₀ alkyl group, a fluorinated C₁-C₂₀ alkyl group, a phenyl group, a deuterated phenyl group, a fluorinated phenyl group, a (C₁-C₂₀ alkyl)phenyl group, a biphenyl group, a deuterated biphenyl group, a fluorinated phenyl group, a (C₁-C₂₀ alkyl)biphenyl group, -Si(Q₃₃)(Q₃₄)(Q₃₅), or any combination thereof,
e71 to e73 may each independently be an integer from 1 to 10,
R₇₁ to R₇₆ may each independently be:
   hydrogen, deuterium, -F, or a cyano group;
   a C₁-C₂₀ alkyl group unsubstituted or substituted with deuterium, -F, a cyano group, or any combination thereof;
   a C₅-C₃₀ carbocyclic group or a C₁-C₃₀ heterocyclic group, each unsubstituted or substituted with deuterium, -F, a cyano group, a C₁-C₂₀ alkyl group, a deuterated C₁-C₂₀ alkyl group, a fluorinated C₁-C₂₀ alkyl group, a phenyl group, a deuterated phenyl group, a fluorinated phenyl group, a (C₁-C₂₀ alkyl)phenyl group, a biphenyl group, a deuterated biphenyl group, a fluorinated phenyl group, a (C₁-C₂₀ alkyl)biphenyl group, -Si(Q₃₃)(Q₃₄)(Q₃₅), or any combination thereof; or

      -Si(Q₃)(Q₄)(Q₅).

Q₃ to Q₅ and Q₃₃ to Q₃₅ may each be the same as described herein.

In one or more embodiments, each of X₇₄ to X₇₆ in Formula 7 may be N.

In one or more embodiments, L₇₁ to L₇₃ in Formula 7 may each independently be a benzene group, a naphthalene group, a triphenylene group, a dibenzofuran group, a dibenzothiophene group, a carbazole group, an indolodibenzofuran group, an indolodibenzothiophene group, an indolocarbazole group, a naphthobenzofuran group, a naphthobenzothiophene group, a benzocarbazole group, a phenanthrenobenzofuran group, a phenanthrenobenzothiophene group, a naphthocarbazole group, a dinaphthofuran group, a dinaphthothiophene group, or a dibenzocarbazole group, each unsubstituted or substituted with deuterium, -F, a cyano group, a C₁-C₂₀ alkyl group, a deuterated C₁-C₂₀ alkyl group, a fluorinated C₁-C₂₀ alkyl group, a phenyl group, a deuterated phenyl group, a fluorinated phenyl group, a (C₁-C₂₀ alkyl)phenyl group, a biphenyl group, a deuterated biphenyl group, a fluorinated phenyl group, a (C₁-C₂₀ alkyl)biphenyl group, -Si(Q₃₃)(Q₃₄)(Q₃₅), or any combination thereof.

In one or more embodiments, in Formula 7, at least one of e71 L₇₁(s), at least one of e72 L₇₂(s), at least one of e73 L₇₃(s), or any combination thereof may each independently be a dibenzofuran group, a dibenzothiophene group, a carbazole group, an indolodibenzofuran group, an indolodibenzothiophene group, an indolocarbazole group, a naphthobenzofuran group, a naphthobenzothiophene group, a benzocarbazole group, a phenanthrenobenzofuran group, a phenanthrenobenzothiophene group, a naphthocarbazole group, a dinaphthofuran group, a dinaphthothiophene group, or a dibenzocarbazole group, each unsubstituted or substituted with deuterium, -F, a cyano group, a C₁-C₂₀ alkyl group, a deuterated C₁-C₂₀ alkyl group, a fluorinated C₁-C₂₀ alkyl group, a phenyl group, a deuterated phenyl group, a fluorinated phenyl group, a (C₁-C₂₀ alkyl)phenyl group, a biphenyl group, a deuterated biphenyl group, a fluorinated phenyl group, a (C₁-C₂₀ alkyl)biphenyl group, -Si(Q₃₃)(Q₃₄)(Q₃₅), or any combination thereof.

In one or more embodiments, in Formula 7, at least one of e71 L₇₁(s), at least one of e72 L₇₂(s), at least one of e73 L₇₃(s), or any combination thereof may each independently include a carbazole group, an indolocarbazole group, a benzocarbazole group, a naphthocarbazole group, or a dibenzocarbazole group, wherein a nitrogen atom of a pyrrole group of the carbazole group, the indolocarbazole group, the benzocarbazole group, the naphthocarbazole group, or the dibenzocarbazole group may be linked to a carbon atom of a 6-membered ring including X₇₄ to X₇₆ in Formula 7, via a single bond or neighboring L₇₁, L₇₂, and/or L₇₃.

In one or more embodiments, in Formula 7, e71 to e73 indicate the numbers of L₇₁ to L₇₃, respectively, and may each independently be 1, 2, 3, 4, or 5.

In one or more embodiments, R₇₁ to R₇₆ in Formula 7 may each independently be:
hydrogen, deuterium, -F, or a cyano group;
a C₁-C₂₀ alkyl group unsubstituted or substituted with deuterium, -F, a cyano group, or any combination thereof;
a benzene group, a naphthalene group, a triphenylene group, a dibenzofuran group, a dibenzothiophene group, a carbazole group, an indolodibenzofuran group, an indolodibenzothiophene group, an indolocarbazole group, a naphthobenzofuran group, a naphthobenzothiophene group, a benzocarbazole group, a phenanthrenobenzofuran group, a phenanthrenobenzothiophene group, a naphthocarbazole group, a dinaphthofuran group, a dinaphthothiophene group, or a dibenzocarbazole group, each unsubstituted or substituted with deuterium, -F, a cyano group, a C₁-C₂₀ alkyl group, a deuterated C₁-C₂₀ alkyl group, a fluorinated C₁-C₂₀ alkyl group, a phenyl group, a deuterated phenyl group, a fluorinated phenyl group, a (C₁-C₂₀ alkyl)phenyl group, a biphenyl group, a deuterated biphenyl group, a fluorinated phenyl group, a (C₁-C₂₀ alkyl)biphenyl group, -Si(Q₃₃)(Q₃₄)(Q₃₅), or any combination thereof; or

   -Si(Q₃)(Q₄)(Q₅).

In one or more embodiments, the electron transport compound may be one of Compounds ETH1 to ETH7:

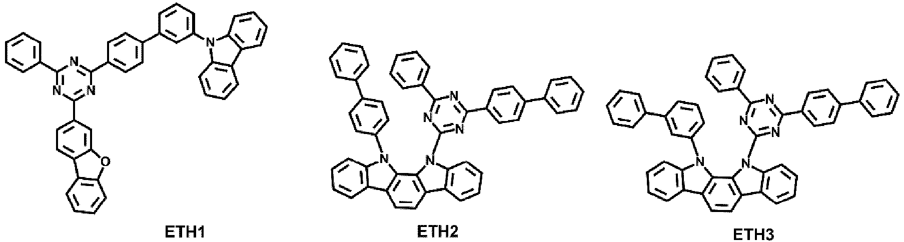

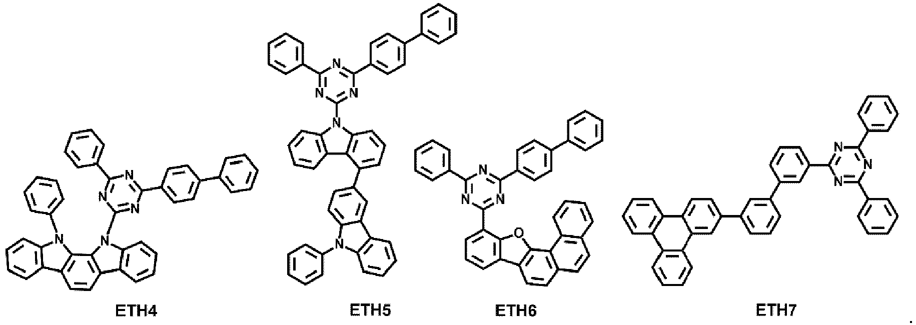

### Description of Formula 30

In the first embodiment and the second embodiment, the organometallic compound may be an organometallic compound represented by Formula 30: 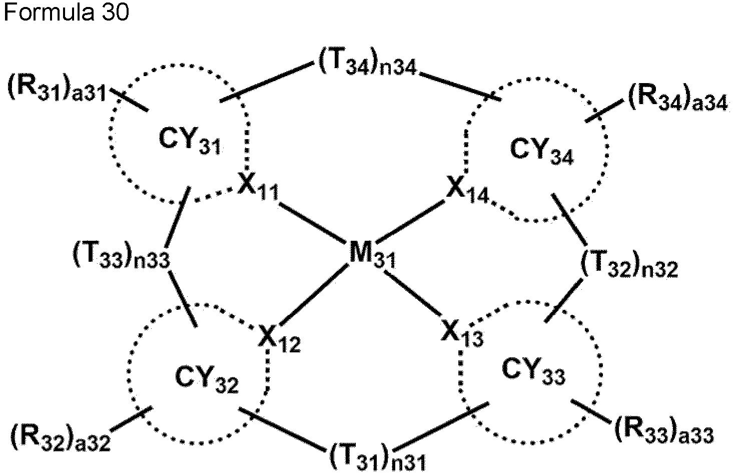 wherein, in Formula 30,
M₃₁ may be a transition metal,
X₁₁ to X₁₄ may each independently be C or **N,**
two bonds of a bond between X₁₁ and M₃₁, a bond between X₁₂ and M₃₁, a bond between X₁₃ and M₃₁, and a bond between X₁₄ and M₃₁ may each be a coordinate bond, and the other two bonds may each be a covalent bond,
ring CY₃₁ to ring CY₃₄ may each independently be a C₅-C₃₀ carbocyclic group or a C₁-C₃₀ heterocyclic group,
T₃₁ may be a single bond, a double bond, *-N(R₃₅ₐ)-*', *-B(R₃₅ₐ)-*', *-P(R₃₅ₐ)-*', *-C(R35a)(R35b)-*', *-Si(R35a)(R35b)-*', *-Ge(R₃₅ₐ)(R_{35b})-*, *-S-*', *-Se-*****', *-O-*', *-C(=O)-*', *-S(=O)-*', *-S(=O)₂-*', *-C(R₃₅ₐ)=*', *=C(R₃₅ₐ)-*', *-C(R₃₅ₐ)=C(R_{35b})-*', *-C(=S)-*', *-C=C-*', a C₅-C₃₀ carbocyclic group unsubstituted or substituted with at least one R₁₀ₐ, or a C₁-C₃₀ heterocyclic group unsubstituted or substituted with at least one R₁₀ₐ,
T₃₂ may be a single bond, a double bond, *-N(R₃₆ₐ)-*', *-B(R₃₆ₐ)-*', *-P(R₃₆ₐ)-*', *-C(R₃₆ₐ)(R_{36b})-*', *-Si(R₃₆ₐ)(R_{36b})-*', *-Ge(R₃₆ₐ)(R_{36b})-*', *-S-*', *-Se-*', *-O-*', *-C(=O)-*', *-S(=O)-*', *-S(=O)₂-*', *-C(R₃₆ₐ)=*', *=C(R₃₆ₐ)-*', *-C(R₃₆ₐ)=C(R_{36b})-*', *-C(=S)-*', *-C≡C-*', a C₅-C₃₀ carbocyclic group unsubstituted or substituted with at least one R₁₀ₐ, or a C₁-C₃₀ heterocyclic group unsubstituted or substituted with at least one R₁₀ₐ,
T₃₃ may be a single bond, a double bond, *-N(R₃₇ₐ)-*', *-B(R₃₇ₐ)-*', *-P(R₃₇ₐ)-*', *-C(R₃₇ₐ)(R_{37b})-*', *-Si(R₃₇ₐ)(R_{37b})-*', *-Ge(R₃₇ₐ)(R_{37b})-*', *-S-*', *-Se-*', *-O-*', *-C(=O)-*', *-S(=O)-*', *-S(=O)₂-*', *-C(R₃₇ₐ)=*', *=C(R₃₇ₐ)-*', *-C(R₃₇ₐ)=C(R_{37b})-*', *-C(=S)-*', *-C=C-*', a C₅-C₃₀ carbocyclic group unsubstituted or substituted with at least one R₁₀ₐ, or a C₁-C₃₀ heterocyclic group unsubstituted or substituted with at least one R₁₀ₐ,
T₃₄ may be a single bond, a double bond, *-N(R₃₈ₐ)-*', *-B(R₃₈ₐ)-*', *-P(R₃₈ₐ)-*', *-C(R₃₈ₐ)(R_{38b})-*', *-Si(R₃₈ₐ)(R_{38b})-*', *-Ge(R₃₈ₐ)(R_{38b})-*', *-S-*', *-Se-*', *-O-*', *-C(=O)-*', *-S(=O)-*', *-S(=O)₂-*', *-C(R₃₈ₐ)=*', *=C(R₃₈ₐ)-*', *-C(R₃₈ₐ)=C(R_{38b})-*', *-C(=S)-*', *-C=C-*', a C₅-C₃₀ carbocyclic group unsubstituted or substituted with at least one R₁₀ₐ, or a C₁-C₃₀ heterocyclic group unsubstituted or substituted with at least one R₁₀ₐ,
n31 to n34 may each independently be an integer from 0 to 5, and three or more of n31 to n34 may each independently be an integer from 1 to 5,
when n31 is 0, T₃₁ may be absent, when n32 is 0, T₃₂ may be absent, when n33 is 0, T₃₃ may be absent, and when n34 is 0, T₃₄ may be absent,
when n31 is 2 or more, two or more of T₃₁ may be identical to or different from each other, when n32 is 2 or more, two or more of T₃₂ may be identical to or different from each other, when n33 is 2 or more, two or more of T₃₃ may be identical to or different from each other, and when n34 is 2 or more, two or more of T₃₄ may be identical to or different from each other,
R₃₁ to R₃₄, R₃₅ₐ, R_{35b}, R₃₆ₐ, R_{36b}, R₃₇ₐ, R_{37b}, R₃₈ₐ, and R_{38b} may each independently be hydrogen, deuterium, -F, -Cl, -Br, -I, -CD₃, -CD₂H, -CDH₂, - CF₃, -CF₂H, -CFH₂, -SF₅, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₇-C₆₀ arylalkyl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₂-C₆₀ heteroarylalkyl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), - Ge(Q₃)(Q₄)(Q₅), -B(Q₆)(Q₇), -P(=O)(Q₈)(Q₉), or -P(Q₈)(Q₉),
a31 to a34 may each independently be an integer from 0 to 20,
two or more of R₃₁ in the number of a31 may optionally be bonded together to form a C₅-C₃₀ carbocyclic group unsubstituted or substituted with at least one R₁₀ₐ or a C₁-C₃₀ heterocyclic group unsubstituted or substituted with at least one R₁₀ₐ,
two or more of R₃₂ in the number of a32 may optionally be bonded together to form a C₅-C₃₀ carbocyclic group unsubstituted or substituted with at least one R₁₀ₐ or a C₁-C₃₀ heterocyclic group unsubstituted or substituted with at least one R₁₀ₐ,
two or more of R₃₃ in the number of a33 may optionally be bonded together to form a C₅-C₃₀ carbocyclic group unsubstituted or substituted with at least one R₁₀ₐ or a C₁-C₃₀ heterocyclic group unsubstituted or substituted with at least one R₁₀ₐ,
two or more of R₃₄ in the number of a34 may optionally be bonded together to form a C₅-C₃₀ carbocyclic group unsubstituted or substituted with at least one R₁₀ₐ or a C₁-C₃₀ heterocyclic group unsubstituted or substituted with at least one R₁₀ₐ,
at least two of R₃₁ to R₃₄, R₃₅ₐ, R_{35b}, R₃₆ₐ, R_{36b}, R₃₇ₐ, R_{37b}, R₃₈ₐ, and R_{38b} may optionally be bonded to each other to form a C₅-C₃₀ carbocyclic group unsubstituted or substituted with at least one R₁₀ₐ or a C₁-C₃₀ heterocyclic group unsubstituted or substituted with at least one R₁₀ₐ, and
R₁₀ₐ is the same as described in connection with R₃₁,
* and *' each indicate a binding site to a neighboring atom,
a substituent of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₂-C₁₀ heterocycloalkenyl group, the substituted C₆-C₆₀ aryl group, the substituted C₇-C₆₀ aryl alkyl group, the substituted C₆-C₆₀ aryloxy group, the substituted C₆-C₆₀ arylthio group, the substituted C₁-C₆₀ heteroaryl group, the substituted C₂-C₆₀ heteroaryl alkyl group, the substituted monovalent non-aromatic condensed polycyclic group, and the substituted monovalent non-aromatic condensed heteropolycyclic group may be:
   deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group;
   a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, - CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), -Ge(Q₁₃)(Q₁₄)(Q₁₅), - B(Q₁₆)(Q₁₇), -P(=O)(Q₁₈)(Q₁₉), -P(Q₁₈)(Q₁₉), or any combination thereof;
   a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with at least one of deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Ge(Q₂₃)(Q₂₄)(Q₂₅), -B(Q₂₆)(Q₂₇), - P(=O)(Q₂₈)(Q₂₉), -P(Q₂₈)(Q₂₉) or any combination thereof;
   -N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), - P(=O)(Q₃₈)(Q₃₉), or -P(Q₃₈)(Q₃₉); or
   any combination thereof, and
   Q₁ to Q₉, Q₁₁ to Q₁₉, Q₂₁ to Q₂₉, and Q₃₁ to Q₃₉ may each independently be hydrogen, deuterium, -F, -Cl, -Br, -I, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group which is unsubstituted or substituted with deuterium, a C₁-C₆₀ alkyl group, a C₆-C₆₀ aryl group, or any combination thereof, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group which is unsubstituted or substituted with deuterium, a C₁-C₆₀ alkyl group, a C₆-C₆₀ aryl group, or any combination thereof, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group.

In an embodiment, M₃₁ in Formula 30 may be Pt, Pd, or Au.

In an embodiment, M₃₁ in Formula 30 may be Pt or Pd.

In one or more embodiments, in Formula 30, a bond between X₁₁ and M₃₁ may be a coordinate bond.

In one or more embodiments, in Formula 30, X₁₁ may be C, and a bond between X₁₁ and M₃₁ may be a coordinate bond. That is, X₁₁ in Formula 30 may be C in a carbene moiety.

In one or more embodiments, ring CY₃₁ to ring CY₃₄ in Formula 30 may each independently be i) a first ring, ii) a second ring, iii) a condensed ring in which two or more first rings are condensed with each other, iv) a condensed ring in which two or more second rings are condensed with each other, or v) a condensed ring in which at least one first ring is condensed with at least one second ring,
wherein the first ring may be a cyclopentane group, a cyclopentadiene group, a furan group, a thiophene group, a pyrrole group, a silole group, an oxazole group, an isoxazole group, an oxadiazole group, an isoxadiazole group, an oxatriazole group, an isoxatriazole group, a thiazole group, an isothiazole group, a thiadiazole group, an isothiadiazole group, a thiatriazole group, an isothiatriazole group, a pyrazole group, an imidazole group, a triazole group, a tetrazole group, an azasilole group, a diazasilole group, or a triazasilole group, and
the second ring may be an adamantane group, a norbornane group, a norbornene group, a cyclohexane group, a cyclohexene group, a benzene group, a pyridine group, a pyrimidine group, a pyrazine group, a pyridazine group, a triazine group, an oxazine group, a thiazine group, a dihydropyrazine group, a dihydropyridine group, or a dihydroazasilane group.

In an embodiment, R₃₁ to R₃₄, R₃₅ₐ, R_{35b}, R₃₆ₐ, R_{36b}, R₃₇ₐ, R_{37b}, R₃₈ₐ, and R_{38b} may each independently be:
hydrogen, deuterium, -F, -Cl, -Br, -I, -CD₃, -CD₂H, -CDH₂, -CF₃, -CF₂H, -CFH₂, a cyano group (CN), a nitro group, an amino group, a C₁-C₂₀ alkyl group, or a C₁-C₂₀ alkoxy group;
a C₁-C₂₀ alkyl group or a C₁-C₂₀ alkoxy group, each substituted with at least one of deuterium, -F, -Cl, -Br, -I, -CD₃, -CD₂H, -CDH₂, -CF₃, -CF₂H, -CFH₂, a cyano group, a nitro group, an amino group, and a phenyl group; or
a cyclopentyl group, a cyclohexyl group, a cycloheptyl group, a cyclopentenyl group, a cyclohexenyl group, a cycloheptenyl group, a phenyl group, a biphenyl group, a naphthyl group, a fluorenyl group, a phenanthrenyl group, or an anthracenyl group, each unsubstituted or substituted with at least one of deuterium, -F, -Cl, -Br, -I, -CD₃, -CD₂H, -CDH₂, -CF₃, -CF₂H, -CFH₂, a cyano group, a nitro group, an amino group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a cycloheptenyl group, a phenyl group, a biphenyl group, a naphthyl group, a fluorenyl group, a phenanthrenyl group, and an anthracenyl group.

In an embodiment, the organometallic compound represented by Formula 30 may be an organometallic compound represented by Formula 30-1 or an organometallic compound represented by Formula 30-2: 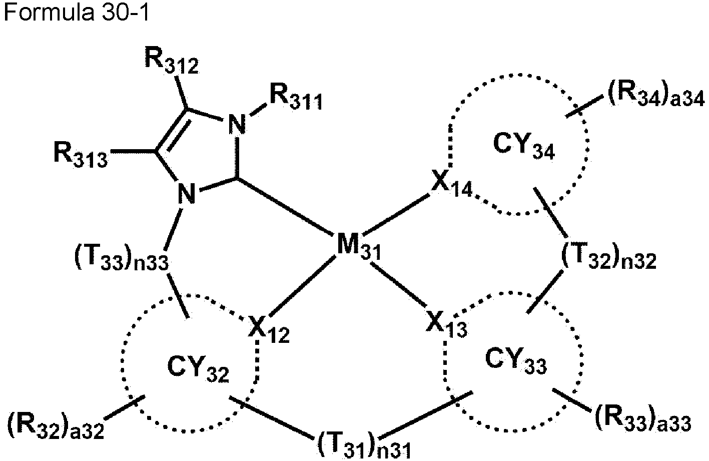 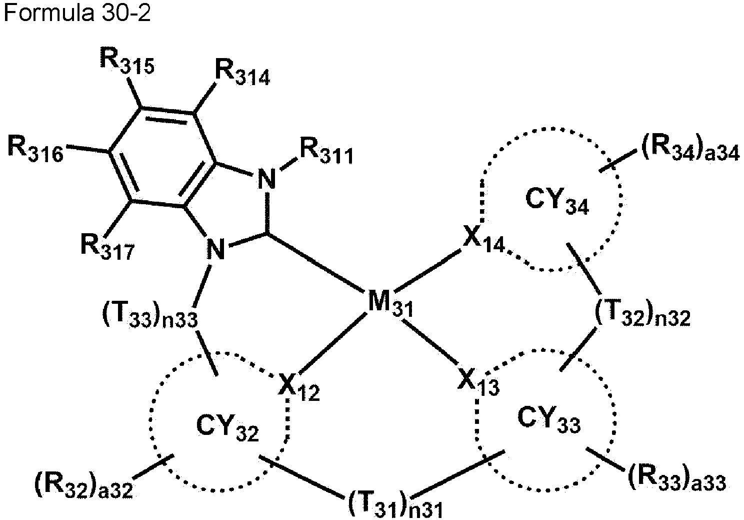 wherein, in Formula 30-1, a bond between carbon of the imidazole group and M₃₁ may be a coordinate bond. That is, in Formula 30-1, the imidazole group may include a carbene moiety bonded to M₃₁.

In Formula 30-2, a bond between carbon of the benzimidazole group and M₃₁ may be a coordinate bond. That is, in Formula 30-2, the benzimidazole group may include a carbene moiety bonded to M₃₁.

Therefore, Formula 30-1' in which the carbon bonded to M₃₁ in the imidazole group is carbene, is the same as Formula 30-1, and Formula 30-2' in which the carbon bonded to M₃₁ in the benzimidazole group is carbene, is the same as Formula 30-2:

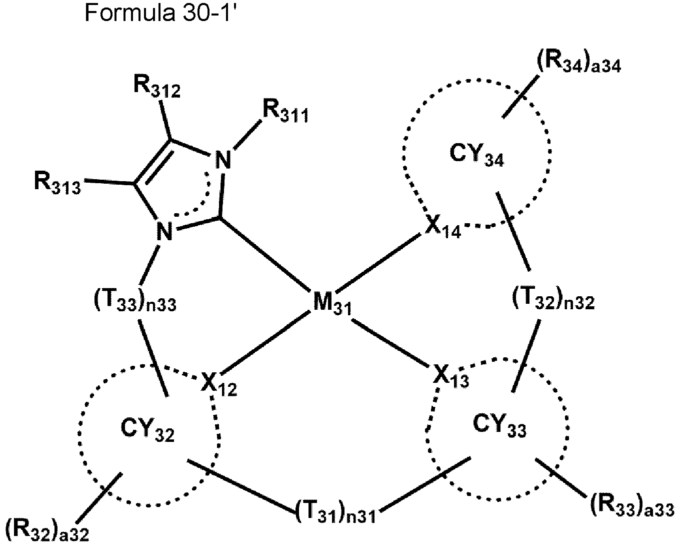

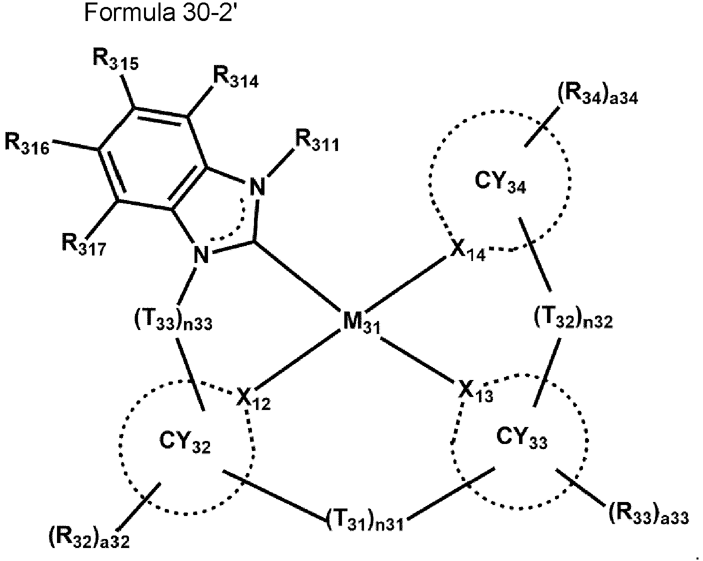

**In** Formulae 30-1 and 30-2,
M₃₁, CY₃₂, CY₃₃, CY₃₄, X₁₂, X₁₃, X₁₄, T₃₁, T₃₂, T₃₃, n31, n32, n33, R₃₂, R₃₃, R₃₄, a32, a33, and a34 may each be the same as described herein, and
R₃₁₁ to R₃₁₇ may each be the same as described in connection with R₃₁ of Formula 30.

In an embodiment, in Formulae 30-1 and 30-2,
R₃₁₁ to R₃₁₇ may each independently be:
hydrogen, deuterium, -F, -Cl, -Br, -I, -CD₃, -CD₂H, -CDH₂, -CF₃, -CF₂H, -CFH₂, -SF₅, a hydroxyl group, a cyano group, a nitro group, an amidino group, a hydrazine group, a hydrazone group, a carboxylic acid group or a salt thereof, a sulfonic acid group or a salt thereof, or a phosphoric acid group or a salt thereof;
a C₁-C₂₀ alkyl group or a C₁-C₂₀ alkoxy group, each unsubstituted or substituted with deuterium, -F, -Cl, -Br, -I, -CD₃, -CD₂H, -CDH₂, -CF₃, -CF₂H, - CFH₂, -SF₅, a hydroxyl group, a cyano group, a nitro group, an amidino group, a hydrazine group, a hydrazone group, or any combination thereof;
a C₃-C₁₀ cycloalkyl group, a C₁-C₁₀ heterocycloalkyl group, a C₆-C₆₀ aryl group, a C₇-C₆₀ arylalkyl group, C₆-C₆₀ aryloxy group, a C₆-C₆₀ arylthio group, a C₁-C₆₀ heteroaryl group, a substituted or unsubstituted C₂-C₆₀ heteroaryl alkyl group, a monovalent non-aromatic condensed polycyclic group, or a monovalent non-aromatic condensed heteropolycyclic group, each unsubstituted or substituted with deuterium, -F, -Cl, -Br, -I, -CD₃, -CD₂H, -CDH₂, -CF₃, -CF₂H, -CFH₂, -SF₅, a hydroxyl group, a cyano group, a nitro group, an amidino group, a hydrazine group, a hydrazone group, a C₁-C₂₀ alkyl group, a C₁-C₂₀ alkoxy group, or any combination thereof; or
-N(Q₁)(Q₂), -Si(Q₃)(Q₄)(Q₅), -Ge(Q₃)(Q₄)(Q₅), -B(Q₆)(Q₇), - P(=O)(Q₈)(Q₉), or -P(Q₈)(Q₉).

In an embodiment, at least one of R₃₁₁ to R₃₁₇ in Formulae 30-1 and 30-2 may include a C₁-C₂₀ alkyl group, a C₆-C₆₀ aryl group, or a C₇-C₆₀ arylalkyl group, each unsubstituted or substituted with deuterium, a methyl group, an ethyl group, an n-propyl group, an isopropyl group, an n-butyl group, an isobutyl group, a sec-butyl group, a tert-butyl group, a phenyl group, a cumyl group, or any combination thereof.

According to an embodiment, the organometallic compound represented by Formula 30 may be an organometallic compound represented by Formula 30-1(1) or an organometallic compound represented by Formula 30-2(1): 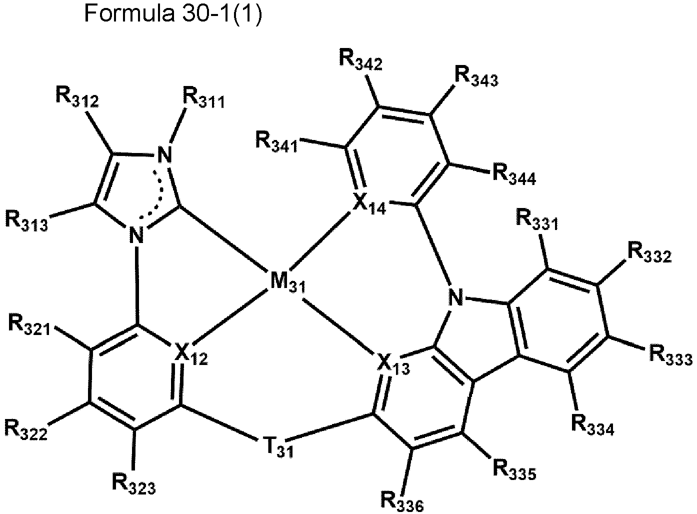 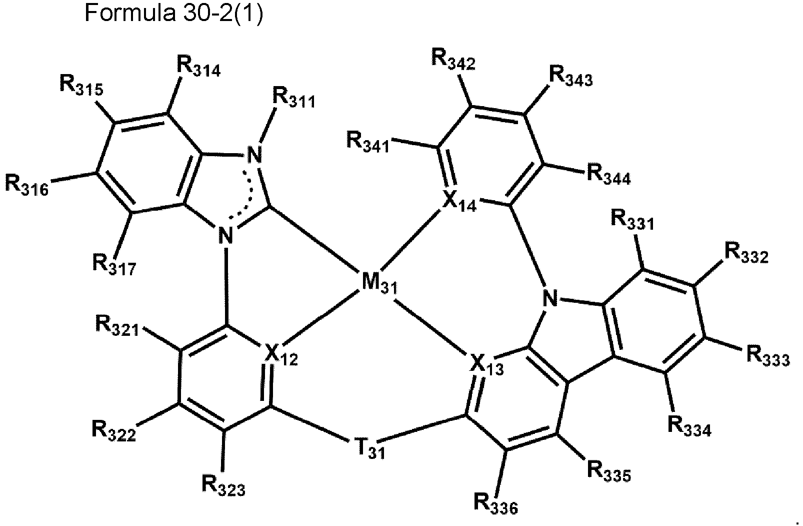 In Formulae 30-1(1) and 30-2(1),
M₃₁, X₁₂, X₁₃, X₁₄, and T₃₁ are each the same as described herein,
R₃₁₁ to R₃₁₇ may each be the same as described in connection with R₃₁,
R₃₂₁ to R₃₂₃ may each be the same as described in connection with R₃₂, and two or more of R₃₂₁ to R₃₂₃ may optionally be bonded together to form a C₅-C₃₀ carbocyclic group unsubstituted or substituted with at least one R₁₀ₐ or a C₁-C₃₀ heterocyclic group unsubstituted or substituted with at least one R₁₀ₐ,
R₃₃₁ to R₃₃₆ may each be the same as described in connection with R₃₃, and two or more of R₃₃₁ to R₃₃₆ may optionally be bonded together to form a C₅-C₃₀ carbocyclic group unsubstituted or substituted with at least one R₁₀ₐ or a C₁-C₃₀ heterocyclic group unsubstituted or substituted with at least one R₁₀ₐ, and
   [0001] R₃₄₁ to R₃₄₄ may each be the same as described in connection with R₃₄, and two or more of R₃₄₁ to R₃₄₄ may optionally be bonded together to form a C₅-C₃₀ carbocyclic group unsubstituted or substituted with at least one R₁₀ₐ or a C₁-C₃₀ heterocyclic group unsubstituted or substituted with at least one R₁₀ₐ.

### Description of Formula 5

In the first embodiment and the second embodiment, the organometallic compound may be an organometallic compound represented by Formula 5:

Formula 5 M₅₁(L₅₁)ₙ₅₁(L₅₂)ₙ₅₂

wherein M₅₁in Formula 5 may be a transition metal.

In an embodiment, M₅₁may be a first-row transition metal, a second-row transition metal, or a third-row transition metal of the Periodic Table of Elements.

In one or more embodiments, M₅₁ may be iridium (Ir), platinum (Pt), osmium (Os), titanium (Ti), zirconium (Zr), hafnium (Hf), europium (Eu), terbium (Tb), thulium (Tm), or rhodium (Rh).

In one or more embodiments, M₅₁ may be Ir, Pt, Os, or Rh.

In one or more embodiments, M₅₁ may be Ir or Os.

In Formula 5, L₅₁ may be a ligand represented by Formula 5A, and L₅₂ may be a ligand represented by Formula 5B: 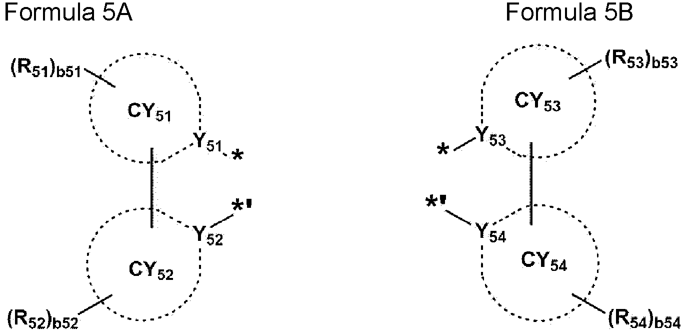 wherein Formulae 5A and 5B may each be the same as described herein.

In Formula 5, n51 may be 1, 2, or 3, wherein, when n51 is 2, two L₅₁ may be identical to or different from each other.

In Formula 5, n52 may be 0, 1, or 2, wherein, when n52 is 2 or more, two or more of L₅₂ may be identical to or different from each other.

The sum of n51 and n52 in Formula 5 may be 2 or 3. For example, the sum of n51 and n52 may be 3.

In an embodiment, in Formula 5, i) M may be Ir, and n51+n52=3; or ii) M may be Pt, and n51 +n52=2.

In one or more embodiments, in Formula 5, M may be Ir, and i) n51 may be 1, and n52 may be 2, or ii) n51 may be 2, and n52 may be 1.

L₅₁ and L₅₂ in Formula 5 may be different from each other.

In Formulae 5A and 5B, Y₅₁ to Y₅₄ may each independently be C or N. For example, Y₅₁ and Y₅₃ may each be **N,** and Y₅₂ and Y₅₄ may each be C.

Ring CY₅₁ to ring CY₅₄ in Formulae 5A and 5B may each independently be a C₅-C₃₀ carbocyclic group or a C₁-C₃₀ heterocyclic group.

For example, ring CY₅₁ to ring CY₅₄ in Formulae 5A and 5B may each independently include (or, be) i) a third ring, ii) a fourth ring, iii) a condensed ring in which two or more third rings are condensed with each other, iv) a condensed ring in which two or more fourth rings are condensed with each other, or v) a condensed ring in which at least one third ring is condensed with at least one fourth ring,
wherein the third ring may be a cyclopentane group, a cyclopentene group, a furan group, a thiophene group, a pyrrole group, a silole group, a borole group, a phosphole group, a germole group, a selenophene group, an oxazole group, an oxadiazole group, an oxatriazole group, a thiazole group, a thiadiazole group, a thiatriazole group, a pyrazole group, an imidazole group, a triazole group, a tetrazole group, or an azasilole group, and
the fourth ring may be an adamantane group, a norbornane group, a norbornene group, a cyclohexane group, a cyclohexene group, a benzene group, a pyridine group, a pyrimidine group, a pyrazine group, a pyridazine group, or a triazine group.

In one or more embodiments, in Formulae 5A and 5B, ring CY₁ to ring CY₄ may each independently be a cyclopentane group, a cyclohexane group, a cyclohexene group, a benzene group, a naphthalene group, an anthracene group, a phenanthrene group, a triphenylene group, a pyrene group, a chrysene group, a 1,2,3,4-tetrahydronaphthalene group, a cyclopentadiene group, a pyrrole group, a furan group, a thiophene group, a silole group, a borole group, a phosphole group, a germole group, a selenophene group, an indene group, an indole group, a benzofuran group, a benzothiophene group, a benzosilole group, a benzoborole group, a benzophosphole group, a benzogermole group, a benzoselenophene group, a fluorene group, a carbazole group, a dibenzofuran group, a dibenzothiophene group, a dibenzosilole group, a dibenzoborole group, a dibenzophosphole group, a dibenzogermole group, a dibenzoselenophene group, a benzofluorene group, a benzocarbazole group, a naphthobenzofuran group, a naphthobenzothiophene group, a naphthobenzosilole group, a naphthobenzoborole group, a naphthobenzophosphole group, a naphthobenzogermole group, a naphthobenzoselenophene group, a dibenzofluorene group, a dibenzocarbazole group, a dinaphthofuran group, a dinaphthothiophene group, a dinaphthosilole group, a dinaphthoborole group, a dinaphthophosphole group, a dinaphthogermole group, a dinaphthoselenophene group, an indenophenanthrene group, an indolophenanthrene group, a phenanthrobenzofuran group, a phenanthrobenzothiophene group, a phenanthrobenzosilole group, a phenanthrobenzoborole group, a phenanthrobenzophosphole group, a phenanthrobenzogermole group, a phenanthrobenzoselenophene group, a dibenzothiophene 5-oxide group, a 9H-fluorene-9-one group, a dibenzothiophene 5,5-dioxide group, an azaindene group, an azaindole group, an azabenzofuran group, an azabenzothiophene group, an azabenzosilole group, an azabenzoborole group, an azabenzophosphole group, an azabenzogermole group, an azabenzoselenophene group, an azafluorene group, an azacarbazole group, an azadibenzofuran group, an azadibenzothiophene group, an azadibenzosilole group, an azadibenzoborole group, an azadibenzophosphole group, an azadibenzogermole group, an azadibenzoselenophene group, an azabenzofluorene group, an azabenzocarbazole group, an azanaphthobenzofuran group, an azanaphthobenzothiophene group, an azanaphthobenzosilole group, an azanaphthobenzoborole group, an azanaphthobenzophosphole group, an azanaphthobenzogermole group, an azanaphthobenzoselenophene group, an azadibenzofluorene group, an azadibenzocarbazole group, an azadinaphthofuran group, an azadinaphthothiophene group, an azadinaphthosilole group, an azadinaphthoborole group, an azadinaphthophosphole group, an azadinaphthogermole group, an azadinaphthoselenophene group, an azaindenophenanthrene group, an azaindolophenanthrene group, an azaphenanthrobenzofuran group, an azaphenanthrobenzothiophene group, an azaphenanthrobenzosilole group, an azaphenanthrobenzoborole group, an azaphenanthrobenzophosphole group, an azaphenanthrobenzogermole group, an azaphenanthrobenzoselenophene group, an azadibenzothiophene 5-oxide group, an aza9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a benzoquinoline group, a benzoisoquinoline group, a benzoquinoxaline group, a benzoquinazoline group, a phenanthroline group, a phenanthridine group, a pyrrole group, a pyrazole group, an imidazole group, a triazole group, an oxazole group, an isooxazole group, a thiazole group, an isothiazole group, an oxadiazole group, a thiadiazole group, an azasilole group, an azaborole group, an azaphosphole group, an azagermole group, an azaselenophene group, a benzopyrrole group, a benzopyrazole group, a benzimidazole group, a benzoxazole group, a benzisoxazole group, a benzothiazole group, a benzisothiazole group, a benzoxadiazole group, a benzothiadiazole group, a pyridinopyrrole group, a pyridinopyrazole group, a pyridinoimidazole group, a pyridinooxazole group, a pyridinoisoxazole group, a pyridinothiazole group, a pyridinoisothiazole group, a pyridinooxadiazole group, a pyridinothiadiazole group, a pyrimidinopyrrole group, a pyrimidinopyrazole group, a pyrimidinoimidazole group, a pyrimidinooxazole group, a pyrimidinoisoxazole group, a pyrimidinothiazole group, a pyrimidinoisothiazole group, a pyrimidinooxadiazole group, a pyrimidinothiadiazole group, a 5,6,7,8-tetrahydroisoquinoline group, a 5,6,7,8-tetrahydroquinoline group, an adamantane group, a norbornane group, a norbornene group, a benzene group condensed with a cyclohexane group, a benzene group condensed with a norbornane group, a pyridine group condensed with a cyclohexane group, or a pyridine group condensed with a norbornane group.

For example, ring CY₅₁ and ring CY₅₃ in Formulae 5A and 5B may be different from each other.

**In** one or more embodiments, ring CY₅₂ and ring CY₅₄ in Formulae 5A and 5B may be different from each other.

In one or more embodiments, ring CY₅₁ to ring CY₅₄ in Formulae 5A and 5B may be different from each other.

R₅₁ to R₅₄ in Formulae 5A and 5B may each independently be hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₅₁)(Q₅₂), -Si(Q₅₃)(Q₅₄)(Q₅₅), -Ge(Q₅₃)(Q₅₄)(Q₅₅), -B(Q₅₆)(Q₅₇), - P(=O)(Q₅₈)(Q₅₉), or -P(Q₅₈)(Q₅₉). Q₅₁ to Q₅₉ are each the same as described in the present specification.

In an embodiment, R₅₁ to R₅₄ in Formulae 5A and 5B may each independently be:
hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, a C₁-C₂₀ alkoxy group, or a C₁-C₂₀ alkylthio group;
a C₁-C₂₀ alkyl group, a C₁-C₂₀ alkoxy group, or a C₁-C₂₀ alkylthio group, each substituted with deuterium, -F, -Cl, -Br, -I, -CD₃, -CD₂H, -CDH₂, -CF₃, - CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.2]octyl group, a (C₁-C₂₀ alkyl)cyclopentyl group, a (C₁-C₂₀ alkyl)cyclohexyl group, a (C₁-C₂₀ alkyl)cycloheptyl group, a (C₁-C₂₀ alkyl)cyclooctyl group, a (C₁-C₂₀ alkyl)adamantanyl group, a (C₁-C₂₀ alkyl)norbornanyl group, a (C₁-C₂₀ alkyl)norbornenyl group, a (C₁-C₂₀ alkyl)cyclopentenyl group, a (C₁-C₂₀ alkyl)cyclohexenyl group, a (C₁-C₂₀ alkyl)cycloheptenyl group, a (C₁-C₂₀ alkyl)bicyclo[1.1.1]pentyl group, a (C₁-C₂₀ alkyl)bicyclo[2.1.1]hexyl group, a (C₁-C₂₀ alkyl)bicyclo[2.2.2]octyl group, a phenyl group, a (C₁-C₂₀ alkyl)phenyl group, a biphenyl group, a terphenyl group, a naphthyl group, a pyridinyl group, a pyrimidinyl group, or any combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.2]octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group or azadibenzothiophenyl group, each unsubstituted or substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a (phenyl)C₁-C₁₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.2]octyl group, a (C₁-C₂₀ alkyl)cyclopentyl group, a (C₁-C₂₀ alkyl)cyclohexyl group, a (C₁-C₂₀ alkyl)cycloheptyl group, a (C₁-C₂₀ alkyl)cyclooctyl group, a (C₁-C₂₀ alkyl)adamantanyl group, a (C₁-C₂₀ alkyl)norbornanyl group, a (C₁-C₂₀ alkyl)norbornenyl group, a (C₁-C₂₀ alkyl)cyclopentenyl group, a (C₁-C₂₀ alkyl)cyclohexenyl group, a (C₁-C₂₀ alkyl)cycloheptenyl group, a (C₁-C₂₀ alkyl)bicyclo[1.1.1]pentyl group, a (C₁-C₂₀ alkyl)bicyclo[2.1.1]hexyl group, a (C₁-C₂₀ alkyl)bicyclo[2.2.2]octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, or any combination thereof; or
-N(Q₅₁)(Q₅₂), -Si(Q₅₃)(Q₅₄)(Q₅₅), -Ge(Q₅₃)(Q₅₄)(Q₅₅), -B(Q₅₆)(Q₅₇), - P(=O)(Q₅₈)(Q₅₉), or -P(Q₅₈)(Q₅₉), and
Q₅₁ to Q₅₉ may each independently be:
   -CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, or - CD₂CDH₂; or
   an n-propyl group, an isopropyl group, an n-butyl group, a sec-butyl group, an isobutyl group, a tert-butyl group, an n-pentyl group, a tert-pentyl group, an neopentyl group, an isopentyl group, a sec-pentyl group, a 3-pentyl group, a sec-isopentyl group, a phenyl group, a biphenyl group, or a naphthyl group, each unsubstituted or substituted with deuterium, a C₁-C₁₀ alkyl group, a phenyl group, or any combination thereof.

In one or more embodiments, R₅₁ to R₅₄ may each independently be:
hydrogen, deuterium, -F, or a cyano group;
a C₁-C₂₀ alkyl group unsubstituted or substituted with deuterium, a cyano group, a C₃-C₁₀ cycloalkyl group, a deuterated C₃-C₁₀ cycloalkyl group, a fluorinated C₃-C₁₀ cycloalkyl group, a (C₁-C₂₀ alkyl)C₃-C₁₀ cycloalkyl group, a C₁-C₁₀ heterocycloalkyl group, a deuterated C₁-C₁₀ heterocycloalkyl group, a fluorinated C₁-C₁₀ heterocycloalkyl group, a (C₁-C₂₀ alkyl)C₁-C₁₀ heterocycloalkyl group, a phenyl group, a deuterated phenyl group, a fluorinated phenyl group, a (C₁-C₂₀ alkyl)phenyl group, a biphenyl group, a deuterated biphenyl group, a fluorinated biphenyl group, a (C₁-C₂₀ alkyl)biphenyl group, a dibenzofuranyl group, a deuterated dibenzofuranyl group, a fluorinated dibenzofuranyl group, a (C₁-C₂₀ alkyl)dibenzofuranyl group, a dibenzothiophenyl group, a deuterated dibenzothiophenyl group, a fluorinated dibenzothiophenyl group, a (C₁-C₂₀ alkyl)dibenzothiophenyl group, or any combination thereof;
a C₃-C₁₀ cycloalkyl group, a C₁-C₁₀ heterocycloalkyl group, a phenyl group, or a biphenyl group, each unsubstituted or substituted with deuterium, a cyano group, a C₁-C₂₀ alkyl group, a deuterated C₁-C₂₀ alkyl group, a fluorinated C₁-C₂₀ alkyl group, a C₁-C₂₀ alkoxy group, a deuterated C₁-C₂₀ alkoxy group, a fluorinated C₁-C₂₀ alkoxy group, a C₃-C₁₀ cycloalkyl group, a deuterated C₃-C₁₀ cycloalkyl group, a fluorinated C₃-C₁₀ cycloalkyl group, a (C₁-C₂₀ alkyl)C₃-C₁₀ cycloalkyl group, a C₁-C₁₀ heterocycloalkyl group, a deuterated C₁-C₁₀ heterocycloalkyl group, a fluorinated C₁-C₁₀ heterocycloalkyl group, a (C₁-C₂₀ alkyl)C₁-C₁₀ heterocycloalkyl group, a phenyl group, a deuterated phenyl group, a fluorinated phenyl group, a (C₁-C₂₀ alkyl)phenyl group, a biphenyl group, a deuterated biphenyl group, a fluorinated biphenyl group, a (C₁-C₂₀ alkyl)biphenyl group, a dibenzofuranyl group, a deuterated dibenzofuranyl group, a fluorinated dibenzofuranyl group, a (C₁-C₂₀ alkyl)dibenzofuranyl group, a dibenzothiophenyl group, a deuterated dibenzothiophenyl group, a fluorinated dibenzothiophenyl group, a (C₁-C₂₀ alkyl)dibenzothiophenyl group, or any combination thereof; or
-Si(Q₅₃)(Q₅₄)(Q₅₅) or -Ge(Q₅₃)(Q₅₄)(Q₅₅).

In Formulae 5A and 5B, b51 to b54 indicate the numbers of R₅₁ to R₅₄, respectively, and may each independently be an integer from 0 to 20. When b51 is 2 or more, two or more of R₅₁ may be identical to or different from each other, when b52 is 2 or more, two or more of R₅₂ may be identical to or different from each other, when b53 is 2 or more, two or more of R₅₃ may be identical to or different from each other, and when b54 is 2 or more, two or more of R₅₄ may be identical to or different from each other. For example, b51 to b54 may each independently be an integer from 0 to 8.

In an embodiment, in Formula 5A, Y₅₂ may be C, a bond between Y₅₂ and M₅₁ may be a covalent bond, and at least one of R₅₂(s) in the number of b52 may be a cyano group or -F.

In one or more embodiments, in Formula 5A, Y₅₁ may be N, a bond between Y₅₁ and M₅₁ may be a coordinate bond, CY₅₁ may be an imidazole group, a triazole group, a benzimidazole group, or a triazolopyridine group, and at least one of R₅₂ in the number of b52 may be a cyano group or -F.

In one or more embodiments, in Formula 5A, Y₅₁ may be C, and a bond between Y₅₁ and M₅₁ may be a coordinate bond.

In one or more embodiments, in Formula 5A, Y₅₁ may be C, a bond between Y₅₁ and M₅₁ may be a coordinate bond, and CY₅₁ may be a benzimidazole group or an imidazopyrazine group.

### Specific examples of organometallic compound of Formula 3 or 5

For example, the organometallic compound represented by Formula 3 or 5 may be one of Compounds P1 to P52 below:

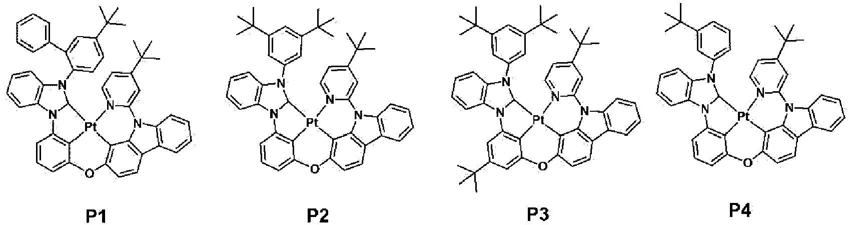

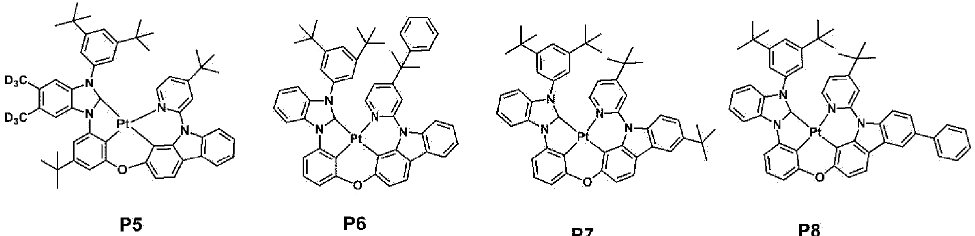

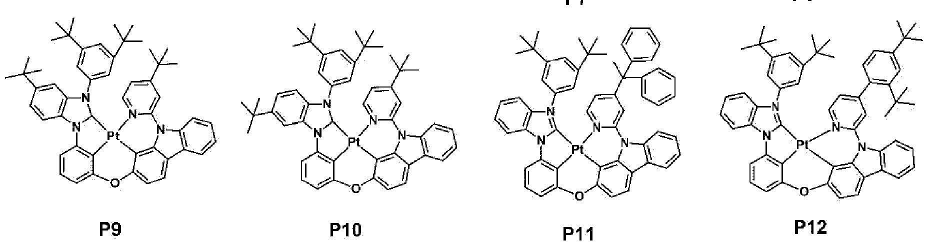

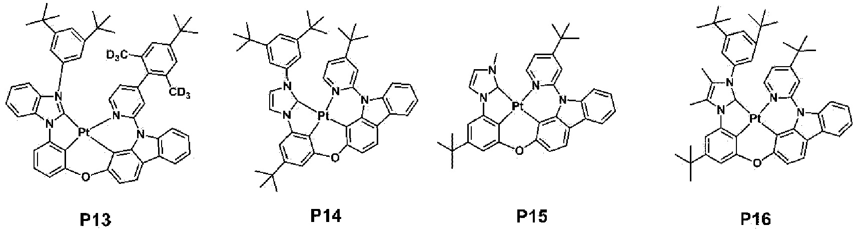

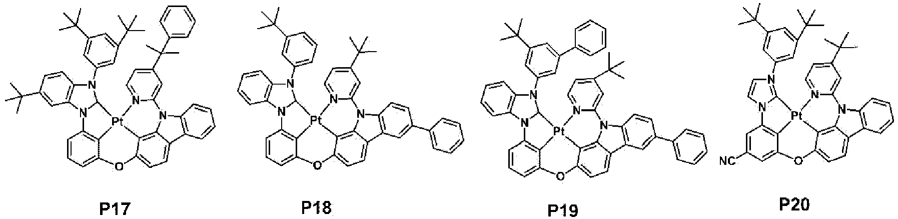

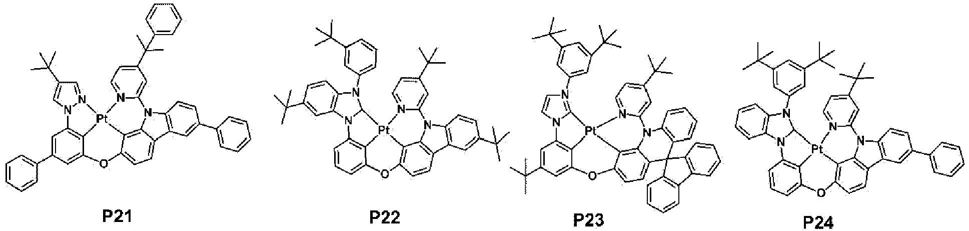

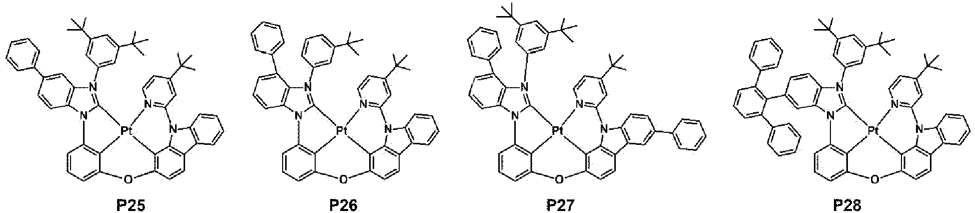

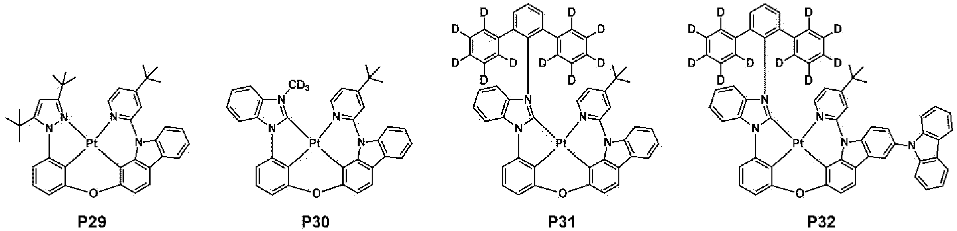

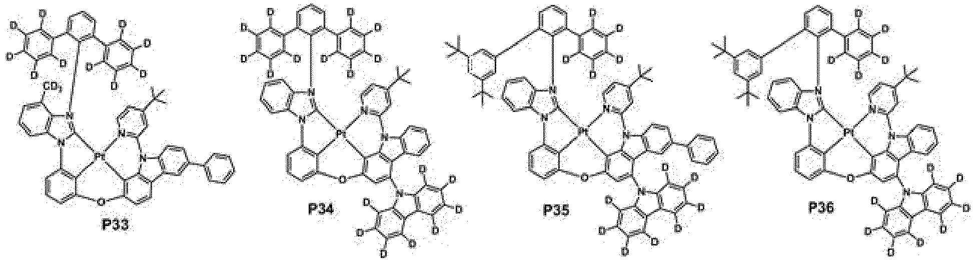

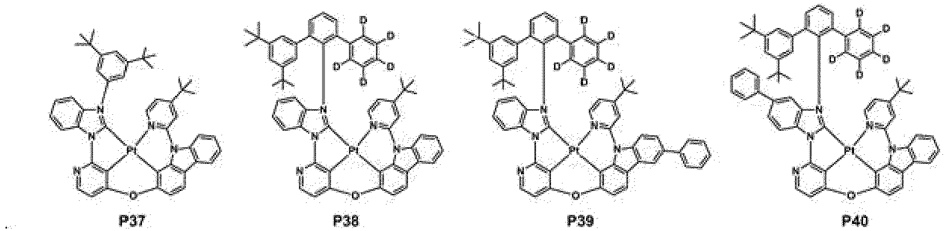

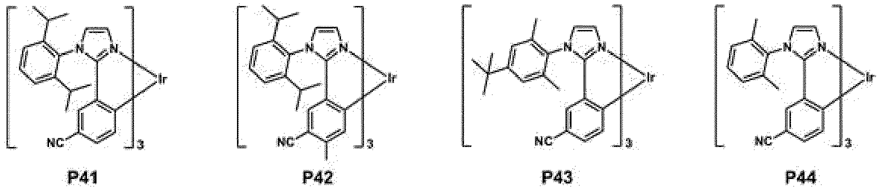

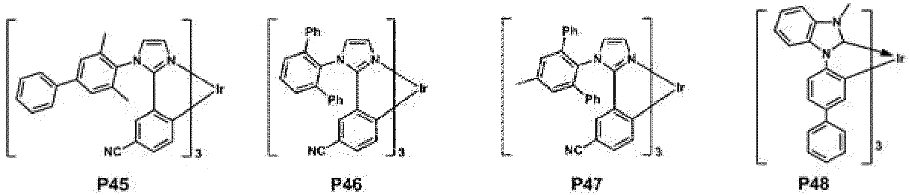

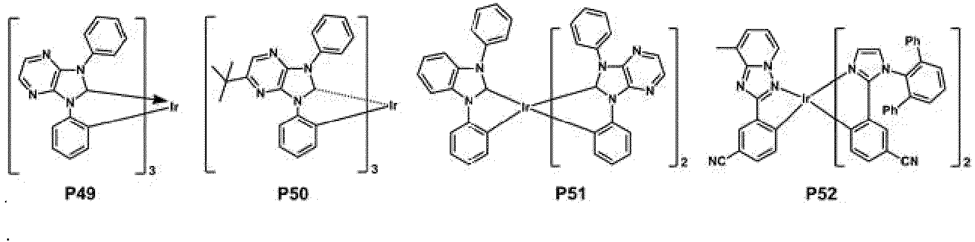

### Description of Formula 4

In the first embodiment and the second embodiment, the delayed fluorescence material may be a polycyclic compound represented by Formula 4: 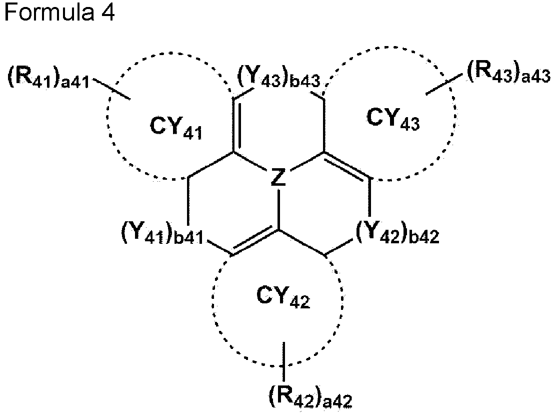 wherein, in Formula 4,
Z may be B or N,
ring CY₄₁ to ring CY₄₃ may each independently be a C₅-C₆₀ carbocyclic group or a C₁-C₆₀ heterocyclic group,
Y₄₁ may be a single bond, *-N(R₄₄)-*', *-B(R₄₄)-*', *-P(R₄₄)-*', *-C(R₄₄)(R₄₅)-*', *-Si(R₄₄)(R₄₅)-*', *-Ge(R₄₄)(R₄₅)-*', *-O-*', *-S-*', *-Se-*', *-C(=O)-*', or *-S(=O)₂-*',
Y₄₂ may be a single bond, *-N(R₄₆)-*', *-B(R₄₆)-*', *-P(R₄₆)-*', *-C(R₄₆)(R₄₇)-*', *-Si(R₄₆)(R₄₇)-*', *-Ge(R₄₆)(R₄₇)-*', *-O-*', *-S-*', *-Se-*', *-C(=O)-*', or *-S(=O)₂-*',
Y₄₃ may be a single bond, *-N(R₄₈)-*', *-B(R₄₈)-*', *-P(R₄₈)-*', *-C(R₄₈)(R₄₉)-*', *-Si(R₄₈)(R₄₉)-*', *-Ge(R₄₈)(R₄₉)-*', *-O-*', *-S-*', *-Se-*', *-C(=O)-*', or *-S(=O)₂-*',
b41 to b43 may each independently be 0 or 1,
when b41 is 0, Y₄₁ is absent, when b42 is 0, Y₄₂ is absent, and when b43 is 0, Y₄₃ is absent,
R₄₁ to R₄₉ may each independently be hydrogen, deuterium, -F, -Cl, - Br, -I, -SF₅, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₇-C₆₀ arylalkyl group, a substituted or unsubstituted C₆-C₆₀ aryloxy group, a substituted or unsubstituted C₆-C₆₀ arylthio group, a substituted or unsubstituted C₂-C₆₀ heteroarylalkyl group, a substituted or unsubstituted C₆-C₆₀ aryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), - Ge(Q₃)(Q₄)(Q₅), -B(Q₆)(Q₇), -P(=O)(Q₈)(Q₉), or -P(Q₈)(Q₉),
a41 to a43 may each independently be an integer from 0 to 20,
two or more of R₄₁ in the number of a41 may optionally be bonded together to form a C₅-C₃₀ carbocyclic group unsubstituted or substituted with at least one R_{10b} or a C₁-C₃₀ heterocyclic group unsubstituted or substituted with at least one R_{10b},
two or more of R₄₂ in the number of a42 may optionally be bonded together to form a C₅-C₃₀ carbocyclic group unsubstituted or substituted with at least one R_{10b} or a C₁-C₃₀ heterocyclic group unsubstituted or substituted with at least one R_{10b},
two or more of R₄₃ in the number of a43 may optionally be bonded together to form a C₅-C₃₀ carbocyclic group unsubstituted or substituted with at least one R_{10b} or a C₁-C₃₀ heterocyclic group unsubstituted or substituted with at least one R_{10b},
two or more of R₄₁ to R₄₉ may optionally be bonded together to form a C₅-C₃₀ carbocyclic group unsubstituted or substituted with at least one R_{10b} or a C₁-C₃₀ heterocyclic group unsubstituted or substituted with at least one R_{10b},
R_{10b} may be the same as described in connection with R₄₁,
* and *' each indicate a binding site to a neighboring atom,
a substituent of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₂-C₁₀ heterocycloalkenyl group, the substituted C₆-C₆₀ aryl group, the substituted C₇-C₆₀ aryl alkyl group, the substituted C₆-C₆₀ aryloxy group, the substituted C₆-C₆₀ arylthio group, the substituted C₁-C₆₀ heteroaryl group, the substituted C₂-C₆₀ heteroaryl alkyl group, the substituted monovalent non-aromatic condensed polycyclic group, and the substituted monovalent non-aromatic condensed heteropolycyclic group may be:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group;
a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, - CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), -Ge(Q₁₃)(Q₁₄)(Q₁₅), - B(Q₁₆)(Q₁₇), -P(=O)(Q₁₈)(Q₁₉), -P(Q₁₈)(Q₁₉), or any combination thereof;
a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with at least one of deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Ge(Q₂₃)(Q₂₄)(Q₂₅), -B(Q₂₆)(Q₂₇), - P(=O)(Q₂₈)(Q₂₉), -P(Q₂₈)(Q₂₉) or any combination thereof;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), - P(=O)(Q₃₈)(Q₃₉), or -P(Q₃₈)(Q₃₉); or
any combination thereof, and
Q₁ to Q₉, Q₁₁ to Q₁₉, Q₂₁ to Q₂₉, and Q₃₁ to Q₃₉ may each independently be hydrogen, deuterium, -F, -Cl, -Br, -I, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group which is unsubstituted or substituted with deuterium, a C₁-C₆₀ alkyl group, a C₆-C₆₀ aryl group, or any combination thereof, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group which is unsubstituted or substituted with deuterium, a C₁-C₆₀ alkyl group, a C₆-C₆₀ aryl group, or any combination thereof, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group.

In an embodiment, ring CY₄₁ to ring CY₄₃ may each independently be i) a benzene group, or ii) a polycyclic group in which two or more C₃-C₃₀ cyclic groups are condensed with each other. Here, two C₃-C₃₀ cyclic groups of the polycyclic group may be condensed with each other while sharing boron (B) or nitrogen (N).

In one or more embodiments, at least one of b41 to b43 may be 1, or at least wo of b41 to b43 may each be 1. In one or more embodiments, two of b41 to b43 may be 1, and the remaining one may be 0.

In one or more embodiments, R₄₁ to R₄₉ may each independently be:
hydrogen, deuterium, -F, -Cl, -Br, -I, a hydroxyl group, a cyano group, a nitro group, a C₁-C₂₀ alkyl group, or a C₁-C₂₀ alkoxy group;
a C₁-C₆₀ alkyl group or a C₁-C₆₀ alkoxy group, each substituted with at least one of deuterium, -F, -Cl, -Br, -I, a hydroxyl group, a cyano group, a nitro group, an amino group, a phenyl group, a biphenyl group, a naphthyl group, a fluorenyl group, a phenanthrenyl group, an anthracenyl group, a fluoranthenyl group, a triphenylenyl group, a pyrenyl group, and a chrysenyl group; or
a cyclopentyl group, a cyclohexyl group, a cycloheptyl group, a cyclopentenyl group, a cyclohexenyl group, a cycloheptenyl group, a phenyl group, a biphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a phenanthrolinyl group, a benzimidazolyl group, a benzofuranyl group, a benzothiophenyl group, a benzoisothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, or a carbazolyl group, each unsubstituted or substituted with at least one of deuterium, -F, -Cl, -Br, -I, a hydroxyl group, a cyano group, a nitro group, an amino group, a C₁-C₆₀ alkyl group, a C₁-C₆₀ alkoxy group, a cyclopentyl group, a cyclohexyl group, a cycloheptyl group, a cyclopentenyl group, a cyclohexenyl group, a cycloheptenyl group, a phenyl group, a biphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a phenanthrolinyl group, a benzimidazolyl group, a benzofuranyl group, a benzothiophenyl group, a benzoisothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, and a carbazolyl group.

In one or more embodiments, the polycyclic compound represented by Formula 4 may be a polycyclic compound represented by one of Formulae 4-1 to 4-9: 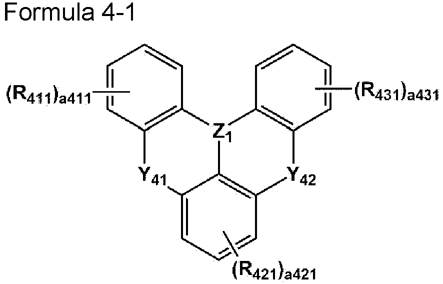 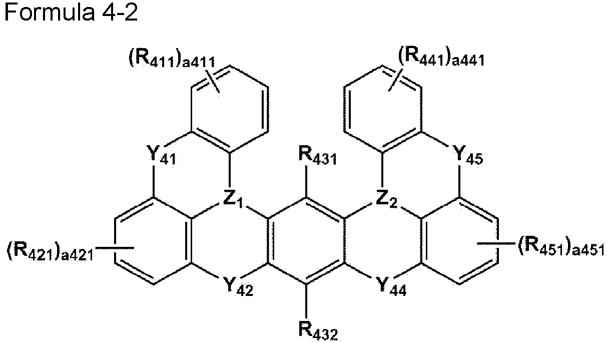 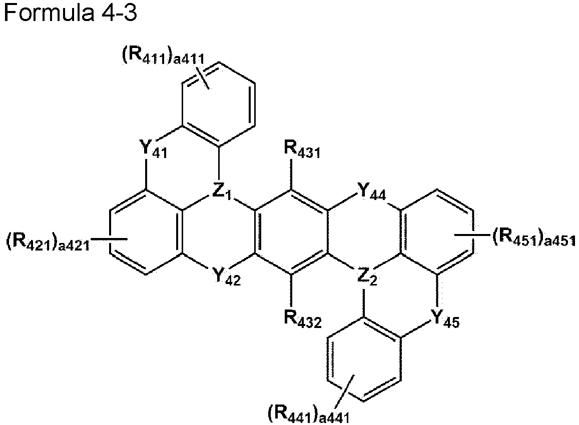 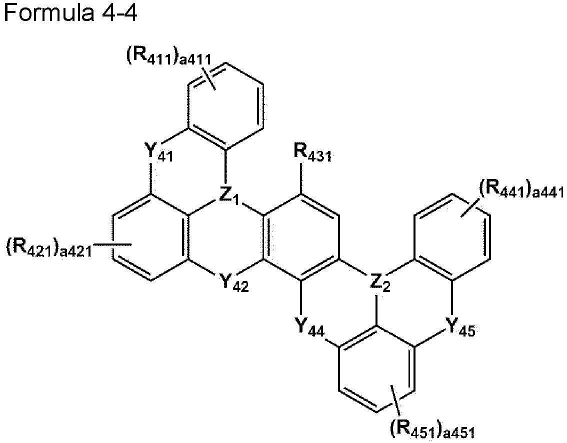 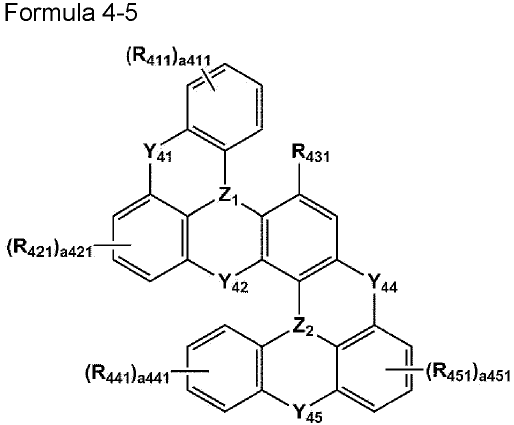 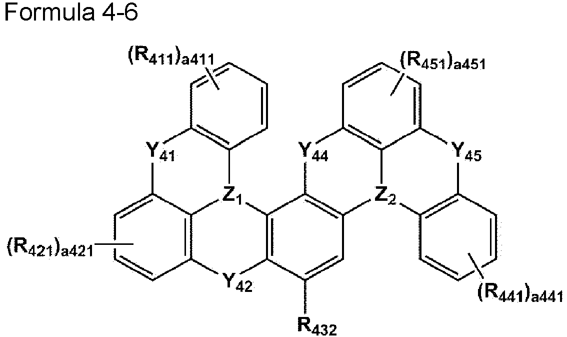 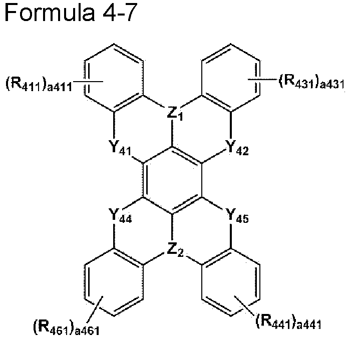 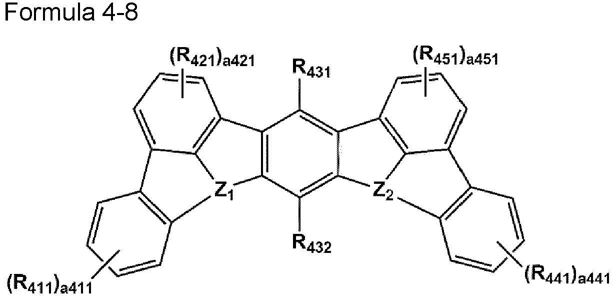 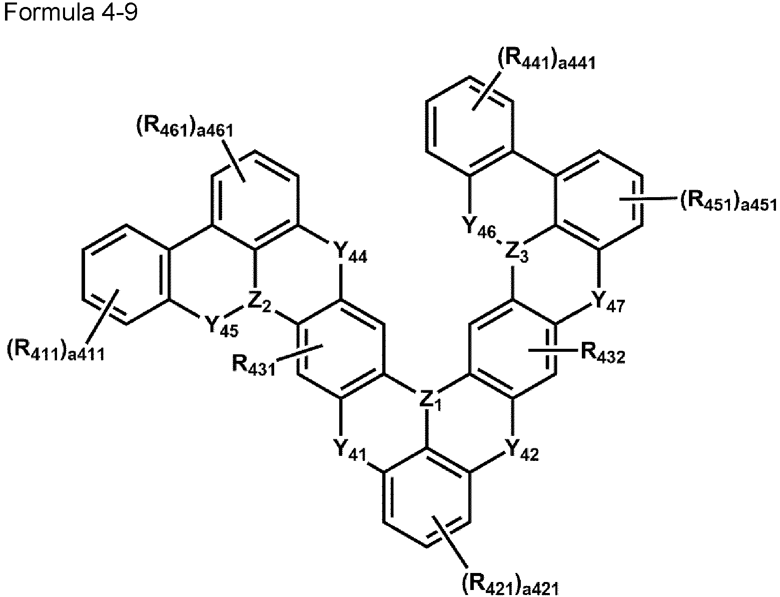 wherein, in Formulae 4-1 to 4-9,
Z₁ to Z₃ may each be the same as described herein in connection with Z of Formula 4,
Y₄₁ and Y₄₂ may be the same as described herein,
Y₄₄ to Y₄₇ may each be the same as described in connection with Y₄₁ and Y₄₂,
R₄₁₁ may be the same as described in connection with R₄₁, R₄₂₁ may be the same as described in connection with R₄₂, R₄₃₁ and R₄₃₂ may each be the same as described in connection with R₄₃, R₄₄₁ may be the same as described in connection with R₄₁, R₄₅₁ may be the same as described in connection with R₄₂, and R₄₆₁ may be the same as described in connection with R₄₃,
a411 may be an integer from 0 to 4,
a421 may be an integer from 0 to 3,
a431 may be an integer from 0 to 4,
a441 may be an integer from 0 to 4,
a451 may be an integer from 0 to 3, and
a461 may be an integer from 0 to 3.

Specific examples of polycyclic compound of Formula 4

The polycyclic compound represented by Formula 4 may be selected from Compounds D1 to D30:

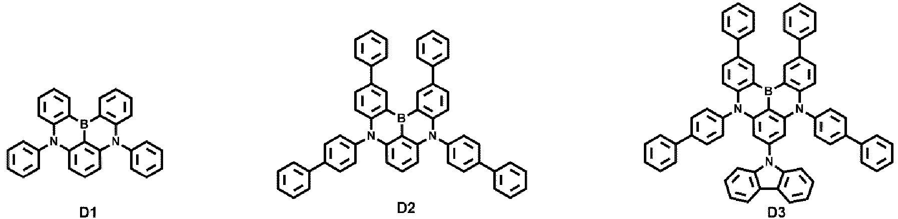

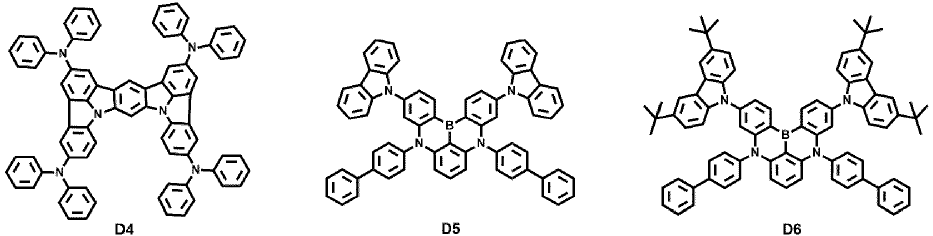

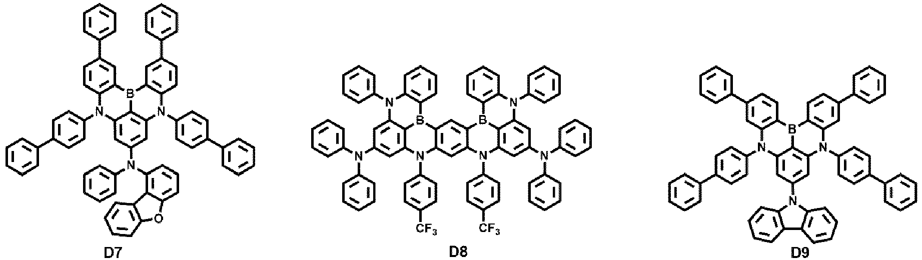

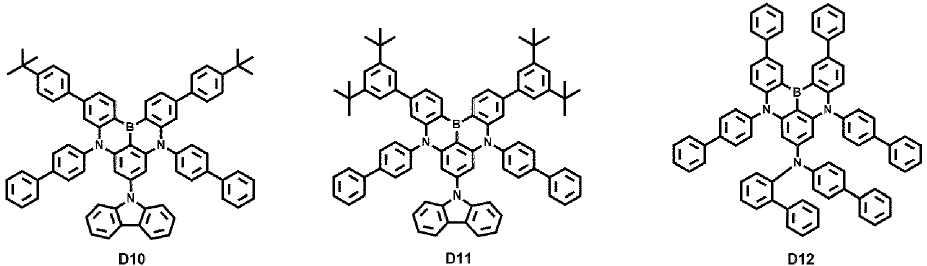

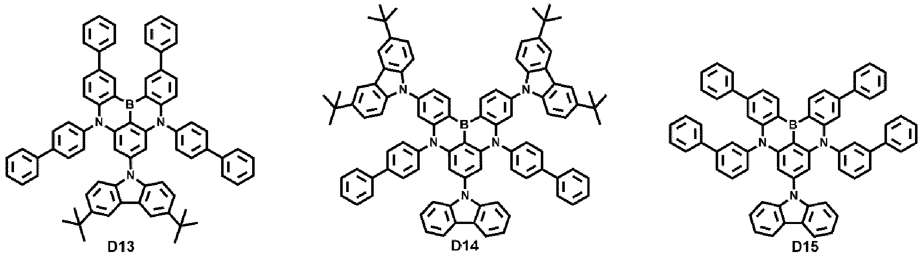

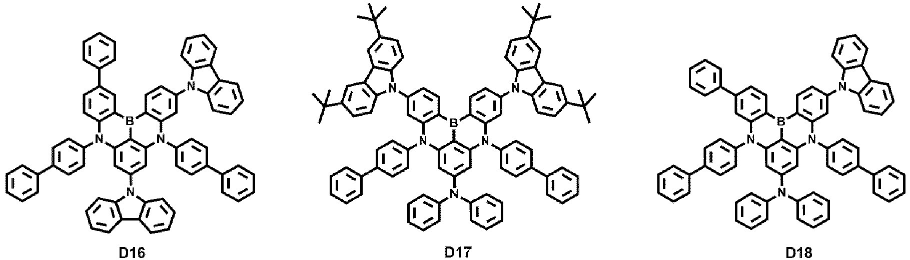

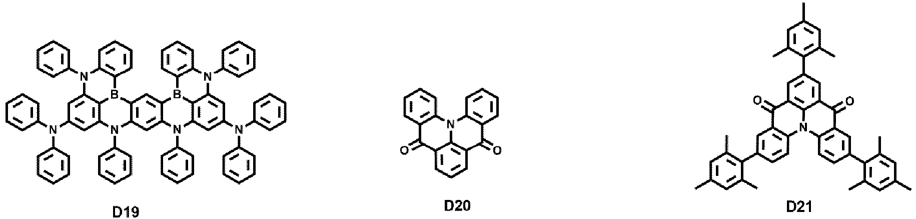

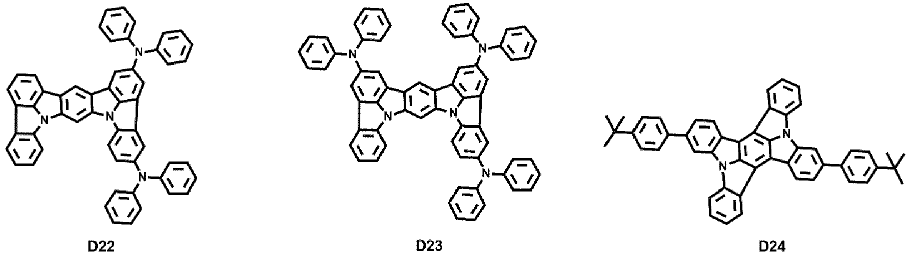

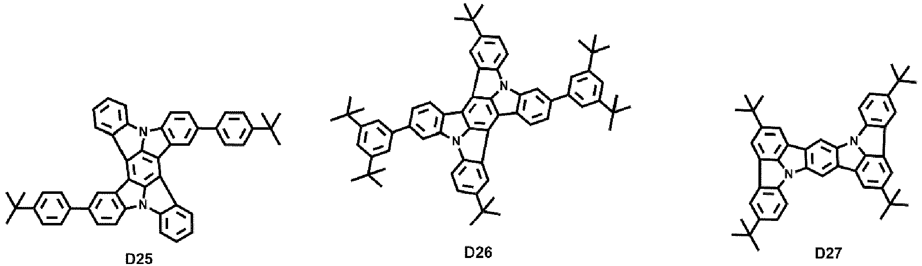

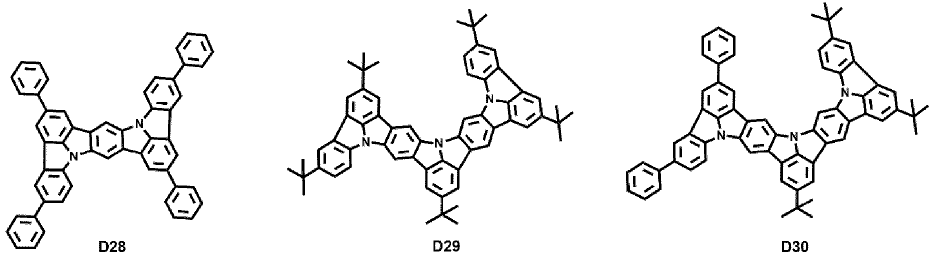

### Description of FIGURE

The FIGURE is a schematic cross-sectional view of an organic light-emitting device 10 according to an embodiment. Hereinafter, the structure and manufacturing method of the organic light-emitting device 10 according to an embodiment will be described in connection with the FIGURE.

In the FIGURE, an organic light-emitting device 10 includes a first electrode 11, a second electrode 19 facing the first electrode 11, and an interlayer 10A arranged between the first electrode 11 and the second electrode 19.

The interlayer 10A includes an emission layer 15, and may further include a hole transport region 12 arranged between the first electrode 11 and the emission layer 15, and an electron transport region 17 arranged between the emission layer 15 and the second electrode 19.

A substrate may be additionally arranged under the first electrode 11 or on the second electrode 19. For use as the substrate, a substrate generally used in organic light-emitting devices, e.g., a glass substrate or a transparent plastic substrate, having excellent mechanical strength, thermal stability, transparency, surface smoothness, ease of handling, and water repellency may be used.

### First electrode 11

The first electrode 11 may be formed by, for example, depositing or sputtering, onto the substrate, a material for forming the first electrode 11. The first electrode 11 may be an anode. The material for forming the first electrode 11 may include materials with a high work function to facilitate hole injection.

The first electrode 11 may be a reflective electrode, a semi-transmissive electrode, or a transmissive electrode. In an embodiment, when the first electrode 11 is a transmissive electrode, the material for forming the first electrode 11 may include indium tin oxide ("ITO"), indium zinc oxide ("IZO"), tin oxide (SnO₂), zinc oxide (ZnO), or any combination thereof. In one or more embodiments, when the first electrode 11 is a semi-transmissive electrode or a reflective electrode, the material for forming the first electrode 11 may include magnesium (Mg), silver (Ag), aluminum (Al), aluminum-lithium (Al-Li), calcium (Ca), magnesium-indium (Mg-In), magnesium-silver (Mg-Ag), or any combination thereof.

The first electrode 11 may have a single-layer structure or a multi-layer structure including a plurality of layers.

### Emission layer 15

A thickness of the emission layer 15 may be in a range of about 100 angstroms (Å) to about 1,000 Å, for example, about 200 Å to about 600 Å. When the thickness of the emission layer 15 is within these ranges, excellent luminescence characteristics may be obtained without a substantial increase in driving voltage.

In an embodiment, the emission layer 15 may include the aforementioned condensed cyclic compound. The emission layer 15 may follow the first embodiment or the second embodiment.

The emission layer 15 may further include, in addition to the condensed cyclic compound, the sensitizer, and the emitter, the host.

### Hole transport region 12

The hole transport region 12 may be arranged between the first electrode 11 and the emission layer 15 of the organic light-emitting device 10.

The hole transport region 12 may have a single-layer structure or a multi-layer structure.

For example, the hole transport region 12 may have a hole injection layer structure, a hole transport layer structure, a hole injection layer/hole transport layer structure, a hole injection layer/first hole transport layer/second hole transport layer structure, a hole injection layer/first hole transport layer/second hole transport layer/electron blocking layer structure, a hole transport layer/organic layer structure, a hole injection layer/hole transport layer/organic layer structure, a hole transport layer/electron blocking layer structure, or a hole injection layer/hole transport layer/electron blocking layer structure.

The hole transport region 12 may include any compound having hole-transporting properties.

For example, the hole transport region 12 may include an amine-based compound.

In an embodiment, the hole transport region 12 may include m-MTDATA, TDATA, 2-TNATA, NPB, β-NPB, TPD, spiro-TPD, spiro-NPB, methylated-NPB, TAPC, HMTPD, 4,4',4"-tris(N-carbazolyl)triphenylam ine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor-sulfonic acid (PANI/CSA), polyaniline/poly(4-styrene sulfonate) (PANI/PSS), a compound represented by one of Formulae 201 to 205, or any combination thereof: 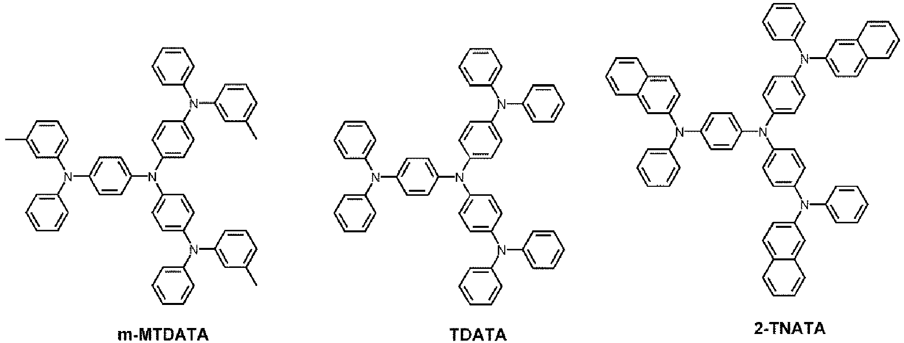 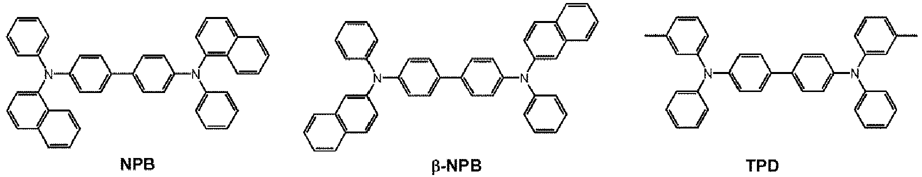 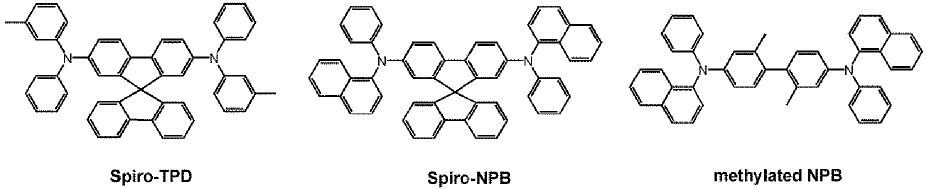 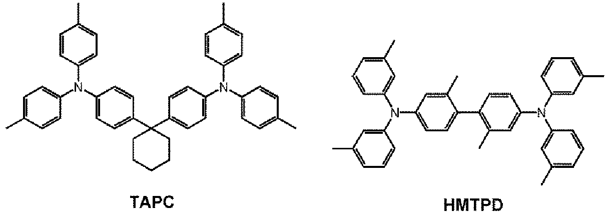 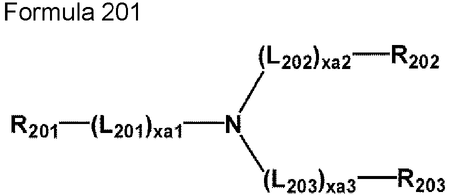 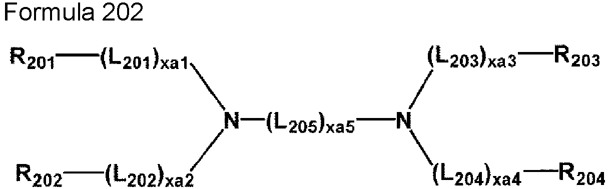 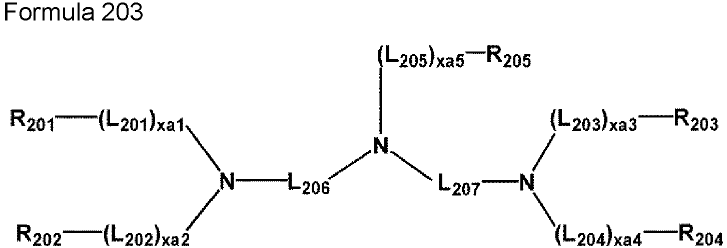 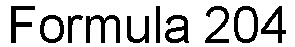 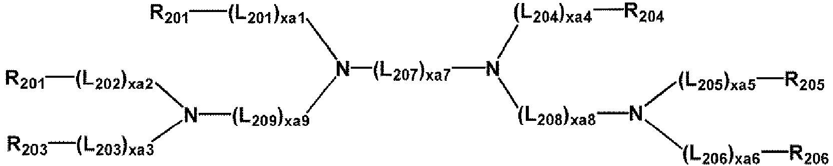 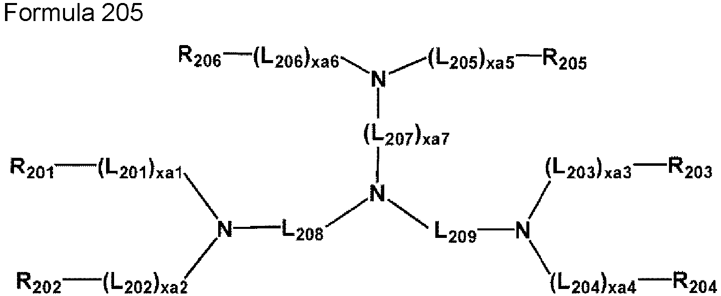 wherein, in Formulae 201 to 205,
L₂₀₁ to L₂₀₉ may each independently be *-O-*', *-S-*', a substituted or unsubstituted C₅-C₆₀ carbocyclic group, or a substituted or unsubstituted C₁-C₆₀ heterocyclic group,
xa1 to xa9 may each independently be an integer from 0 to 5, and
R₂₀₁ to R₂₀₆ may each independently be a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, or a substituted or unsubstituted monovalent non-aromatic condensed heteropolycyclic group, wherein neighboring two groups of R₂₀₁ to R₂₀₆ may optionally be linked to each other via a single bond, a dimethyl-methylene group, or a diphenyl-methylene group.

For example, L₂₀₁ to L₂₀₉ may each independently be a benzene group, a heptalene group, an indene group, a naphthalene group, an azulene group, a an indacene group, an acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentaphene group, a rubicene group, a coronene group, an ovalene group, a pyrrole group, an isoindole group, an indole group, a furan group, a thiophene group, a benzofuran group, a benzothiophene group, a benzocarbazole group, a dibenzocarbazole group, a dibenzofuran group, a dibenzothiophene group, a dibenzothiophene sulfone group, a carbazole group, a dibenzosilole group, an indenocarbazole group, an indolocarbazole group, a benzofurocarbazole group, a benzothienocarbazole group, or a triindolobenzene group, each unsubstituted or substituted with deuterium, a C₁-C₁₀ alkyl group, a C₁-C₁₀ alkoxy group, a phenyl group, a naphthyl group, a fluorenyl group, a carbazolyl group, a dibenzofuranyl group, a dibenzothiophenyl group, a triphenylenyl group, a biphenyl group, a terphenyl group, a quaterphenyl group, -Si(Q₁₁)(Q₁₂)(Q₁₃), or any combination thereof,
xa1 to xa9 may each independently be 0, 1, or 2,
R₂₀₁ to R₂₀₆ may each independently be a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an indeno carbazolyl group, an indolocarbazolyl group, a benzofurocarbazolyl group, or a benzothienocarbazolyl group, each unsubstituted or substituted with deuterium, -F, -Cl, -Br, -I, a hydroxyl group, a cyano group, a nitro group, an amidino group, a hydrazine group, a hydrazone group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, - Si(Q₃₁)(Q₃₂)(Q₃₃), -N(Q₃₁)(Q₃₂), or any combination thereof,
Q₁₁ to Q₁₃ and Q₃₁ to Q₃₃ may each independently be a C₁-C₁₀ alkyl group, a C₁-C₁₀ alkoxy group, a phenyl group, a biphenyl group, a terphenyl group, or a naphthyl group.

In one or more embodiments, the hole transport region 12 may include a carbazole-containing amine-based compound.

In one or more embodiments, the hole transport region 12 may include a carbazole-containing amine-based compound and a carbazole-free amine-based compound.

The carbazole-containing amine-based compound may include, for example, compounds represented by Formula 201 including a carbazole group and further including at least one of a dibenzofuran group, a dibenzothiophene group, a fluorene group, a spiro-bifluorene group, an indenocarbazole group, an indolocarbazole group, a benzofurocarbazole group, and a benzothienocarbazole group.

The carbazole-free amine-based compound may include, for example, compounds represented by Formula 201 not including a carbazole group and including at least one of a dibenzofuran group, a dibenzothiophene group, a fluorene group, a spiro-bifluorene group, an indenocarbazole group, an indolocarbazole group, a benzofurocarbazole group, and a benzothienocarbazole group.

In one or more embodiments, the hole transport region 12 may include a compound represented by Formula 201, a compound represented by Formula 202, or any combination thereof.

In one or more embodiments, the hole transport region 12 may include a compound represented by Formula 201-1, 202-1, or 201-2, or any combination thereof: 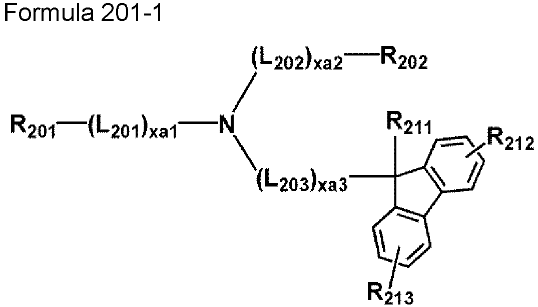 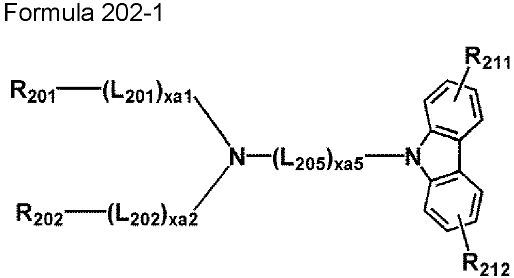 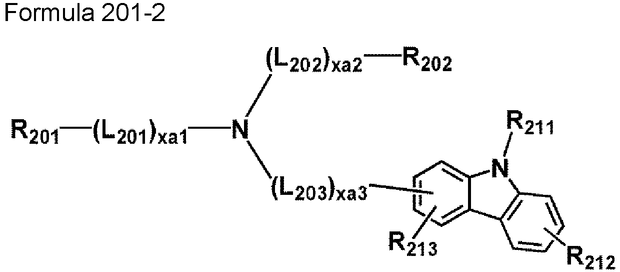 wherein, in Formulae 201-1, 202-1, and 201-2, L₂₀₁ to L₂₀₃, L₂₀₅, xa1 to xa3, xa5, R₂₀₁ and R₂₀₂ may each be the same as described herein in connect with Formulae 201 to 205, and R₂₁₁ to R₂₁₃ may each independently be hydrogen, deuterium, -F, -Cl, -Br, -I, a hydroxyl group, a cyano group, a nitro group, an amidino group, a hydrazine group, a hydrazone group, a C₁-C₂₀ alkyl group, a C₁-C₂₀ alkoxy group, a phenyl group, a biphenyl group, a terphenyl group, a phenyl group substituted with a C₁-C₁₀ alkyl group, a phenyl group substituted with -F, a naphthyl group, a fluorenyl group, a spiro-bifluorenyl group, a dimethylfluorenyl group, a diphenylfluorenyl group, a triphenylenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, or a pyridinyl group.

In an embodiment, the hole transport region 12 may include one of Compounds HT1 to HT39 or any combination thereof:

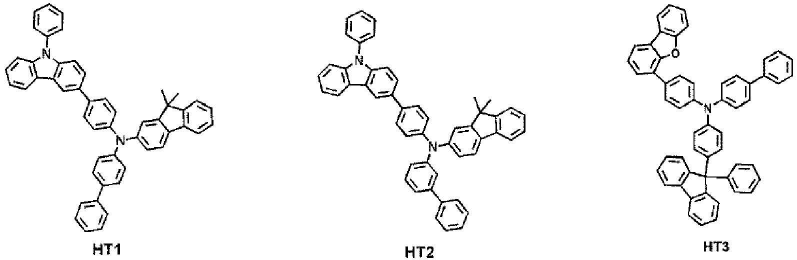

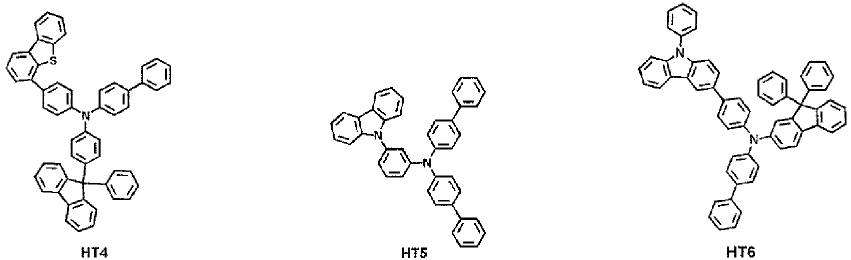

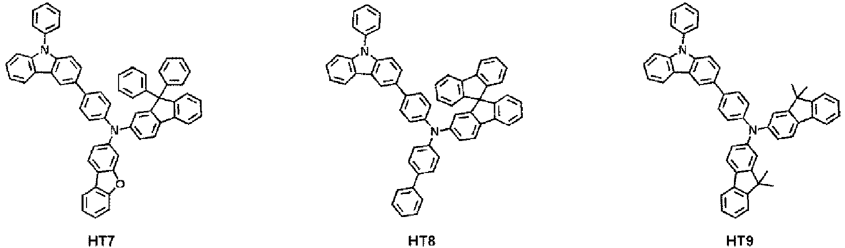

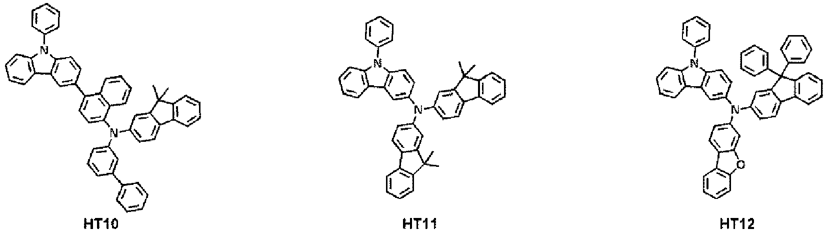

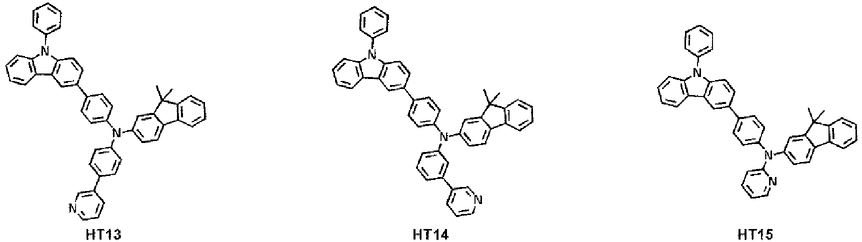

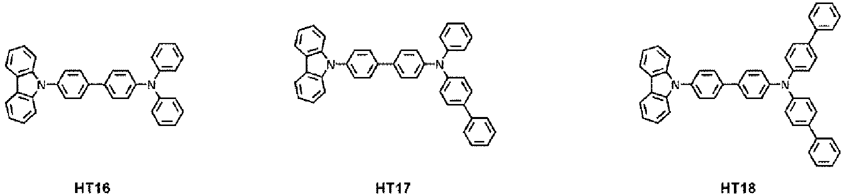

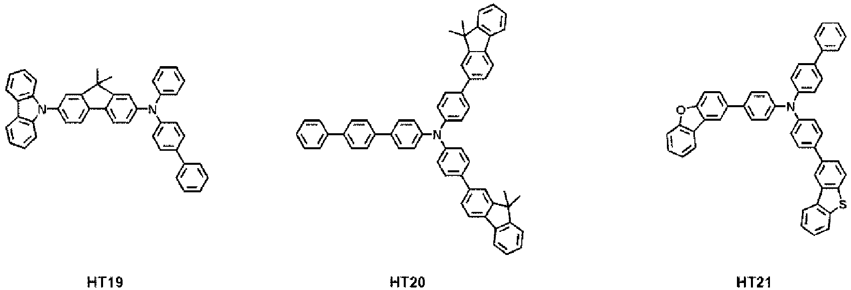

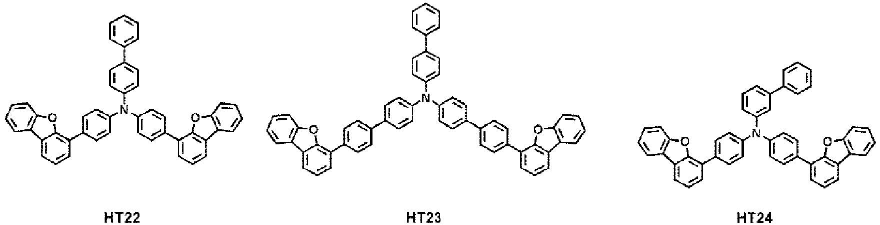

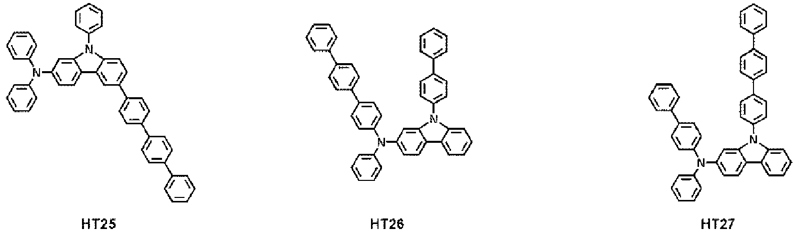

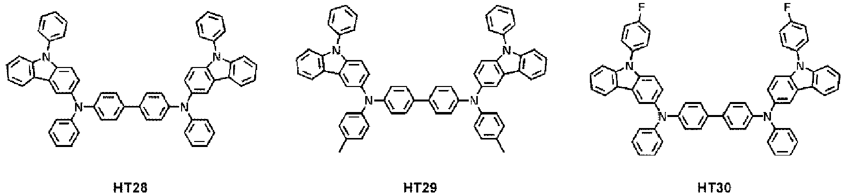

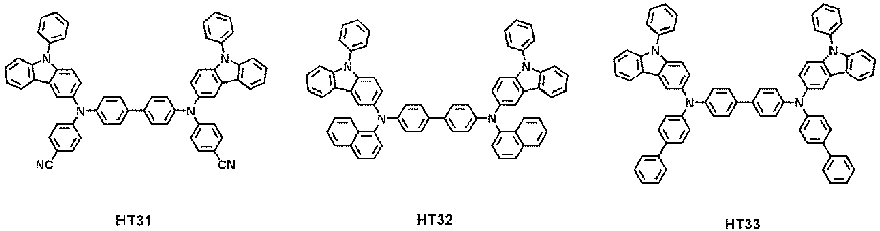

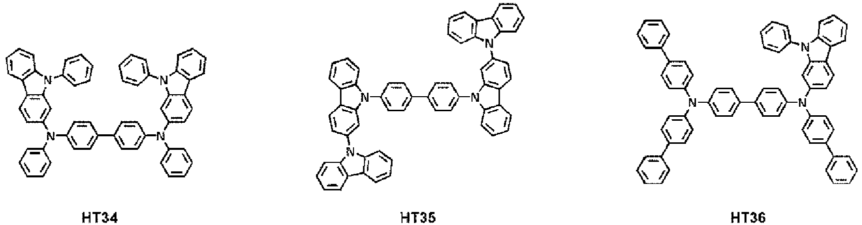

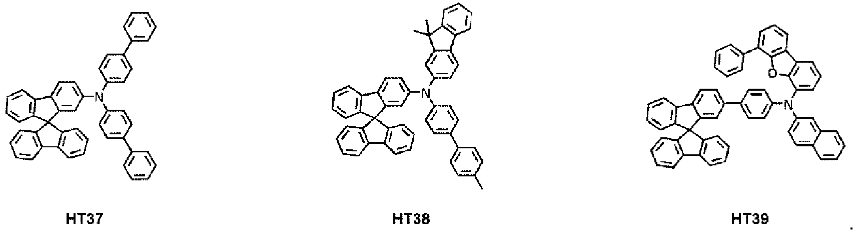

In one or more embodiments, the hole transport region 12 of the organic light-emitting device 10 may further include a p-dopant. When the hole transport region 12 further includes a p-dopant, the hole transport region 12 may have a matrix (for example, at least one of the compounds represented by Formulae 201 to 205) and a p-dopant included in the matrix. The p-dopant may be uniformly or non-uniformly doped in the hole transport region 12.

In an embodiment, the LUMO energy level of the p-dopant may be -3.5 electron volts (eV) or less.

The p-dopant may include a quinone derivative, a metal oxide, a cyano group-containing compound, or any combination thereof.

For example, the p-dopant may include:
a quinone derivative, such as tetracyanoquinodimethane (TCNQ),2,3,5,6-tetrafluoro-7,7,8,8-tetracyanobenzoquinodimethane (F4-TCNQ), and F6-TCNNQ;
a metal oxide, such as tungsten oxide or molybdenum oxide;
1,4,5,8,9,12-hexaazatriphenylene-hexacarbonitrile (HAT-CN);
a compound represented by Formula 221; or
any combination thereof: 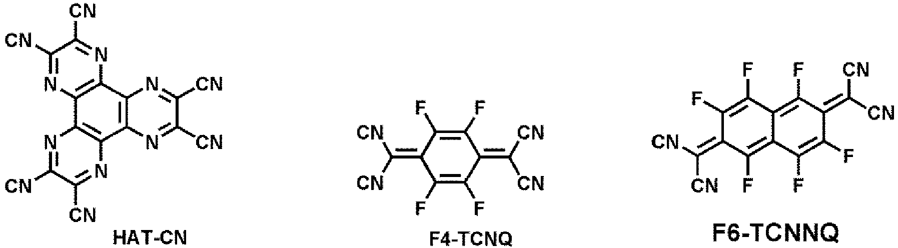 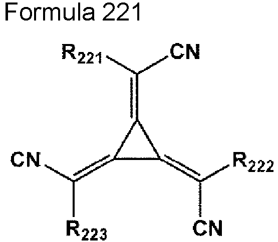
wherein, in Formula 221,
R₂₂₁ to R₂₂₃ may each independently be a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₁-C₆₀ heteroaryl group, a substituted or unsubstituted monovalent non-aromatic condensed polycyclic group, or a substituted or unsubstituted monovalent non-aromatic condensed heteropolycyclic group, wherein at least one substituent of R₂₂₁ to R₂₂₃ may be: a cyano group; -F; -Cl; -Br; -I; a C₁-C₂₀ alkyl group substituted with -F; a C₁-C₂₀ alkyl group substituted with -Cl; a C₁-C₂₀ alkyl group substituted with -Br; a C₁-C₂₀ alkyl group substituted with -I; or any combination thereof.

The compound represented by Formula 221 may include, for example, Compound HT-D2:

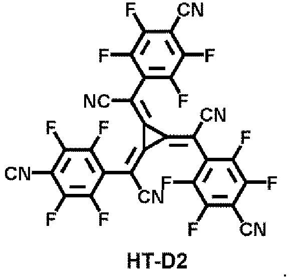

The hole transport region 12 may have a thickness in a range from about 100 Å to about 10,000 Å, for example, about 400 Å to about 2,000 Å, and the emission layer 15 may have a thickness in a range of about 100 Å to about 3,000 Å, for example, about 300 Å to about 1,000 Å. When the thickness of each of the hole transport region 12 and the emission layer 15 is within these ranges, satisfactory hole transportation characteristics and/or luminescence characteristics may be obtained without a substantial increase in driving voltage.

The hole transport region 12 may further include a buffer layer.

The buffer layer may compensate for an optical resonance distance according to a wavelength of light emitted from the emission layer 15, and thus, efficiency of a formed organic light-emitting device may be improved.

The hole transport region 12 may further include an electron blocking layer. The electron blocking layer may include a known material, for example, mCP or DBFPO:

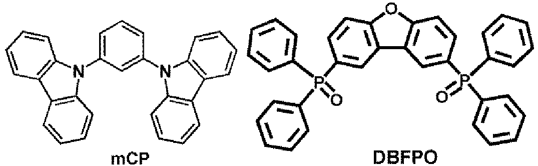

### Electron transport region 17

The electron transport region 17 is arranged between the emission layer 15 and the second electrode 19 of the organic light-emitting device 10.

The electron transport region 17 may have a single-layer structure or a multi-layer structure.

For example, the electron transport region 17 may have an electron transport layer, an electron transport layer/electron injection layer structure, a buffer layer/electron transport layer structure, hole blocking layer/electron transport layer structure, a buffer layer/electron transport layer/electron injection layer structure, or a hole blocking layer/electron transport layer/electron injection layer structure. The electron transport region 17 may further include an electron control layer.

The electron transport region 17 may include known electron-transporting materials.

The electron transport region 17 (for example, a buffer layer, a hole blocking layer, an electron control layer, or an electron transport layer in the electron transport region) may include a metal-free compound containing at least one π electron-deficient nitrogen-containing C₁-C₆₀ cyclic group. The π electron-deficient nitrogen-containing C₁-C₆₀ cyclic group is the same as described herein.

For example, the electron transport region 17 may include a compound represented by Formula 601:

Formula 601 [Ar₆₀₁]ₓₑ₁₁-[(L₆₀₁)ₓₑ₁-R₆₀₁]ₓₑ₂₁.

wherein, in Formula 601,
Ar₆₀₁ and L₆₀₁ may each independently be a C₅-C₆₀ carbocyclic group unsubstituted or substituted with at least one R₆₀₁ₐ or a C₁-C₆₀ heterocyclic group unsubstituted or substituted with at least one R₆₀₁ₐ,
xe11 may be 1, 2, or 3,
xe1 may be an integer from 0 to 5,
R₆₀₁ₐ and R₆₀₁ may each independently be a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -Si(Q₆₀₁)(Q₆₀₂)(Q₆₀₃), -C(=O)(Q₆₀₁), -S(=O)₂(Q₆₀₁), or - P(=O)(Q₆₀₁)(Q₆₀₂),
Q₆₀₁ to Q₆₀₃ may each independently be a C₁-C₁₀ alkyl group, a C₁-C₁₀ alkoxy group, a phenyl group, a biphenyl group, a terphenyl group, or a naphthyl group, and
xe21 may be an integer from 1 to 5.

In an embodiment, at least one of Ar₆₀₁ in the number of xe11 and R₆₀₁ in the number of xe21 may include the π electron-deficient nitrogen-containing C₁-C₆₀ cyclic group.

In an embodiment, Ar₆₀₁ and L₆₀₁ in Formula 601 may each independently be a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, or an azacarbazole group, each unsubstituted or substituted with deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, -Si(Q₃₁)(Q₃₂)(Q₃₃), -S(=O)₂(Q₃₁), -P(=O)(Q₃₁)(Q₃₂), or any combination thereof, and
Q₃₁ to Q₃₃ may each independently be a C₁-C₁₀ alkyl group, a C₁-C₁₀ alkoxy group, a phenyl group, a biphenyl group, a terphenyl group, or a naphthyl group.

When xe11 in Formula 601 is 2 or more, two or more of Ar₆₀₁ may be linked to each other via a single bond.

In one or more embodiments, Ar₆₀₁ in Formula 601 may be an anthracene group.

In one or more embodiments, the compound represented by Formula 601 may be represented by Formula 601-1: 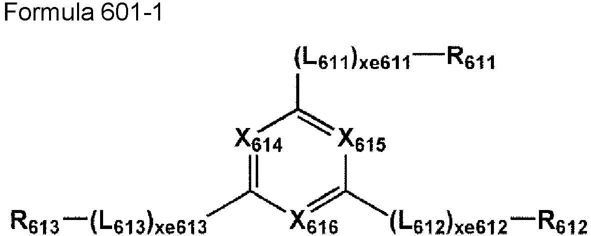 wherein, in Formula 601-1,
X₆₁₄ may be N or C(R₆₁₄), X₆₁₅ may be N or C(R₆₁₅), X₆₁₆ may be N or C(R₆₁₆), and at least one of X₆₁₄ to X₆₁₆ may be N,
L₆₁₁ to L₆₁₃ may each independently be the same as described in connection with L₆₀₁,
xe611 to xe613 may each independently be the same as described in connection with xe1,
R₆₁₁ to R₆₁₃ may each independently be the same as described in connection with R₆₀₁, and
R₆₁₄ to R₆₁₆ may each independently be hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, or a naphthyl group.

In one or more embodiments, xe1 and xe611 to xe613 in Formulae 601 and 601-1 may each independently be 0, 1, or 2.

In one or more embodiments, R₆₀₁ and R₆₁₁ to R₆₁₃ in Formulae 601 and 601-1 may each independently be a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, or an azacarbazolyl group, each unsubstituted or substituted with deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group, or any combination thereof; or
-S(=O)₂(Q₆₀₁), or -P(=O)(Q₆₀₁)(Q₆₀₂), and
Q₆₀₁ and Q₆₀₂ may each be the same as described herein.

The electron transport region 17 may include one of Compounds ET1 to ET36 or any combination thereof:

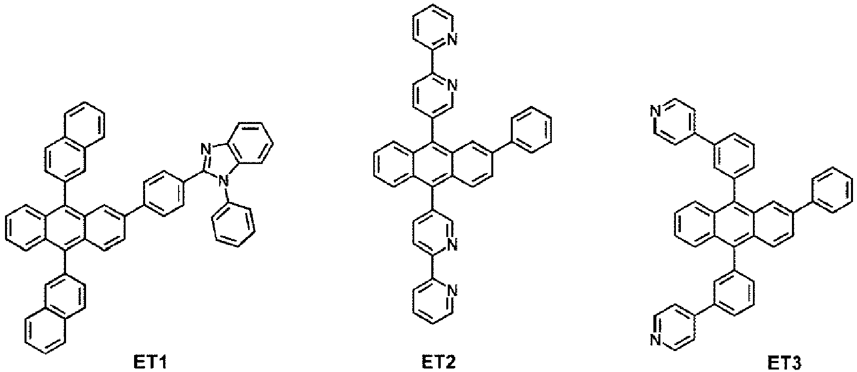

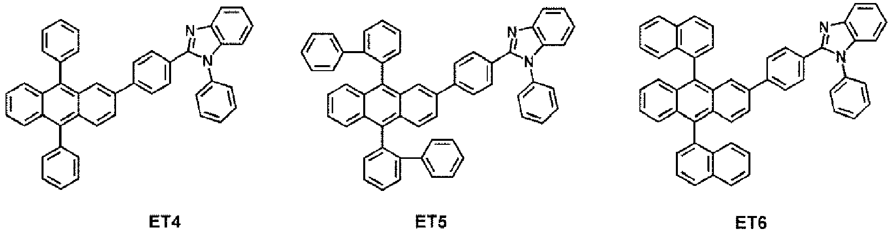

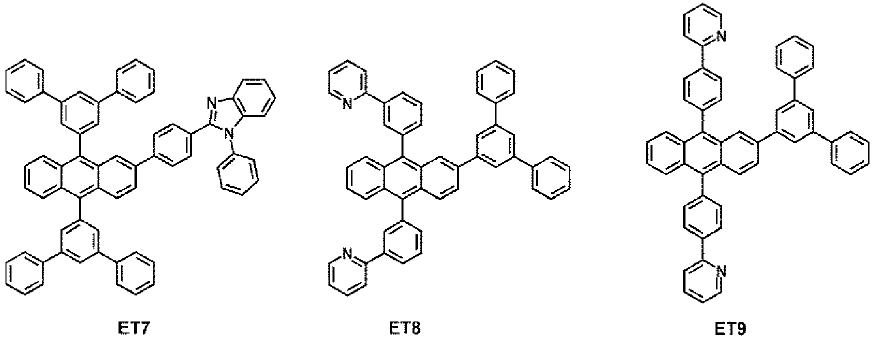

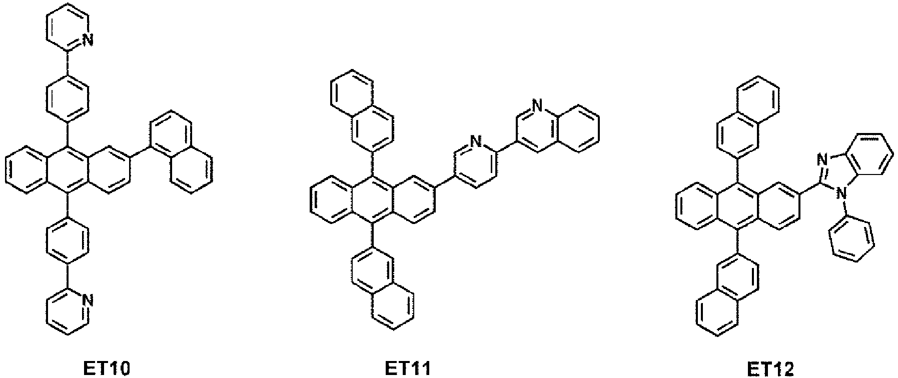

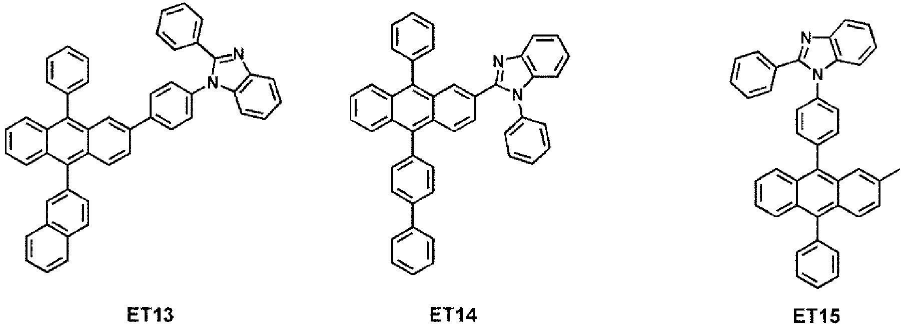

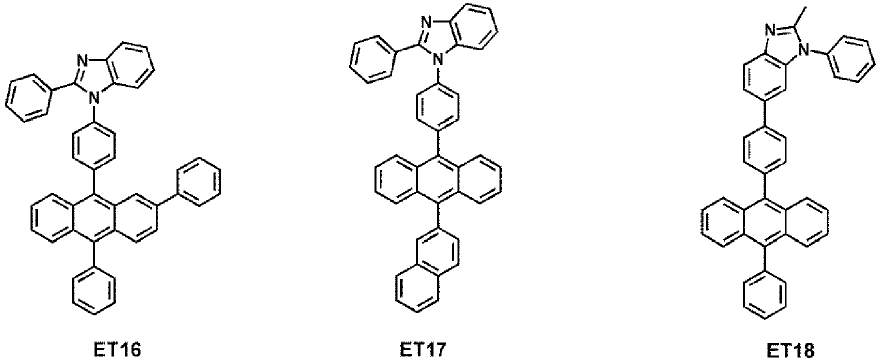

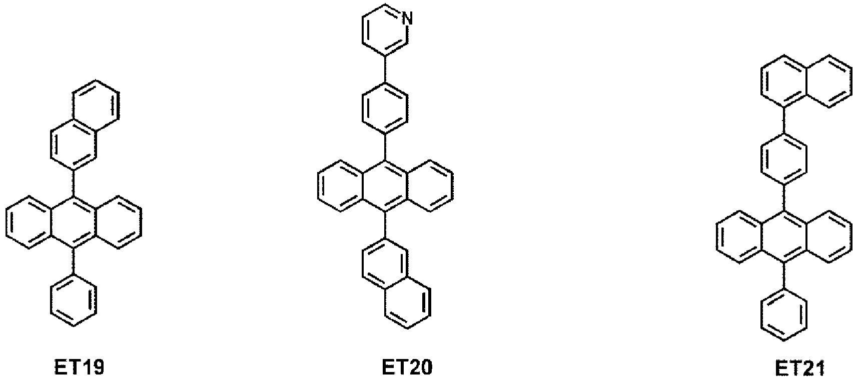

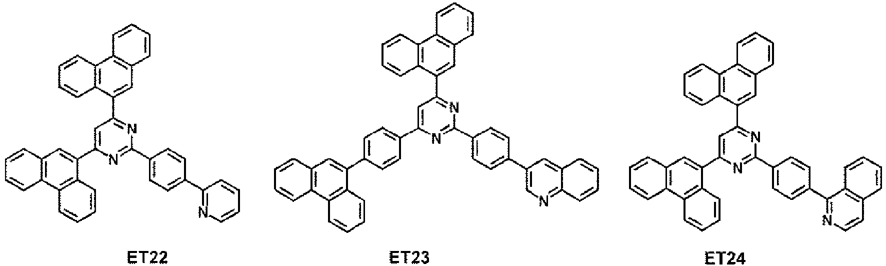

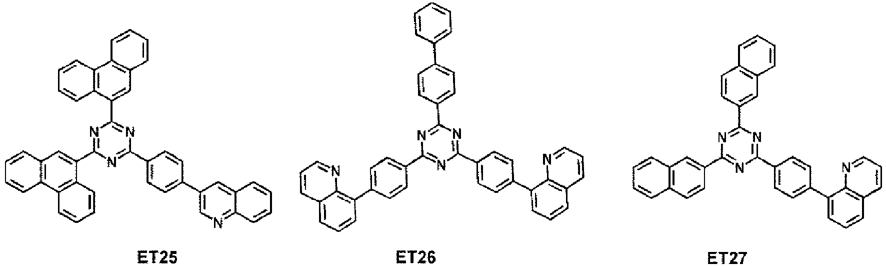

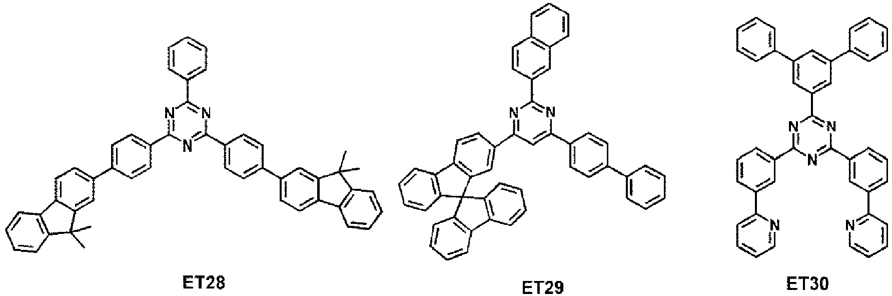

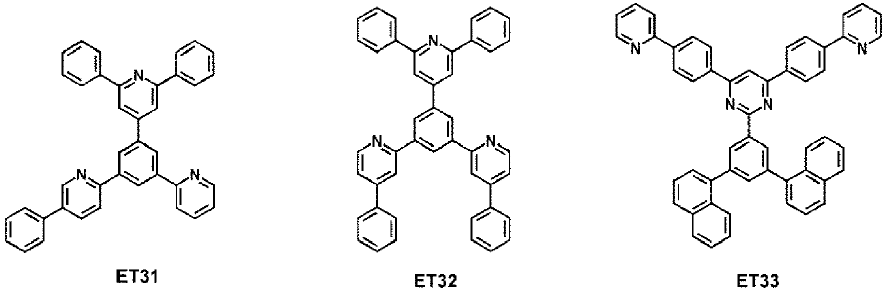

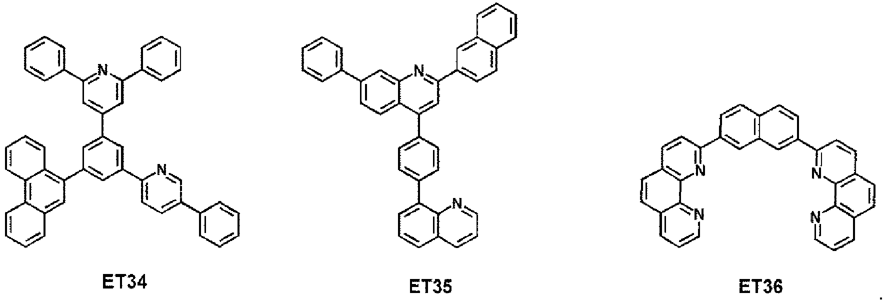

In one or more embodiments, the electron transport region 17 may include 2,9-dimethyl-4,7-diphenyl-1,10-phenanthroline (BCP), 4,7-diphenyl-1,10-phenanthroline (Bphen), Alq₃, BAlq, 3-(biphenyl-4-yl)-5-(4-tert-butylphenyl)-4-phenyl-4H-1,2,4-triazole (TAZ), NTAZ, DBFPO, or any combination thereof. For example, when the electron transport region 17 includes a hole blocking layer, the hole blocking layer may include BCP or Bphen:

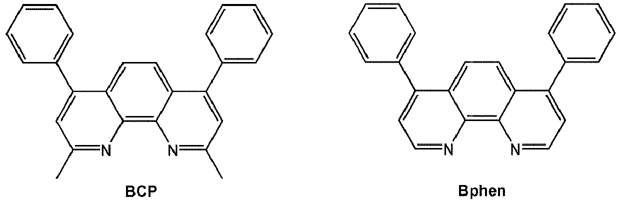

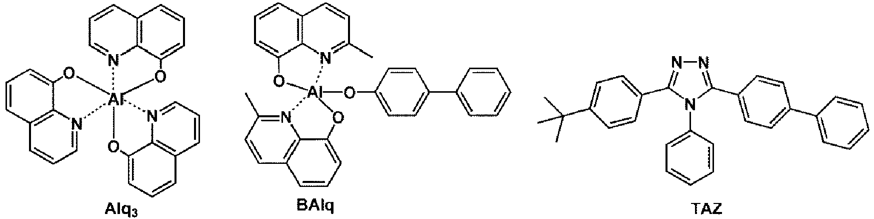

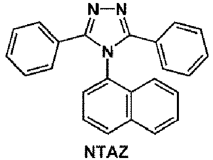

Thicknesses of the buffer layer, the hole blocking layer, and the electron control layer may each independently be in a range of about 20 Å to about 1,000 Å, for example, about 30 Å to about 300 Å. When the thicknesses of the buffer layer, the hole blocking layer, and the electron control layer are within these ranges, excellent hole blocking characteristics or excellent electron control characteristics may be obtained without a substantial increase in driving voltage.

A thickness of the electron transport layer may be in a range of about 100 Å to about 1,000 Å, for example, about 150 Å to about 500 Å. When the thickness of the electron transport layer is within these ranges, satisfactory electron transporting characteristics may be obtained without a substantial increase in driving voltage.

The electron transport region 17 (for example, the electron transport layer in the electron transport region 17) may further include, in addition to the aforementioned materials, a metal-containing material.

The metal-containing material may include an alkali metal complex, an alkaline earth metal complex, or any combination thereof. A metal ion of the alkali metal complex may include a Li ion, a Na ion, a K ion, a Rb ion, a Cs ion, or any combination thereof, and a metal ion of the alkaline earth metal complex may include a Be ion, a Mg ion, a Ca ion, a Sr ion, a Ba ion, or any combination thereof. A ligand coordinated with the metal ion of the alkali metal complex or the alkaline earth-metal complex may include a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxydiphenyloxadiazole, a hydroxydiphenylthiadiazole, a hydroxyphenylpyridine, a hydroxyphenylbenzimidazole, a hydroxyphenylbenzothiazole, a bipyridine, a phenanthroline, a cyclopentadiene, or any combination thereof.

For example, the metal-containing material may include a Li complex. The Li complex may include, for example, Compound ET-D1 (LiQ) or ET-D2:

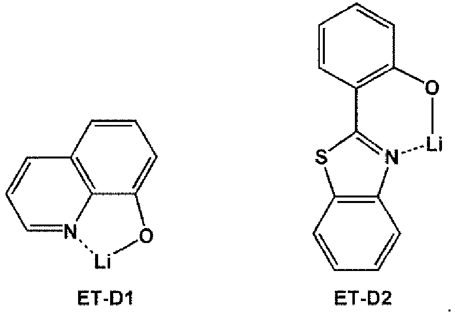

The electron transport region 17 may include an electron injection layer that facilitates the injection of electrons from the second electrode 19. The electron injection layer may directly contact the second electrode 19.

The electron injection layer may have i) a single-layer structure consisting of a single layer including a single material, ii) a single-layer structure consisting of a single layer including multiple materials that are different from each other, or iii) a multi-layer structure consisting of multiple layers including multiple materials that are different from each other.

The electron injection layer may include an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof.

The alkali metal may include Li, Na, K, Rb, Cs, or any combination thereof. In an embodiment, the alkali metal may be Li, Na, or Cs. In one or more embodiments, the alkali metal may be Li or Cs.

The alkaline earth metal may include Mg, Ca, Sr, Ba, or any combination thereof.

The rare earth metal may include Sc, Y, Ce, Tb, Yb, Gd, or any combination thereof.

The alkali metal compound, the alkaline earth metal compound, and the rare earth metal compound may include oxides and halides (for example, fluorides, chlorides, bromides, or iodides) of the alkali metal, the alkaline earth metal, and the rare earth metal, or any combination thereof.

The alkali metal compound may include: one of alkali metal oxides such as Li₂O, Cs₂O, K₂O, and the like; one of alkali metal halides such as LiF, NaF, CsF, KF, Lil, Nal, Csl, KI, and the like; or any combination thereof. In an embodiment, the alkali metal compound may include LiF, Li₂O, NaF, Lil, Nal, Csl, KI, or any combination thereof.

The alkaline earth-metal compound may include one of alkaline earth-metal compounds, such as BaO, SrO, CaO, BaₓSr₁₋ₓO (wherein 0<x<1), or BaₓCa₁₋ₓO (wherein 0<x<1), or any combination thereof. In an embodiment, the alkaline earth metal compound may include BaO, SrO, CaO, or any combination thereof.

The rare earth metal compound may include YbF₃, ScF₃, ScO₃, Y₂O₃, Ce₂O₃, GdF₃, TbF₃, or any combination thereof. In an embodiment, the rare earth metal compound may include YbF₃, ScF₃, TbF₃, YbI₃, ScI₃, TbI₃, or any combination thereof.

The alkali metal complex, the alkaline earth metal complex, and the rare earth metal complex may include an ion of alkali metal, alkaline earth metal, and rare earth metal as described above, and a ligand coordinated with a metal ion of the alkali metal complex, the alkaline earth metal complex, or the rare earth metal complex may include hydroxy quinoline, hydroxy isoquinoline, hydroxy benzoquinoline, hydroxy acridine, hydroxy phenanthridine, hydroxy phenyloxazole, hydroxy phenylthiazole, hydroxy diphenyloxadiazole, hydroxy diphenylthiadiazole, hydroxy phenylpyridine, hydroxy phenylbenzimidazole, hydroxy phenylbenzothiazole, bipyridine, phenanthroline, cyclopentadiene, or any combination thereof.

The electron injection layer may consist of an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof, as described above. In one or more embodiments, the electron injection layer may further include an organic material. When the electron injection layer further includes an organic material, an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combination thereof may be homogeneously or non-homogeneously dispersed in a matrix including the organic material.

A thickness of the electron injection layer may be in a range of about 1 Å to about 100 Å, for example, about 3 Å to about 90 Å. When the thickness of the electron injection layer is within these ranges, satisfactory electron injection characteristics may be obtained without a substantial increase in driving voltage.

### Second electrode 19

The second electrode 19 is arranged on the aforementioned interlayer 10A. The second electrode 19 may be a cathode which is an electron injection electrode, and in this regard, a material for forming the second electrode 19 may be selected from a metal, an alloy, an electrically conductive compound, and a combination thereof, which have a relatively low work function.

The second electrode 19 may include Li, Ag, Mg, Al, Al-Li, Ca, Mg-In, Mg-Ag, ITO, IZO, or any combination thereof. The second electrode 19 may be a transmissive electrode, a semi-transmissive electrode, or a reflective electrode.

The second electrode 19 may have a single-layer structure having a single layer or a multi-layer structure including two or more layers.

### Explanation of terms

The term "C₁-C₆₀ alkyl group" as used herein refers to a linear or branched saturated aliphatic hydrocarbons monovalent group having 1 to 60 carbon atoms, and the term "C₁-C₆₀ alkylene group" as used herein refers to a divalent group having the same structure as the C₁-C₆₀ alkyl group.

Examples of the C₁-C₆₀ alkyl group, the C₁-C₂₀ alkyl group, and/or the C₁-C₁₀ alkyl group include a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, or a tert-decyl group, each unsubstituted or substituted with a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, or any combination thereof.

The term "C₁-C₆₀ alkoxy group" as used herein refers to a monovalent group represented by -OA₁₀₁ (wherein A₁₀₁ is the C₁-C₆₀ alkyl group), and examples thereof include a methoxy group, an ethoxy group, a propoxy group, a butoxy group, a pentoxy group, and the like. The term "C₁-C₆₀ alkylthio group" as used herein refers to a monovalent group represented by -SA₁₀₁ (wherein A₁₀₁ is the C₁-C₆₀ alkyl group), and examples thereof include a methylthio group, an ethylthio group, a propylthio group, a butylthio group, a pentylthio group, and the like.

The term "C₂-C₆₀ alkenyl group" as used herein refers to a hydrocarbon group formed by substituting at least one carbon-carbon double bond in the middle or at the terminus of the C₂-C₆₀ alkyl group, and examples thereof include an ethenyl group, a propenyl group, a butenyl group, and the like. The term "C₂-C₆₀ alkenylene group" as used herein refers to a divalent group having the same structure as the C₂-C₆₀ alkenyl group.

The term "C₂-C₆₀ alkynyl group" as used herein refers to a hydrocarbon group formed by substituting at least one carbon-carbon triple bond in the middle or at the terminus of the C₂-C₆₀ alkyl group, and examples thereof include an ethynyl group, a propynyl group, and the like. The term "C₂-C₆₀ alkynylene group" as used herein refers to a divalent group having the same structure as the C₂-C₆₀ alkynyl group.

The term "C₃-C₁₀ cycloalkyl group" as used herein refers to a monovalent saturated hydrocarbon cyclic group having 3 to 10 carbon atoms as a ring-forming atom, and the term "C₃-C₁₀ cycloalkylene group" as used herein refers to a divalent group having the same structure as the C₃-C₁₀ cycloalkyl group.

Examples of the C₃-C₁₀ cycloalkyl group include a cyclopropyl group, a cyclobutyl group, a cyclopentyl group, cyclohexyl group, a cycloheptyl group, a cyclooctyl group, an adamantanyl group, a norbornanyl group (a bicyclo[2.2.1]heptyl group), a bicyclo[1.1.1]pentyl group, a bicyclo[2.1.1]hexyl group, and a bicyclo[2.2.2]octyl group.

The term "C₁-C₁₀ heterocycloalkyl group" as used herein refers to a monovalent saturated cyclic group that includes at least one heteroatom selected from N, O, P, Si, S, Se, Ge, and B as a ring-forming atom and 1 to 10 carbon atoms as a ring-forming atom, and the term "C₁-C₁₀ heterocycloalkylene group" as used herein refers to a divalent group having the same structure as the C₁-C₁₀ heterocycloalkyl group.

Examples of the C₁-C₁₀ heterocycloalkyl group include a silolanyl group, a silinanyl group, tetrahydrofuranyl group, a tetrahydro-2H-pyranyl group, a tetrahydrothiophenyl group, and the like.

The term "C₃-C₁₀ cycloalkenyl group" as used herein refers to a monovalent hydrocarbon cyclic group that includes 3 to 10 carbon atoms as a ring-forming atom and at least one carbon-carbon double bond in the ring thereof and has no aromaticity, and examples thereof include a cyclopentenyl group, a cyclohexenyl group, and a cycloheptenyl group. The term "C₃-C₁₀ cycloalkenylene group" as used herein refers to a divalent group having the same structure as the C₃-C₁₀ cycloalkenyl group.

The term "C₂-C₁₀ heterocycloalkenyl group" as used herein refers to a monovalent cyclic group that has at least one hetero atom selected from N, O, P, Si, S, Se, Ge, and B as a ring-forming atom, 2 to 10 carbon atoms as a ring-forming atom, and at least one carbon-carbon double bond in the ring thereof, and has no aromaticity. Examples of the C₂-C₁₀ heterocycloalkenyl group include a 2,3-dihydrofuranyl group, a 2,3-dihydrothiophenyl group, and the like. The term "C₂-C₁₀ heterocycloalkenylene group" as used herein refers to a divalent group having the same structure as the C₂-C₁₀ heterocycloalkenyl group.

The term "C₆-C₆₀ aryl group" as used herein refers to a monovalent group having a carbocyclic aromatic system having 6 to 60 carbon atoms, and the term "C₆-C₆₀ arylene group" as used herein refers to a divalent group having a carbocyclic aromatic system having 6 to 60 carbon atoms. Examples of the C₆-C₆₀ aryl group include a phenyl group, a naphthyl group, an anthracenyl group, a phenanthrenyl group, a pyrenyl group, a chrysenyl group, and the like. When the C₆-C₆₀ aryl group and the C₆-C₆₀ arylene group each include two or more rings, the rings may be fused to each other.

The term "C₁-C₆₀ heteroaryl group" as used herein refers to a monovalent group that includes a heterocyclic aromatic system having at least one heteroatom selected from N, O, P, Si, S, Se, Ge, and B as a ring-forming atom and 1 to 60 carbon atoms as a ring-forming atom, and the term "C₁-C₆₀ heteroarylene group" as used herein refers to a divalent group that includes a heterocyclic aromatic system having at least one heteroatom selected from N, O, P, Si, S, Se, Ge, and B as a ring-forming atom and 1 to 60 carbon atoms as a ring-forming atom. Examples of the C₁-C₆₀ heteroaryl group include a pyridinyl group, a pyrimidinyl group, a pyrazinyl group, a pyridazinyl group, a triazinyl group, a quinolinyl group, an isoquinolinyl group, and the like. When the C₁-C₆₀ heteroaryl group and the C₁-C₆₀ heteroarylene group each include two or more rings, the rings may be fused to each other.

The term "C₆-C₆₀ aryloxy group" as used herein indicates -OA₁₀₂ (wherein A₁₀₂ is the C₆-C₆₀ aryl group), and the term "C₆-C₆₀ arylthio group" as used herein indicates -SA₁₀₃ (wherein A₁₀₃ is the C₆-C₆₀ aryl group).

The term "monovalent non-aromatic condensed polycyclic group" as used herein refers to a monovalent group in which two or more rings are condensed with each other, only carbon is used as a ring-forming atom (for example, the number of carbon atoms may be 8 to 60), and the whole molecule is a non-aromaticity group. An example of the monovalent non-aromatic condensed polycyclic group is a fluorenyl group. The term "divalent non-aromatic condensed polycyclic group" as used herein refers to a divalent group having the same structure as the monovalent non-aromatic condensed polycyclic group described above.

The term "monovalent non-aromatic condensed heteropolycyclic group" as used herein refers to a monovalent group having two or more rings condensed with each other, a heteroatom selected from N, O, P, Si, S, Se, Ge, and B, other than carbon atoms (for example, having 1 to 60 carbon atoms), as a ring-forming atom, and no aromaticity in the entire molecular structure thereof. Examples of the monovalent non-aromatic condensed heteropolycyclic group include a carbazolyl group and the like. The term "divalent non-aromatic condensed heteropolycyclic group" as used herein refers to a divalent group having the same structure as the monovalent non-aromatic condensed heteropolycyclic group.

The term "π electron-depleted nitrogen-containing C₁-C₆₀ cyclic group" as used herein refers to a cyclic group having 1 to 60 carbon atoms and including at least one *-N=*' (wherein * and *' each indicate a binding site to an adjacent atom) as a ring-forming moiety. For example, the π electron-depleted nitrogen-containing C₁-C₆₀ cyclic group may be a) a first ring, b) a condensed ring in which at least two first rings are condensed, or c) a condensed ring in which at least one first ring and at least one second ring are condensed.

The term "π electron-rich C₃-C₆₀ cyclic group" as used herein refers to a cyclic group having 3 to 60 carbon atoms and not including at least one *-N=*' (wherein * and *' each indicate a binding site to an adjacent atom) as a ring-forming moiety. For example, the π electron-rich C₃-C₆₀ cyclic group may be a) a second ring or b) a condensed ring in which at least two second rings are condensed.

The term "C₅-C₆₀ carbocyclic group" and "C₃-C₆₀ carbocyclic group" as used herein refers to a monocyclic or polycyclic group only having 5 to 60 or 5 to 30 carbon atoms, as a ring-forming atom, respectively, and may be, for example, a) a third ring or b) a condensed ring in which two or more third rings are condensed with each other.

The term "C₁-C₆₀ heterocyclic group" and "C₁-C₃₀ heterocyclic group" as used herein refers to a monocyclic or polycyclic group that has 1 to 60 or 1 to 30 carbon atoms and at least one heteroatom selected from N, O, P, Si, S, Se, Ge, and B as a ring-forming atom, respectively, and may be, for example, a) a fourth ring, b) a condensed ring in which two or more fourth rings are condensed with each other, or c) a condensed ring in which at least one third ring is condensed with at least one fourth ring.

The "first ring" as used herein may be an imidazole group, a pyrazole group, a thiazole group, an isothiazole group, an oxazole group, an isoxazole group, a pyridine group, a pyrazine group, a pyridazine group, a pyrimidine group, a triazole group, a tetrazole group, an oxadiazole group, a triazine group, or a thiadiazole group.

The "second ring" as used herein may be a benzene group, a cyclopentadiene group, a pyrrole group, a furan group, a thiophene group, or a silole group.

The "third ring" as used herein may be a cyclopentane group, a cyclopentadiene group, an indene group, an adamantane group, a norbornene group, a bicyclo[1.1.1]pentane group, a bicyclo[2.1.1]hexane group, a bicyclo[2.2.1]heptane group (a norbornane group), a bicyclo[2.2.2]octane group, a cyclohexane group, a cyclohexene group, or a benzene group.

The "fourth ring" as used herein may be a furan group, a thiophene group, a pyrrole group, a silole group, an oxazole group, an isoxazole group, an oxadiazole group, an isoxadiazole group, an oxatriazole group, an isoxatriazole group, a thiazole group, an isothiazole group, a thiadiazole group, an isothiadiazole group, a thiatriazole group, an isothiatriazole group, a pyrazole group, an imidazole group, a triazole group, a tetrazole group, an azasilole group, a diazasilole group, a triazasilole group, a pyridine group, a pyrimidine group, a pyrazine group, a pyridazine group, or a triazine group.

For example, the π electron-depleted nitrogen-containing C₁-C₆₀ cyclic group may be an imidazole group, a pyrazole group, a thiazole group, an isothiazole group, an oxazole group, an isoxazole group, a pyridine group, a pyrazine group, a pyridazine group, a pyrimidine group, an indazole group, a purine group, a quinoline group, an isoquinoline group, a benzoquinoline group, a benzoisoquinoline group, a phthalazine group, a naphthyridine group, a quinoxaline group, a benzoquinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, an azacarbazole group, an azadibenzofuran group, an azadibenzothiophene group, an azadibenzosilole group, an acridine group, or a pyridopyrazine group.

For example, the π electron-rich C₃-C₆₀ cyclic group may be a benzene group, a heptalene group, an indene group, a naphthalene group, an azulene group, an indacene group, an acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentaphene group, a rubicene group, a coronene group, an ovalene group, a pyrrole group, a furan group, a thiophene group, an isoindole group, an indole group, an indene group, a benzofuran group, a benzothiophene group, a benzosilole group, a naphthopyrrole group, a naphthofuran group, a naphthothiophene group, a naphthosilole group, a benzocarbazole group, a dibenzocarbazole group, a dibenzofuran group, a dibenzothiophene group, a carbazole group, a dibenzosilole group, an indenocarbazole group, an indolocarbazole group, a benzofurocarbazole group, a benzothienocarbazole group, a benzosilolocarbazole group, a triindolobenzene group, a pyrrolophenanthrene group, a furanophenanthrene group, a thienophenanthrene group, a benzonaphthofuran group, a benzonapthothiophene group, an (indolo)phenanthrene group, a (benzofurano)phenanthrene group, or a (benzothieno)phenanthrene group.

For example, the C₅-C₆₀ carbocyclic group or the C₅-C₃₀ carbocyclic group may be a cyclopentane group, a cyclohexane group, a cyclohexene group, a benzene group, a naphthalene group, an anthracene group, a phenanthrene group, an indene group, a triphenylene group, a pyrene group, a chrysene group, a 1,2,3,4-tetrahydronaphthalene group, a cyclopentadiene group, an indene group, a fluorene group, a 5,6,7,8-tetrahydroisoquinoline group, a 5,6,7,8-tetrahydroquinoline group, an adamantane group, a norbornane group, or a norbornene group.

For example, the C₁-C₆₀ heterocyclic group or the C₁-C₃₀ heterocyclic group may be a thiophene group, a furan group, a pyrrole group, a cyclopentadiene group, a silole group, a borole group, a phosphole group, a selenophene group, a germole group, a benzothiophene group, a benzofuran group, an indole group, a benzosilole group, a benzoborole group, a benzophosphole group, a benzoselenophene group, a benzogermole group, a dibenzothiophene group, a dibenzofuran group, a carbazole group, a dibenzosilole group, a dibenzoborole group, a dibenzophosphole group, a dibenzoselenophene group, a dibenzogermole group, a dibenzothiophene 5-oxide group, a 9H-fluorene-9-one group, a dibenzothiophene 5,5-dioxide group, an azabenzothiophene group, an azabenzofuran group, an azaindole group, an azaindene group, an azabenzosilole group, an azabenzoborole group, an azabenzophosphole group, an azabenzoselenophene group, an azabenzogermole group, an azadibenzothiophene group, an azadibenzofuran group, an azacarbazole group, an azafluorene group, an azadibenzosilole group, an azadibenzoborole group, an azadibenzophosphole group, an azadibenzoselenophene group, an azadibenzogermole group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, or a benzothiadiazole group.

The terms "a π electron-deficient nitrogen-containing C₁-C₆₀ cyclic group", "a π electron-rich C₃-C₆₀ cyclic group", "a C₅-C₆₀ carbocyclic group", "a C₅-C₃₀ carbocyclic group", "a C₁-C₆₀ heterocyclic group" and "a C₁-C₃₀ heterocyclic group" as used herein each refer to a part of a condensed ring or a monovalent, a divalent, a trivalent, a tetravalent, a pentavalent, or a hexavalent group, depending on the formula structure.

Substituents of the substituted π electron-deficient nitrogen-containing C₁-C₆₀ cyclic group, the substituted π electron-rich C₃-C₆₀ cyclic group, the substituted C₅-C₆₀ carbocyclic group, the substituted C₅-C₃₀ carbocyclic group, the substituted C₁-C₆₀ heterocyclic group, the substituted C₁-C₃₀ heterocyclic group, the substituted C₁-C₆₀ alkylene group, the substituted C₂-C₆₀ alkenylene group, the substituted C₂-C₆₀ alkynylene group, the substituted C₃-C₁₀ cycloalkylene group, the substituted C₁-C₁₀ heterocycloalkylene group, the substituted C₃-C₁₀ cycloalkenylene group, the substituted C₂-C₁₀ heterocycloalkenylene group, the substituted C₆-C₆₀ arylene group, the substituted C₁-C₆₀ heteroarylene group, the substituted divalent non-aromatic condensed polycyclic group, the substituted divalent non-aromatic condensed heteropolycyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₂-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group may each independently be:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, or a C₁-C₆₀ alkylthio group;
a C₁-C₆₀ alkyl group, a C₂-C₆₀ alkenyl group, a C₂-C₆₀ alkynyl group, a C₁-C₆₀ alkoxy group, or a C₁-C₆₀ alkylthio group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅),-Ge(Q₁₃)(Q₁₄)(Q₁₅), -B(Q₁₆)(Q₁₇), -P(=O)(Q₁₈)(Q₁₉), -P(Q₁₈)(Q₁₉), or any combination thereof;
a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₁-C₆₀ alkylthio group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), -Si(Q₂₃)(Q₂₄)(Q₂₅), -Ge(Q₂₃)(Q₂₄)(Q₂₅),-B(Q₂₆)(Q₂₇), -P(=O)(Q₂₈)(Q₂₉), -P(Q₂₈)(Q₂₉), or any combination thereof;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇),-P(=O)(Q₃₈)(Q₃₉), or -P(Q₃₈)(Q₃₉); or
any combination thereof.

Q₁ to Q₉, Q₁₁ to Q₁₉, Q₂₁ to Q₂₉, and Q₃₁ to Q₃₉ may each independently be hydrogen, deuterium, -F, or a C₁-C₆₀ alkyl group, a C₂-C₆₀ alkenyl group, a C₂-C₆₀ alkynyl group, a C₁-C₆₀ alkoxy group, a C₁-C₆₀ alkylthio group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with deuterium, -F, a cyano group, a C₁-C₆₀ alkyl group, a C₆-C₆₀ aryl group, or any combination thereof.

Unless otherwise defined, Q₁ to Q₉, Q₁₁ to Q₁₉, Q₂₁ to Q₂₉, and Q₃₁ to Q₃₉ may each independently be, for example:
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, or-CD₂CDH₂; or
an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, a phenyl group, a biphenyl group, or a naphthyl group, each unsubstituted or substituted with deuterium, a C₁-C₁₀ alkyl group, a phenyl group, or any combination thereof..

The term "room temperature" as used herein refers to a temperature of about 25 °C.

The terms "a biphenyl group, a terphenyl group, and a quaterphenyl group" used herein respectively refer to monovalent groups in which two, three, or four phenyl groups which are linked together via a single bond.

A term "(C₁-C₂₀ alkyl) X group" (e.g., (C₁-C₂₀ alkyl) phenyl group) used herein refers to an X group (e.g., a phenyl group) substituted with at least one C₁-C₂₀ alkyl.

Hereinafter, a compound and an organic light-emitting device according to embodiments are described in detail with reference to Synthesis Examples and Examples. However, the organic light-emitting device is not limited thereto. The wording "'B' was used instead of 'A'" used in describing Synthesis Examples means that an amount of 'A' used was identical to an amount of 'B' used, in terms of a molar equivalent.

### Examples

### Synthesis Example 1 (Compound 1)

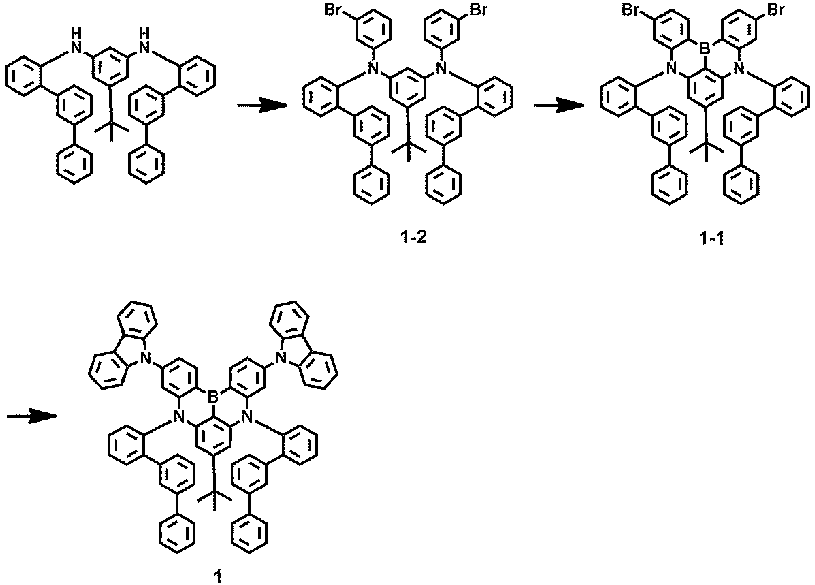

### Synthesis of Compound 1-2

A mixture of N1,N3-di([1,1':3',1"-terphenyl]-2-yl)-5-(tert-butyl)benzene-1,3-diamine [15 grams (g), 24.16 millimoles (mmol)), 1-bromo-3-iodobenzene (136.7 g, 483.3 mmol), Cul (0.46 g, 2.42 mmol), and K₂CO₃ (11.69 g, 84.56 mmol) was heated at 200 °C in a nitrogen atmosphere for 24 hours, and then an ammonium chloride solution was added to the reaction mixture. The organic phase was extracted with dichloromethane and the resulting extract was dried with MgSO₄. The dried extract was then purified by column chromatography using methylene chloride (MC) and hexane (in a volume ratio of 1:3), to provide 12.94 g of Compound 1-2 (white solid).

Liquid chromatography-mass spectrometry (LCMS) with a calculated mass-to-charge ratio (m/z) of 930.83 grams per mole (g/mol), measured: [M+] 931.720 g/mol.

### Synthesis of Compound 1-1

A mixture of Compound 1-2 (10.4 g, 11.17 mmol), 100 milliliters (ml) of 1,2-dichlorobenzene, and BI₃ (6.56 g, 16.76 mmol) was stirred in a nitrogen atmosphere for 24 hours after the temperature was raised to 150 °C. The resulting solution was cooled to room temperature and a Na₂CO₃ solution was added to the reaction mixture. The organic phase was extracted with dichloromethane was performed on the reaction product and dried with MgSO₄. The dried extract was purified by column chromatography using MC and hexane (in a volume ratio of 1:3), to provide 3.03 g of Compound 1-1 (solid).

LCMS (m/z) calculated: 938.61 g/mol, measured: [M+] 939.159 g/mol.

### Synthesis of Compound 1

Compound 1-1 (1 g, 1.07 mmol), carbazole (0.35 g, 2.09 mmol), Pd₂(dba)₃ (0.001 g, 0.001 mmol), S-Phos (0.008 g, 0.02 mmol), and NaOtBu (0.31 g, 3.21 mmol) were mixed with toluene, and the mixture was heated at 110 °C in a nitrogen atmosphere for 5 hours. The reaction mixture was cooled to room temperature and an ammonium chloride solution was added to the reaction mixture. The organic phase was extracted with dichloromethane and dried with MgSO₄. The dried extract was purified by column chromatography using MC and hexane (in a volume ratio of 1:2), to provide 0.49 g of Compound 1 (yellow solid).

LCMS (m/z) calculated: 1111.21 g/mol, measured: [M+] 1110.11 g/mol.

### Synthesis Example 2 (Compound 121)

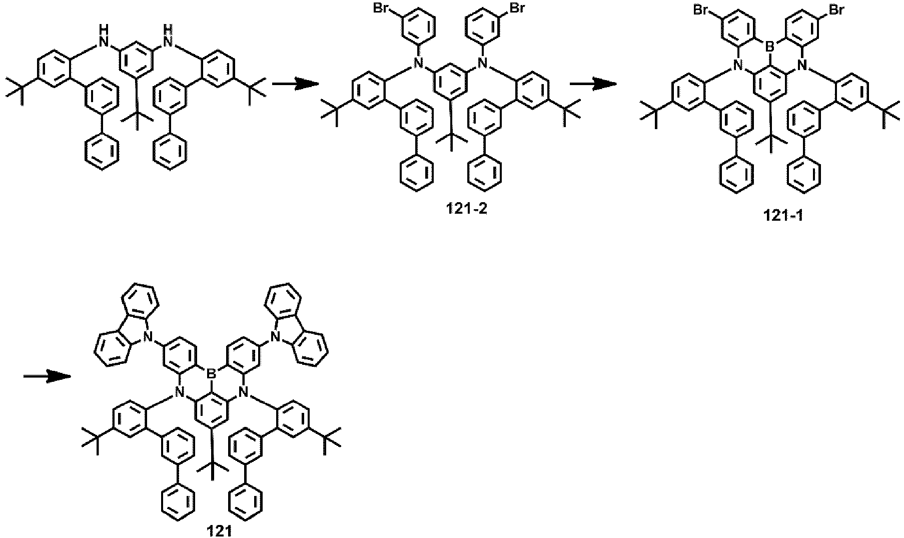

### Synthesis of Compound 121-2

A mixture of 5-(tert-butyl)-N1,N3-bis(5-(tert-butyl)-[1,1':3',1"-terphenyl]-2-yl)benzene-1,3-diamine (23 g, 31.37 mmol), 1-bromo-3-iodobenzene (177.53 g, 627.5 mmol), Cul (0.6 g, 3.14 mmol), and K₂CO₃ (15.17 g, 109.79 mmol) was heated at 200 °C in a nitrogen atmosphere for 24 hours, and then an ammonium chloride solution was added to the reaction mixture. The organic phase was extracted with dichloromethane and dried with MgSO₄. The dried extract was then purified by column chromatography using MC and hexane (in a volume ratio of 1:3), to provide 23.05 g of Compound 121-2 (white solid).

LCMS (m/z) calculated: 1043.04 g/mol, measured: [M+] 1043.3024 g/mol.

### Synthesis of Compound 121-1

A mixture of Compound 121-2 (10 g, 9.59 mmol), 100 ml of 1,2-dichlorobenzene, and BI₃ (5.63 g, 14.38 mmol) was stirred in a nitrogen atmosphere for 24 hours after the temperature was raised to 150 °C. The resulting solution was cooled to room temperature and a Na₂CO₃ solution was added to the reaction mixture. The organic phase was extracted with dichloromethane and dried with MgSO₄. The dried extract was purified by column chromatography using MC and hexane (in a volume ratio of 1:3), to provide 3.03 g of Compound 121-1 (solid).

LCMS (m/z) calculated: 1050.83 g/mol, measured: [M+] 1050.159 g/mol.

### Synthesis of Compound 121

Compound 121-1 (1.7 g, 1.61 mmol), carbazole (0.33 g, 1.97 mmol), Pd₂(dba)₃ (0.014 g, 0.015 mmol), S-Phos (0.013 g, 0.032 mmol), and NaOtBu (0.46 g, 4.79 mmol) were mixed with toluene, and the mixture was heated at 110 °C in a nitrogen atmosphere for 5 hours. The reaction solution was cooled to room temperature, and an ammonium chloride solution was added to the reaction mixture. The organic phase was extracted with dichloromethane and dried with MgSO₄. The dried extract was purified by column chromatography using MC and hexane (in a volume ratio of 1:2), to provide 1.2 g of Compound 121 (yellow solid).

LCMS (m/z) calculated: 1223.43 g/mol, measured: [M+] 1223.31 g/mol.

### Synthesis Example 3 (Compound 576)

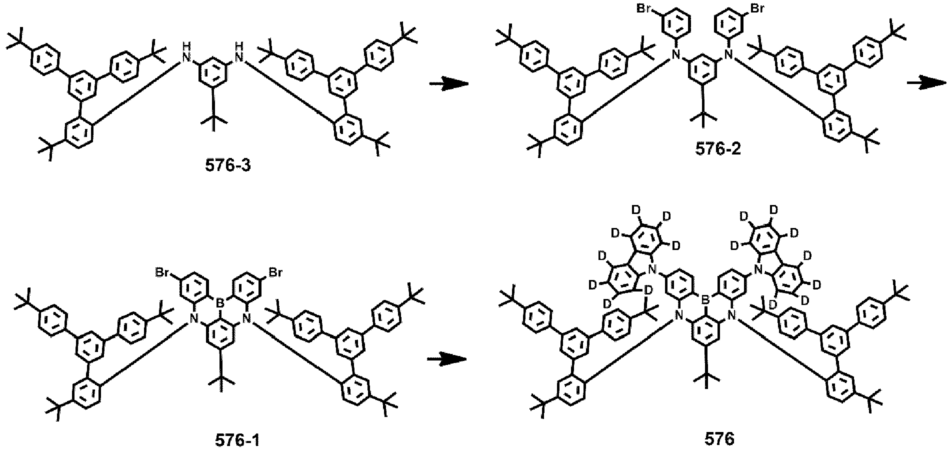

### Synthesis of Compound 576-2

Compound 576-2 was synthesized in the same manner as in Synthesis of Compound 1-2 in Synthesis Example 1, except that Compound 576-3 was used instead of N1,N3-di([1,1':3',1"-terphenyl]-2-yl)-5-(tert-butyl)benzene-1,3-diamine.

### Synthesis of Compound 576-1

Compound 576-1 was synthesized in the same manner as in Synthesis of Compound 1-1 in Synthesis Example 1, except that Compound 576-2 was used instead of Compound 1-2.

### Synthesis of Compound 576

Compound 576 (1.6 g, yellow solid) was synthesized in the same manner as in Synthesis of Compound 1 in Synthesis Example 1, except that Compound 576-1 was used instead of Compound 1-1 and 9H-carbazole-1,2,3,4,5,6,7,8-d₈ was used instead of carbazole.

LCMS (m/z) calculated: 1615.022 g/mol, measured: [M+] 1616.027 g/mol. Synthesis Example 4 (Compound 516)

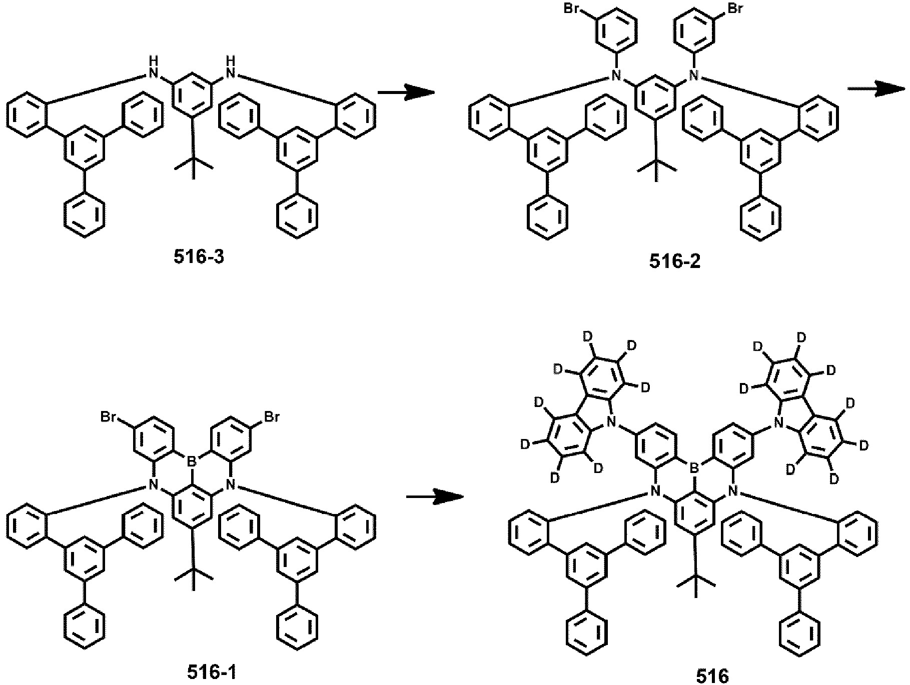

### Synthesis of Compound 516-2

Compound 516-2 was synthesized in the same manner as in Synthesis of Compound 1-2 in Synthesis Example 1, except that Compound 516-3 was used instead of N1,N3-di([1,1':3',1"-terphenyl]-2-yl)-5-(tert-butyl)benzene-1,3-diamine.

### Synthesis of Compound 516-1

Compound 516-1 was synthesized in the same manner as in Synthesis of Compound 1-1 in Synthesis Example 1, except that Compound 516-2 was used instead of Compound 1-2.

### Synthesis of Compound 516

Compound 516 (0.75 g, yellow solid) was synthesized in the same manner as in Synthesis of Compound 1 in Synthesis Example 1, except that Compound 516-1 was used instead of Compound 1-1 and 9H-carbazole-1,2,3,4,5,6,7,8-da was used instead of carbazole.

LCMS (m/z) calculated: 1278.77 g/mol, measured: [M+] 1278.757 g/mol.

### Comparative Synthesis Example 1 (Compound R3)

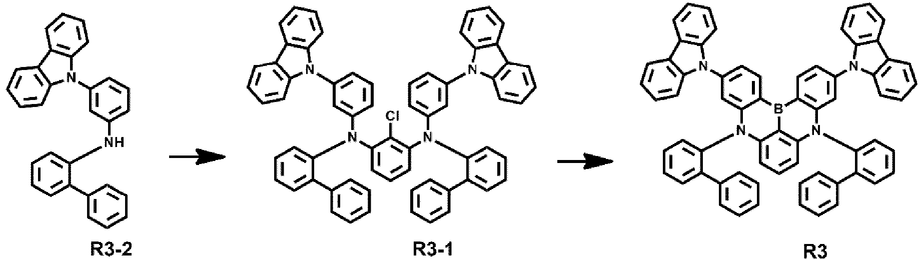

### Synthesis of Compound R3-1

1-Bromo-2,3-dichlorobenzene (1.25 g, 5.53 mmol), Compound R3-2 (4.996 g, 12.17 mmol), Pd₂(dba)₃ (0.504 g, 0.55 mmol), S-Phos (0.456 g, 1.11 mmol), and NaOtBu (1.86 g, 19.37 mmol) were mixed with 110 ml of xylene, and the mixture was refluxed for 30 minutes. Then an ammonium chloride solution was added to the reaction mixture, the organic phase was extracted with ethyl acetate and dried with MgSO₄. The dried extract was then purified by column chromatography using MC and hexane (in a volume ratio of 1:3), to provide 4.3 g of Compound R3-1 (white solid).

LCMS (m/z) calculated : 928.333 g/mol, measured: [M+] 929.336 g/mol.

### Synthesis of Compound R3

Compound R3-1 (3.26 g, 3.51 mmol) was dissolved in 87 ml of t-butylbenzene, and the resulting solution was cooled to -78 °C. Then, after adding t-BuLi (5.48 ml, 8.77 mmol) thereto, the temperature was raised to 60 °C, and the resulting solution was stirred for one and half hours. After the reaction mixture was cooled to 0 °C, boron tribromide (1.76 ml, 7.01 mmol) was added and the reaction solution was stirred at room temperature for one and half hours. The reaction mixture was cooled to 0 °C and N,N-diisopropylethyl amine (1.19 ml, 7.01mmol) was added thereto, the reaction solution was stirred at 120 °C for 4 hours. Then, sodium acetate solution (1.0 M) was added to the reaction mixture. The organic phase was extracted with ethyl acetate and dried with MgSO₄ . The dried extract was purified by column chromatography using MC and hexane (in a volume ratio of 1:4), to provide 0.6 g of Compound R3 (solid).

LCMS (m/z) calculated : 902.368 g/mol, measured: [M+] 903.353 g/mol.

### Evaluation Example 1

For each of Compounds 1, 121, 576, 516, and R1 to R4, a HOMO energy level, a LUMO energy level, a singlet (S₁) energy level, a triplet (T₁) energy level, and ΔEst (an absolute value of difference between S₁ energy level and T₁ energy level values) were evaluated by density functional theory (DFT) calculations using a Gaussian 16 program structurally optimized at a level of B3LYP/6-31G(D,P), and the results are shown in Table 2. Compounds R1, R2 and R4 can be obtained by referring to the methods in the Synthesis Examples and Comparative Synthesis Example 1.

**Table 2**

| Compound No. | HOMO energy level (eV) | LUMO energy level (eV) | S₁ energy level (eV) | T₁ energy level (eV) | ΔEst (eV) |
|---|---|---|---|---|---|
| 1 | -5.025 | -1.476 | 2.661 | 2.562 | 0.099 |
| 121 | -4.938 | -1.360 | 2.667 | 2.578 | 0.089 |
| 576 | -4.846 | -1.314 | 2.698 | 2.593 | 0.105 |
| 516 | -4.974 | -1.381 | 2.758 | 2.648 | 0.110 |
| R1 | -4.642 | -0.997 | 2.678 | 2.546 | 0.132 |
| R2 | -5.011 | -0.452 | 2.667 | 2.531 | 0.136 |
| R3 | -5.047 | -1.464 | 2.678 | 2.551 | 0.127 |
| R4 | -4.576 | -1.013 | 2.684 | 2.578 | 0.105 |

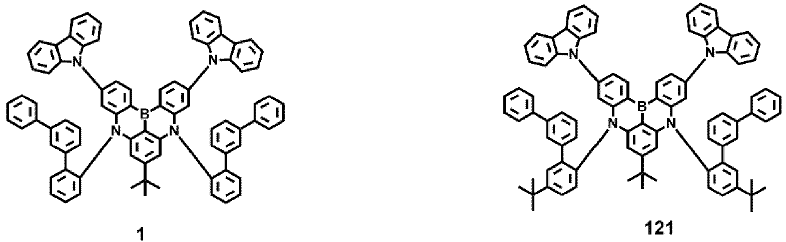

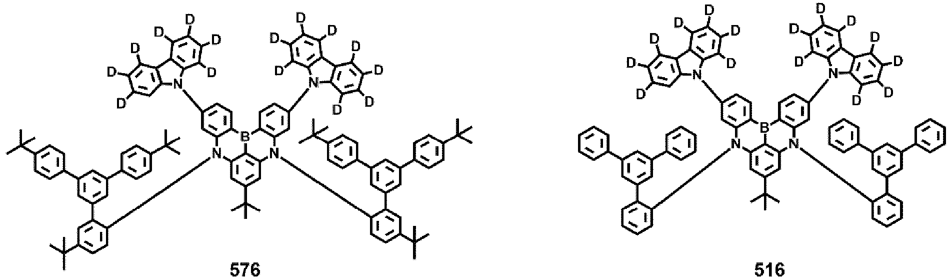

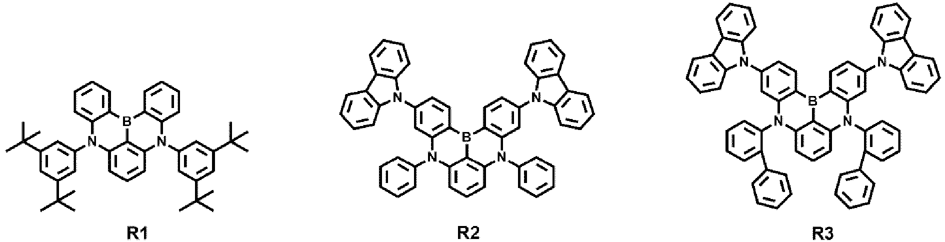

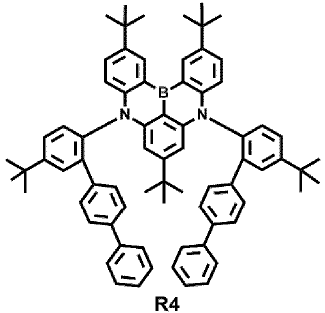

As shown in Table 2, Compounds 1, 121, 576, and 516 had electrical properties suitable for light-emitting devices.

### Example 1

A glass substrate with a 1,500 Å -thick ITO electrode (i.e., anode) formed thereon was cut to a size of 50 millimeters (mm) x 50 mm x 0.5 mm, sonicated with acetone isopropyl alcohol and pure water each for 15 minutes, and cleaned by UV-ozone-irradiation for 30 minutes.

Subsequently, m-MTDATA was deposited on the ITO electrode of the glass substrate to form a hole injection layer having a thickness of 600 Å, and α-NPD was deposited on the hole injection layer to form a hole transport layer having a thickness of 250 Å.

Next, Compound 1 (emitter) and mCP (host) were co-deposited on the hole transport layer in a weight ratio of 10:90 to form an emission layer having a thickness of 400 Å.

BAlq was deposited on the emission layer to form a hole blocking layer having a thickness of 50 Å, Alq₃ was deposited on the hole blocking layer to form an electron transport layer having a thickness of 300 Å, LiF was deposited on the electron transport layer to form an electron injection layer having a thickness of 10 Å, and Al was vacuum-deposited on the electron injection layer to form a second electrode (i.e., cathode) having a thickness of 1,200 Å, thereby completing the manufacture of a light-emitting device having a structure of ITO / m-MTDATA (600 Å) / α-NPD (250 Å) / mCP + Compound 1 (10 weight percent (wt%)) (400 Å) / BAlq (50 Å) / Alq₃ (300 Å) / LiF (10 Å) / Al (1,200 Å).

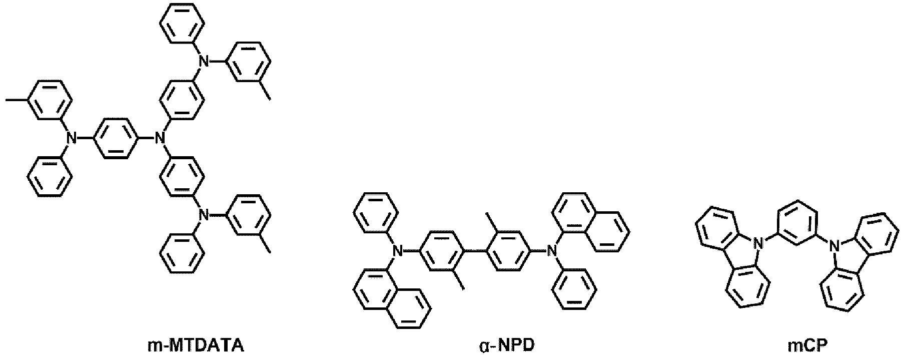

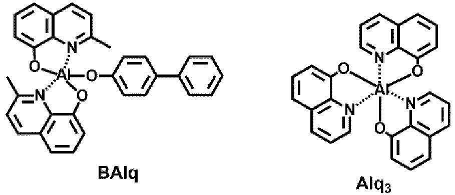

### Examples 2 to 4 and Comparative Examples R1 to R3

Light-emitting devices were manufactured in the same manner as in Example 1, except that compounds shown in Table 3 were each used instead of Compound 1 in forming an emission layer.

### Evaluation Example 2

For each of the light-emitting devices of Examples 1 to 4 and Comparative Examples R1 to R3, emission peak wavelengths (i.e., maximum emission peak wavelength) of the electroluminescence (EL) spectrum, full width at half maximum (FWHM), driving voltage, external quantum efficiency at 1,000 nit (EQE at 1,000 nit), and lifetime (LT₉₅ at 1,000 nit) were evaluated, and the results are shown in Table 3. The emission peak wavelengths of the EL spectrum and the FWHM were evaluated for each light-emitting device based on the EL spectrum measured by using a luminance meter (Minolta Cs-1000A). The driving voltage and the EQE were evaluated by using a current-voltage meter (Keithley 2400) and a luminance meter (Minolta Cs-1000A). The lifetime (LT₉₅ at 1,000 nit) is the time (hr) required for the luminance to reach 95 % of the initial luminance of 100 %, and is expressed by converting the measured time into relative values (%).

**Table 3**

| | Emitter Compo und No. | Emission peak waveleng th (nm) | FWHM (nm) | Driving voltage (V) | EQE at 1,000 nit (%) | LT₉₅ at 1,000 nit (relative value, %) |
|---|---|---|---|---|---|---|
| Example 1 | 1 | 466 | 24 | 5.0 | 13.0 | 374 |
| Example 2 | 121 | 465 | 23 | 4.8 | 14.6 | 449 |
| Example 3 | 576 | 466 | 24 | 4.9 | 14 | 239 |
| Example 4 | 516 | 466 | 26 | 4.8 | 13.2 | 314 |
| Comparative Example R1 | R1 | 463 | 23 | 5.5 | 7.9 | 60 |
| Comparative Example R2 | R2 | 465 | 22 | 5.1 | 11.7 | 100 |
| Comparative Example R3 | R3 | 463 | 26 | 5.6 | 9.7 | 82 |

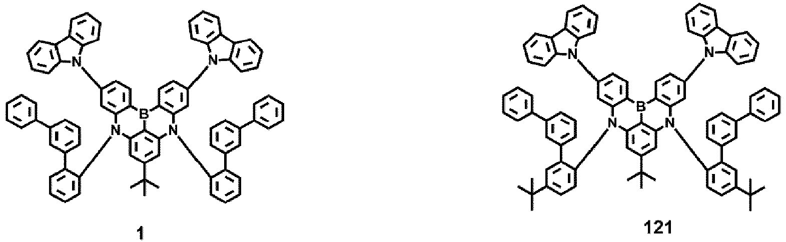

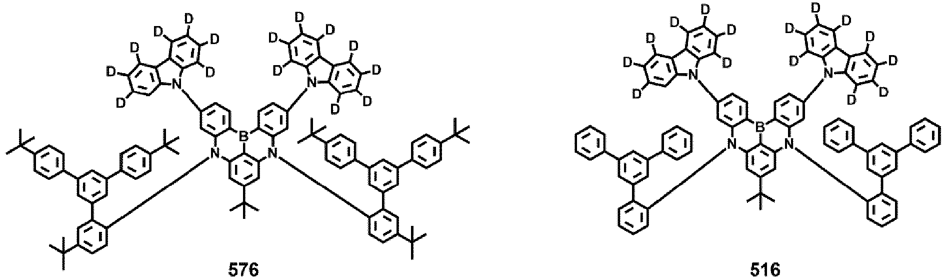

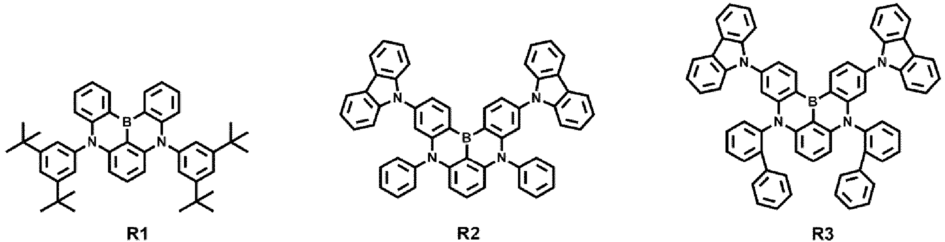

As shown in Table 3, the light-emitting devices of Examples 1 to 4 emitting blue light had improved driving voltage, improved EQE, and improved lifespan characteristics, compared to the light-emitting devices of Comparative Examples R1 to R3.

According to the one or more embodiments, a condensed cyclic compound may emit blue light having a relatively large oscillator strength, a relatively narrow FWHM, and thus an electronic device, e.g., a light-emitting device, including the condensed cyclic compound may have improved driving voltage, improved external quantum efficiency, and improved lifespan characteristics.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the scope as defined by the following claims.

## Claims

1. A condensed cyclic compound represented by Formula 1: 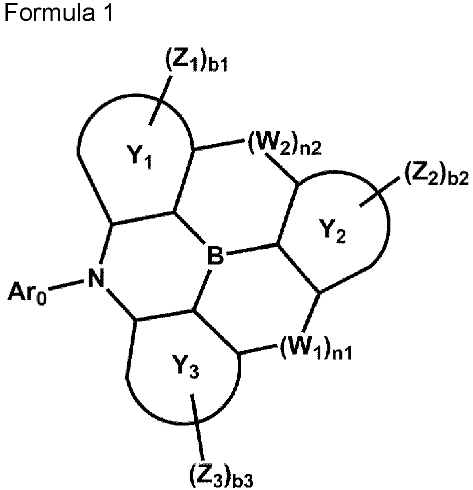 wherein, in Formula 1, Ar₀ is a group represented by Formula 2, 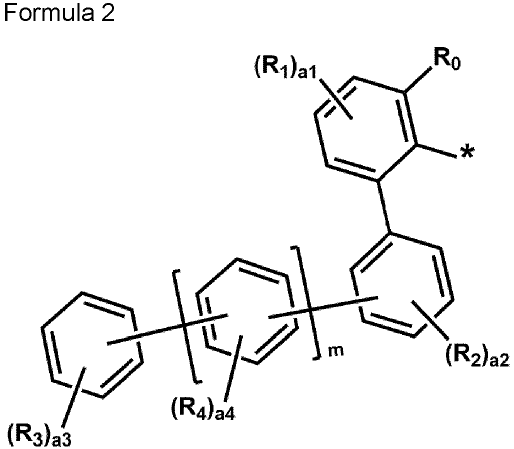 wherein, in Formulae 1 and 2,
ring Y₁ to ring Y₃ are each independently a C₅-C₆₀ carbocyclic group or a C₁-C₆₀ heterocyclic group,
W₁ is a single bond, O, S, N(Ar1), N(T₁₁), C(T₁₂)(T₁₃), or Si(T₁₂)(T₁₃),
W₂ is a single bond, O, S, N(Ar₂), N(T₂₁), C(T₂₂)(T₂₃), or Si(T₂₂)(T₂₃),
n1 and n2 are each independently 0 or 1,
when n1 is 0, *-(W₁)ₙ₁-*' is absent,
when n2 is 0, *-(W₂)ₙ₂-*' is absent,
the sum of n1 and n2 is 1 or greater,
each of Ar₁ and Ar₂ is a group represented by Formula 2,
R₀ is:
hydrogen, deuterium, -F, or a cyano group; or
a C₁-C₆₀ alkyl group unsubstituted or substituted with deuterium, -F, a cyano group, or any combination thereof,
R₁ to R₄ are each independently:
hydrogen, deuterium, -F, or a cyano group;
a C₁-C₆₀ alkyl group unsubstituted or substituted with deuterium, -F, a cyano group, or any combination thereof; or
a C₆-C₆₀ aryl group unsubstituted or substituted with deuterium, -F, a cyano group, a C₁-C₆₀ alkyl group, a C₆-C₆₀ aryl group, or any combination thereof,
a1 is an integer from 0 to 3,
a2 and a4 are each independently an integer from 0 to 4,
a3 and m are each independently an integer from 0 to 5,
* in Formula 2 indicates a binding site to the compound of Formula 1,
Z₁ to Z₃, T₁₁ to T₁₃, and T₂₁ to T₂₃ are each independently hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), -P(=O)(Q₈)(Q₉),-P(Q₈)(Q₉), or a group represented by Formula 2,
b1 and b2 are each independently an integer from 0 to 10,
b3 is an integer from 1 to 5,
Formula 1 satisfies at least one of Conditions 1 and 2:
Condition 1
b1 is 1 or greater, and at least one of Z₁ in the number of b1 comprises a carbazole group; and
Condition 2
b2 is 1 or greater, and at least one of Z₂ in the number of b2 comprises a carbazole group,
at least one of Z₃ in the number of b3 is a C₁-C₆₀ alkyl group unsubstituted or substituted with deuterium, -F, a cyano group, or any combination thereof,
two or more of Z₁, Z₂, Z₃, T₁₁, T₁₂, T₁₃, T₂₁, T₂₂ and T₂₃ are optionally linked together to form a substituted or unsubstituted C₅-C₃₀ carbocyclic group or a substituted or unsubstituted C₁-C₃₀ heterocyclic group,
at least one substituent of the substituted C₅-C₃₀ carbocyclic group, the substituted C₁-C₃₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₁-C₆₀ alkylthio group, the substituted C₃-C₁₀ cycloalkyl group, the substituted C₁-C₁₀ heterocycloalkyl group, the substituted C₃-C₁₀ cycloalkenyl group, the substituted C₂-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group is:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, or a C₁-C₆₀ alkylthio group;
a C₁-C₆₀ alkyl group, a C₂-C₆₀ alkenyl group, a C₂-C₆₀ alkynyl group, a C₁-C₆₀ alkoxy group, or a C₁-C₆₀ alkylthio group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂),-Si(Q₁₃)(Q₁₄)(Q₁₅), -Ge(Q₁₃)(Q₁₄)(Q₁₅), -B(Q₁₆)(Q₁₇), -P(=O)(Q₁₈)(Q₁₉), -P(Q₁₈)(Q₁₉), or any combination thereof;
a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₁-C₆₀ alkylthio group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group,-N(Q₂₁)(Q₂₂), -Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉),-P(Q₂₈)(Q₂₉), or any combination thereof;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇),-P(=O)(Q₃₈)(Q₃₉), or -P(Q₃₈)(Q₃₉); or
any combination thereof, and
Q₁ to Q₉, Q₁₁ to Q₁₉, Q₂₁ to Q₂₉, and Q₃₁ to Q₃₉ are each independently: hydrogen; deuterium; -F; or a C₁-C₆₀ alkyl group, a C₂-C₆₀ alkenyl group, a C₂-C₆₀ alkynyl group, a C₁-C₆₀ alkoxy group, a C₁-C₆₀ alkylthio group, a C₃-C₁₀ cycloalkyl group, a C₁-C₁₀ heterocycloalkyl group, a C₃-C₁₀ cycloalkenyl group, a C₂-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with deuterium, -F, a cyano group, a C₁-C₆₀ alkyl group, a C₆-C₆₀ aryl group, or any combination thereof.

2. The condensed cyclic compound of claim 1, wherein ring Y₁ to ring Y₃ are each independently a benzene group, a naphthalene group, a phenanthrene group, a pyridine group, a pyrimidine group, a pyridazine group, a pyrazine group, a quinoline group, an isoquinoline group, a dibenzofuran group, a dibenzothiophene group, a carbazole group, a fluorene group, a dibenzosilole group, an azadibenzofuran group, an azadibenzothiophene group, an azacarbazole group, an azafluorene group, or an azadibenzosilole group; and/or
wherein n1 is 1, and W₁ is N(Ar₁) or N(T₁₁).

3. The condensed cyclic compound of claims 1 or 2, wherein
R₀ is:
hydrogen, deuterium, -F, or a cyano group; or
a C₁-C₂₀ alkyl group unsubstituted or substituted with deuterium, -F, a cyano group, or any combination thereof, and
R₁ to R₄ are each independently:
hydrogen, deuterium, -F, or a cyano group;
a C₁-C₂₀ alkyl group unsubstituted or substituted with deuterium, -F, a cyano group, or any combination thereof; or
a phenyl group unsubstituted or substituted with deuterium, -F, a cyano group, a C₁-C₂₀ alkyl group, a phenyl group, or any combination thereof.

4. The condensed cyclic compound of any of claims 1-3, wherein
a2 is 1, and
R₂ is a phenyl group unsubstituted or substituted with deuterium, -F, a cyano group, a C₁-C₂₀ alkyl group, a phenyl group, or any combination thereof.

5. The condensed cyclic compound of any of claims 1-4, wherein
Z₁ to Z₃, T₁₁ to T₁₃, and T₂₁ to T₂₃ are each independently:
hydrogen, deuterium, -F, or a cyano group;
a C₁-C₂₀ alkyl group, a phenyl group, a biphenyl group, a dibenzofuranyl group, a dibenzothiophenyl group, a carbazolyl group, a fluorenyl group, or a dibenzosilolyl group, each unsubstituted or substituted with deuterium, -F, cyano group, a C₁-C₂₀ alkyl group, a phenyl group, a dibenzofuranyl group, a dibenzothiophenyl group, a carbazolyl group, a fluorenyl group, a dibenzosilolyl group, or any combination thereof; or
-N(Q₁)(Q₂), and
Q₁ and Q₂ are each independently a C₁-C₂₀ alkyl group, a phenyl group, a biphenyl group, a dibenzofuranyl group, a dibenzothiophenyl group, a carbazolyl group, a fluorenyl group, or a dibenzosilolyl group, each unsubstituted or substituted with deuterium, -F, a cyano group, a C₁-C₂₀ alkyl group, a phenyl group, a dibenzofuranyl group, a dibenzothiophenyl group, a carbazolyl group, a fluorenyl group, a dibenzosilolyl group, or any combination thereof.

6. The condensed cyclic compound of any of claims 1-5, wherein
each of b1 and b2 is 1 or greater, and
at least one of Z₁ and Z₂ is a group represented by Formula 3: 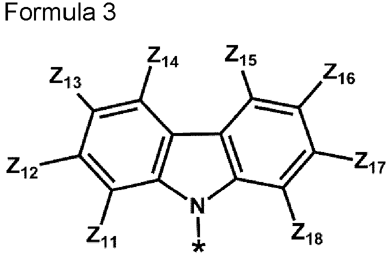
wherein, in Formula 3,
Z₁₁ to Z₁₈ are each independently:
hydrogen, deuterium, -F, or a cyano group;
a C₁-C₂₀ alkyl group unsubstituted or substituted with deuterium, -F, a cyano group, or any combination thereof; or
a phenyl group unsubstituted or substituted with deuterium, -F, a cyano group, a C₁-C₂₀ alkyl group, a phenyl group, or any combination thereof, and
* indicates a binding site to Formula 1.

7. The condensed cyclic compound of any of claims 1-6, wherein the group represented by Formula 2 is a group represented by one of Formulae 2-1 to 2-3: 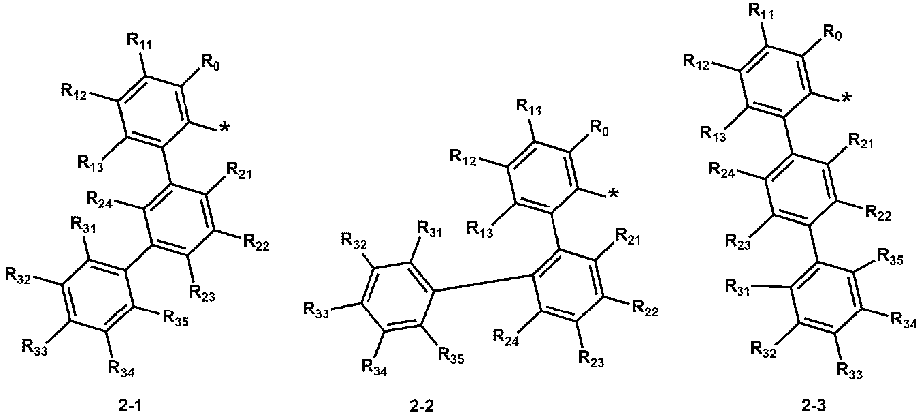 wherein, in Formulae 2-1 to 2-3,
R₀ is as defined in claim 1,
R₁₁ to R₁₃ are each defined as for R₁,
R₂₁ to R₂₄ are each defined as for R₂,
R₃₁ to R₃₅ are each defined as for R₃, and
* indicates a binding site to Formula 1.

8. The condensed cyclic compound of any of claims 1-7, wherein the group represented by Formula 2 is a group represented by Formula 2-1(1): 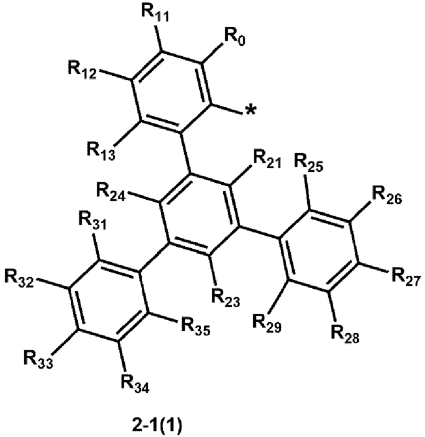 wherein, in Formula 2-1(1),
R₀ is as defined in claim 1,
R₁₁ to R₁₃ are each defined as for R₁ in claim 1,
R₂₁, R₂₃, and R₂₄ are each defined as for R₂ in claim 1,
R₃₁ to R₃₅ are each defined as for R₃ in claim 1,
R₂₅ to R₂₉ are each independently:
hydrogen, deuterium, -F, or a cyano group; or
a C₁-C₂₀ alkyl group unsubstituted or substituted with deuterium, -F, a cyano group, or any combination thereof, and
* indicates a binding site to Formula 1; and/or
wherein the condensed cyclic compound is represented by Formula 1A: 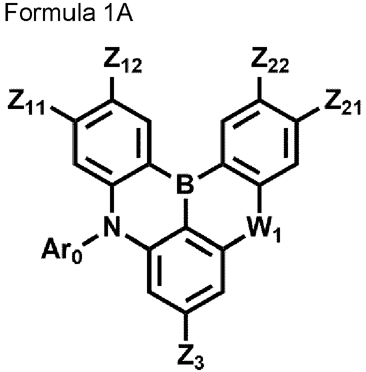
wherein, in Formula 1A,
W₁ is O, S, N(Ar1), N(T₁₁), C(T₁₂)(T₁₃), or Si(T₁₂)(T₁₃),
Ar₀, Ar₁, and T₁₁ to T₁₃ are each as defined in claim 1,
Z₁₁ and Z₁₂ are each defined as for Z₁ in claim 1,
Z₂₁ and Z₂₂ are each defined as for Z₂ in claim 1,
at least one of Z₁₁, Z₁₂, Z₂₁, and Z₂₂ is an N-carbazolyl group unsubstituted or substituted with deuterium, -F, a cyano group, a C₁-C₂₀ alkyl group, a phenyl group, or any combination thereof, and
Z₃ is a C₁-C₂₀ alkyl group unsubstituted or substituted with deuterium, -F, a cyano group, or any combination thereof.

9. The condensed cyclic compound of any of claims 1-8, wherein the condensed cyclic compound comprises at least one deuterium, at least one tert-butyl group, or any combination thereof.

10. A light-emitting device comprising:
a first electrode;
a second electrode; and
an interlayer arranged between the first electrode and the second electrode and comprising an emission layer,
wherein the interlayer comprises at least one condensed cyclic compound of any of claims 1-9.

11. The light-emitting device of claim 10, wherein the at least one condensed cyclic compound is included in the emission layer.

12. The light-emitting device of claim 11, wherein light emitted from the emission layer has an emission peak wavelength in a range of 440 nm to 470 nm; and/or
wherein the at least one condensed cyclic compound included in the emission layer is an emitter.

13. The light-emitting device of claims 11 or 12, wherein the emission layer further comprises a sensitizer, and the sensitizer and the condensed cyclic compound are different from each other;
preferably wherein the sensitizer is an organometallic compound, a delayed fluorescence material, a prompt fluorescence material, or any combination thereof;
preferably wherein
the organometallic compound comprises a transition metal and a tetradentate ligand bonded to the transition metal,
the transition metal is platinum or palladium, and
the tetradentate ligand comprises a carbene moiety bonded to the transition metal.

14. The light-emitting device of any of claims 11-13, wherein the at least one condensed cyclic compound included in the emission layer is a sensitizer.

15. An electronic apparatus comprising the light-emitting device of any of claims 10-14.

## Patentansprüche

1. Kondensierte cyclische Verbindung dargestellt durch Formel 1: 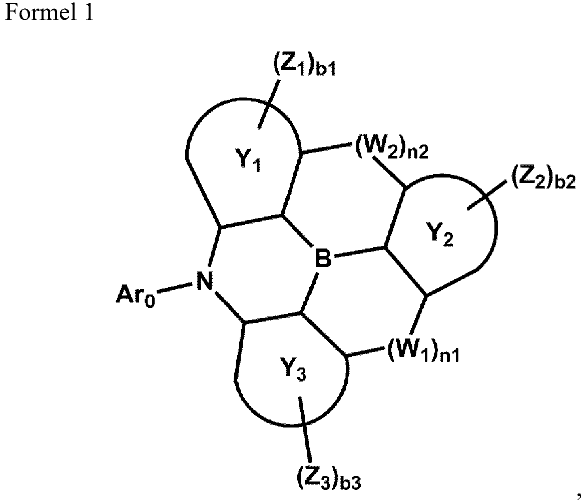 wobei in Formel 1 Ar₀ eine Gruppe dargestellt durch Formel 2 ist, 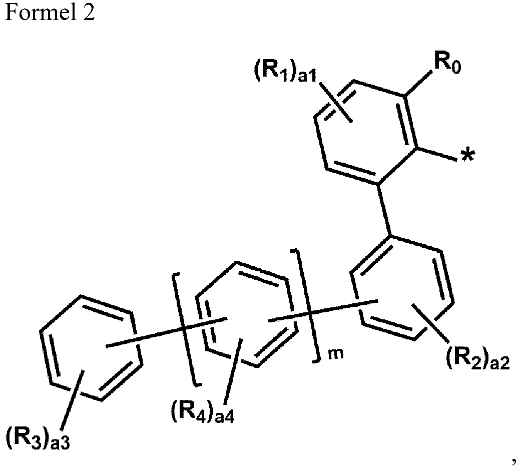 wobei in Formel 1 und 2
Ring Y₁ bis Ring Y₃ jeweils unabhängig eine carbocyclische C₃-C₆₀-Gruppe oder eine heterocyclische C₁-C₆₀-Gruppe sind,
W₁ eine Einfachbindung, O, S, N(Ar₁), N(T₁₁), C(T₁₂)(T₁₃) oder Si(T₁₂)(T₁₃) ist,
W₂ eine Einfachbindung, O, S, N(Ar₂), N(T₂₁), C(T₂₂)(T₂₃) oder Si(T₂₂)(T₂₃) ist,
n1 und n2 jeweils unabhängig 0 oder 1 sind,
wenn n1 0 ist, *-(W₁)ₙ₁-*' fehlt,
wenn n2 0 ist, *-(W₂)ₙ₂-*' fehlt,
die Summe von n1 und n2 1 oder größer ist,
jedes von Ar₁ und Ar₂ eine Gruppe dargestellt durch Formel 2 ist,
R₀ wie folgt ist:
Wasserstoff, Deuterium, -F oder eine Cyanogruppe; oder
eine C₁-C₆₀-Alkylgruppe, unsubstituiert oder substituiert mit Deuterium, -F, einer Cyanogruppe oder einer beliebigen Kombination davon,
R₁ bis R₄ jeweils unabhängig wie folgt sind:
Wasserstoff, Deuterium, -F oder eine Cyanogruppe;
eine C₁-C₆₀-Alkylgruppe, unsubstituiert oder substituiert mit Deuterium, -F, einer Cyanogruppe oder einer beliebigen Kombination davon; oder
eine C₆-C₆₀-Arylgruppe, unsubstituiert oder substituiert mit Deuterium, -F, einer Cyanogruppe, einer C₁-C₆₀-Alkylgruppe, einer C₆-C₆₀-Arylgruppe oder einer beliebigen Kombination davon,
a1 eine ganze Zahl von 0 bis 3 ist,
a2 und a4 jeweils unabhängig eine ganze Zahl von 0 bis 4 sind,
a3 und m jeweils unabhängig eine ganze Zahl von 0 bis 5 sind,
* in Formel 2 eine Bindungsstelle an die Verbindung von Formel 1 angibt,
Z₁ bis Z₃, T₁₁ bis T₁₃ und T₂₁ bis T₂₃ jeweils unabhängig Wasserstoff, Deuterium, -F, -Cl, -Br, -I, -SF₅, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine substituierte oder unsubstituierte C₁-C₆₀-Alkylgruppe, eine substituierte oder unsubstituierte C₂-C₆₀-Alkenylgruppe, eine substituierte oder unsubstituierte C₂-C₆₀-Alkinylgruppe, eine substituierte oder unsubstituierte C₁-C₆₀-Alkoxygruppe, eine substituierte oder unsubstituierte C₁-C₆₀-Alkylthiogruppe, eine substituierte oder unsubstituierte C₃-C₁₀-Cycloalkylgruppe, eine substituierte oder unsubstituierte C₁-C₁₀-Heterocycloalkylgruppe, eine substituierte oder unsubstituierte C₃-C₁₀-Cycloalkenylgruppe, eine substituierte oder unsubstituierte C₂-C₁₀-Heterocycloalkenylgruppe, eine substituierte oder unsubstituierte C₆-C₆₀-Arylgruppe, eine substituierte oder unsubstituierte C₆-C₆₀-Aryloxygruppe, eine substituierte oder unsubstituierte C₆-C₆₀-Arylthiogruppe, eine substituierte oder unsubstituierte C₁-C₆₀-Heteroarylgruppe, eine substituierte oder unsubstituierte einwertige nichtaromatische kondensierte polycyclische Gruppe, eine substituierte oder unsubstituierte einwertige nichtaromatische kondensierte heteropolycyclische Gruppe, -N(Q₁)(Q₂), -Si(Q₃)(Q₄)(Q₅),-Ge(Q₃)(Q₄)(Q₅), -B(Q₆)(Q₇), -P(=O)(Q₈)(Q₉), -P(Q₈)(Q₉) oder eine Gruppe dargestellt durch Formel 2 sind,
b1 und b2 jeweils unabhängig eine ganze Zahl von 0 bis 10 sind,
b3 eine ganze Zahl von 1 bis 5 ist,
Formel 1 zumindest eines von Bedingung 1 und 2 erfüllt:
Bedingung 1
b1 ist 1 oder größer und zumindest eines von Z₁ in der Anzahl von b1 umfasst eine Carbazolgruppe; und
Bedingung 2
b2 ist 1 oder größer und zumindest eines von Z₂ in der Anzahl von b2 umfasst eine Carbazolgruppe,
zumindest eines von Z₃ in der Anzahl von b3 eine C₁-C₆₀-Alkylgruppe, unsubstituiert oder substituiert mit Deuterium, -F, einer Cyanogruppe oder einer beliebigen Kombination davon, ist,
zwei oder mehr von Z₁, Z₂, Z₃, T₁₁, T₁₂, T₁₃, T₂₁, T₂₂ und T₂₃ optional miteinander verknüpft sind, um eine substituierte oder unsubstituierte carbocyclische C₅-C₃₀-Gruppe oder eine substituierte oder unsubstituierte heterocyclische C₁-C₃₀-Gruppe zu bilden,
zumindest ein Substituent der substituierten carbocyclischen C₅-C₃₀-Gruppe, der substituierten heterocyclischen C₁-C₃₀-Gruppe, der substituierten C₁-C₆₀-Alkylgruppe, der substituierten C₂-C₆₀-Alkenylgruppe, der substituierten C₂-C₆₀-Alkinylgruppe, der substituierten C₁-C₆₀-Alkoxygruppe, der substituierten C₁-C₆₀-Alkylthiogruppe, der substituierten C₃-C₁₀-Cycloalkylgruppe, der substituierten C₁-C₁₀-Heterocycloalkylgruppe, der substituierten C₃-C₁₀-Cycloalkenylgruppe, der substituierten C₂-C₁₀-Heterocycloalkenylgruppe, der substituierten C₆-C₆₀-Arylgruppe, der substituierten C₆-C₆₀-Aryloxygruppe, der substituierten C₆-C₆₀-Arylthiogruppe, der substituierten C₁-C₆₀-Heteroarylgruppe, der substituierten einwertigen nichtaromatischen kondensierten polycyclischen Gruppe und der substituierten einwertigen nichtaromatischen kondensierten heteropolycyclischen Gruppe wie folgt ist:
Deuterium, -F, -Cl, -Br, -I, -CD₃, -CD₂H, -CDH₂, -CF₃, -CF₂H, -CFH₂, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine C₁-C₆₀-Alkylgruppe, eine C₂-C₆₀-Alkenylgruppe, eine C₂-C₆₀-Alkinylgruppe, eine C₁-C₆₀-Alkoxygruppe oder eine C₁-C₆₀-Alkylthiogruppe;
eine C₁-C₆₀-Alkylgruppe, eine C₂-C₆₀-Alkenylgruppe, eine C₂-C₆₀-Alkinylgruppe, eine C₁-C₆₀-Alkoxygruppe oder eine C₁-C₆₀-Alkylthiogruppe, jeweils substituiert mit Deuterium, -F, -Cl, -Br, -I, -CD₃, -CD₂H, -CDH₂, -CF₃, -CF₂H, -CFH₂, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₃-C₁₀-Cycloalkylgruppe, einer C₁-C₁₀-Heterocycloalkylgruppe, einer C₃-C₁₀-Cycloalkenylgruppe, einer C₂-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer einwertigen nichtaromatischen kondensierten polycyclischen Gruppe, einer einwertigen nichtaromatischen kondensierten heteropolycyclischen Gruppe, -N(Q₁₁)(Q₁₂), -Si(Q₁₃)(Q₁₄)(Q₁₅), -Ge(Q₁₃)(Q₁₄)(Q₁₅), -B(Q₁₆)(Q₁₇), -P(=O)(Q₁₈)(Q₁₉), -P(Q₁₈)(Q₁₉) oder einer beliebigen Kombination davon;
eine C₃-C₁₀-Cycloalkylgruppe, eine C₁-C₁₀-Heterocycloalkylgruppe, eine C₃-C₁₀-Cycloalkenylgruppe, eine C₂-C₁₀-Heterocycloalkenylgruppe, eine C₆-C₆₀-Arylgruppe, eine C₆-C₆₀-Aryloxygruppe, eine C₆-C₆₀-Arylthiogruppe, eine C₁-C₆₀-Heteroarylgruppe, eine einwertige nichtaromatische kondensierte polycyclische Gruppe oder eine einwertige nichtaromatische kondensierte heteropolycyclische Gruppe, jeweils unsubstituiert oder substituiert mit Deuterium, -F, -Cl, -Br, -I, -CD₃, -CD₂H, -CDH₂, -CF₃, -CF₂H, -CFH₂, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Sulfonsäuregruppe oder einem Salz davon, einer Phosphorsäuregruppe oder einem Salz davon, einer C₁-C₆₀-Alkylgruppe, einer C₂-C₆₀-Alkenylgruppe, einer C₂-C₆₀-Alkinylgruppe, einer C₁-C₆₀-Alkoxygruppe, einer C₁-C₆₀-Alkylthiogruppe, einer C₃-C₁₀-Cycloalkylgruppe, einer C₁-C₁₀-Heterocycloalkylgruppe, einer C₃-C₁₀-Cycloalkenylgruppe, einer C₂-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer einwertigen nichtaromatischen kondensierten polycyclischen Gruppe, einer einwertigen nichtaromatischen kondensierten heteropolycyclischen Gruppe, -N(Q₂₁)(Q₂₂), -Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉), -P(Q₂₈)(Q₂₉) oder einer beliebigen Kombination davon;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), -P(=O)(Q₃₈)(Q₃₉) oder -P(Q₃₈)(Q₃₉); oder
eine beliebige Kombination davon, und
Q₁ bis Q₉, Q₁₁ bis G₁₉, Q₂₁ bis Q₂₉ und Q₃₁ bis Q₃₉ jeweils unabhängig wie folgt sind: Wasserstoff; Deuterium; -F; oder eine C₁-C₆₀-Alkylgruppe, eine C₂-C₆₀-Alkenylgruppe, eine C₂-C₆₀-Alkinylgruppe, eine C₁-C₆₀-Alkoxygruppe, eine C₁-C₆₀-Alkylthiogruppe, eine C₃-C₁₀-Cycloalkylgruppe, eine C₁-C₁₀-Heterocycloalkylgruppe, eine C₃-C₁₀-Cycloalkenylgruppe, eine C₂-C₁₀-Heterocycloalkenylgruppe, eine C₆-C₆₀-Arylgruppe, eine C₆-C₆₀-Aryloxygruppe, eine C₆-C₆₀-Arylthiogruppe, eine C₁-C₆₀-Heteroarylgruppe, eine einwertige nichtaromatische kondensierte polycyclische Gruppe oder eine einwertige nichtaromatische kondensierte heteropolycyclische Gruppe, jeweils unsubstituiert oder substituiert mit Deuterium, -F, einer Cyanogruppe, einer C₁-C₆₀-Alkylgruppe, einer C₆-C₆₀-Arylgruppe oder einer beliebigen Kombination davon.

2. Kondensierte cyclische Verbindung nach Anspruch 1, wobei Ring Y₁ bis Ring Y₃ jeweils unabhängig eine Benzolgruppe, eine Naphthalengruppe, eine Phenanthrengruppe, eine Pyridingruppe, eine Pyrimidingruppe, eine Pyridazingruppe, eine Pyrazingruppe, eine Chinolingruppe, eine Isochinolingruppe, eine Dibenzofurangruppe, eine Dibenzothiophengruppe, eine Carbazolgruppe, eine Fluorengruppe, eine Dibenzosilolgruppe, eine Azadibenzofurangruppe, eine Azadibenzothiophengruppe, eine Azacarbazolgruppe, eine Azafluorengruppe oder eine Azadibenzosilolgruppe sind; und/oder
wobei n1 1 ist und W₁ N(Ar₁) oder N(T₁₁) ist.

3. Kondensierte cyclische Verbindung nach Anspruch 1 oder 2, wobei
R₀ wie folgt ist:
Wasserstoff, Deuterium, -F oder eine Cyanogruppe; oder
eine C₁-C₂₀-Alkylgruppe, unsubstituiert oder substituiert mit Deuterium, -F, einer Cyanogruppe oder einer beliebigen Kombination davon, und
R₁ bis R₄ jeweils unabhängig wie folgt sind:
Wasserstoff, Deuterium, -F oder eine Cyanogruppe;
eine C₁-C₂₀-Alkylgruppe, unsubstituiert oder substituiert mit Deuterium, -F, einer Cyanogruppe oder einer beliebigen Kombination davon; oder
eine Phenylgruppe, unsubstituiert oder substituiert mit Deuterium, -F, einer Cyanogruppe, einer C₁-C₂₀-Alkylgruppe, einer Phenylgruppe oder einer beliebigen Kombination davon.

4. Kondensierte cyclische Verbindung nach einem der Ansprüche 1-3, wobei
a2 1 ist, und
R₂ eine Phenylgruppe, unsubstituiert oder substituiert mit Deuterium, -F, einer Cyanogruppe, einer C₁-C₂₀-Alkylgruppe, einer Phenylgruppe oder einer beliebigen Kombination davon, ist.

5. Kondensierte cyclische Verbindung nach einem der Ansprüche 1-4, wobei
Z₁ bis Z₃, T₁₁ bis T₁₃ und T₂₁ bis T₂₃ jeweils unabhängig wie folgt sind:
Wasserstoff, Deuterium, -F oder eine Cyanogruppe;
eine C₁-C₂₀-Alkylgruppe, eine Phenylgruppe, eine Biphenylgruppe, eine Dibenzofuranylgruppe, eine Dibenzothiophenylgruppe, eine Carbazolylgruppe, eine Fluorenylgruppe oder eine Dibenzosilolylgruppe, jeweils unsubstituiert oder substituiert mit Deuterium, -F, Cyanogruppe, einer C₁-C₂₀-Alkylgruppe, einer Phenylgruppe, einer Dibenzofuranylgruppe, einer Dibenzothiophenylgruppe, einer Carbazolylgruppe, einer Fluorenylgruppe, einer Dibenzosilolylgruppe oder einer beliebigen Kombination davon; oder
-N(Q₁)(Q₂), und
Q₁ und Q₂ jeweils unabhängig eine C₁-C₂₀-Alkylgruppe, eine Phenylgruppe, eine Biphenylgruppe, eine Dibenzofuranylgruppe, eine Dibenzothiophenylgruppe, eine Carbazolylgruppe, eine Fluorenylgruppe oder eine Dibenzosilolylgruppe sind, jeweils unsubstituiert oder substituiert mit Deuterium, -F, einer Cyanogruppe, einer C₁-C₂₀-Alkylgruppe, einer Phenylgruppe, einer Dibenzofuranylgruppe, einer Dibenzothiophenylgruppe, einer Carbazolylgruppe, einer Fluorenylgruppe, einer Dibenzosilolylgruppe oder einer beliebigen Kombination davon.

6. Kondensierte cyclische Verbindung nach einem der Ansprüche 1-5, wobei
jedes von b1 und b2 1 oder größer ist, und
zumindest eines von Z₁ und Z₂ eine Gruppe dargestellt durch Formel 3 ist: 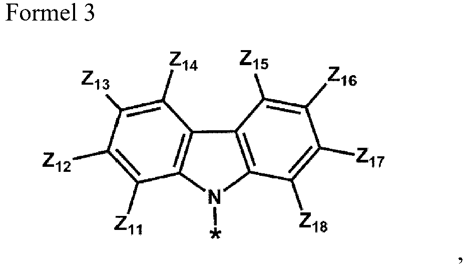
wobei in Formel 3
Z₁₁ bis Z₁₈ jeweils unabhängig wie folgt sind:
Wasserstoff, Deuterium, -F oder eine Cyanogruppe;
eine C₁-C₂₀-Alkylgruppe, unsubstituiert oder substituiert mit Deuterium, -F, einer Cyanogruppe oder einer beliebigen Kombination davon; oder
eine Phenylgruppe, unsubstituiert oder substituiert mit Deuterium, -F, einer Cyanogruppe, einer C₁-C₂₀-Alkylgruppe, einer Phenylgruppe oder einer beliebigen Kombination davon, und
* eine Bindungsstelle an Formel 1 angibt.

7. Kondensierte cyclische Verbindung nach einem der Ansprüche 1-6, wobei die Gruppe dargestellt durch Formel 2 eine Gruppe dargestellt durch eine der Formeln 2-1 bis 2-3 ist: 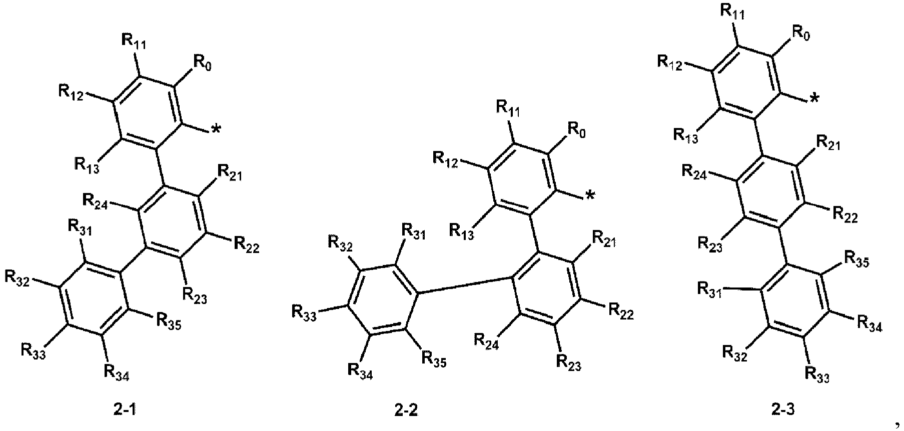 wobei in Formel 2-1 bis 2-3
R₀ wie in Anspruch 1 definiert ist,
R₁₁ bis R₁₃ jeweils wie für R₁ definiert sind,
R₂₁ bis R₂₄ jeweils wie für R₂ definiert sind,
R₃₁ bis R₃₅ jeweils wie für R₃ definiert sind, und
* eine Bindungsstelle an Formel 1 angibt.

8. Kondensierte cyclische Verbindung nach einem der Ansprüche 1-7, wobei die Gruppe dargestellt durch Formel 2 eine Gruppe dargestellt durch Formel 2-1(1) ist: 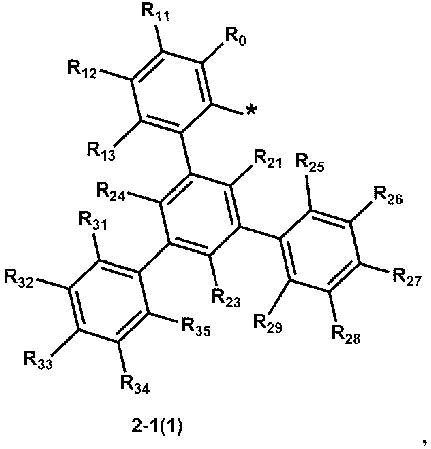 wobei in Formel 2-1(1)
R₀ wie in Anspruch 1 definiert ist,
R₁₁ bis R₁₃ jeweils wie für R₁ in Anspruch 1 definiert sind,
R₂₁, R₂₃ und R₂₄ jeweils wie für R₂ in Anspruch 1 definiert sind,
R₃₁ bis R₃₅ jeweils wie für R₃ in Anspruch 1 definiert sind,
R₂₅ bis R₂₉ jeweils unabhängig wie folgt sind:
Wasserstoff, Deuterium, -F oder eine Cyanogruppe; oder
eine C₁-C₂₀-Alkylgruppe, unsubstituiert oder substituiert mit Deuterium, -F, einer Cyanogruppe oder einer beliebigen Kombination davon, und
* eine Bindungsstelle an Formel 1 angibt; und/oder
wobei die kondensierte cyclische Verbindung dargestellt ist durch Formel 1A: 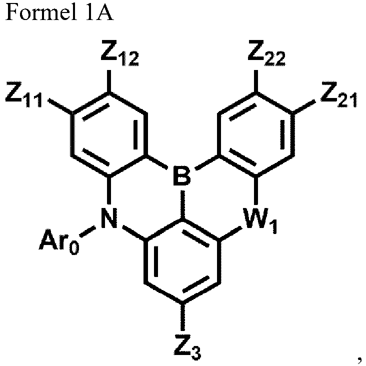
wobei in Formel 1A
W₁ O, S, N(Ar1), N(T₁₁), C(T₁₂)(T₁₃) oder Si(T₁₂)(T₁₃) ist,
Ar₀, Ar₁ und T₁₁ bis T₁₃ jeweils wie in Anspruch 1 definiert sind,
Z₁₁ und Z₁₂ jeweils wie für Z₁ in Anspruch 1 definiert sind,
Z₂₁ und Z₂₂ jeweils wie für Z₂ in Anspruch 1 definiert sind,
zumindest eines von Z₁₁, Z₁₂, Z₂₁ und Z₂₂ eine N-Carbazolylgruppe, unsubstituiert oder substituiert mit Deuterium, -F, einer Cyanogruppe, einer C₁-C₂₀-Alkylgruppe, einer Phenylgruppe oder einer beliebigen Kombination davon, ist, und
Z₃ eine C₁-C₂₀-Alkylgruppe, unsubstituiert oder substituiert mit Deuterium, -F, einer Cyanogruppe oder einer beliebigen Kombination davon, ist.

9. Kondensierte cyclische Verbindung nach einem der Ansprüche 1-8, wobei die kondensierte cyclische Verbindung zumindest ein Deuterium, zumindest eine tert-Butylgruppe oder eine beliebige Kombination davon umfasst.

10. Lichtemittierende Vorrichtung, umfassend:
eine erste Elektrode;
eine zweite Elektrode; und
eine Zwischenschicht, die zwischen der ersten Elektrode und der zweiten Elektrode angeordnet ist und eine Emissionsschicht umfasst,
wobei die Zwischenschicht zumindest eine kondensierte cyclische Verbindung nach einem der Ansprüche 1-9 umfasst.

11. Lichtemittierende Vorrichtung nach Anspruch 10, wobei die zumindest eine kondensierte cyclische Verbindung in der Emissionsschicht enthalten ist.

12. Lichtemittierende Vorrichtung nach Anspruch 11, wobei Licht, das von der Emissionsschicht emittiert wird, eine Emissionsspitzenwellenlänge in einem Bereich von 440 nm bis 470 nm aufweist; und/oder
wobei die zumindest eine kondensierte cyclische Verbindung, die in der Emissionsschicht enthalten ist, ein Emitter ist.

13. Lichtemittierende Vorrichtung nach Anspruch 11 oder 12, wobei die Emissionsschicht ferner einen Sensibilisator umfasst und sich der Sensibilisator und die kondensierte cyclische Verbindung voneinander unterscheiden;
wobei bevorzugt der Sensibilisator eine organometallische Verbindung, ein verzögertes Fluoreszenzmaterial, ein sofortiges Fluoreszenzmaterial oder eine beliebige Kombination davon ist;
wobei bevorzugt
die organometallische Verbindung ein Übergangsmetall und einen vierzähnigen Liganden, der an das Übergangsmetall gebunden ist, umfasst,
das Übergangsmetall Platin oder Palladium ist, und
der vierzähnige Ligand eine Carbeneinheit umfasst, die an das Übergangsmetall gebunden ist.

14. Lichtemittierende Vorrichtung nach einem der Ansprüche 11-13, wobei die zumindest eine kondensierte cyclische Verbindung, die in der Emissionsschicht enthalten ist, ein Sensibilisator ist.

15. Elektronische Einrichtung, umfassend die lichtemittierende Vorrichtung nach einem der Ansprüche 10-14.

## Revendications

1. Composé cyclique condensé représenté par la Formule 1 : 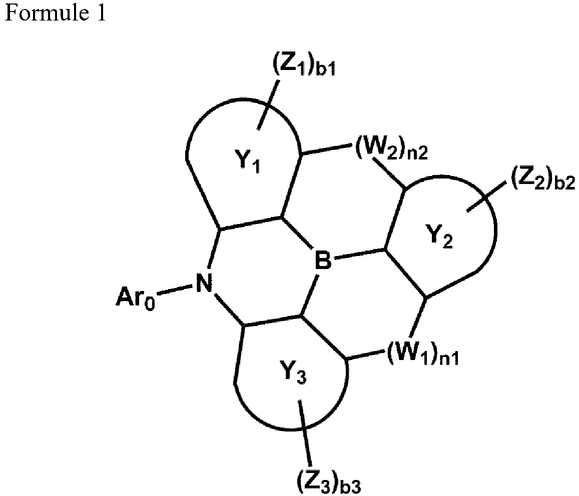 dans lequel, dans la Formule 1, Ar₀ est un groupe représenté par la Formule 2, 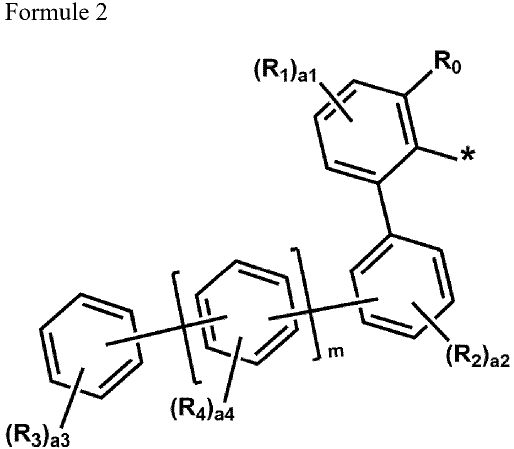 dans lequel, dans les Formules 1 et 2,
les cycles Y₁ à Y₃ sont chacun indépendamment un groupe carbocyclique en C₅-C₆₀ ou un groupe hétérocyclique en C₁-C₆₀,
W₁ est une liaison simple, O, S, N(Ar1), N(T₁₁), C(T₁₂)(T₁₃) ou Si(T₁₂)(T₁₃),
W₂ est une liaison simple, O, S, N(Ar₂), N(T₂₁), C(T₂₂)(T₂₃) ou Si(T₂₂)(T₂₃),
n1 et n2 sont chacun indépendamment 0 ou 1,
lorsque n1 est 0, *-(W₁)ₙ₁-*' est absent,
lorsque n2 est 0, *-(W₂)ₙ₂-*' est absent,
la somme de n1 et n2 est égale ou supérieure à 1,
chacun de Ar₁ et Ar₂ est un groupe représenté par la Formule 2,
R₀ est :
un hydrogène, un deutérium, -F ou un groupe cyano ; ou
un groupe alkyle en C₁-C₆₀ non substitué ou substitué par un deutérium, -F, un groupe cyano, ou toute combinaison de ceux-ci,
R₁ à R₄ sont chacun indépendamment :
un hydrogène, un deutérium, -F ou un groupe cyano ;
un groupe alkyle en C₁-C₆₀ non substitué ou substitué par un deutérium, -F, un groupe cyano, ou toute combinaison de ceux-ci ; ou
un groupe aryle en C₆-C₆₀ non substitué ou substitué par un deutérium, -F, un groupe cyano, un groupe alkyle en C₁-C₆₀, un groupe aryle en C₆-C₆₀, ou toute combinaison de ceux-ci,
a1 est un nombre entier de 0 à 3,
a2 et a4 sont chacun indépendamment un nombre entier de 0 à 4,
a3 et m sont chacun indépendamment un nombre entier de 0 à 5,
* dans la Formule 2 indique un site de liaison au composé de Formule 1,
Z₁ à Z₃, T₁₁ à T₁₃ et T₂₁ à T₂₃ sont chacun indépendamment un hydrogène, un deutérium, - F, -Cl, -Br, -I, -SF₅, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₆₀ substitué ou non substitué, un groupe alcényle en C₂-C₆₀ substitué ou non substitué, un groupe alkynyle en C₂-C₆₀ substitué ou non substitué, un groupe alcoxy en C₁-C₆₀ substitué ou non substitué, un groupe alkylthio en C₁-C₆₀ substitué ou non substitué, un groupe cycloalkyle en C₃-C₁₀ substitué ou non substitué, un groupe hétérocycloalkyle en C₁-C₁₀ substitué ou non substitué, un groupe cycloalcényle en C₃-C₁₀ substitué ou non substitué, un groupe hétérocycloalcényle en C₂-C₁₀ substitué ou non substitué, un groupe aryle en C₆-C₆₀ substitué ou non substitué, un groupe aryloxy en C₆-C₆₀ substitué ou non substitué, un groupe arylthio en C₆-C₆₀ substitué ou non substitué, un groupe hétéroaryle en C₁-C₆₀ substitué ou non substitué, un groupe polycyclique condensé non aromatique monovalent substitué ou non substitué, un groupe hétéropolycyclique condensé non aromatique monovalent substitué ou non substitué, -N(Q₁)(Q₂), -Si(Q₃)(Q₄)(Q₅), -Ge(Q₃)(Q₄)(Q₅), -B(Q₆)(Q₇), -P(=O)(Q₈)(Q₉),-P(Q₈)(Q₉), ou un groupe représenté par la Formula 2,
b1 et b2 sont chacun indépendamment un nombre entier de 0 à 10,
b3 est un nombre entier de 1 à 5,
la Formule 1 satisfait au moins à l'une des conditions 1 et 2 :
Condition 1
b1 est égal ou supérieur à 1, et au moins l'un de Z₁ dans le nombre de b1 comprend un groupe carbazole ; et
Condition 2
b2 est égal ou supérieur à 1, et au moins l'un de Z₂ dans le nombre de b2 comprend un groupe carbazole,
au moins l'un de Z₃ dans le nombre de b3 est un groupe alkyle en C₁-C₆₀ substitué ou non substitué par un deutérium, -F, un groupe cyano, ou toute combinaison de ceux-ci,
deux ou plus de Z₁, Z₂, Z₃, T₁₁, T₁₂, T₁₃, T₂₁, T₂₂ et T₂₃ sont éventuellement liés entre eux pour former un groupe carbocyclique en C₅-C₃₀ substitué ou non substitué ou un groupe hétérocyclique en C₁-C₃₀ substitué ou non substitué,
au moins un substituant du groupe carbocyclique en C₅-C₃₀ substitué, du groupe hétéropolycyclique en C₁-C₃₀ substitué, du groupe alkyle en C₁-C₆₀ substitué, du groupe alcényle en C₂-C₆₀ substitué, du groupe alkynyle en C₂-C₆₀ substitué, du groupe alcoxy en C₁-C₆₀ substitué, du groupe alkylthio en C₁-C₆₀ substitué, du groupe cycloalkyle en C₃-C₁₀ substitué, du groupe hétérocycloalkyle en C₁-C₁₀ substitué, du groupe cycloalcényle en C₃-C₁₀ substitué, du groupe hétérocycloalcényle en C₂-C₁₀ substitué, du groupe aryle en C₆-C₆₀ substitué, du groupe aryloxy en C₆-C₆₀ substitué, du groupe arylthio en C₆-C₆₀ substitué, du groupe hétéroaryle en C₁-C₆₀ substitué, du groupe polycyclique condensé non aromatique monovalent substitué et du groupe hétéropolycyclique condensé non aromatique monovalent substitué est :
un deutérium, -F, -Cl, -Br, -I, -CD₃, -CD₂H, -CDH₂, -CF₃, -CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₆₀, un groupe alcényle en C₂-C₆₀, un groupe alkynyle en C₂-C₆₀, un groupe alcoxy en C₁-C₆₀ ou un groupe alkylthio en C₁-C₆₀ ;
un groupe alkyle en C₁-C₆₀, un groupe alcényle en C₂-C₆₀, un groupe alkynyle en C₂-C₆₀, un groupe alcoxy en C₁-C₆₀ ou un groupe alkylthio en C₁-C₆₀, chacun étant substitué par un deutérium, -F, -Cl, -Br, -I, -CD₃, -CD₂H, -CDH₂, -CF₃, -CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe cycloalkyle en C₃-C₁₀, un groupe hétérocycloalkyle en C₁-C₁₀, un groupe cycloalcényle en C₃-C₁₀, un groupe hétérocycloalcényle en C₂-C₁₀, un groupe aryle en C₆-C₆₀, un groupe aryloxy en C₆-C₆₀, un groupe arylthio en C₆-C₆₀, un groupe hétéroaryle en C₁-C₆₀, un groupe polycyclique condensé non aromatique monovalent, un groupe hétéropolycyclique condensé non aromatique monovalent,-N(Q₁₁)(Q₁₂), -Si(Q₁₃)(Q₁₄)(Q₁₅), -Ge(Q₁₃)(Q₁₄)(Q₁₅), -B(Q₁₆)(Q₁₇), -P(=O)(Q₁₈)(Q₁₉),-P(Q₁₈)(Q₁₉), ou toute combinaison de ceux-ci ;
un groupe cycloalkyle en C₃-C₁₀, un groupe hétérocycloalkyle en C₁-C₁₀, un groupe cycloalcényle en C₃-C₁₀, un groupe hétérocycloalcényle en C₂-C₁₀, un groupe aryle en C₆-C₆₀, un groupe aryloxy en C₆-C₆₀, un groupe arylthio en C₆-C₆₀, un groupe hétéroaryle en C₁-C₆₀, un groupe polycyclique condensé non aromatique monovalent ou un groupe hétéropolycyclique condensé non aromatique monovalent, chacun étant non substitué ou substitué par un deutérium, -F, -Cl, -Br, -I, -CD₃, -CD₂H, -CDH₂, -CF₃, -CF₂H, -CFH₂, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁-C₆₀, un groupe alcényle en C₂-C₆₀, un groupe alkynyle en C₂-C₆₀, un groupe alcoxy en C₁-C₆₀, un groupe alkylthio en C₁-C₆₀, un groupe cycloalkyle en C₃-C₁₀, un groupe hétérocycloalkyle en C₁-C₁₀, un groupe cycloalcényle en C₃-C₁₀, un groupe hétérocycloalcényle en C₂-C₁₀, un groupe aryle en C₆-C₆₀, un groupe aryloxy en C₆-C₆₀, un groupe arylthio en C₆-C₆₀, un groupe hétéroaryle en C₁-C₆₀, un groupe polycyclique condensé non aromatique monovalent, un groupe hétéropolycyclique condensé non aromatique monovalent, -N(Q₂₁)(Q₂₂), -Si(Q₂₃)(Q₂₄)(Q₂₅), -Ge(Q₂₃)(Q₂₄)(Q₂₅),-B(Q₂₆)(Q₂₇), -P(=O)(Q₂₈)(Q₂₉), -P(Q₂₈)(Q₂₉), ou toute combinaison de ceux-ci ;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), -P(=O)(Q₃₈)(Q₃₉) ou-P(Q₃₈)(Q₃₉) ; ou
toute combinaison de ceux-ci, et
Q₁ à Q₉, Q₁₁ à G₁₉, Q₂₁ à Q₂₉ et Q₃₁ à Q₃₉ sont chacun indépendamment : un hydrogène; un deutérium; -F ; ou un groupe alkyle en C₁-C₆₀, un groupe alcényle en C₂-C₆₀, un groupe alkynyle en C₂-C₆₀, un groupe alkoxy en C₁-C₆₀, un groupe alkylthio en C₁-C₆₀, un groupe cycloalkyle en C₃-C₁₀, un groupe hétérocycloalkyle en C₁-C₁₀, un groupe cycloalcényle en C₃-C₁₀, un groupe hétérocycloalcényle en C₂-C₁₀, un groupe aryle en C₆-C₆₀, un groupe aryloxy en C₆-C₆₀, un groupe arylthio en C₆-C₆₀, un groupe hétéroaryle en C₁-C₆₀, un groupe polycyclique condensé non aromatique monovalent ou un groupe hétéropolycyclique condensé non aromatique monovalent, chacun étant non substitué ou substitué par un deutérium, -F, un groupe cyano, un groupe alkyle en C₁-C₆₀, un groupe aryle en C₆-C₆₀, ou toute combinaison de ceux-ci.

2. Composé cyclique condensé de la revendication 1, dans lequel les cycles Y₁ à Y₃ sont chacun indépendamment un groupe benzène, un groupe naphthalène, un groupe phénanthrène, un groupe pyridine, un groupe pyrimidine, un groupe pyridazine, un groupe pyrazine, un groupe quinoline, un groupe isoquinoline, un groupe dibenzofurane, un groupe dibenzothiophène, un groupe carbazole, un groupe fluorène, un groupe dibenzosilole, un groupe azadibenzofurane, un groupe azadibenzothiophène, un groupe azacarbazole, un groupe azafluorène ou un groupe azadibenzosilole ; et/ou
dans lequel n1 est 1, et W₁ est N(Ar₁) ou N(T₁₁).

3. Composé cyclique condensé de la revendication 1 ou 2, dans lequel
R₀ est :
un hydrogène, un deutérium, -F ou un groupe cyano ; ou
un groupe alkyle en C₁-C₂₀ non substitué ou substitué par un deutérium, -F, un groupe cyano, ou toute combinaison de ceux-ci, et
R₁ à R₄ sont chacun indépendamment :
un hydrogène, un deutérium, -F ou un groupe cyano ;
un groupe alkyle en C₁-C₂₀ non substitué ou substitué par un deutérium, -F, un groupe cyano, ou toute combinaison de ceux-ci ; ou
un groupe phényle non substitué ou substitué par un deutérium, -F, un groupe cyano, un groupe alkyle en C₁-C₂₀, un groupe phényle, ou toute combinaison de ceux-ci.

4. Composé cyclique condensé de l'une quelconque des revendications 1 à 3, dans lequel
a2 est 1, et
R₂ est un groupe phényle non substitué ou substitué par un deutérium, -F, un groupe cyano, un groupe alkyle en C₁-C₂₀, un groupe phényle, ou toute combinaison de ceux-ci.

5. Composé cyclique condensé de l'une quelconque des revendications 1 à 4, dans lequel
Z₁ à Z₃, T₁₁ à T₁₃ et T₂₁ à T₂₃ sont chacun indépendamment :
un hydrogène, un deutérium, -F ou un groupe cyano ;
un groupe alkyle en C₁-C₂₀, un groupe phényle, un groupe biphényle, un groupe dibenzofuranyle, un groupe dibenzothiophényle, un groupe carbazolyle, un groupe fluorényle ou un groupe dibenzosilolyle, chacun étant non substitué ou substitué par un deutérium, -F, un groupe cyano, un groupe alkyle en C₁-C₂₀, un groupe phényle, un groupe dibenzofuranyle, un groupe dibenzothiophényle, un groupe carbazolyle, un groupe fluorényle, un groupe dibenzosilolyle, ou toute combinaison de ceux-ci ; ou
-N(Q₁)(Q₂), et
Q₁ et Q₂ sont chacun indépendamment un groupe alkyle en C₁-C₂₀, un groupe phényle, un groupe biphényle, un groupe dibenzofuranyle, un groupe dibenzothiophényle, un groupe carbazolyle, un groupe fluorényle ou un groupe dibenzosilolyle, chacun étant non substitué ou substitué par un deutérium, -F, un groupe cyano, un groupe alkyle en C₁-C₂₀, un groupe phényle, un groupe dibenzofuranyle, un groupe dibenzothiophényle, un groupe carbazolyle, un groupe fluorényle, un groupe dibenzosilolyle, ou toute combinaison de ceux-ci.

6. Composé cyclique condensé de l'une quelconque des revendications 1 à 5, dans lequel
chacun de b1 et b2 est égal ou supérieur à 1, et
au moins l'un de Z₁ et Z₂ est un groupe représenté par la Formule 3 : 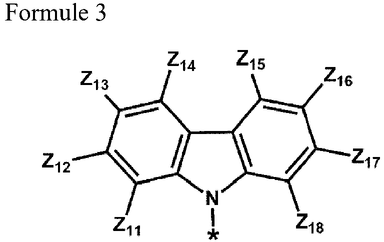
dans lequel, dans la Formule 3,
Z₁₁ à Z₁₈ sont chacun indépendamment :
un hydrogène, un deutérium, -F ou un groupe cyano ;
un groupe alkyle en C₁-C₂₀ non substitué ou substitué par un deutérium, -F, un groupe cyano, ou toute combinaison de ceux-ci ; ou
un groupe phényle non substitué ou substitué par un deutérium, -F, un groupe cyano, un groupe alkyle en C₁-C₂₀, un groupe phényle, ou toute combinaison de ceux-ci, et
* indique un site de liaison à la Formule 1.

7. Composé cyclique condensé de l'une quelconque des revendications 1 à 6, dans lequel le groupe représenté par la Formule 2 est un groupe représenté par l'une des Formules 2-1 à 2-3 : 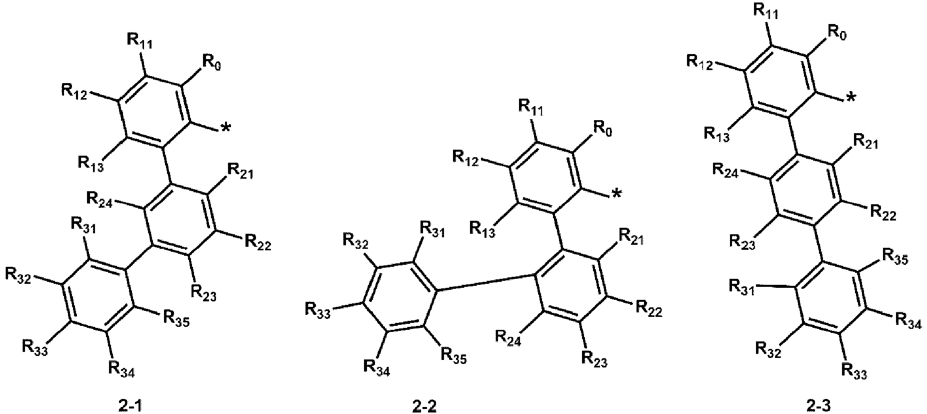 dans lequel, dans les Formules 2-1 à 2-3,
R₀ est tel que défini dans la revendication 1,
R₁₁ à R₁₃ sont chacun définis comme pour R₁,
R₂₁ à R₂₄ sont chacun définis comme pour R₂,
R₃₁ à R₃₅ sont chacun définis comme pour R₃, et
* indique un site de liaison à la Formule 1.

8. Composé cyclique condensé de l'une quelconque des revendications 1 à 7, dans lequel le groupe représenté par la Formule 2 est un groupe représenté par la Formule 2-1(1) : 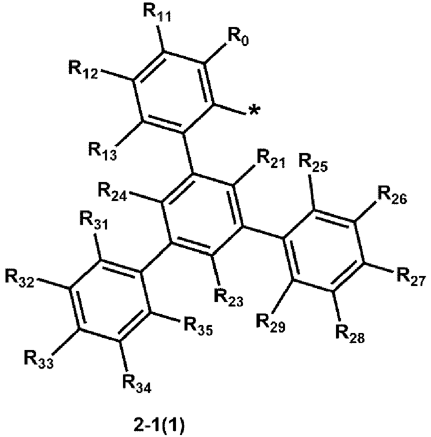 dans lequel, dans la Formule 2-1(1),
R₀ est tel que défini dans la revendication 1,
R₁₁ à R₁₃ sont chacun définis comme pour R₁ dans la revendication 1,
R₂₁, R₂₃ et R₂₄ sont chacun définis comme pour R₂ dans la revendication 1,
R₃₁ à R₃₅ sont chacun définis comme pour R₃ dans la revendication 1,
R₂₅ à R₂₉ sont chacun indépendamment :
un hydrogène, un deutérium, -F ou un groupe cyano ; ou
un groupe alkyle en C₁-C₂₀ non substitué ou substitué par un deutérium, -F, un groupe cyano, ou toute combinaison de ceux-ci, et
* indique un site de liaison à la Formule 1 ; et/ou
dans lequel le composé cyclique condensé est représenté par la Formule 1A : 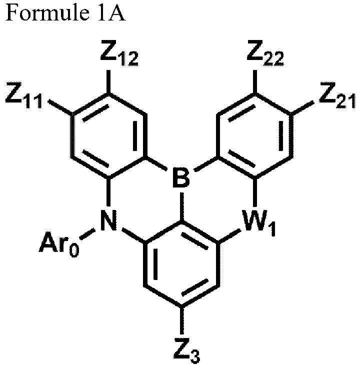
dans lequel, dans la Formule 1A,
W₁ est O, S, N(Ar₁), N(T₁₁), C(T₁₂)(T₁₃) ou Si(T₁₂)(T₁₃),
Ar₀, Ar₁ et T₁₁ à T₁₃ sont chacun tels que définis dans la revendication 1,
Z₁₁ et Z₁₂ sont chacun définis comme pour Z₁ dans la revendication 1,
Z₂₁ et Z₂₂ sont chacun définis comme pour Z₂ dans la revendication 1,
au moins l'un de Z₁₁, Z₁₂, Z₂₁ et Z₂₂ est un groupe N-carbazolyle non substitué ou substitué par un deutérium, -F, un groupe cyano, un groupe alkyle en C₁-C₂₀, un groupe phényle, ou toute combinaison de ceux-ci, et
Z₃ est un groupe alkyle en C₁-C₂₀ non substitué ou substitué par un deutérium, -F, un groupe cyano, ou toute combinaison de ceux-ci.

9. Composé cyclique condensé de l'une quelconque des revendications 1 à 8, ledit composé cyclique condensé comprenant au moins un deutérium, au moins un groupe tert-butyle, ou toute combinaison de ceux-ci.

10. Dispositif électroluminescent comprenant :
une première électrode ;
une seconde électrode ; et
une couche intermédiaire agencée entre la première électrode et la seconde électrode et comprenant une couche d'émission,
ladite couche intermédiaire comprenant au moins un composé cyclique condensé de l'une quelconque des revendications 1 à 9.

11. Dispositif électroluminescent de la revendication 10, dans lequel au moins un composé cyclique condensé est compris dans la couche d'émission.

12. Dispositif électroluminescent de la revendication 11, dans lequel la lumière émise par la couche d'émission présente une longueur d'onde de pic d'émission dans une plage de 440 nm à 470 nm ; et/ou
dans lequel au moins un composé cyclique condensé compris dans la couche d'émission est un émetteur.

13. Dispositif électroluminescent de la revendication 11 ou 12, dans lequel la couche d'émission comprend en outre un sensibilisateur, et le sensibilisateur et le composé cyclique condensé sont différents l'un de l'autre ;
de préférence dans lequel le sensibilisateur est un composé organométallique, un matériau à fluorescence retardée, un matériau à fluorescence rapide, ou toute combinaison de ceux-ci ;
de préférence dans lequel
le composé organométallique comprend un métal de transition et un ligand tétradenté lié au métal de transition,
le métal de transition est le platine ou le palladium, et
le ligand tétradenté comprend un fragment carbène lié au métal de transition.

14. Dispositif électroluminescent de l'une quelconque des revendications 11 à 13, dans lequel au moins un composé cyclique condensé compris dans la couche d'émission est un sensibilisateur.

15. Appareil électronique comprenant le dispositif électroluminescent de l'une quelconque des revendications 10 à 14.
